(19) Europäisches Patentamt
European Patent Office
Office européen des brevets

(11) **EP 4 773 296 A1**

(12) **EUROPEAN PATENT APPLICATION**
published in accordance with Art. 153(4) EPC

(43) Date of publication:
**08.07.2026 Bulletin 2026/28**

(21) Application number: **24872551.7**

(22) Date of filing: **27.09.2024**

(51) International Patent Classification (IPC):
***H01M 10/0567*** *(2010.01)* ***C07D 263/44*** *(2006.01)*
***C07F 9/24*** *(2006.01)* ***C07F 9/26*** *(2006.01)*
***C07F 9/40*** *(2006.01)* ***C07F 9/42*** *(2006.01)*
***H01G 11/60*** *(2013.01)* ***H01G 11/62*** *(2013.01)*
***H01G 11/64*** *(2013.01)* ***H01M 10/052*** *(2010.01)*
***H01M 10/054*** *(2010.01)* ***H01M 10/0568*** *(2010.01)*
***H01M 10/0569*** *(2010.01)*

(52) Cooperative Patent Classification (CPC):
**C07D 263/44; C07F 9/24; C07F 9/26; C07F 9/40; C07F 9/42; H01G 11/60; H01G 11/62; H01G 11/64; H01M 10/052; H01M 10/054; H01M 10/0567; H01M 10/0568; H01M 10/0569;** Y02E 60/10

(86) International application number:
**PCT/JP2024/034775**

(87) International publication number:
**WO 2025/070770 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 JP 2023165239**

(71) Applicant: **Central Glass Co., Ltd.**
**Yamaguchi 755-0001 (JP)**

(72) Inventors:
- **KAWAHARA, Kei**
  **Tokyo 101-0054 (JP)**
- **IWASAKI, Susumu**
  **Tokyo 101-0054 (JP)**
- **MORINAKA, Takayoshi**
  **Tokyo 101-0054 (JP)**
- **TAKAHASHI, Mikihiro**
  **Tokyo 101-0054 (JP)**

(74) Representative: **Manitz Finsterwald**
**Patent- und Rechtsanwaltspartnerschaft mbB**
**Martin-Greif-Straße 1**
**80336 München (DE)**

(54) **COMPOUND HAVING OXAZOLIDINE STRUCTURE, NONAQUEOUS ELECTROLYTE CONTAINING SAID COMPOUND, AND NONAQUEOUS ELECTROLYTE BATTERY CONTAINING SAID NONAQUEOUS ELECTROLYTE**

(57) A nonaqueous electrolyte solution capable of exhibiting superior cycle characteristic to that of a case containing a known compound of the related art is provided. The nonaqueous electrolyte solution contains (I) a solute, (II) a nonaqueous organic solvent, and (III) at least one selected from the group consisting of compounds represented by the following formulae (1) to (8).

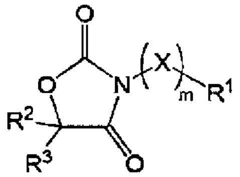 X = 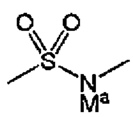 or 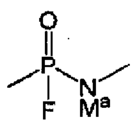 (1)

EP 4 773 296 A1

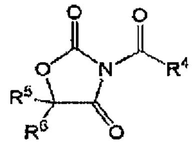 (2) 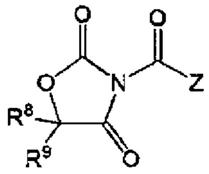 Z = 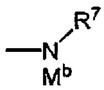 or 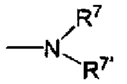 (3)

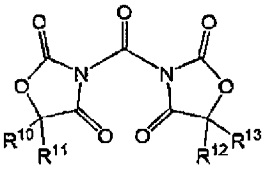 (4) 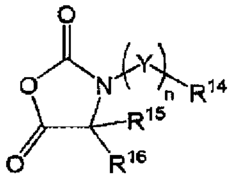 Y = 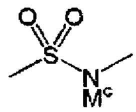 or 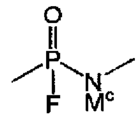 (5)

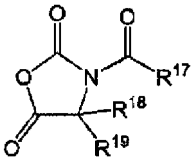 (6) 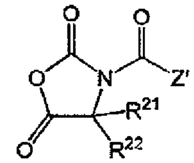 Z' = 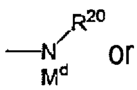 or 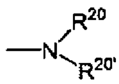 (7)

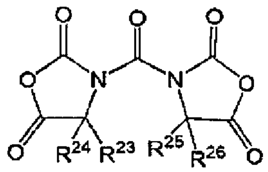 (8)

## Description

### TECHNICAL FIELD

The present disclosure relates to an oxazolidine compound having a specific structure, a nonaqueous electrolyte solution containing the same, and a nonaqueous electrolyte solution battery containing the nonaqueous electrolyte solution.

### BACKGROUND ART

Recently, electrical storage systems for applications for information-related devices or telecommunication devices, namely devices having a small size and requiring a high energy density, such as personal computers, video cameras, digital still cameras, and cellular phones, as well as electrical storage systems for applications for devices having a large size and requiring power, such as electric vehicles, hybrid vehicles, auxiliary power supplies for fuel cell vehicles, and electricity storages, have been attracting attentions. As one candidate therefor, nonaqueous electrolyte solution secondary batteries, such as lithium ion batteries, lithium batteries, lithium ion capacitors, and sodium ion batteries have actively been developed.

For nonaqueous electrolyte solution secondary batteries, as battery characteristics, for example, it is required to achieve various characteristics such as cycle characteristic, input/output characteristic, storage characteristic, continuous charge characteristic, and safety at a high level. Moreover, in recent years, in in-vehicle batteries, there has been a growing demand for higher capacity for increasing the cruising range.

Many of these nonaqueous electrolyte solution secondary batteries have already been put into practical use, but further improvement is desired in increasing the capacity. In particular, it has been known that when an alloy-based active material, particularly an active material containing Si atoms, is used as the negative electrode active material, it is expected that the capacity of the battery is increased, but the battery performance deteriorates greatly with long-term use. Therefore, attempts have been made to improve the capacity and the durability for increasing the cruising range, for example, for automobiles.

As measures for improving the high-temperature characteristic of a nonaqueous electrolyte solution secondary battery and the battery characteristics (cycle characteristic) thereof after repeating charging and discharging, optimization of various constituting elements of the battery including active materials of a positive electrode and a negative electrode has been hitherto studied. Techniques relating to a nonaqueous electrolyte solution have also been studied, and use of a variety of additives to prevent deterioration of an electrolyte solution due to decomposition of the electrolyte solution at surfaces of active positive and negative electrodes has been proposed.

PTL 1 reports an electrolyte solution for a nonaqueous electrolyte solution secondary battery containing a nitrogen-containing heterocyclic compound such as a pyrrolidine compound, an imidazolidine compound, and an oxazolidine compound as a nonaqueous electrolyte solution having excellent battery characteristics, for example, high charge-discharge efficiency and capacity retention rate.

In addition, in PTL 2, a nonaqueous electrolyte solution containing an oxazolidine compound or the like in a solvent containing a 5- to 7-membered ring lactone compound has been reported.

Further, in PTL 3, it is reported that when a nitrogen-containing annular compound containing an ester bond is contained in a nonaqueous electrolyte solution, the capacity retention rate at 20°C and 60°C is improved, and the battery can exhibit flame retardancy.

### CITATION LIST

### PATENT LITERATURE

PTL 1: JP5109213B
PTL 2: JP4302366B
PTL 3: JP5439345B

### SUMMARY OF INVENTION

### TECHNICAL PROBLEM

The present inventors have examined the electrolyte solutions containing the oxazolidine compounds specifically disclosed in these patent documents (for example, 3-methyl-2-oxazolidone used in Example 2 of PTL 1 and Example 24 of PTL 2, 5,5-dimethyl-1,3-oxazolidin-2-one used in Example 1 of PTL 3, 3,5,5-trimethyloxazolidine-2,4-dione used in Example 4 of PTL 3, and 5,5-dimethyl-3-ethyl-2,4-oxazolidinone used in Example 5 of PTL 3) and found that there is room for improvement from the viewpoint of the discharge capacity retention rate after a cycle test (hereinafter also referred to as "cycle characteristic").

Accordingly, the present disclosure has been made in view of the above circumstances, and an object of the present disclosure is to provide a nonaqueous electrolyte solution capable of exhibiting superior cycle characteristic to that of a case containing a known compound of the related art by containing an oxazolidine compound having a specific structure in the molecule, a nonaqueous electrolyte solution battery containing the same, and an oxazolidine compound having such a specific structure.

SOLUTION TO PROBLEM

As the present inventors have conducted intensive studies on the above problems, the present inventors have found that, when an oxazolidine compound having a specific structure in the molecule, of oxazolidine compounds, is contained in a nonaqueous electrolyte solution, superior cycle characteristic to that of a case containing a known compound of the related art is exhibited in a nonaqueous electrolyte solution battery, and the present invention has been thus completed.

That is, the present invention relates to the following invention.

A nonaqueous electrolyte solution containing:

(I) a solute (hereinafter sometimes referred to as "component (I)");
(II) a nonaqueous organic solvent (hereinafter sometimes referred to as "component (II)"); and
(III) at least one selected from the group consisting of a compound represented by the following general formula (1), a compound represented by the general formula (2), a compound represented by the general formula (3), a compound represented by the general formula (4), a compound represented by the general formula (5), a compound represented by the general formula (6), a compound represented by the general formula (7), and a compound represented by the general formula (8) (hereinafter sometimes referred to as "component (III)").

[Chem. 1]

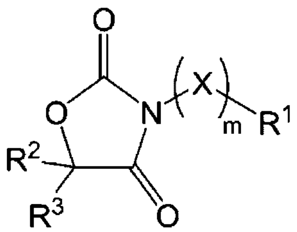

X = 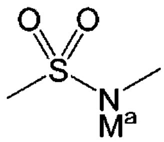 or 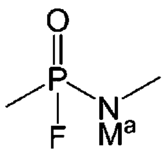 (1)

[In the general formula (1),

m represents an integer of 0 or 1.
$M^a$ represents a hydrogen atom or $(M_1^{A+})_a$. Here, $M_1^{A+}$ represents a metal cation or an onium cation, and A represents the valence of the cation. a represents a number satisfying $A \times a = 1$.
$R^1$ represents $-S(=O)_2R^a$ or $-P(=O)(R^b)(R^c)$.
$R^a$, $R^b$, and $R^c$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_2^{B+})_b$. Here, $M_2^{B+}$ represents a proton, a metal cation, or an onium cation, and B represents the valence of the cation. b represents a number satisfying $B \times b = 1$. Here, $R^b$ and $R^c$ may together form a 5-membered ring or a 6-membered ring together with the phosphorus atom.

$R^2$ and $R^3$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^2$ and $R^3$ may be integrated to form a carbonyl group.]

Here, $(X)_mR^1$ in the general formula (1) may be $S(=O)_2F$, $S(=O)_2OLi$, $P(=O)(OCH_2C{\equiv}CH)_2$, $S(=O)_2OCH_3$, $S(=O)_2NHS(=O)_2F$, $PF(=O)NLiS(=O)_2F$, $S(=O)_2CF_3$, $S(=O)_2CH_3$, $P(=O)(OCHCH_3CH_3)_2$, or $P(=O)(-OCH_2\text{-}CH_2O\text{-})$.

From the viewpoint of the cycle characteristic, $(X)_mR^1$ may be preferably $S(=O)_2OLi$, $P(=O)F_2$, $P(=O)(OCH_2C{\equiv}CH)_2$, $S(=O)_2OCH_3$, $S(=O)_2NHS(=O)_2F$, or $PF(=O)NLiS(=O)_2F$, more preferably $S(=O)_2OLi$, $P(=O)F_2$, $P(=O)(OCH_2C{=}CH)_2$, $S(=O)_2NHS(=O)_2F$, or $PF(=O)NLiS(=O)_2F$, and particularly preferably $S(=O)_2OLi$, $P(=O)F_2$, $P(=O)(OCH_2C{\equiv}CH)_2$, or $PF(=O)NLiS(=O)_2F$.

Moreover, from the viewpoint of the effect of reducing the initial resistance described later (hereinafter sometimes referred to as "initial resistance characteristic"), $(X)_mR^1$ may be preferably $S(=O)_2F$, $P(=O)F_2$, $S(=O)_2OLi$, $P(=O)(OCH_2C{=}CH)_2$, $S(=O)_2OCH_3$, $S(=O)_2NHS(=O)_2F$, $PF(=O)NLiS(=O)_2F$, or $S(=O)_2CF_3$, more preferably $S(=O)_2F$, $P(=O)F_2$, $S(=O)_2OLi$, $P(=O)(OCH_2C{\equiv}CH)_2$, $S(=O)_2OCH_3$, $S(=O)_2NHS(=O)_2F$, or $PF(=O)NLiS(=O)_2F$, and particularly preferably $S(=O)_2F$, $P(=O)F_2$, $S(=O)_2OLi$, or $P(=O)(OCH_2C{\equiv}CH)_2$.

[Chem. 2]

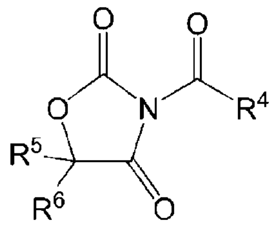

(2)

[In the general formula (2),

$R^4$ represents a halogen atom, $-(O)_{x1}\text{-}S(=O)_2R^d$, $-(O)_{y1}\text{-}P(=O)(R^e)(R^f)$, or $-O^-(M_3^{C+})_c$. Here, $M_3^{C+}$ represents a proton, a metal cation, or an onium cation, and C represents the valence of the cation. c represents a number satisfying $C \times c = 1$, and x1 and y1 each represent an integer of 0 or 1.

$R^d$, $R^e$, and $R^f$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_4^{D+})_d$. Here, $M_4^{D+}$ represents a proton, a metal cation, or an onium cation, and D represents the valence of the cation. d represents a number satisfying $D \times d = 1$.

$R^5$ and $R^6$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^5$ and $R^6$ may be integrated to form a carbonyl group.]

Here, $R^4$ in the general formula (2) may be OLi, F, $P(=O)(OCH_2C{\equiv}CH)_2$, $OS(=O)_2F$, $P(=O)F_2$, or $S(=O)_2F$.

From the viewpoint of the cycle characteristic, $R^4$ may be preferably OLi, F, $P(=O)(OCH_2C{\equiv}CH)_2$, or $OS(=O)_2F$, more preferably OLi, F, or $P(=O)(OCH_2C{\equiv}CH)_2$, and particularly preferably OLi or F.

Moreover, from the viewpoint of the initial resistance characteristic, $R^4$ may be preferably $OS(=O)_2F$, $S(=O)_2F$, $P(=O)F_2$, F, or OLi, more preferably $OS(=O)_2F$, $S(=O)_2F$, $P(=O)F_2$, or F, and particularly preferably $OS(=O)_2F$, $S(=O)_2F$, or $P(=O)F_2$.

[Chem. 3]

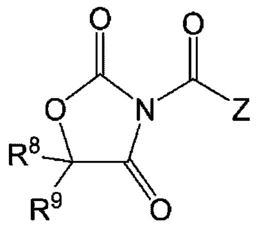 Z = 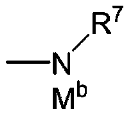 or 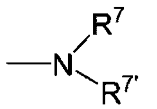 (3)

[In the general formula (3),

$M^b$ represents a hydrogen atom or $(M_5^{E+})_e$. Here, $M_5^{E+}$ represents a metal cation or an onium cation, and E represents the valence of the cation. e represents a number satisfying $E \times e = 1$.

$R^7$ and $R^{7'}$ each independently represent a hydrogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), $-S(=O)_2R^g$, or $-P(=O)(R^h)(R^i)$.

$R^g$, $R^h$, and $R^i$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_6^{F+})_f$. Here, $M_6^{F+}$ represents a proton, a metal cation, or an onium cation, and F represents the valence of the cation. f represents a number satisfying $F \times f = 1$.

$R^8$ and $R^9$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^8$ and $R^9$ may be integrated to form a carbonyl group.]

Here, Z in the general formula (3) may be $NLi\text{-}S(=O)_2F$, $NLi\text{-}P(=O)F_2$, $NLi\text{-}S(=O)_2(OCH_2C{\equiv}CH)$, $NLi\text{-}P(=O)(OC_2H_5)_2$, $N(CH_2C{=}N)_2$, or $NNa\text{-}S(=O)_2F$.

From the viewpoint of the cycle characteristic, Z may be preferably $NLi\text{-}S(=O)_2F$, $NLi\text{-}S(=O)_2(OCH_2C{\equiv}CH)$, or $NLi\text{-}P(=O)F_2$, and more preferably $NLi\text{-}S(=O)_2F$, or $NLi\text{-}S(=O)_2(OCH_2C{\equiv}CH)$.

Moreover, from the viewpoint of the initial resistance characteristic, Z may be preferably $NLi\text{-}S(=O)_2F$, $NLi\text{-}P(=O)F_2$, $NLi\text{-}S(=O)_2(OCH_2C{\equiv}CH)$, or $NLi\text{-}P(=O)(OC_2H_5)_2$, more preferably $NLi\text{-}S(=O)_2F$, $NLi\text{-}P(=O)F_2$, or $NLi\text{-}S(=O)_2$

$(OCH_2C{\equiv}CH)$, and particularly preferably $NLi\text{-}S(=O)_2F$ or $NLi\text{-}P(=O)F_2$.

[Chem. 4]

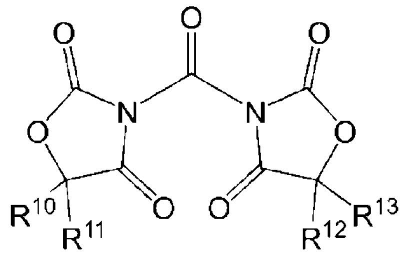

(4)

[In the general formula (4), $R^{10}$, $R^{11}$, $R^{12}$, and $R^{13}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{10}$ and $R^{11}$ or $R^{12}$ and $R^{13}$ may be each independently integrated to form a carbonyl group.]

Here, the general formula (4) may be a structure in which $R^{10}$ to $R^{13}$ are all H, a structure in which $R^{10}$ to $R^{13}$ are all F, a structure in which $R^{10}$ to $R^{13}$ are all $CH_3$, or a structure in which $R^{10}$ and $R^{11}$ are H and $CH_3$, and $R^{12}$ and $R^{13}$ are H and $CH_3$.

From the viewpoint of the cycle characteristic, the general formula (4) may be preferably a structure in which $R^{10}$ and $R^{11}$ are H and $CH_3$, and $R^{12}$ and $R^{13}$ are H and $CH_3$, a structure in which $R^{10}$ to $R^{13}$ are all H, or a structure in which $R^{10}$ to $R^{13}$ are all $CH_3$, more preferably a structure in which $R^{10}$ and $R^{11}$ are H and $CH_3$, and $R^{12}$ and $R^{13}$ are H and $CH_3$ or a structure in which $R^{10}$ to $R^{13}$ are all H, and particularly preferably a structure in which $R^{10}$ and $R^{11}$ are H and $CH_3$, and $R^{12}$ and $R^{13}$ are H and $CH_3$.

Moreover, from the viewpoint of the initial resistance characteristic, the general formula (4) may be preferably a structure in which $R^{10}$ to $R^{13}$ are all F, a structure in which $R^{10}$ to $R^{13}$ are all $CH_3$, or a structure in which $R^{10}$ and $R^{11}$ are H and $CH_3$, and $R^{12}$ and $R^{13}$ are H and $CH_3$.

[Chem. 5]

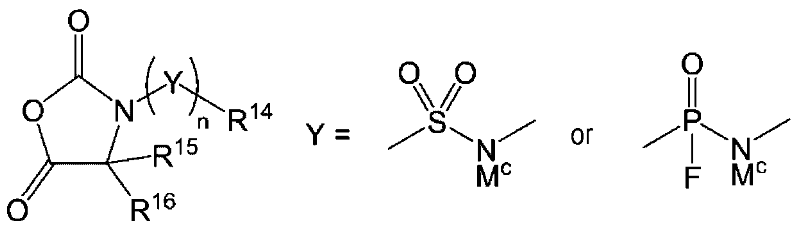

Y = or

(5)

[In the general formula (5),

n represents an integer of 0 or 1.

$M^c$ represents a hydrogen atom or $(M_7^{G+})_g$. Here, $M_7^{G+}$ represents a metal cation or an onium cation, and G represents the valence of the cation. g represents a number satisfying $G \times g = 1$.

$R^{14}$ represents $-S(=O)_2Rj$ or $-P(=O)(R^k)(R^l)$, and here, $R^j$, $R^k$, and $R^l$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a

carbonyl group, an oxygen atom, a sulfur atom, or a nitrogen atom may be interposed between any carbon-carbon atoms of the hydrocarbon group), or $-O^-(M_8^{H+})_h$. Here, $M_8^{H+}$ represents a proton, a metal cation, or an onium cation, and H represents the valence of the cation. h represents a number satisfying $H \times h = 1$.

$R^{15}$ and $R^{16}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{15}$ and $R^{16}$ may be integrated to form a carbonyl group.]

Here, $(Y)_nR^{14}$ in the general formula (5) may be $S(=O)_2OLi$, $S(=O)_2F$, $P(=O)F_2$, or $P(=O)(OCH_3)_2$.

From the viewpoint of the cycle characteristic and the initial resistance characteristic, $(Y)_nR^{14}$ may be preferably $S(=O)_2OLi$, $S(=O)_2F$, or $P(=O)F_2$, and more preferably $S(=O)_2OLi$ or $S(=O)_2F$.

[Chem. 6]

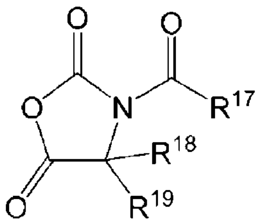

(6)

[In the general formula (6),

$R^{17}$ represents a halogen atom, $-(O)_{x2}-S(=O)_2R^m$, $-(O)_{y2}-P(=O)(R^n)(R^o)$, or $-O^-(M_9^{I+})_i$. Here, $M_9^{I+}$ represents a proton, a metal cation, or an onium cation, and I represents the valence of the cation. i represents a number satisfying $I \times i = 1$.

x2 and y2 each represent an integer of 0 or 1.

$R^m$, $R^n$, and $R^o$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_{10}^{J+})_j$. Here, $M_{10}^{J+}$ represents a proton, a metal cation, or an onium cation, and J represents the valence of the cation. j represents a number satisfying $J \times j = 1$.

$R^{18}$ and $R^{19}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{18}$ and $R^{19}$ may be integrated to form a carbonyl group.]

Here, $R^{17}$ in the general formula (6) may be $OP(=O)F_2$, $S(=O)_2(OCH_2C{\equiv}CH)$, OLi, or F.

From the viewpoint of the cycle characteristic and the initial resistance characteristic, $R^{17}$ may be preferably $OP(=O)F_2$, $S(=O)_2(OCH_2C{\equiv}CH)$, or OLi, and more preferably $OP(=O)F_2$ or $S(=O)_2(OCH_2C{\equiv}CH)$.

[Chem. 7]

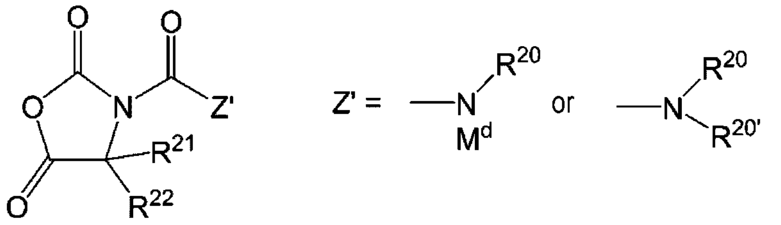

(7)

[In the general formula (7),

$M^d$ represents a hydrogen atom or $(M_{11}{}^{K+})_k$. Here, $M_{11}{}^{K+}$ represents a metal cation or an onium cation, and K represents the valence of the cation. k represents a number satisfying K × k = 1.

$R^{20}$ and $R^{20'}$ each independently represent a hydrogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), $-S(=O)_2R^p$, or $-P(=O)(R^q)(R^r)$.

$R^p$, $R^q$, and $R^r$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_{12}{}^{L+})_1$. Here, $M_{12}{}^{L+}$ represents a proton, a metal cation, or an onium cation, and L represents the valence of the cation. l represents a number satisfying L × l = 1.

$R^{21}$ and $R^{22}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{21}$ and $R^{22}$ may be integrated to form a carbonyl group.]

Here, Z' in the general formula (7) may be $NLi-S(=O)_2F$, $NLi-P(=O)(OCH_2CF_3)_2$, or $NH-P(=O)F_2$.
From the viewpoint of the cycle characteristic, Z' may be preferably $NLi-P(=O)(OCH_2CF_3)_2$.
Moreover, from the viewpoint of the initial resistance characteristic, Z' may be preferably $NLi-S(=O)_2F$ or $NH-P(=O)F_2$, and more preferably $NLi-S(=O)_2F$.

[Chem. 8]

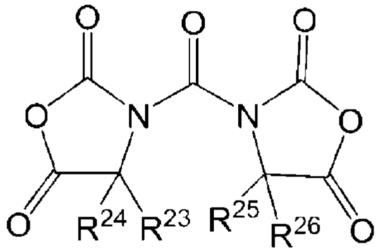

(8)

[In the general formula (8),
$R^{23}$, $R^{24}$, $R^{25}$, and $R^{26}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{23}$ and $R^{24}$ or $R^{25}$ and $R^{26}$ may be each independently integrated to form a carbonyl group.]

Here, the general formula (8) may be a structure in which $R^{23}$ and $R^{24}$ are H and $CH_3$, and $R^{25}$ and $R^{26}$ are H and $CH_3$ or a structure in which $R^{23}$ to $R^{26}$ are all $CF_3$.

From the viewpoint of the cycle characteristic, the general formula (8) may be preferably a structure in which $R^{23}$ and $R^{24}$ are H and $CH_3$, and $R^{25}$ and $R^{26}$ are H and $CH_3$.

Moreover, from the viewpoint of the initial resistance characteristic, the general formula (8) may be preferably a structure in which $R^{23}$ to $R^{26}$ are all $CF_3$.

2. The nonaqueous electrolyte solution according to 1 above in which the concentration of the (III) is 0.009 to 11.5% by mass with respect to the total amount of the nonaqueous electrolyte solution.

3. The nonaqueous electrolyte solution according to 1 or 2 above in which the (III) is at least one selected from the group consisting of the compound represented by the general formula (1), the compound represented by the general formula (2), the compound represented by the general formula (3), the compound represented by the general formula (4), the compound represented by the general formula (5), the compound represented by the general formula (6), and the compound represented by the general formula (7).

4. The nonaqueous electrolyte solution according to any one of 1 to 3 above in which the (III) is at least one selected from the group consisting of the compound represented by the general formula (1), the compound represented by the general formula (2), the compound represented by the general formula (3), and the compound represented by the general formula (4).

5. The nonaqueous electrolyte solution according to any one of 1 to 4 above in which the compound represented by the general formula (1) is at least one selected from the group consisting of the following compounds.

[Chem. 9]

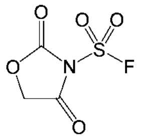
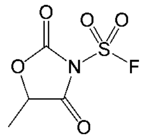
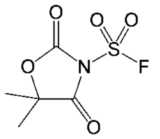
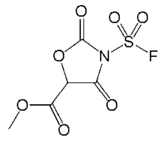
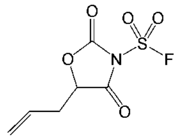
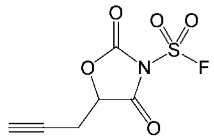
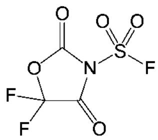
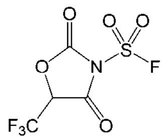
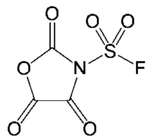
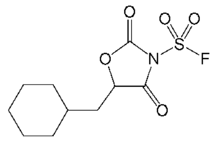
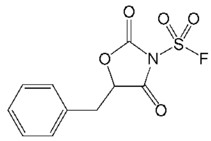
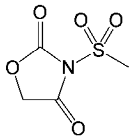
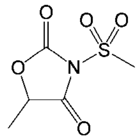
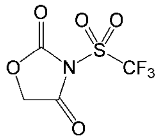
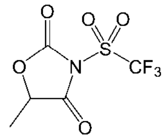
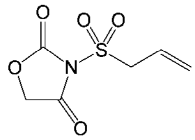
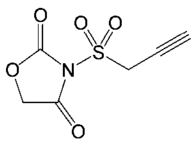
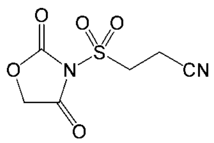
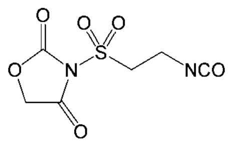
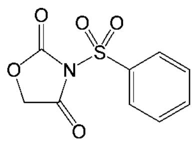

[Chem. 10]

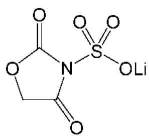
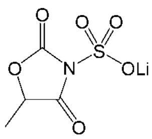
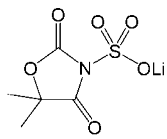
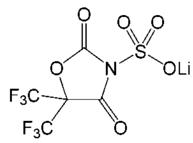
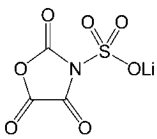
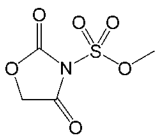
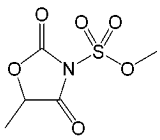
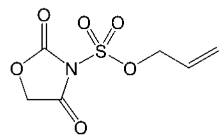
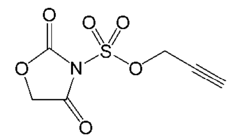
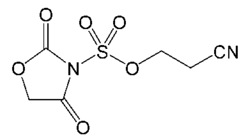
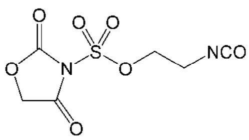
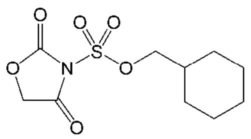
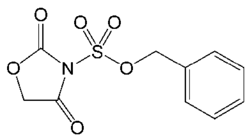
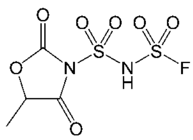
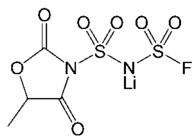

[Chem. 11]

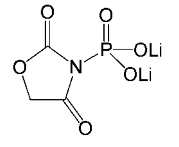 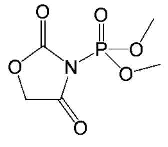 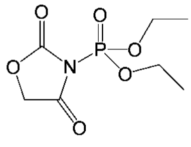 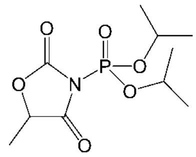 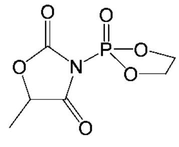

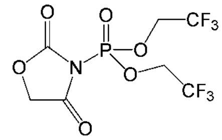 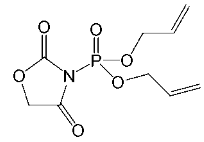 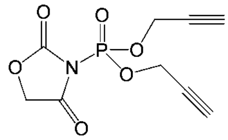 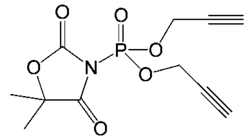 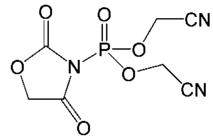

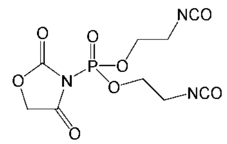 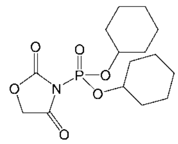 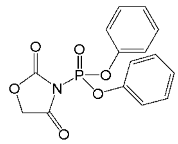 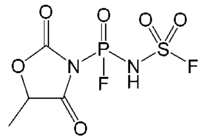 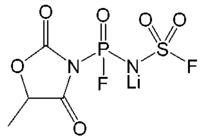

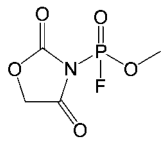 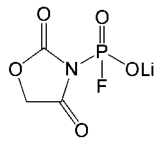 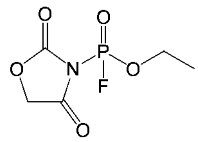 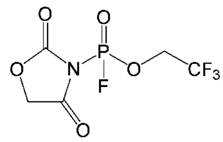 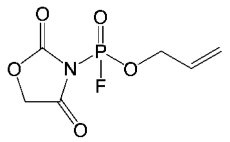

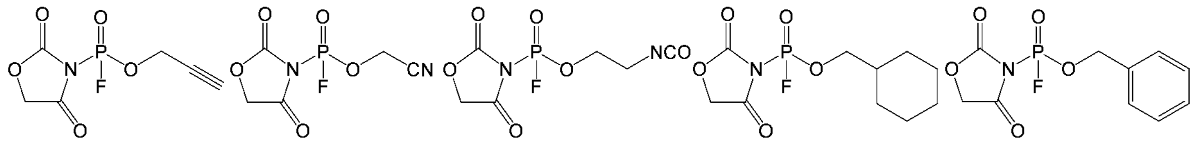

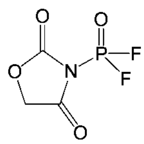 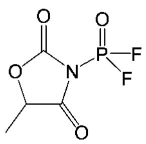 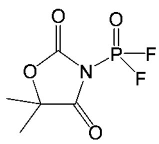 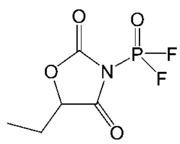 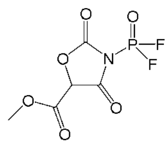 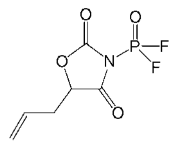

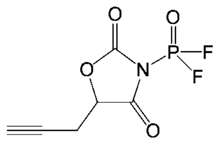 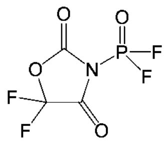 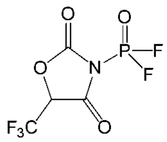 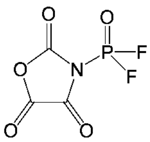 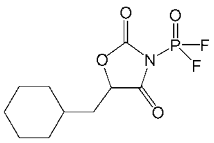 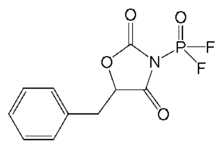

6. The nonaqueous electrolyte solution according to any one of 1 to 4 above in which the compound represented by the general formula (2) is at least one selected from the group consisting of the following compounds.

[Chem. 12]

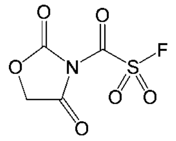 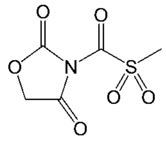 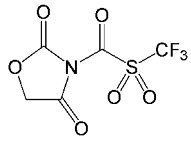 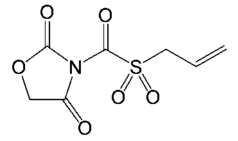 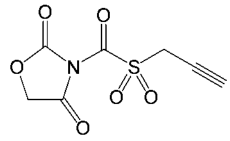

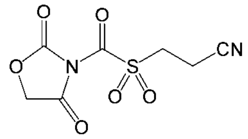 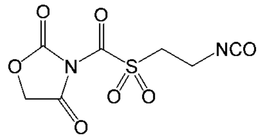 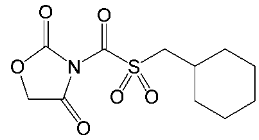 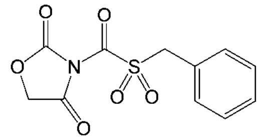

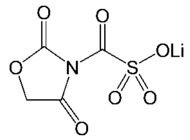 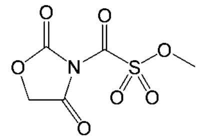 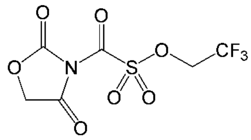 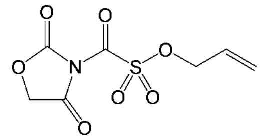

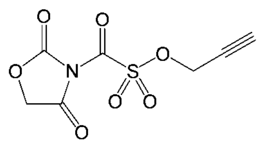 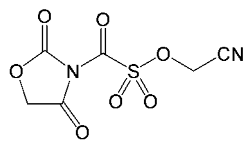 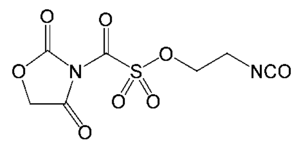 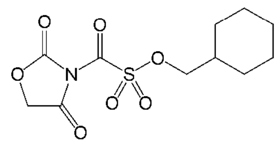

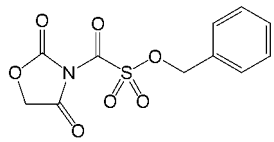 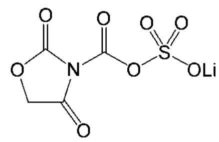 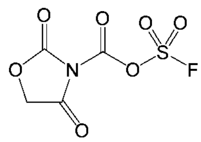 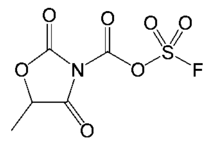

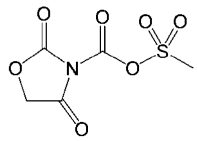 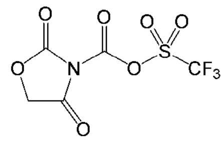 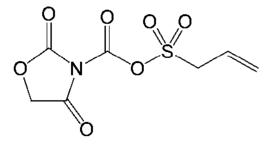 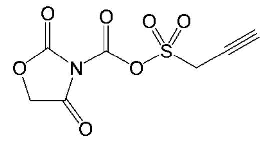

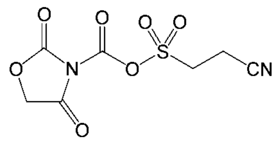 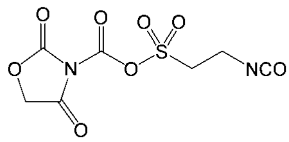 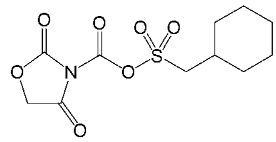 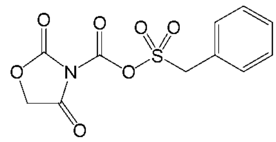

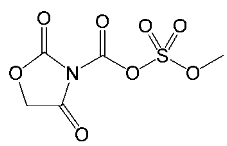 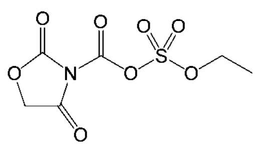 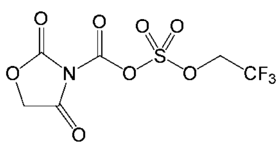 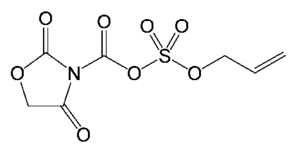

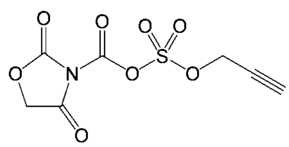 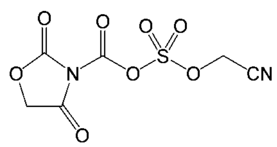 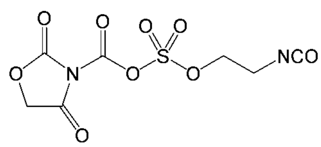

[Chem. 13]

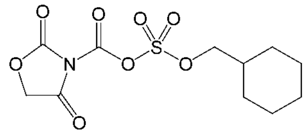
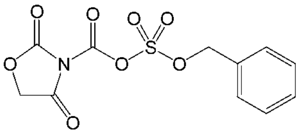

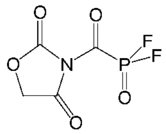
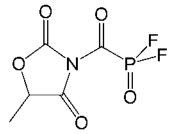
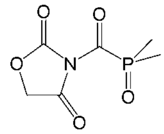
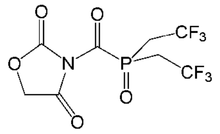
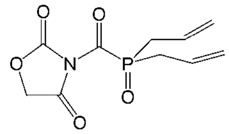

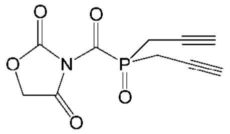
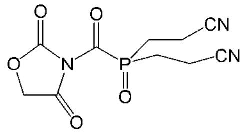
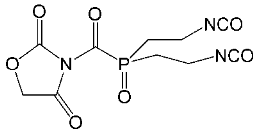

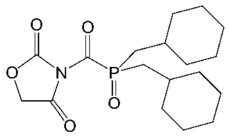
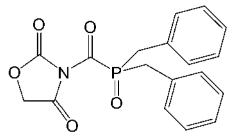

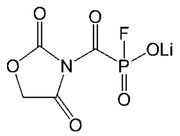
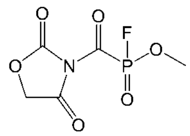
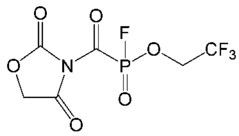
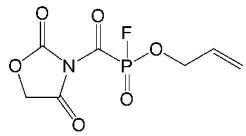
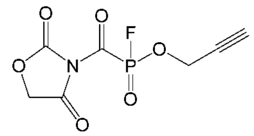

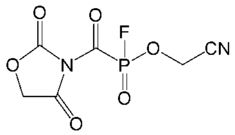
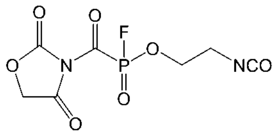
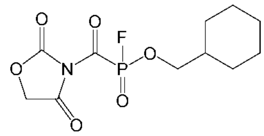
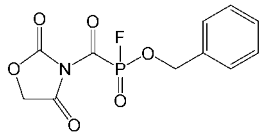

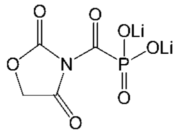
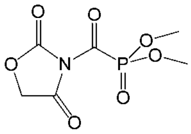
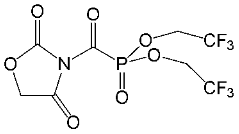
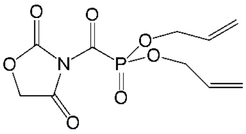

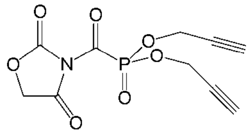
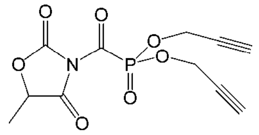
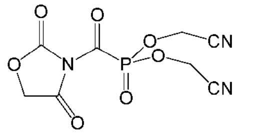

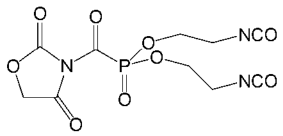
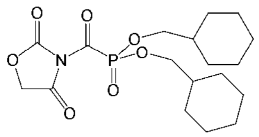
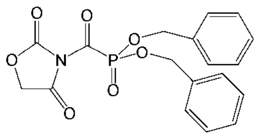

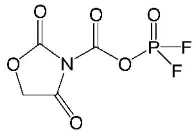
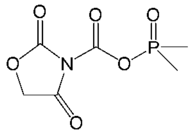
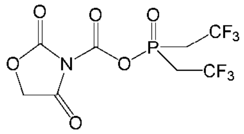
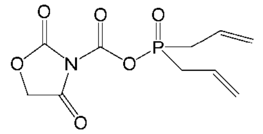

[Chem. 14]

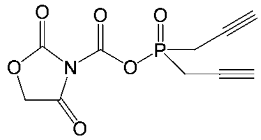 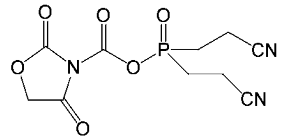 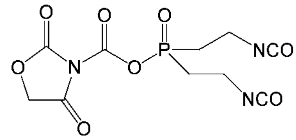 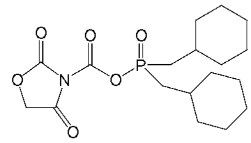

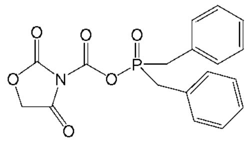 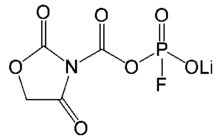 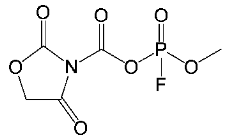 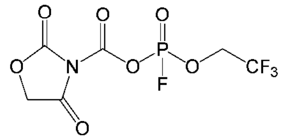

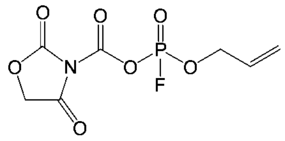 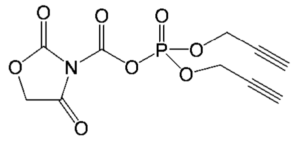 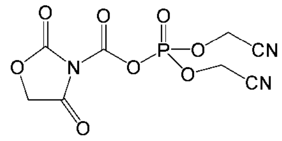 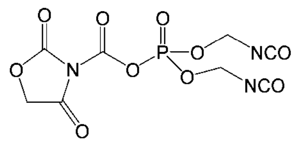

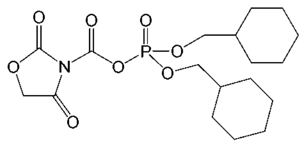 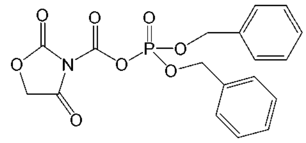

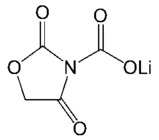 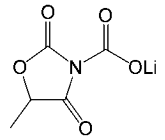 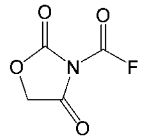 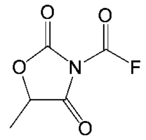 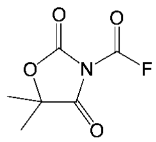

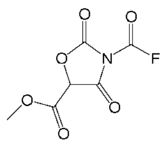 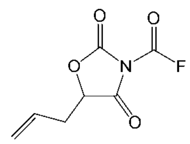 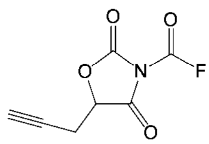 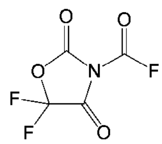

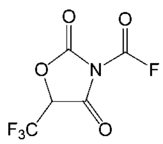 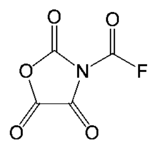 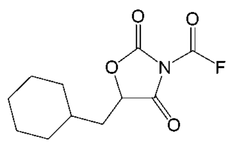 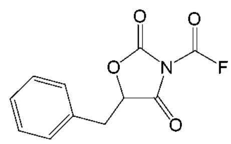

7. The nonaqueous electrolyte solution according to any one of 1 to 4 above in which the compound represented by the general formula (3) is at least one selected from the group consisting of the following compounds.

[Chem. 15]

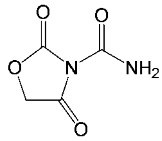
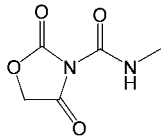
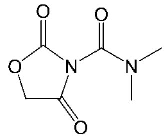
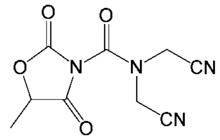
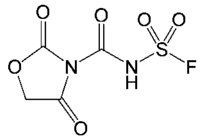
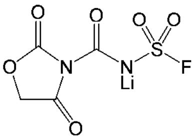
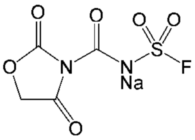
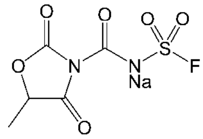
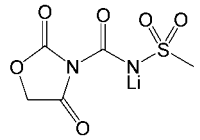
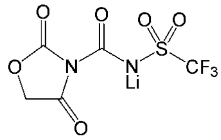
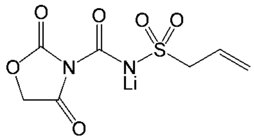
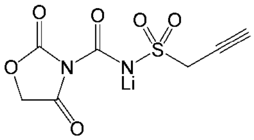
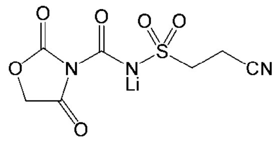
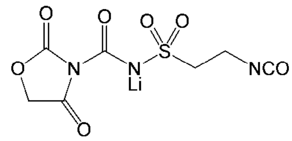
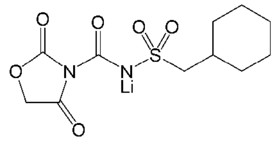
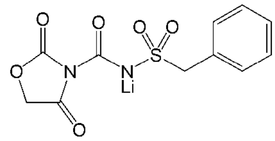
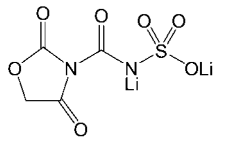
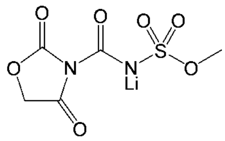
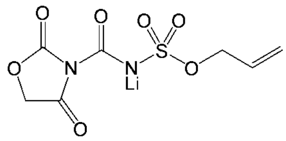
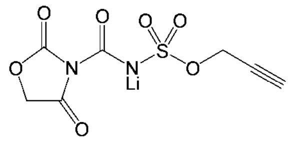
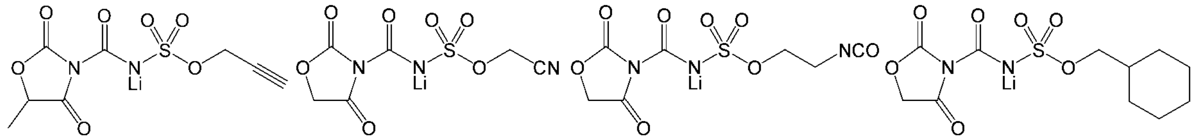
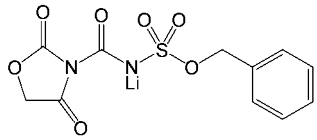
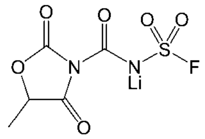
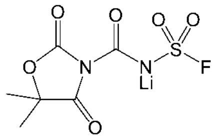
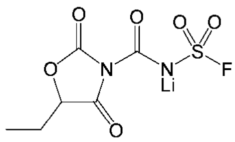
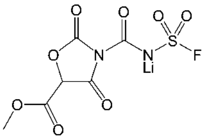
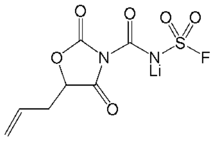
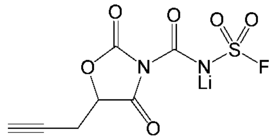
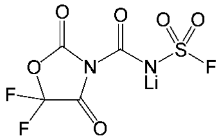
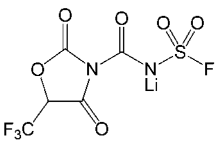
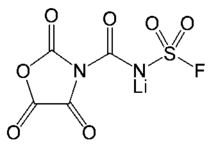
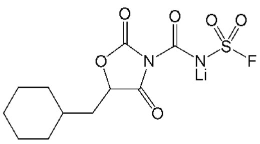
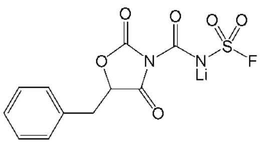
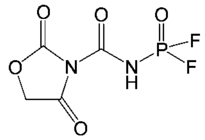
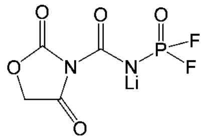
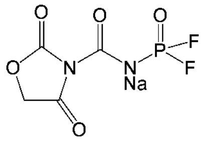
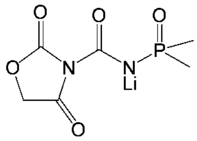
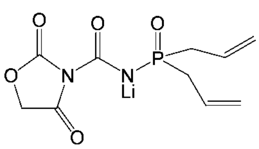

[Chem. 16]

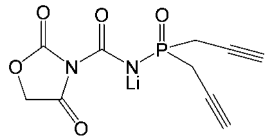
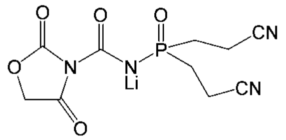
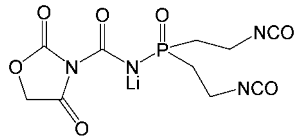

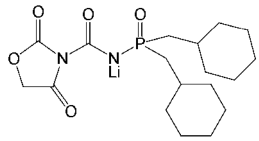
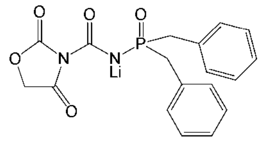

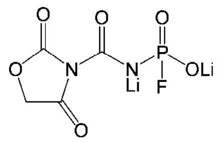
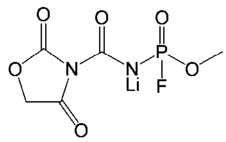
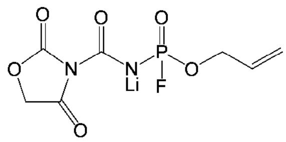
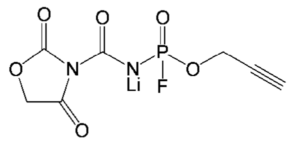

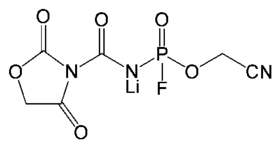
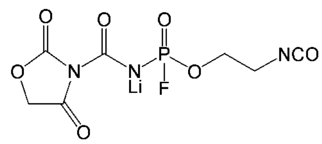
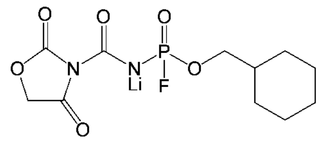

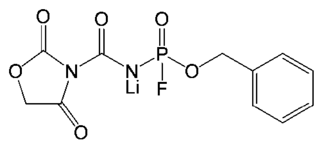
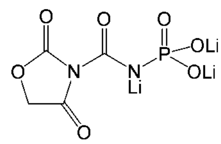
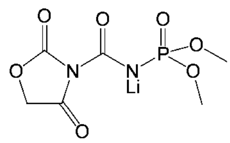
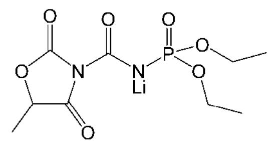

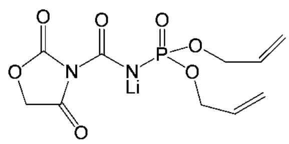
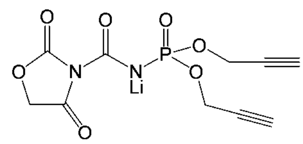
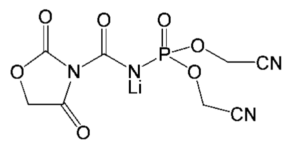

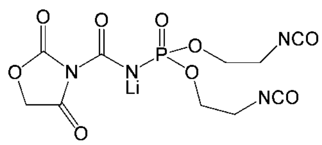
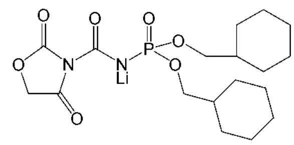
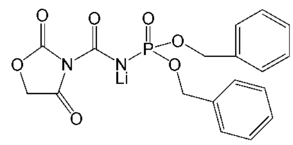

8. The nonaqueous electrolyte solution according to any one of 1 to 4 above in which the compound represented by the general formula (4) is at least one selected from the group consisting of the following compounds.

[Chem. 17]

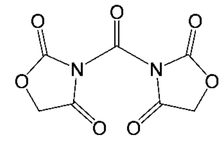 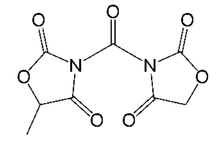 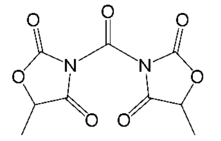 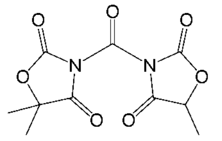 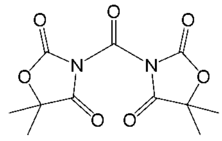

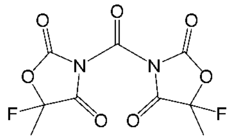 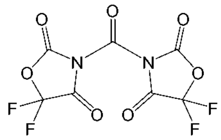 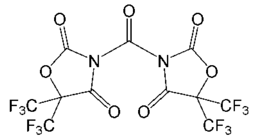 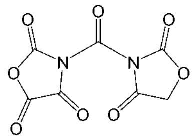 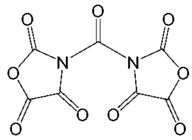

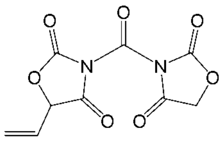 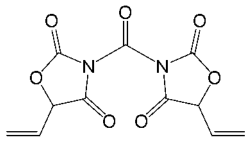 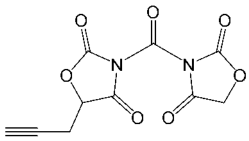 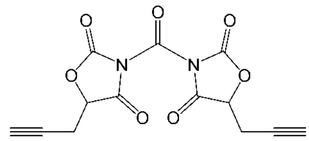

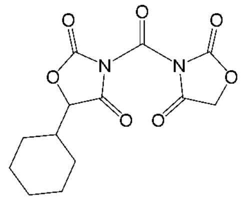 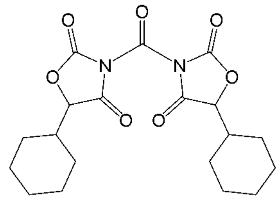 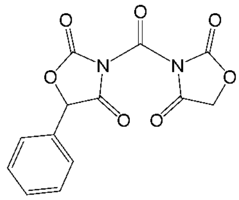 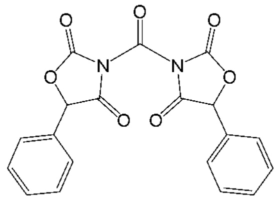

9. The nonaqueous electrolyte solution according to any one of 1 to 3 above in which the compound represented by the general formula (5) is at least one selected from the group consisting of the following compounds.

[Chem. 18]

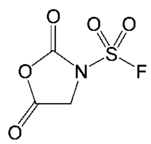 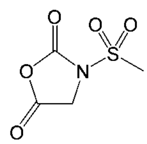 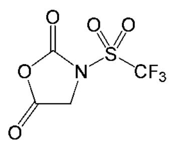 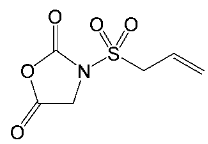

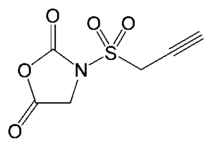 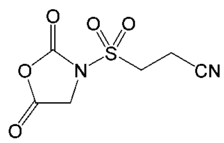 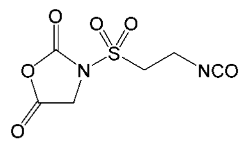 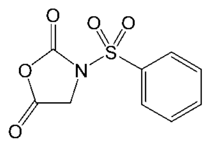

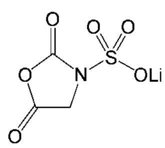 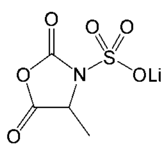 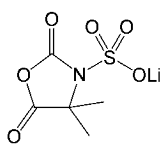 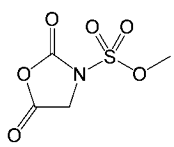 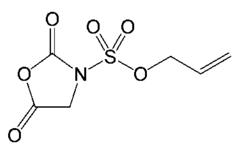

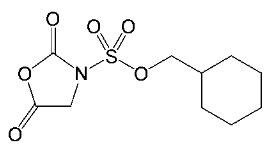 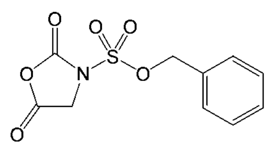 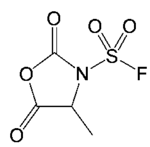 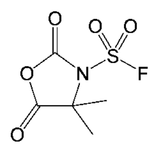 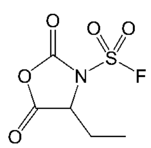

[Chem. 19]

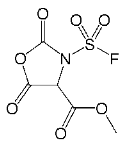 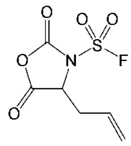 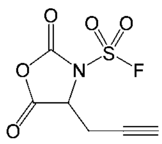 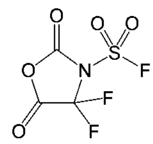

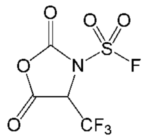 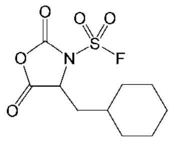 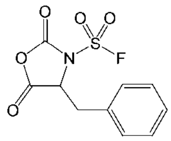

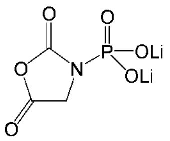 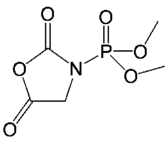 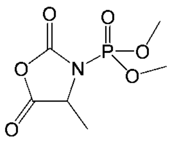 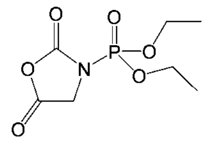 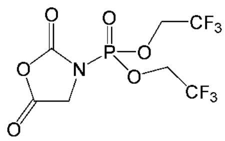

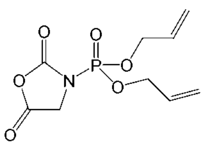 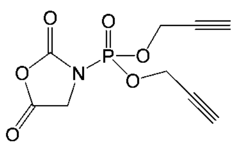 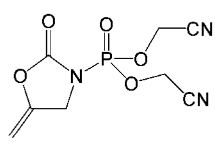 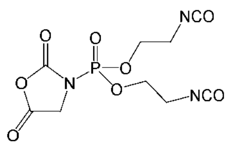

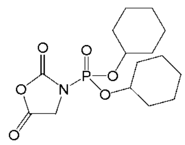 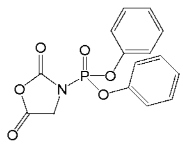 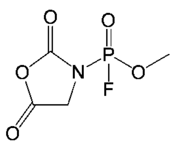 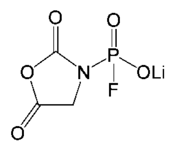 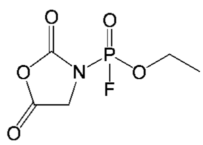

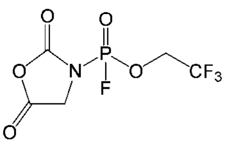 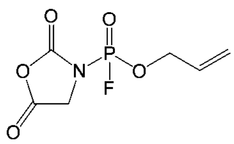 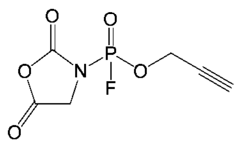

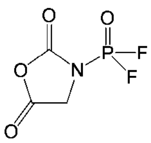 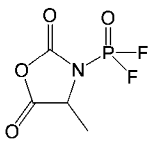 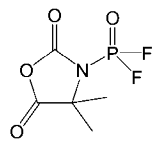

Chem. 0. The nonaqueous electrolyte solution according to any one of 1 to 3 above in which the compound represented by the general formula (6) is at least one selected from the group consisting of the following compounds.

[Chem. 20]

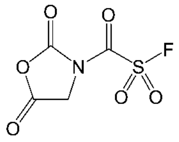 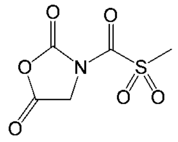 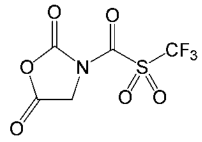 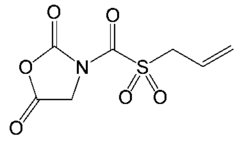 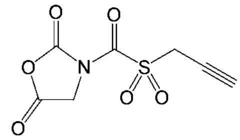

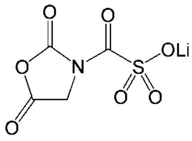 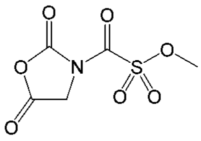 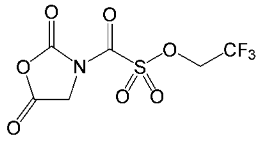 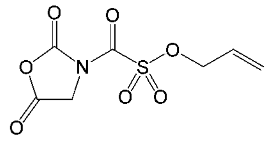

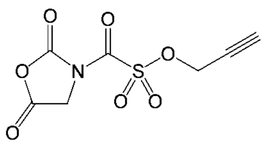 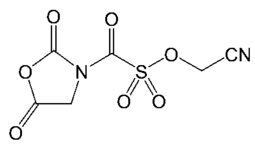 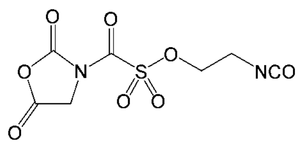

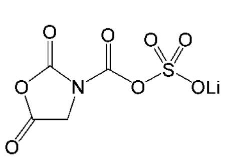 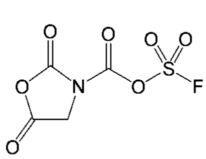 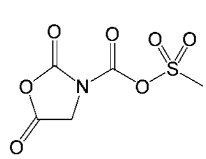 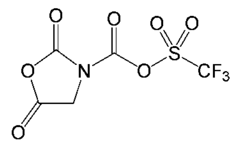

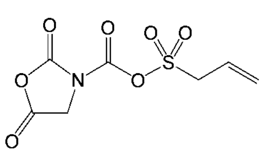 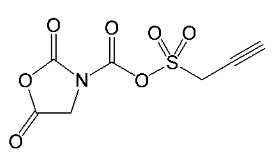 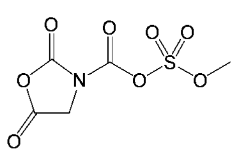 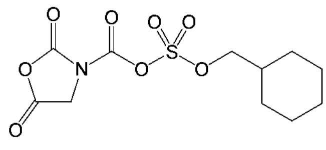

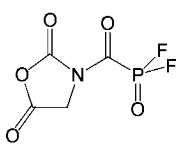 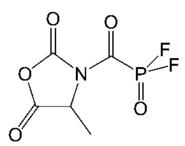 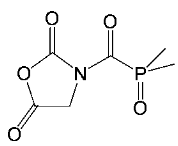 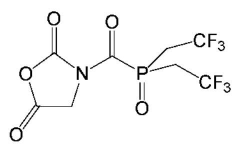

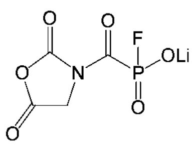 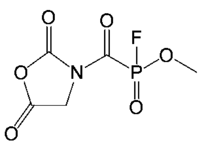 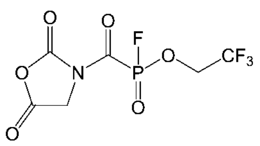 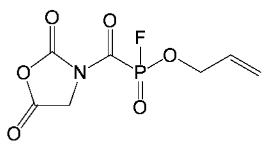

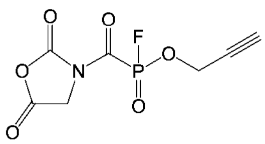 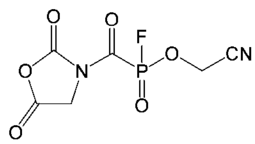 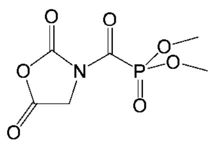 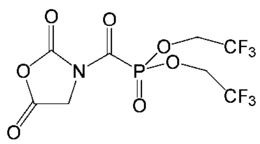

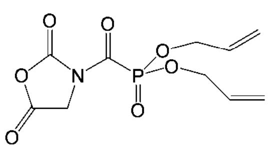 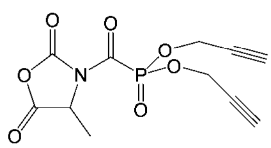 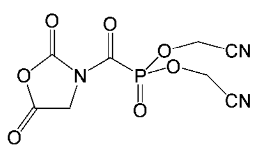 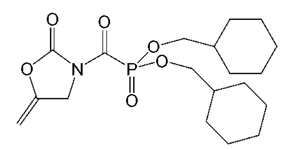

[Chem. 21]

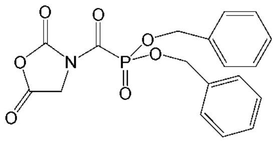
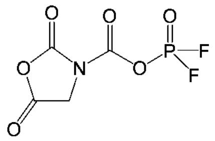
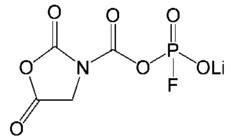

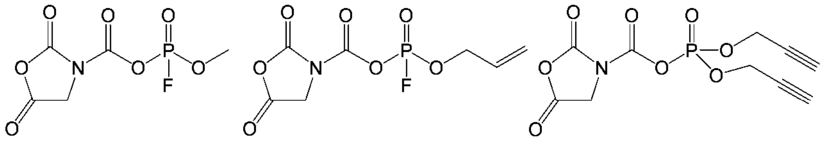

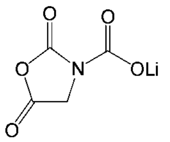
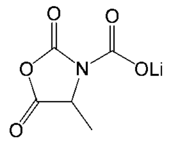
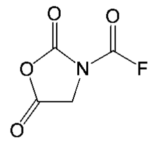
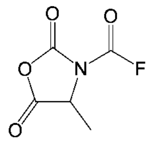

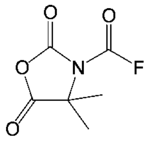
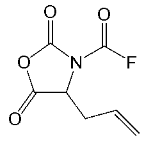
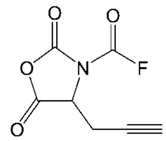
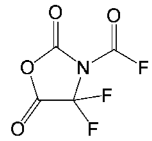

11. The nonaqueous electrolyte solution according to any one of 1 to 3 above in which the compound represented by the general formula (7) is at least one selected from the group consisting of the following compounds.

[Chem. 22]

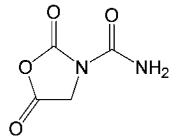
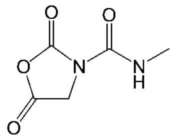
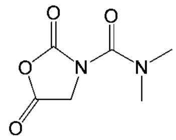
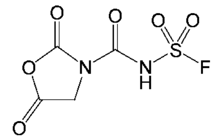
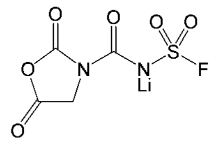

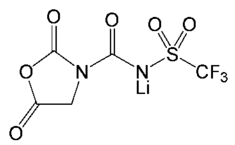
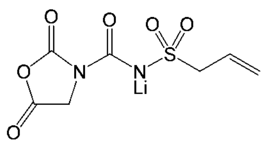
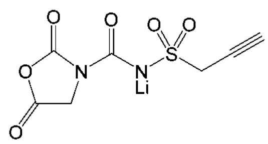
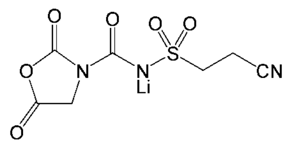

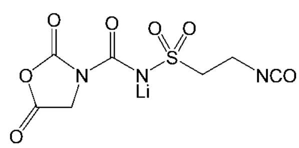
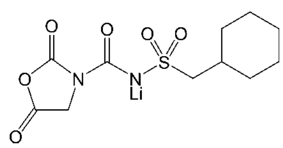
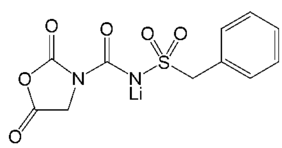
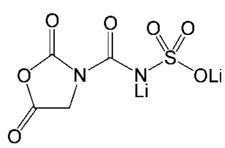

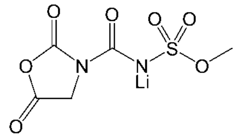
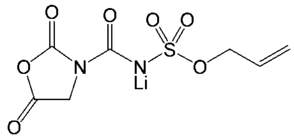
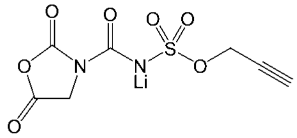
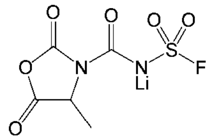

[Chem. 23]

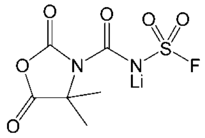 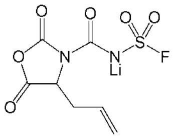 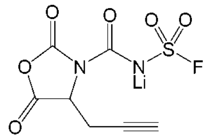 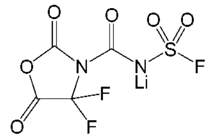

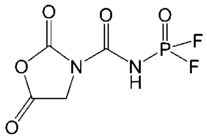 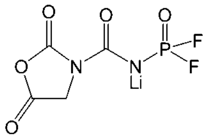 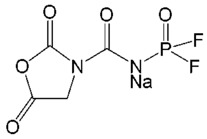 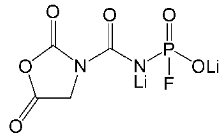

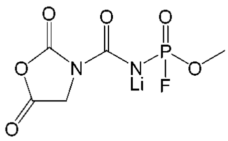 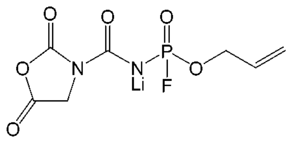 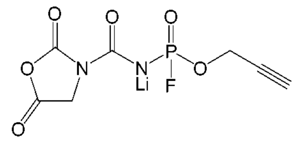 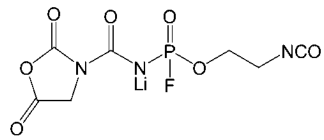

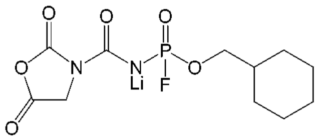 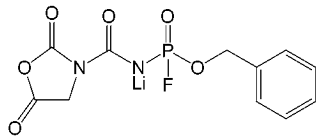 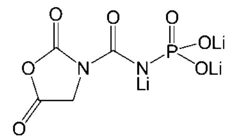 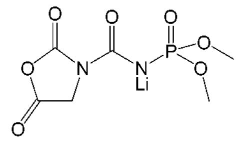

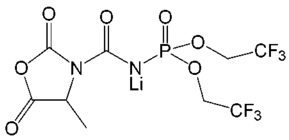 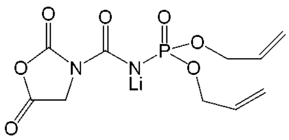 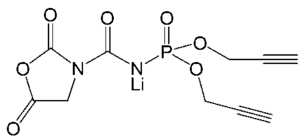 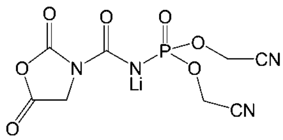

12. The nonaqueous electrolyte solution according to 1 or 2 above in which the compound represented by the general formula (8) is at least one selected from the group consisting of the following compounds.

[Chem. 24]

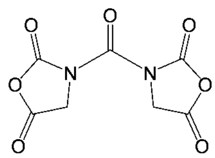 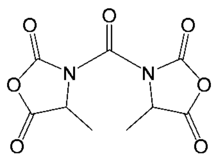 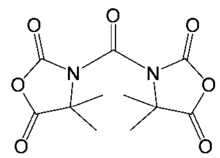 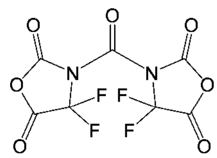

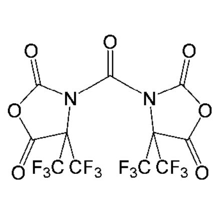 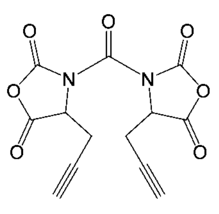 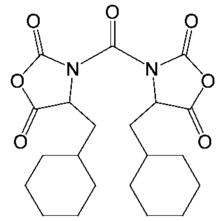 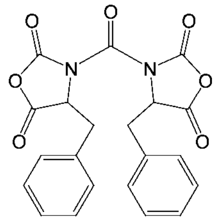

13. The nonaqueous electrolyte solution according to any one of 1 to 12 above in which the (I) is at least one selected from the group consisting of $LiPF_6$, $LiBF_4$, $LiSbF_6$, $LiAsF_6$, $LiClO_4$, $LiN(SO_2F)_2$ (hereinafter sometimes referred to as "FSI"), $LiAlO_2$, $LiAlCl_4$, LiCl, and LiI or at least one selected from the group consisting of $NaPF_6$, $NaBF_4$, $NaSbF_6$, $NaAsF_6$, $NaClO_4$, $NaN(SO_2F)_2$, $NaAlO_2$, $NaAlCl_4$, NaCl, and NaI.

14. The nonaqueous electrolyte solution according to 1 or 2 above in which the (II) contains at least one selected from the group consisting of a cyclic ester, a chain ester, a cyclic ether, a chain ether, a sulfone compound, a sulfoxide compound, and an ionic liquid.

15. The nonaqueous electrolyte solution according to 14 above in which the cyclic ester contains a cyclic

carbonate.

16. The nonaqueous electrolyte solution according to 15 above in which the cyclic carbonate contains at least one selected from the group consisting of ethylene carbonate and propylene carbonate.

17. The nonaqueous electrolyte solution according to 14 above in which the chain ester contains a chain carbonate.

18. The nonaqueous electrolyte solution according to 17 above in which the chain carbonate contains at least one selected from the group consisting of ethyl methyl carbonate, dimethyl carbonate, diethyl carbonate, and methyl propyl carbonate.

19. The nonaqueous electrolyte solution according to any one of 1 to 18 above further containing at least one selected from the group consisting of cyclohexylbenzene, cyclohexylfluorobenzene, biphenyl, 2-fluorobiphenyl, t-butylbenzene, t-amylbenzene, 2-fluorotoluene, fluorobenzene, vinylene carbonate (hereinafter sometimes referred to as "VC"), an oligomer of vinylene carbonate (having a number average molecular weight in terms of polystyrene of 170 to 5000), vinylethylene carbonate, difluoroanisole, fluoroethylene carbonate (hereinafter sometimes referred to as "FEC"), 1,6-diisocyanatohexane, ethynylethylene carbonate, trans-difluoroethylene carbonate, methyl propargyl carbonate, ethyl propargyl carbonate, dipropargyl carbonate, maleic anhydride, succinic anhydride, 1,3-propanesultone, 1,3-propenesultone, 1,4-butanesultone, dimethylvinylene carbonate, 1,3,2-dioxathiolane-2,2-dioxide (hereinafter sometimes referred to as "DTD"), 4-propyl-1,3,2-dioxathiolane-2,2-dioxide, methylene methanedisulfonate, dimethylene methanedisulfonate, trimethylene methanedisulfonate, methyl methanesulfonate, methanesulfonyl fluoride, ethenesulfonyl fluoride, phenyl difluorophosphate, 1,2-ethanedisulfonic anhydride, tris(trimethylsilyl)borate, succinonitrile, (ethoxy)pentafluorocyclotriphosphazene, tetrafluoro(picolinato)phosphate, difluoro(picolinato)borate, 1,3-dimethyl-1,3-divinyl-1,3-di(1,1,1,3,3,3-hexafluoroisopropyl)disiloxane, lithium difluorophosphate (hereinafter sometimes referred to as "DFP"), sodium difluorophosphate, lithium fluorosulfate (hereinafter sometimes referred to as "FS"), sodium fluorosulfate, lithium trifluoromethanesulfonate, sodium trifluoromethanesulfonate, lithium nonafluorobutanesulfonate, sodium nonafluorobutanesulfonate, lithium difluorobis(oxalato)phosphate (hereinafter sometimes referred to as "DFBOP"), sodium difluorobis(oxalato)phosphate, lithium difluorooxalatoborate (hereinafter sometimes referred to as "DFOB"),
sodium difluorooxalatoborate, lithium bis(oxalato)borate (hereinafter sometimes referred to as "BOB"), sodium bis(oxalato)borate, lithium tetrafluorooxalatophosphate (hereinafter sometimes referred to as "TFOP"), sodium tetrafluorooxalatophosphate, lithium tris(oxalato)phosphate, sodium tris(oxalato)phosphate, lithium difluoro(malonato)borate, sodium difluoro(malonato)borate, lithium tetrafluoro(malonato)phosphate, sodium tetrafluoro(malonato)phosphate, lithium monofluorophosphate, sodium monofluorophosphate, lithium bis(difluorophosphoryl)imide, sodium bis(difluorophosphoryl)imide, lithium (difluorophosphoryl)(fluorosulfonyl)imide (hereinafter sometimes referred to as "DFPFSI"), sodium (difluorophosphoryl)(fluorosulfonyl)imide, lithium bis(trifluoromethanesulfonyl)imide, sodium bis(trifluoromethanesulfonyl)imide, lithium bis(pentafluoroethanesulfonyl)imide, sodium bis(pentafluoroethanesulfonyl)imide, lithium (trifluoromethanesulfonyl)(pentafluoroethanesulfonyl)imide, sodium (trifluoromethanesulfonyl)(pentafluoroethanesulfonyl) imide, lithium (trifluoromethanesulfonyl)(fluorosulfonyl)imide, sodium (trifluoromethanesulfonyl)(fluorosulfonyl)imide, lithium (pentafluoroethanesulfonyl)(fluorosulfonyl)imide, sodium (pentafluoroethanesulfonyl)(fluorosulfonyl)imide, lithium tris(trifluoromethanesulfonyl)methide, sodium tris(trifluoromethanesulfonyl)methide, lithium acrylate, sodium acrylate, lithium methacrylate, sodium methacrylate, lithium methyl sulfate, sodium methyl sulfate, lithium ethyl sulfate, sodium ethyl sulfate, lithium nitrate, lithium nitrite, sodium nitrate, and sodium nitrite.

20. The nonaqueous electrolyte solution according to any one of 1 to 19 above further containing a compound represented by the following general formula (9).

[Chem. 25]

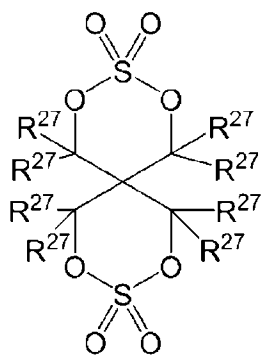

(9)

[In the general formula (9), $R^{27}$'s each independently represent a hydrogen atom, a halogen atom, or an alkyl group having 1 to 10 carbon atoms, and an oxygen atom may be included in any carbon atom-carbon atom bond in the alkyl group. Moreover, any hydrogen atom of the alkyl group may be substituted with a fluorine atom.]

21. The nonaqueous electrolyte solution according to 20 above in which the compound represented by the general formula (9) is at least one selected from the group consisting of the following compounds 1-1 (hereinafter sometimes referred to as "TDUTO") and 1-2.

[Chem. 26]

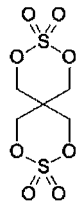

Compound 1-1

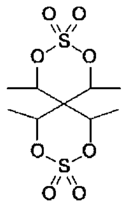

Compound 1-2

22. The nonaqueous electrolyte solution according to any one of 1 to 21 above further containing a compound represented by the following general formula (10).

[Chem. 27]

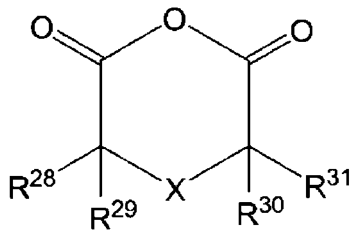

(10)

[In the general formula (10), $R^{28}$ to $R^{31}$ each independently represent a hydrogen atom, a halogen atom, an alkyl group, an alkenyl group, or an aryl group. X represents an oxygen atom, a sulfur atom, or an $SO_2$ group.]

23. The nonaqueous electrolyte solution according to 22 above in which the compound of the formula (10) is a compound represented by the following formula.

[Chem. 28]

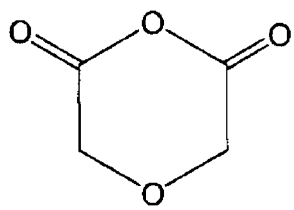

24. A nonaqueous electrolyte solution battery containing at least a positive electrode, a negative electrode, a separator, and the nonaqueous electrolyte solution according to any one of 1 to 23 above.

25. A compound represented by any of the following general formulae (1) to (8).

[Chem. 29]

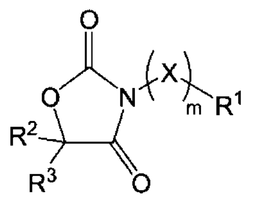

X =

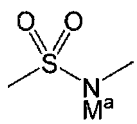

or

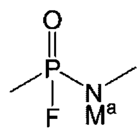

(1)

[In the general formula (1),
m represents an integer of 0 or 1.

$M^a$ represents a hydrogen atom or $(M_1^{A+})_a$. Here, $M_1^{A+}$ represents a metal cation or an onium cation, and A represents the valence of the cation. a represents a number satisfying $A \times a = 1$.

$R^1$ represents $-S(=O)_2R^a$ or $-P(=O)(R^b)(R^c)$.

$R^a$, $R^b$, and $R^c$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_2^{B+})_b$. Here, $M_2^{B+}$ represents a proton, a metal cation, or an onium cation, and B represents the valence of the cation. b represents a number satisfying $B \times b = 1$. Here, $R^b$ and $R^c$ may together form a 5-membered ring or a 6-membered ring together with the phosphorus atom.

$R^2$ and $R^3$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^2$ and $R^3$ may be integrated to form a carbonyl group.]

[Chem. 30]

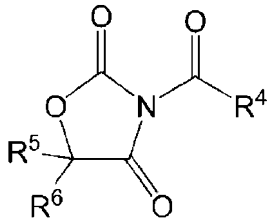

(2)

[In the general formula (2),

$R^4$ represents a halogen atom, $-(O)_{x1}-S(=O)_2R^d$, $-(O)_{y1}-P(=O)(R^e)(R^f)$, or $-O^-(M_3^{C+})_c$. Here, $M_3^{C+}$ represents a proton, a metal cation, or an onium cation, and C represents the valence of the cation. c represents a number satisfying $C \times c = 1$, and x1 and y1 each represent an integer of 0 or 1.

$R^d$, $R^e$, and $R^f$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_4^{D+})_d$. Here, $M_4^{D+}$ represents a proton, a metal cation, or an onium cation, and D represents the valence of the cation. d represents a number satisfying $D \times d = 1$.

$R^5$ and $R^6$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen

atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^5$ and $R^6$ may be integrated to form a carbonyl group.]

[Chem. 31]

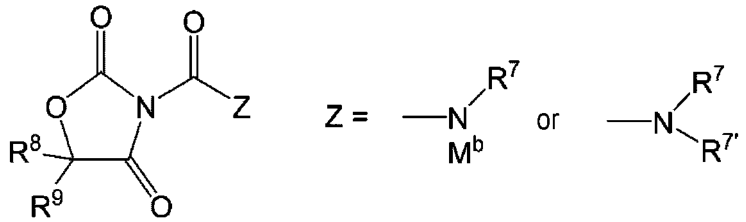

(3)

[In the general formula (3),

$M^b$ represents a hydrogen atom or $(M_5^{E+})_e$. Here, $M_5^{E+}$ represents a metal cation or an onium cation, and E represents the valence of the cation. e represents a number satisfying $E \times e = 1$.

$R^7$ and $R^{7'}$ each independently represent a hydrogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), $-S(=O)_2R^g$, or $-P(=O)(R^h)(R^i)$.

$R^g$, $R^h$, and $R^i$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_6^{F+})_f$. Here, $M_6^{F+}$ represents a proton, a metal cation, or an onium cation, and F represents the valence of the cation. f represents a number satisfying $F \times f = 1$.

$R^8$ and $R^9$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^8$ and $R^9$ may be integrated to form a carbonyl group.]

[Chem. 32]

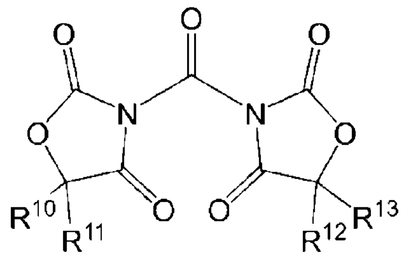

(4)

[In the general formula (4),
$R^{10}$, $R^{11}$, $R^{12}$, and $R^{13}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{10}$ and $R^{11}$ or $R^{12}$ and $R^{13}$ may be each independently integrated to form a carbonyl group.]

[Chem. 33]

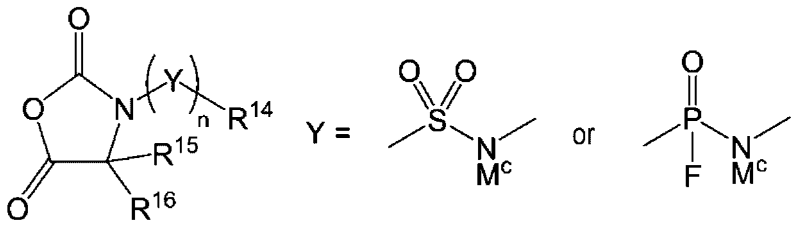

(5)

[In the general formula (5),
n represents an integer of 0 or 1.

$M^c$ represents a hydrogen atom or $(M_7{}^{G+})_g$. Here, $M_7{}^{G+}$ represents a metal cation or an onium cation, and G represents the valence of the cation. g represents a number satisfying $G \times g = 1$.

$R^{14}$ represents $-S(=O)_2R^j$ or $-P(=O)(R^k)(R^l)$, and here, $R^j$, $R^k$, and $R^l$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_8{}^{H+})_h$. Here, $M_8{}^{H+}$ represents a proton, a metal cation, or an onium cation, and H represents the valence of the cation. h represents a number satisfying $H \times h = 1$.

$R^{15}$ and $R^{16}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{15}$ and $R^{16}$ may be integrated to form a carbonyl group.]

[Chem. 34]

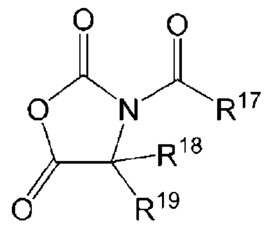

(6)

[In the general formula (6),

$R^{17}$ represents a halogen atom, $-(O)_{x2}-S(=O)_2R^m$, $-(O)_{y2}-P(=O)(R^n)(R^o)$, or $-O^-(M_9^{l+})_i$. Here, $M_9^{l+}$ represents a proton, a metal cation, or an onium cation, and l represents the valence of the cation. i represents a number satisfying $l \times i = 1$.

x2 and y2 each represent an integer of 0 or 1.

$R^m$, $R^n$, and $R^o$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_{10}^{J+})_j$. Here, $M_{10}^{J+}$ represents a proton, a metal cation, or an onium cation, and J represents the valence of the cation. j represents a number satisfying $J \times j = 1$.

$R^{18}$ and $R^{19}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{18}$ and $R^{19}$ may be integrated to form a carbonyl group.]

[Chem. 35]

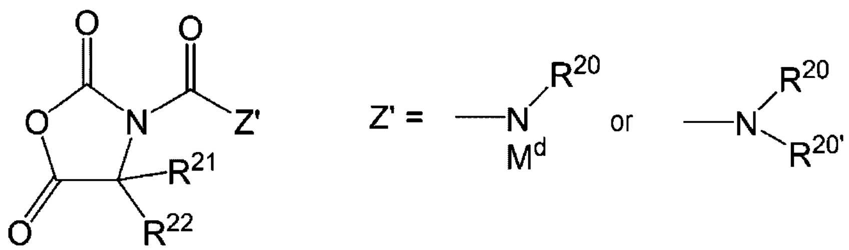

(7)

[In the general formula (7),

$M^d$ represents a hydrogen atom or $(M_{11}^{K+})_k$. Here, $M_{11}^{K+}$ represents a metal cation or an onium cation, and K represents the valence of the cation. k represents a number satisfying $K \times k = 1$.

$R^{20}$ and $R^{20'}$ each independently represent a hydrogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8

carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), $-S(=O)_2R^p$, or $-P(=O)(R^q)(R^r)$.

$R^p$, $R^q$, and $R^r$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_{12}{}^{L+})_l$. Here, $M_{12}{}^{L+}$ represents a proton, a metal cation, or an onium cation, and L represents the valence of the cation. l represents a number satisfying L x l = 1.

$R^{21}$ and $R^{22}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{21}$ and $R^{22}$ may be integrated to form a carbonyl group.]

[Chem. 36]

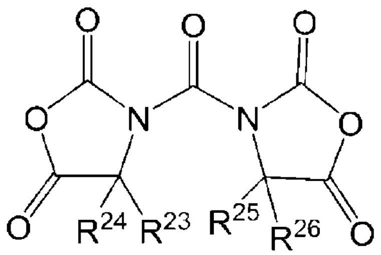

(8)

[In the general formula (8),
$R^{23}$, $R^{24}$, $R^{25}$, and $R^{26}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group). Here, $R^{23}$ and $R^{24}$ or $R^{25}$ and $R^{26}$ may be each independently integrated to form a carbonyl group.]

26. The compound according to 25 above in which the compound represented by the general formula (1) is selected from the following compounds.

[Chem. 37]

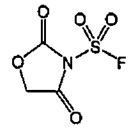 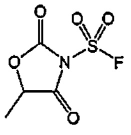 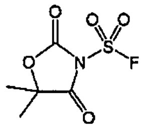 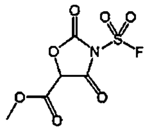 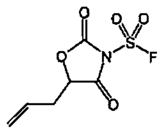 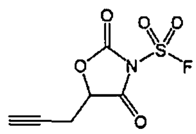

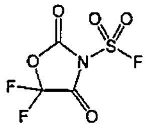 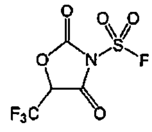 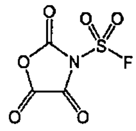 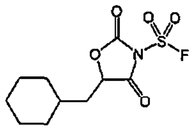 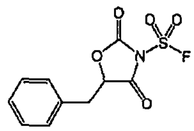

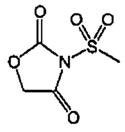 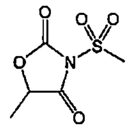 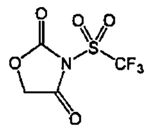 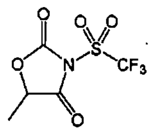 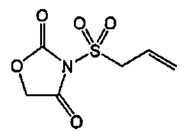

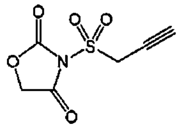 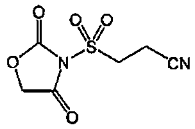 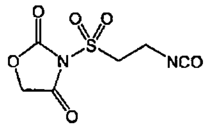 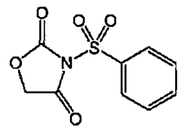

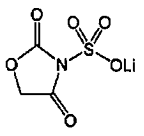 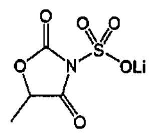 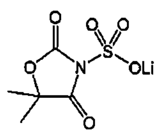 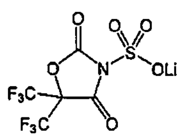 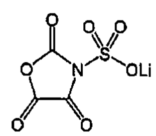

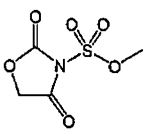 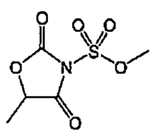 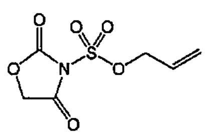 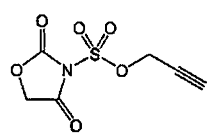 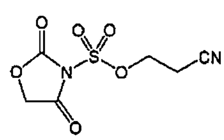

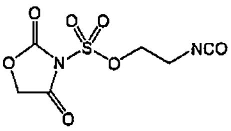 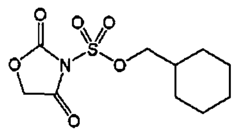 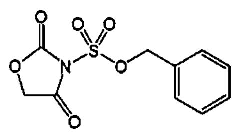 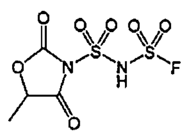 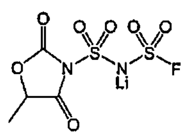

[Chem. 38]

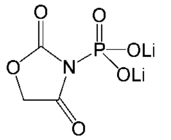 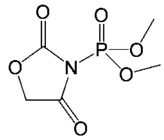 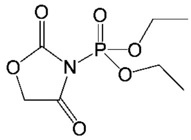 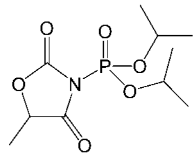 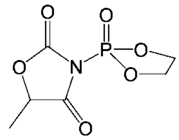

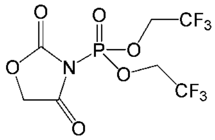 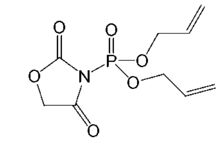 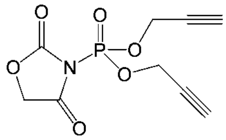 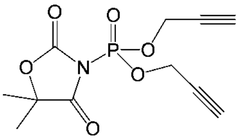 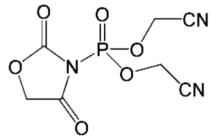

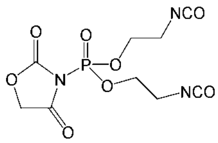 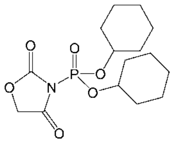 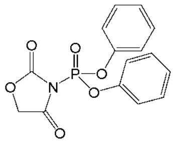 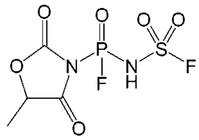 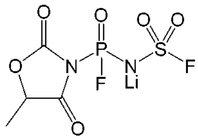

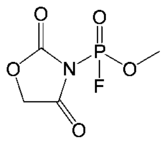 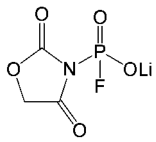 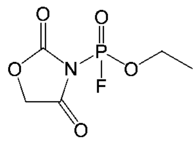 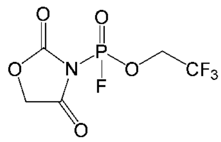 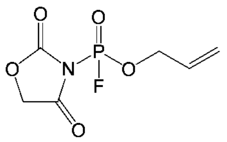

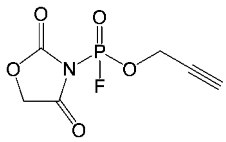 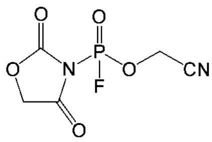 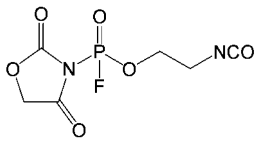 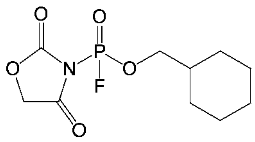 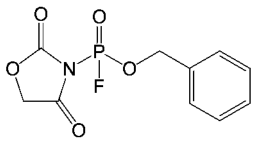

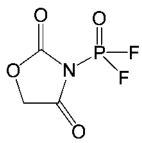 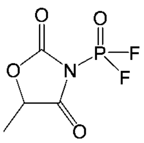 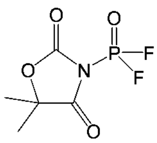 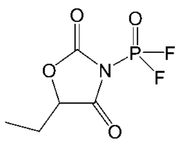 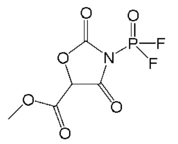 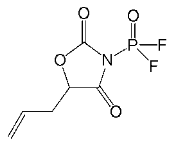

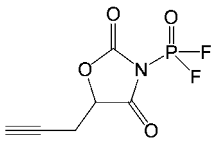 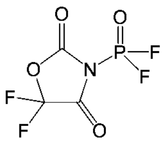 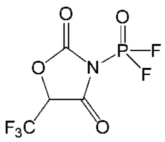 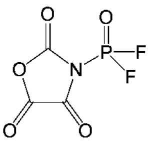 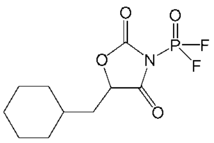 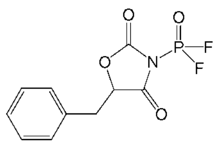

27. The compound according to 25 above in which the compound represented by the general formula (2) is selected from the following compounds.

[Chem. 39]

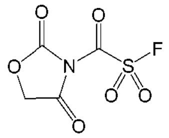 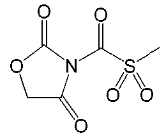 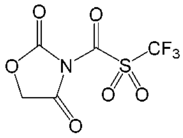 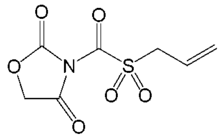 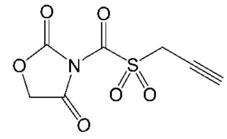

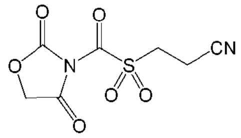 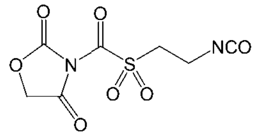 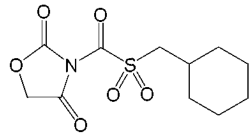 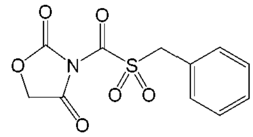

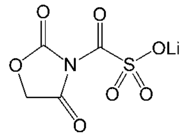 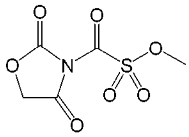 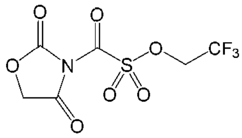 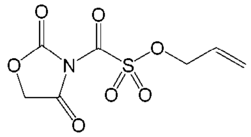

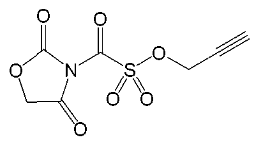 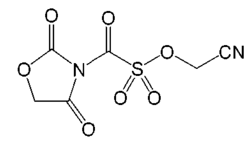 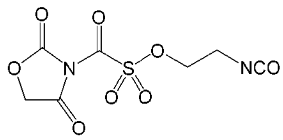 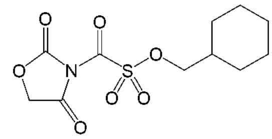

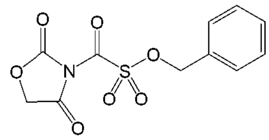 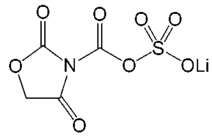 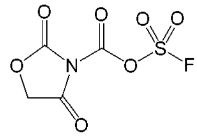 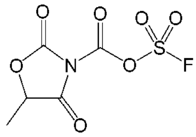

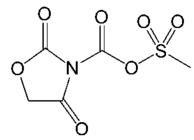 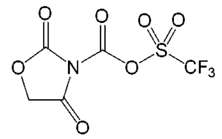 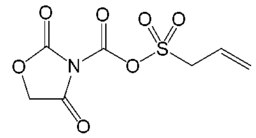 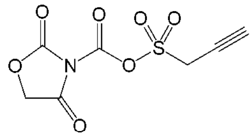

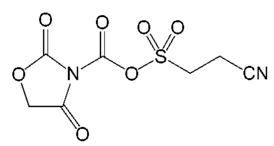 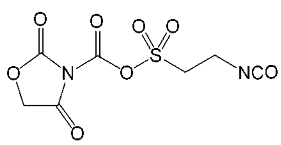 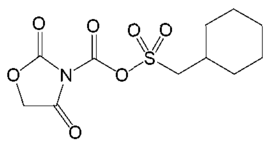 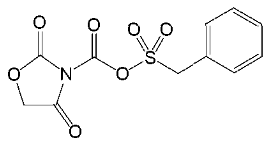

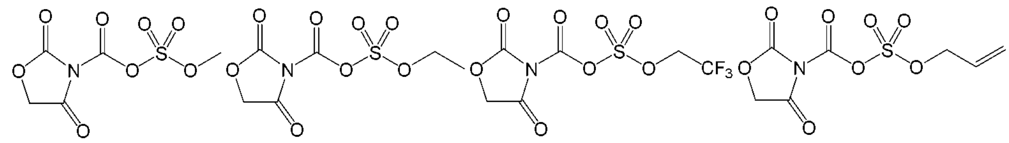

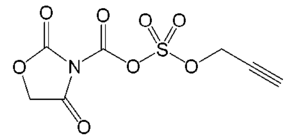 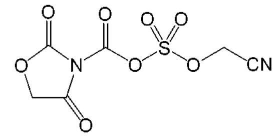 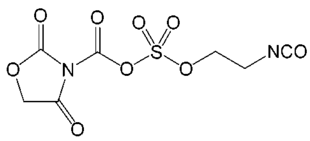

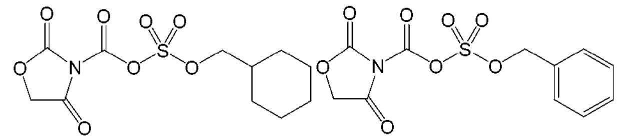

[Chem. 40]

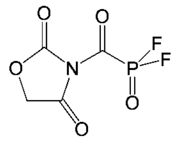 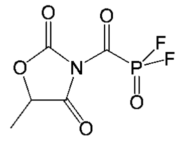 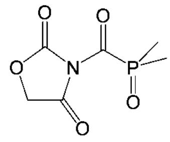 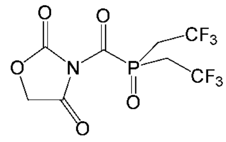 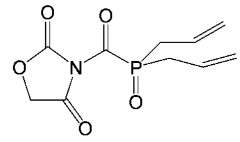

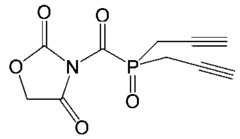 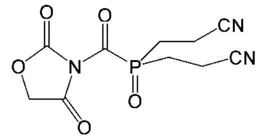 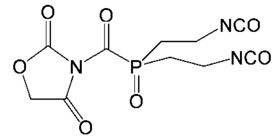

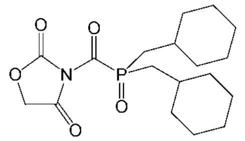 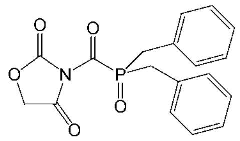

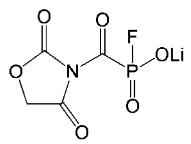 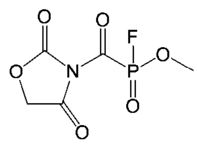 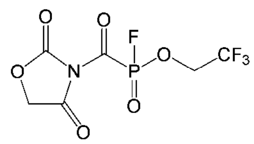 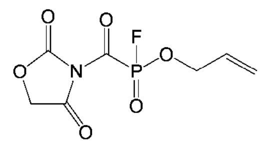 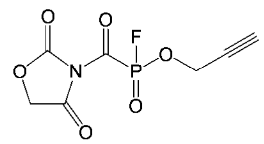

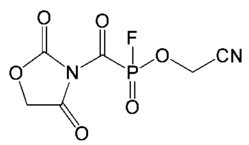 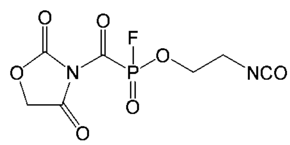 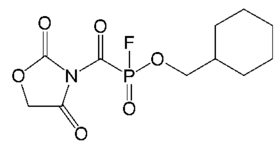 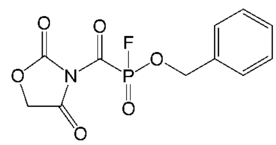

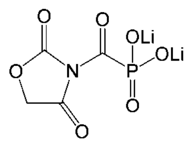 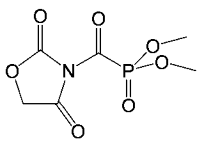 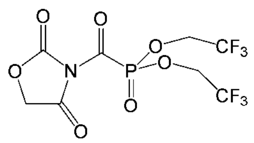 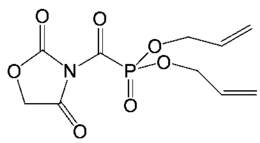

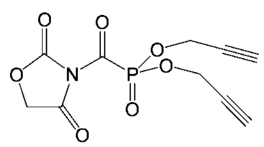 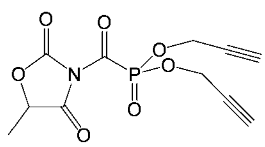 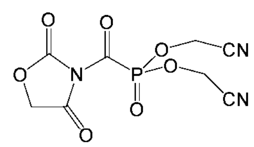

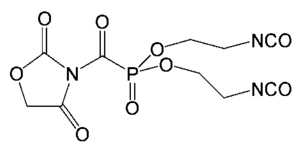 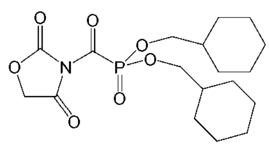 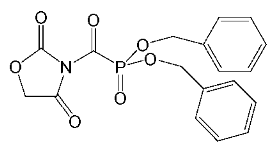

[Chem. 41]

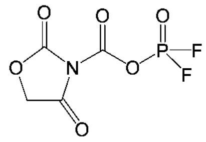
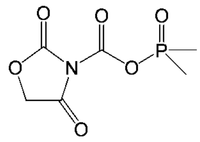
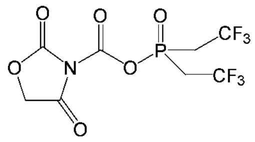
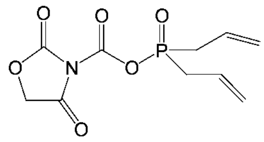
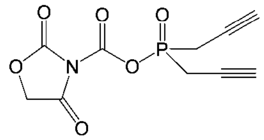
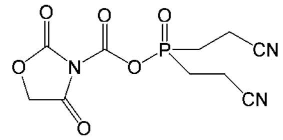
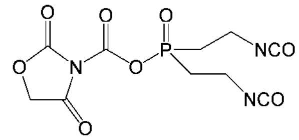
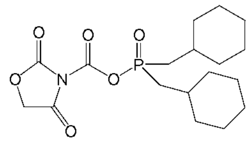
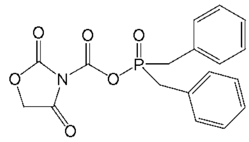
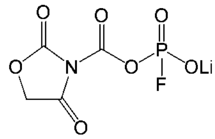
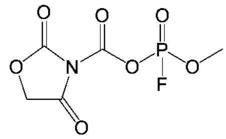
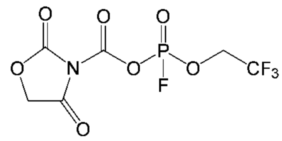
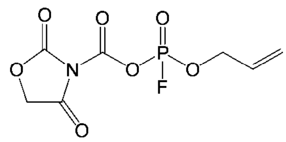
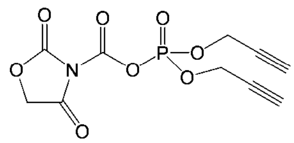
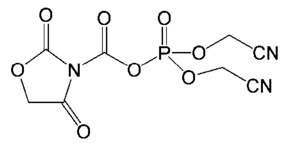
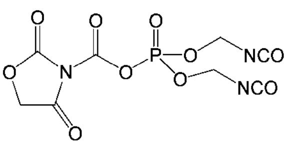
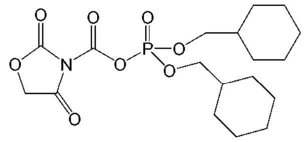
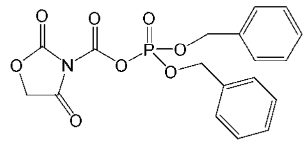

[Chem. 42]

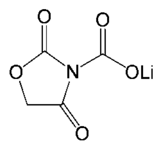
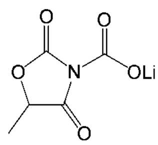
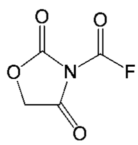
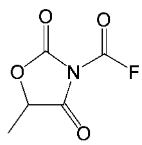
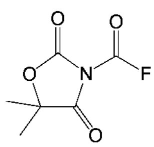
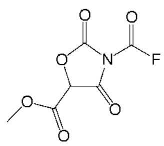
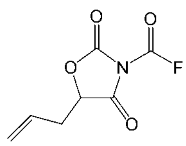
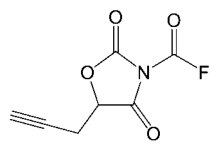
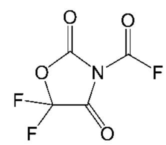
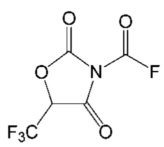
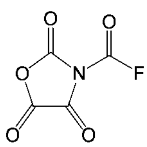
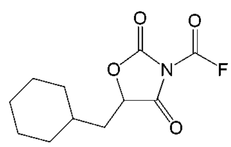
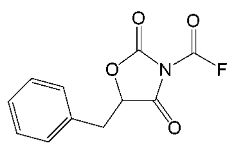

28. The compound according to 25 above in which the compound represented by the general formula (3) is selected from the following compounds.

[Chem. 43]

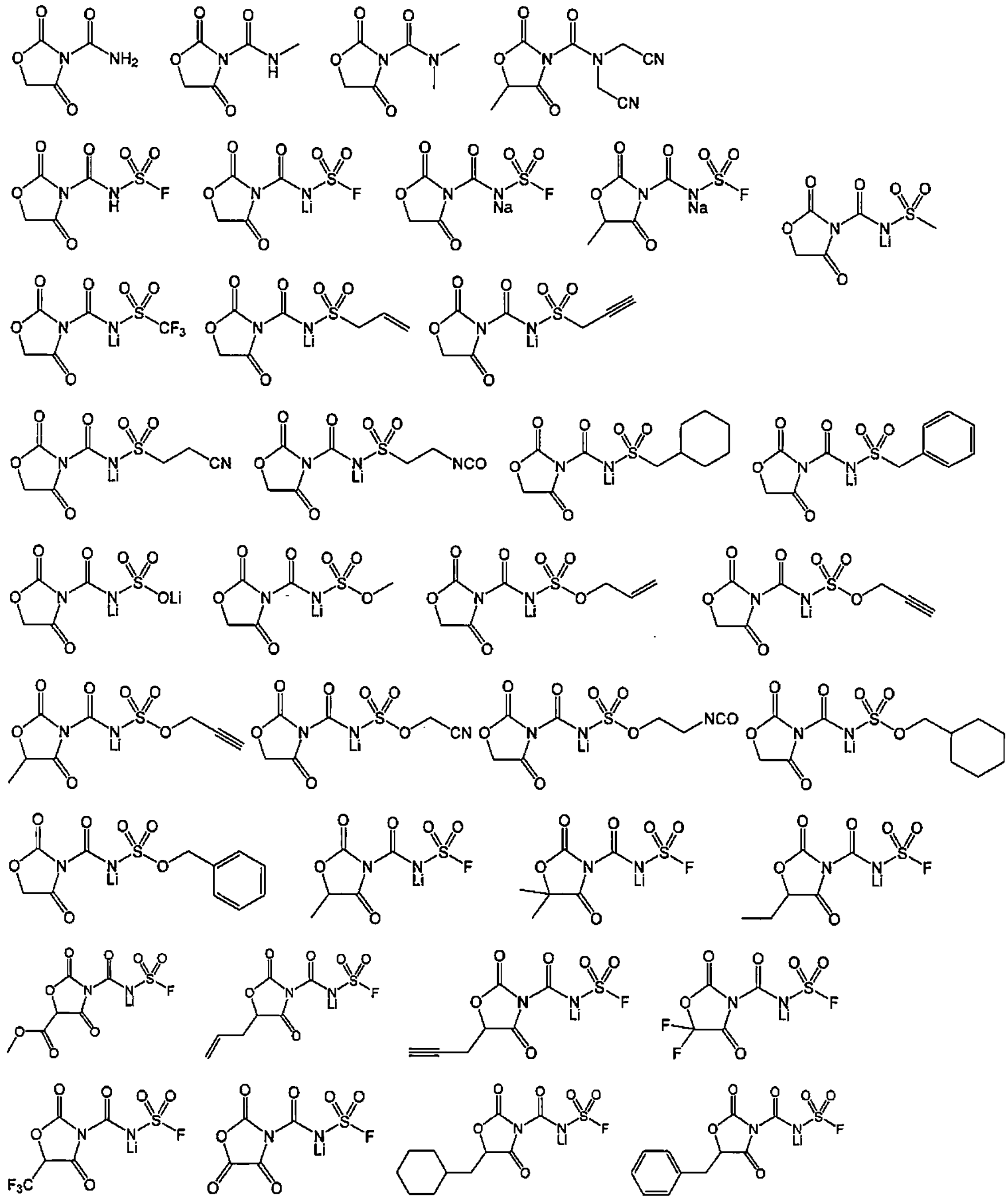

[Chem. 44]

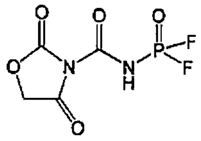 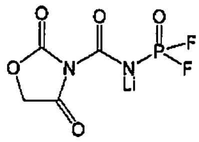 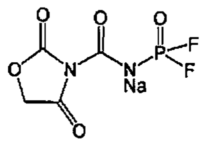 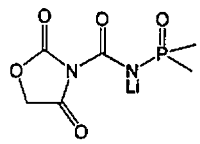 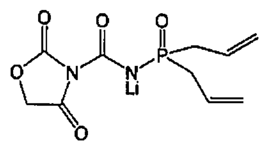

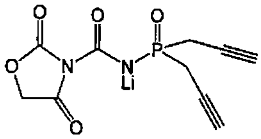 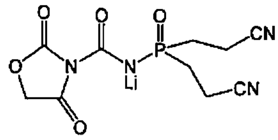 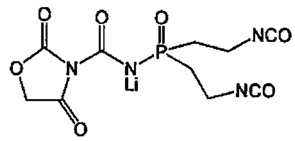

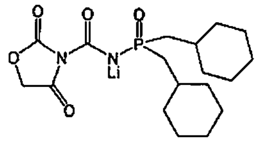 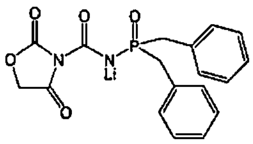

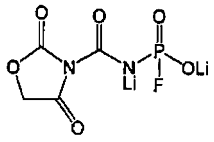 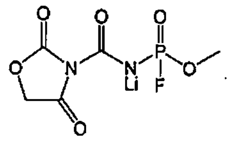 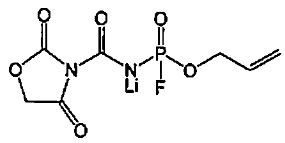 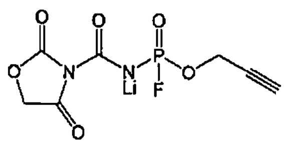

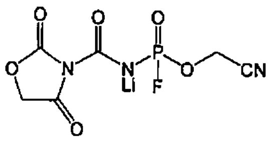 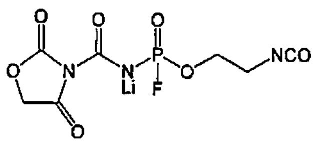 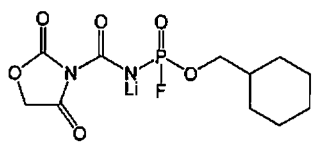

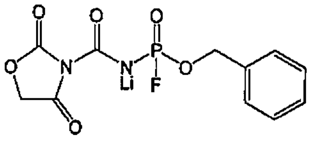 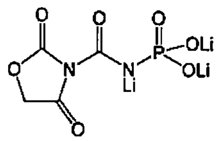 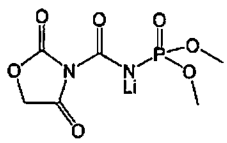 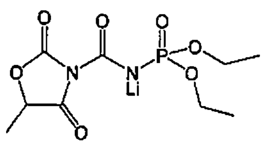

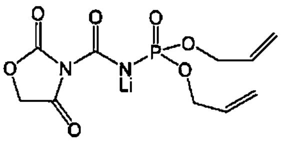 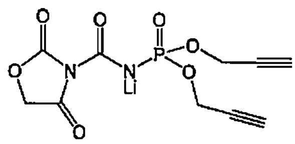 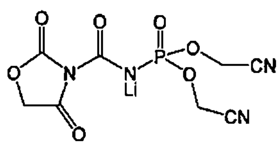

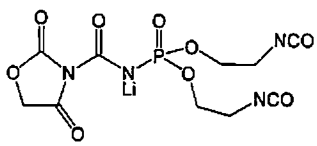 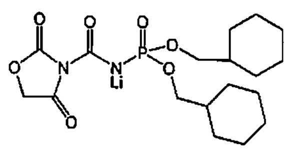 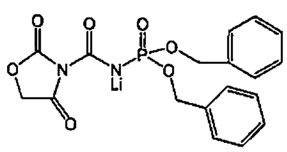

29. The compound according to 25 above in which the compound represented by the general formula (4) is selected from the following compounds.

[Chem. 45]

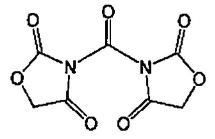 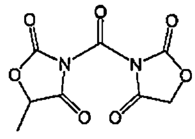 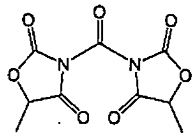 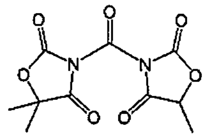 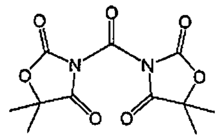

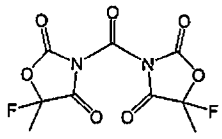 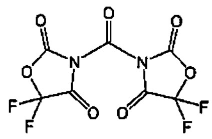 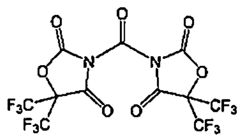 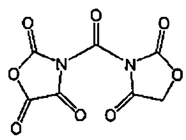 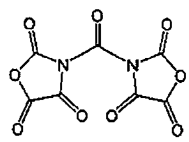

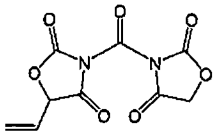 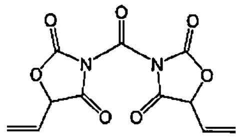 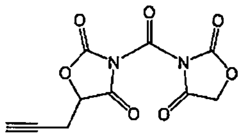 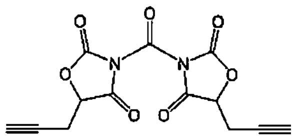

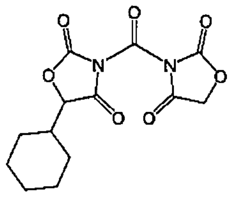 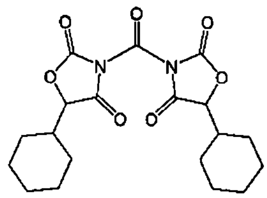 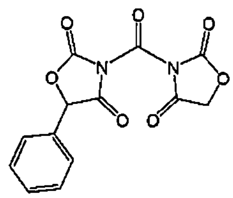 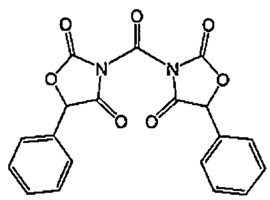

30. The compound according to 25 above in which the compound represented by the general formula (5) is selected from the following compounds.

[Chem. 46]

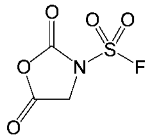 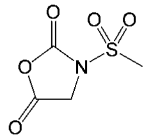 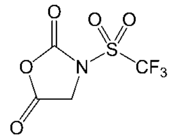 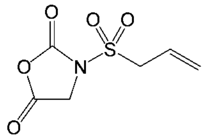

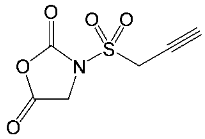 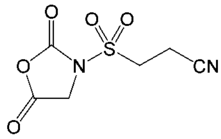 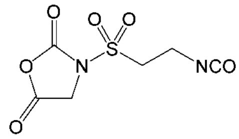 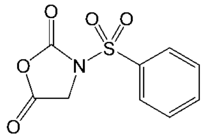

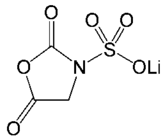 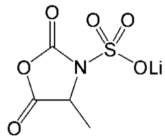 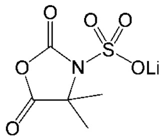 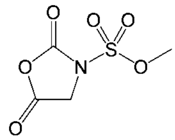 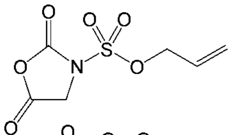

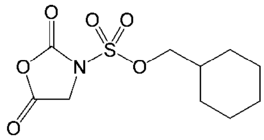 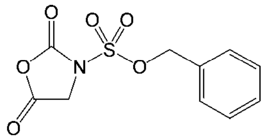 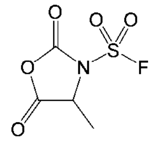 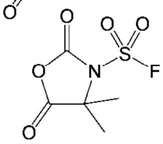 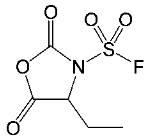

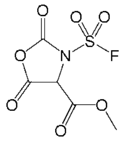 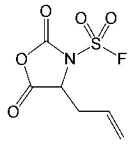 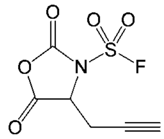 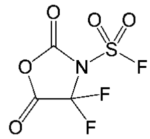

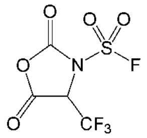 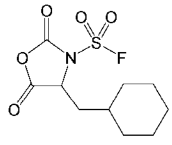 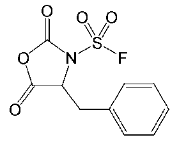

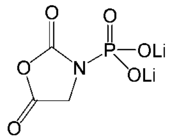 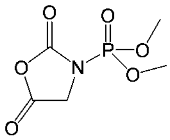 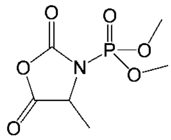 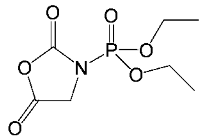 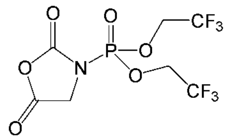

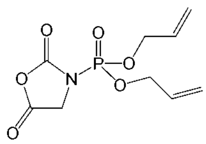 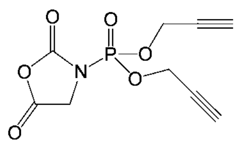 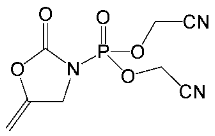 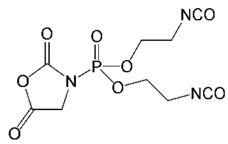

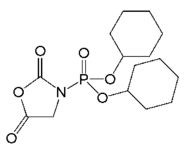 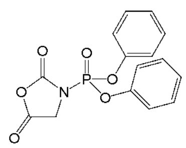 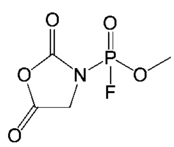 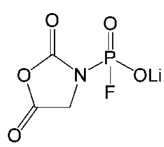 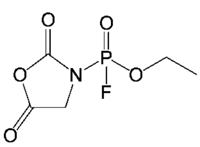

[Chem. 47]

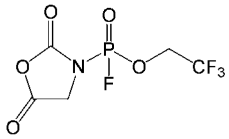 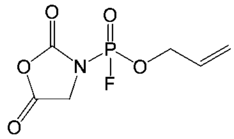 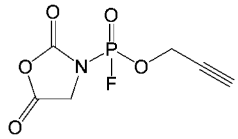

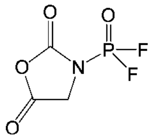 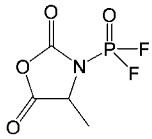 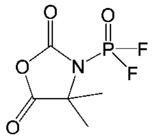

31. The compound according to 25 above in which the compound represented by the general formula (6) is selected from the following compounds.

[Chem. 48]

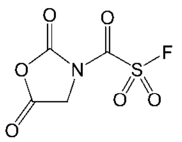 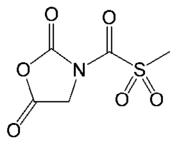 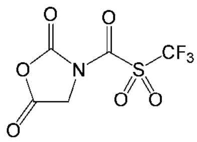 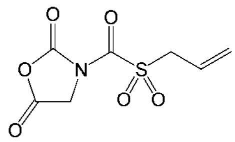 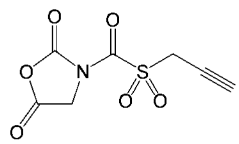

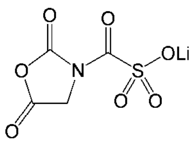 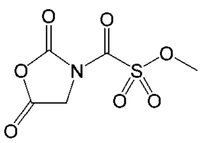 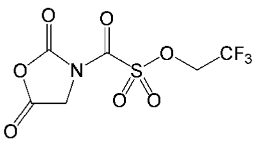 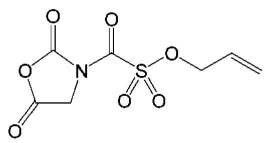

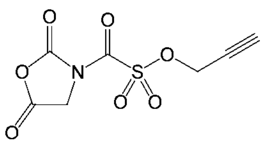 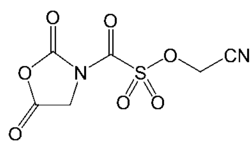 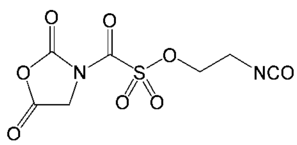

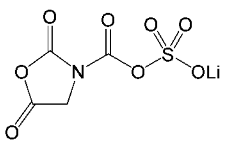 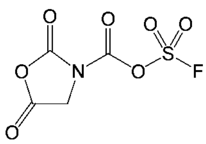 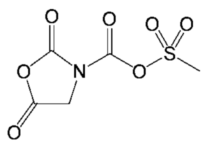 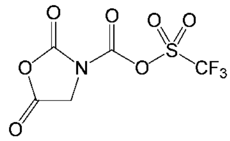

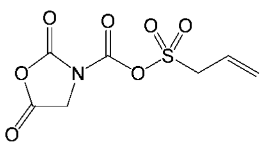 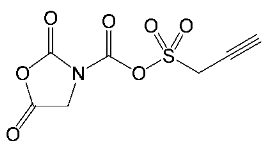 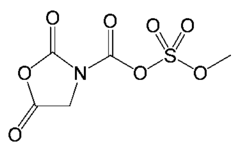 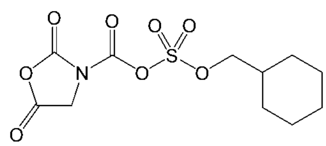

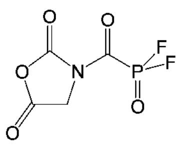 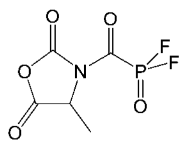 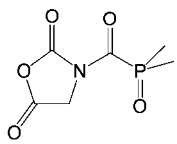 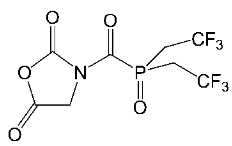

[Chem. 49]

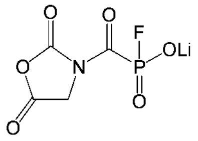 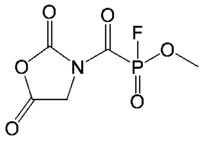 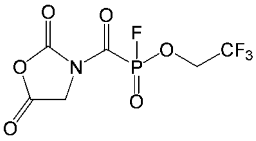 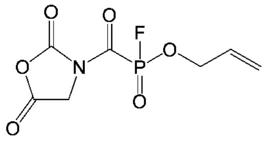

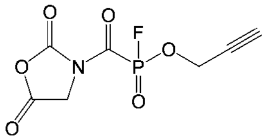 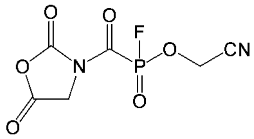 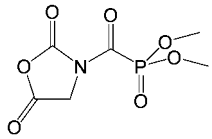 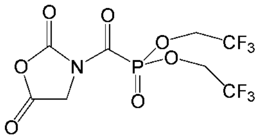

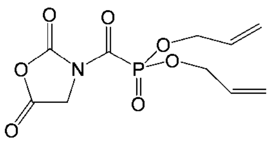 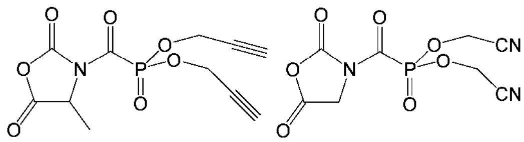 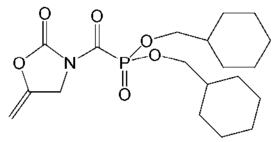

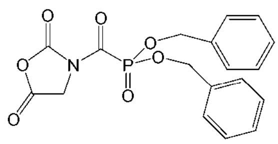 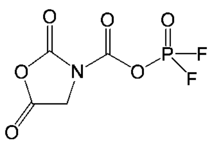 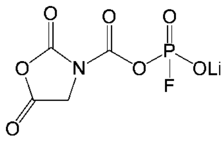

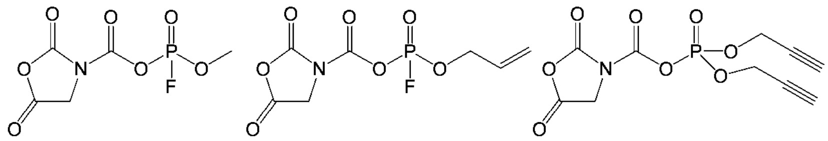

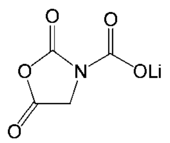 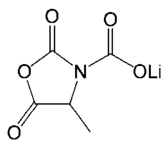 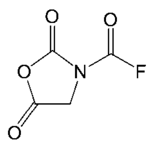 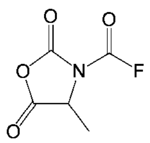

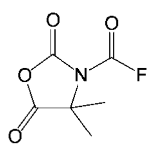 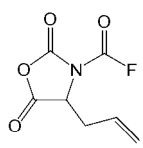 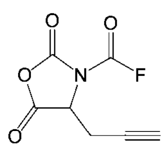 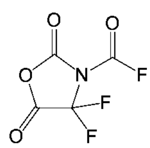

32. The compound according to 25 above in which the compound represented by the general formula (7) is selected from the following compounds.

[Chem. 50]

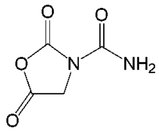
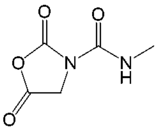
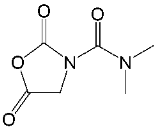
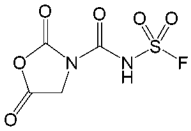
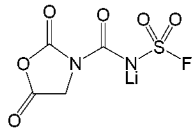
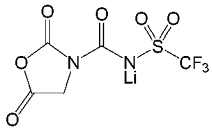
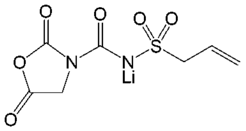
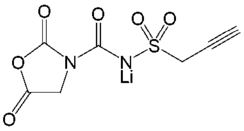
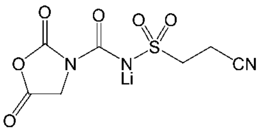
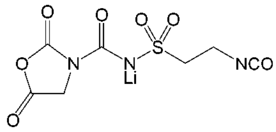
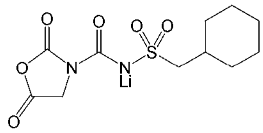
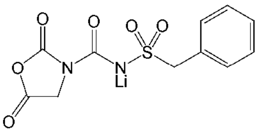
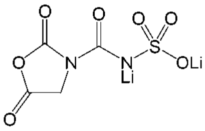
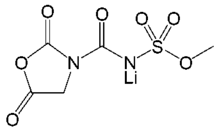
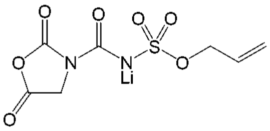
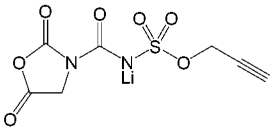
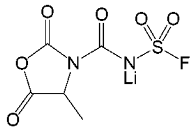
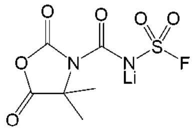
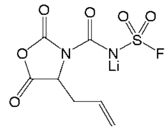
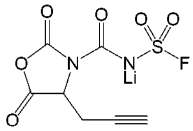
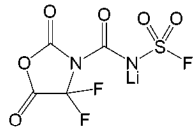
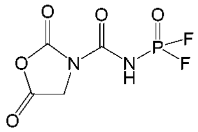
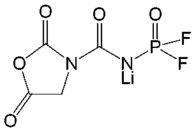
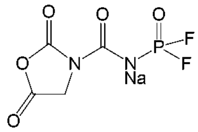
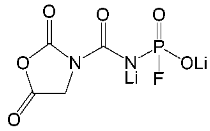
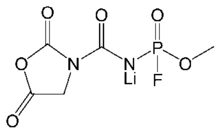
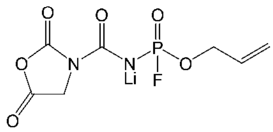
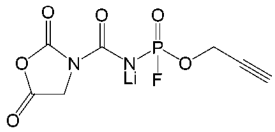
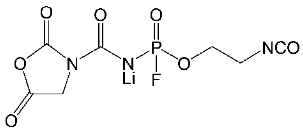
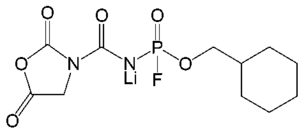
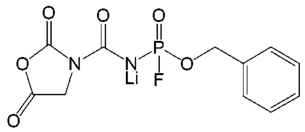
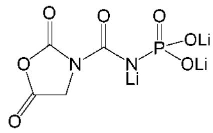
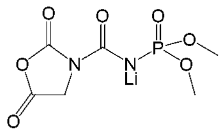
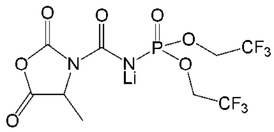
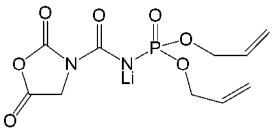
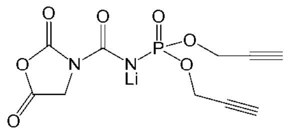
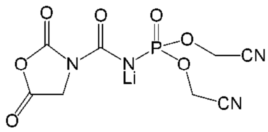

33. The compound according to 25 above in which the compound represented by the general formula (8) is selected from the following compounds.

[Chem. 51]

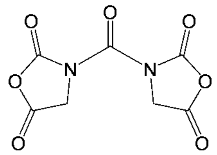 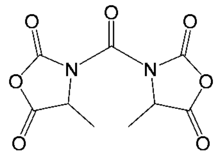 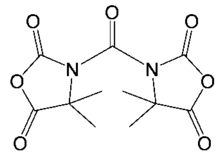 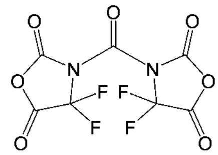

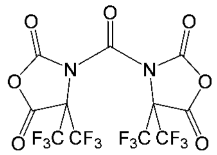 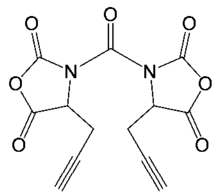 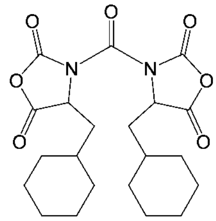 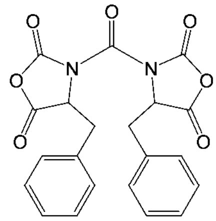

ADVANTAGEOUS EFFECTS OF INVENTION

According to the present disclosure, a nonaqueous electrolyte solution capable of exhibiting superior cycle characteristic to that of a case containing a known compound of the related art by containing an oxazolidine compound having a specific structure in the molecule, a nonaqueous electrolyte solution battery containing the same, and an oxazolidine compound having such a specific structure can be provided.

DESCRIPTION OF EMBODIMENTS

Hereinafter, the configuration of the nonaqueous electrolyte solution according to the present disclosure will be explained in detail.

Component (I) (Solute)

As the solute used in the nonaqueous electrolyte solution in the present disclosure, various solutes which have been employed in the field of such a nonaqueous electrolyte solution can be employed without particular restriction. Such a solute is preferably an ionic salt composed of a pair of a cation and an anion and is not particularly limited as long as the solute is present in an ionic state as a cation and an anion in a nonaqueous organic solvent, and various solutes can be used.

Suitable examples of such a solute include specifically at least one selected from the group consisting of $LiPF_6$, $LiBF_4$, $LiSbF_6$, $LiAsF_6$, $LiClO_4$, $LiN(SO_2F)_2$, $LiAlO_2$, $LiAlCl_4$, LiCl, and LiI or at least one selected from the group consisting of $NaPF_6$, $NaBF_4$, $NaSbF_6$, $NaAsF_6$, $NaClO_4$, $NaN(SO_2F)_2$, $NaAlO_2$, $NaAlCl_4$, NaCl, and NaI.

Of these, in view of energy density, output characteristic, life, and the like of the nonaqueous electrolyte solution battery, the cation is preferably at least one selected from the group consisting of lithium, sodium, potassium, magnesium, and quaternary ammonium, and the anion is preferably at least one selected from the group consisting of a hexafluorophosphate anion, a tetrafluoroborate anion, and a bis(fluorosulfonyl)imide anion.

As the solute in the present disclosure, one type may be used alone, or two or more types may be used in any combination at any ratio according to the application.

The lower limit of the concentration of the solute in the present disclosure may be, for example, 0.5 mol/L or more, preferably 0.7 mol/L or more, and further preferably 0.9 mol/L or more, with respect to the total amount of the nonaqueous electrolyte solution. The upper limit thereof may be 5 mol/L or less, preferably 4 mol/L or less, and further preferably 2 mol/L or less. When the solute concentration is 0.5 mol/L or more, the ionic conductivity hardly decreases, and the cycle characteristic and the output characteristic of the nonaqueous electrolyte solution battery hardly deteriorate, which is preferable. On the other hand, when the solute concentration is 5 mol/L or less, the viscosity of the nonaqueous electrolyte solution hardly increases, and the ionic conductivity hardly decreases, which is preferable. Here, when two or more types are used as the solute, the total concentration of these solutes is preferably in the above range.

Here, when the solute is dissolved in the nonaqueous organic solvent which is explained below, the liquid temperature is not particularly limited but is, for example, preferably -20 to 80°C, and more preferably 0 to 60°C.

Component (II) (Nonaqueous Organic Solvent)

The nonaqueous organic solvent used in the nonaqueous electrolyte solution in the present disclosure is not particularly limited as long as the component (I) and the additive (III) which is explained below can be dissolved, and any

nonaqueous organic solvent can be used.

The nonaqueous organic solvent is preferably at least one selected from the group consisting of a cyclic ester, a chain ester, a cyclic ether, a chain ether, a sulfone compound, a sulfoxide compound, an amide compound, a nitrile compound, and an ionic liquid, and more preferably contains at least one selected from the group consisting of a cyclic ester, a chain ester, a cyclic ether, a chain ether, a sulfone compound, a sulfoxide compound, and an ionic liquid. Here, the cyclic carbonate is a subordinate concept of the cyclic ester, and the chain carbonate is a subordinate concept of the chain ester.

Specific examples of the nonaqueous organic solvent used in the present disclosure include ethyl methyl carbonate (hereinafter also referred to as "EMC"), dimethyl carbonate (hereinafter also referred to as "DMC"), diethyl carbonate (hereinafter also referred to as "DEC"), methyl propyl carbonate, ethyl propyl carbonate, methyl butyl carbonate, 2,2,2-trifluoroethyl methyl carbonate, 2,2,2-trifluoroethyl ethyl carbonate, 2,2,2-trifluoroethyl propyl carbonate, bis(2,2,2-trifluoroethyl) carbonate, 1,1,1,3,3,3-hexafluoro-1-propylmethyl carbonate, 1,1,1,3,3,3-hexafluoro-1-propylethyl carbonate, 1,1,1,3,3,3-hexafluoro-1-propylpropyl carbonate, bis(1,1,1,3,3,3-hexafluoro-1-propyl) carbonate, ethylene carbonate (hereinafter also referred to as "EC"), propylene carbonate (hereinafter also referred to as "PC"), butylene carbonate, methyl acetate, ethyl acetate, methyl propionate, ethyl propionate, methyl 2-fluoropropionate, ethyl 2-fluoropropionate, diethyl ether, dibutyl ether, diisopropyl ether, 1,2-dimethoxyethane, tetrahydrofuran, 2-methyltetrahydrofuran, furan, tetrahydropyran, 1,3-dioxane, 1,4-dioxane, N,N-dimethylformamide, acetonitrile, propionitrile, dimethyl sulfoxide, sulfolane, γ-butyrolactone, and γ-valerolactone, and at least one of the specific examples may be contained.

The nonaqueous organic solvent preferably contains at least one selected from the group consisting of a cyclic ester and a chain ester because the input and output characteristic at a low temperature is excellent.

Further, the nonaqueous organic solvent preferably contains at least one selected from the group consisting of a cyclic carbonate and a chain carbonate because the cycle characteristic at a high temperature is excellent.

It is preferable that the nonaqueous organic solvent contains a cyclic ester and that the cyclic ester contains a cyclic carbonate.

Specific examples of the cyclic carbonate include, for example, EC, PC, and butylene carbonate, and of these, at least one selected from the group consisting of EC and PC is preferably contained.

It is also preferable that the nonaqueous organic solvent contains a chain ester and that the chain ester contains a chain carbonate.

Specific examples of the chain carbonate include, for example, EMC, DMC, DEC, methyl propyl carbonate, ethyl propyl carbonate, 2,2,2-trifluoroethyl methyl carbonate, 2,2,2-trifluoroethyl ethyl carbonate, 1,1,1,3,3,3-hexafluoro-1-propylmethyl carbonate, and 1,1,1,3,3,3-hexafluoro-1-propylethyl carbonate, and of these, at least one selected from the group consisting of EMC, DMC, DEC, and methyl propyl carbonate is preferably contained.

Specific examples of the chain ester include, for example, methyl acetate, ethyl acetate, methyl propionate, ethyl propionate, methyl 2-fluoropropionate, ethyl 2-fluoropropionate, and methyl 3,3,3-trifluoropropionate.

As the nonaqueous organic solvent of the present disclosure, one type may be used alone, or two or more types may be used in any combination at any ratio according to the application. Of these, from the viewpoint of the electrochemical stability against oxidation and reduction thereof and the chemical stability relating to heat or the reaction with the solute, in particular, at least one selected from the group consisting of PC, EC, DEC, DMC, and EMC is preferably contained.

The concentration of the nonaqueous organic solvent in the present disclosure is not particularly limited as long as the nonaqueous organic solvent has a function as a nonaqueous organic solvent, but the concentration may be, for example, 40 to 99% by mass, preferably 50 to 95% by mass, and particularly preferably 70 to 93% by mass with respect to the total amount (100% by mass) of the nonaqueous electrolyte solution.

<u>Component (III)</u>

The additives used in the nonaqueous electrolyte solution in the present disclosure are the eight types of compounds explained above.

Here, the meanings of the functional groups described in each formula are as follows.

The halogen atom is selected from, for example, halogen atoms such as a fluorine atom, a chlorine atom, and a bromine atom. The halogen atom is preferably a fluorine atom.

The alkyl group having 1 to 10 carbon atoms may be linear or branched-chain, preferably has 1 to 10 carbon atoms and preferably has 1 to 5 carbon atoms. Examples of such an alkyl group include a methyl group, an ethyl group, an n-propyl group, an isopropyl group, an n-butyl group, a sec-butyl group, a tert-butyl group, an n-pentyl group, an n-hexyl group, an n-octyl group, and an n-decyl group.

Examples of the alkoxy group having 1 to 10 carbon atoms include alkoxy groups corresponding to the alkyl groups.

Examples of the alkenyl group having 2 to 10 carbon atoms include a linear or branched-chain alkenyl group, and

an alkenyl group having 2 to 5 carbon atoms is preferable. Moreover, the alkenyl group may have two or more unsaturated bonds. Suitable examples of such an alkenyl group include a vinyl group, a 1-propenyl group, a 2-propenyl group, a 1-methylvinyl group, a 1-butenyl group, a 2-butenyl group, a 3-butenyl group, a 1-methylpropenyl group, a 1-pentenyl group, and a 1,3-butadienyl group.

Examples of the alkenyloxy group having 2 to 10 carbon atoms include alkenyloxy groups corresponding to the alkenyl groups.

Examples of the alkynyl group having 2 to 10 carbon atoms include a linear or branched-chain alkynyl group, and an alkynyl group having 2 to 5 carbon atoms is preferable. Moreover, the alkynyl group may have two or more unsaturated bonds. Suitable examples of such an alkynyl group include an ethynyl group, a 1-propynyl group, a 2-propynyl group, a 1-methylethynyl group, a 1-butynyl group, a 2-butynyl group, a 3-butynyl group, a 1-methylpropynyl group, a 1,1-dimethyl-2-propynyl group, a 1-pentynyl group, and a 1,3-butadienyl group.

Examples of the alkynyloxy group having 2 to 10 carbon atoms include alkynyloxy groups corresponding to the alkynyl groups.

The cycloalkyl group having 3 to 8 carbon atoms is preferably a cycloalkyl group having 3 to 5 carbon atoms, and suitable examples of such a cycloalkyl group include a cyclopropyl group, a cyclobutyl group, a cyclopentyl group, and a cyclohexyl group. Moreover, the cycloalkyl group also includes those having a linking group such as a methylene group, and suitable examples thereof include a cyclohexylmethyl group.

Suitable examples of the cycloalkoxy group having 3 to 8 carbon atoms include cycloalkoxy groups corresponding to the cycloalkyl groups.

The cycloalkenyl group having 3 to 8 carbon atoms is preferably a cycloalkenyl group having 3 to 5 carbon atoms, and suitable examples of such a cycloalkenyl group include a cyclopropenyl group, a cyclobutenyl group, a cyclopentenyl group, and a cyclohexenyl group. Moreover, the cycloalkenyl group also includes those having a linking group such as a methylene group, and suitable examples thereof include a cyclohexenylmethyl group.

Suitable examples of the cycloalkenyloxy group having 3 to 8 carbon atoms include cycloalkenyloxy groups corresponding to the cycloalkenyl groups.

Suitable examples of the aryl group having 6 to 10 carbon atoms include a phenyl group and a naphthalene group. Moreover, the aryl group also includes those having a linking group such as a methylene group, and suitable examples thereof include a benzyl group.

Suitable examples of the aryloxy group having 6 to 10 carbon atoms include a phenoxy group and a naphthoxy group.

The heteroaryl group having 6 to 10 carbon atoms is an aryl group containing a heteroatom such as an oxygen atom, a sulfur atom, and a nitrogen atom, and suitable examples of such a heteroaryl group include a furyl group, a thiophenyl group, a pyrrole group, an imidazole group, a pyrazole group, an oxazole group, a thiooxazole group, a pyridyl group, a pyridazine group, a pyrimidine group, a pyrazine group, a triazine group, an indole group, a purine group, and a quinolyl group. Suitable examples of the heteroaryloxy group having 6 to 10 carbon atoms include heteroaryloxy groups corresponding to the heteroaryl groups.

A hydrogen atom of the hydrocarbon group may be optionally substituted with a halogen atom, a cyano group, an isocyano group, or the like. In addition, a carbonyl group, an oxygen atom, a sulfur atom, a nitrogen atom, or the like may be interposed between any carbon-carbon atoms of the hydrocarbon group, and these may be combined and interposed.

For example, when the hydrocarbon group is an alkyl group, suitable examples thereof include a trifluoromethyl group and a 1,1,1-trifluoroethyl group.

Suitable examples of the metal cation include alkali metal cations such as a lithium ion, a sodium ion, and a potassium ion and alkaline earth metal cations such as a magnesium ion and a calcium ion.

Examples of the onium cation include a trialkylammonium ion, a tetraalkylammonium ion, a tetraalkylphosphonium ion, an imidazolium ion, an ammonium ion having a spiro skeleton, and a nitronium cation.

Examples of the compound represented by the general formula (1) include the following compounds.

[Chem. 52]

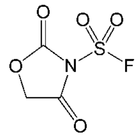 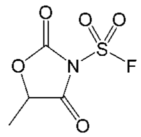 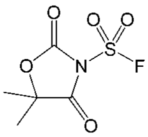 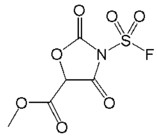 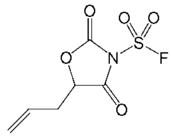 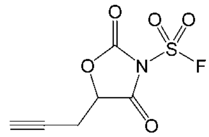

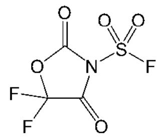 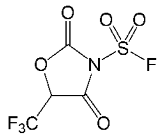 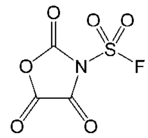 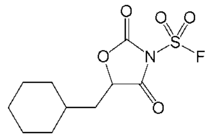 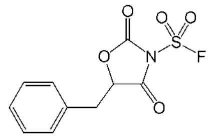

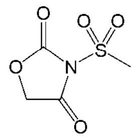 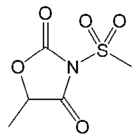 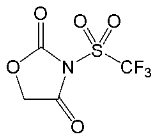 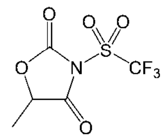 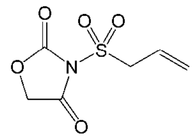

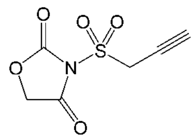 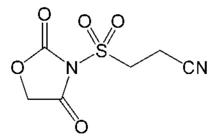 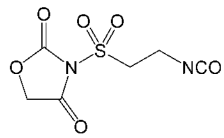 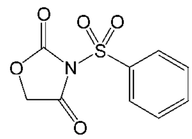

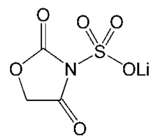 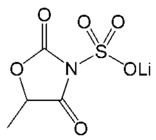 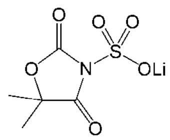 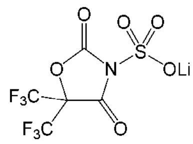 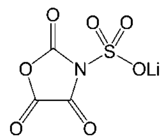

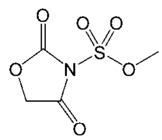 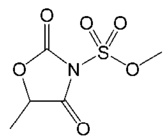 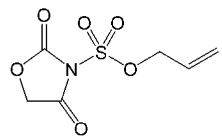 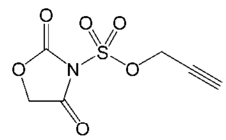 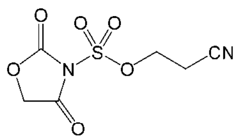

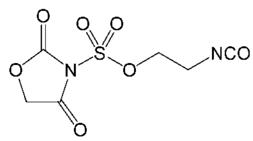 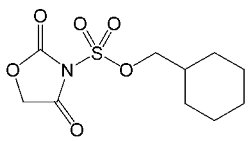 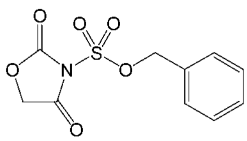 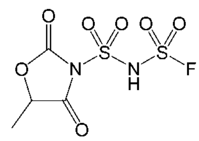 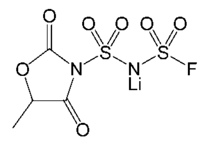

[Chem. 53]

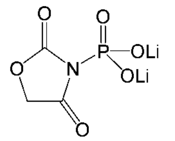 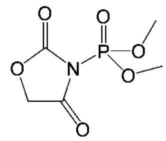 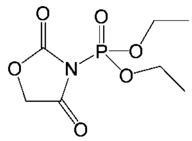 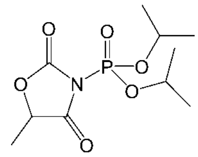 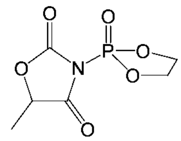

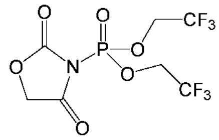 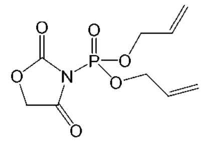 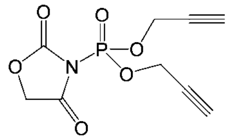 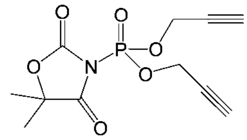 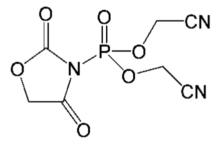

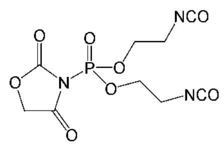 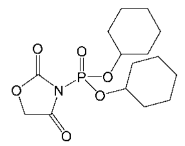 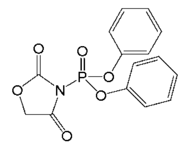 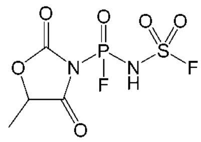 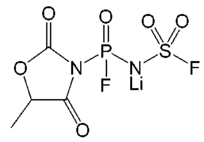

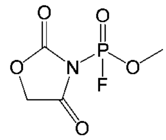 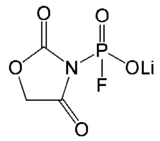 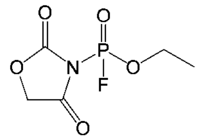 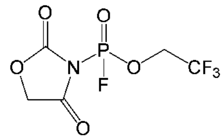 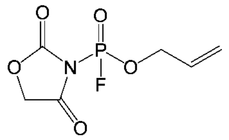

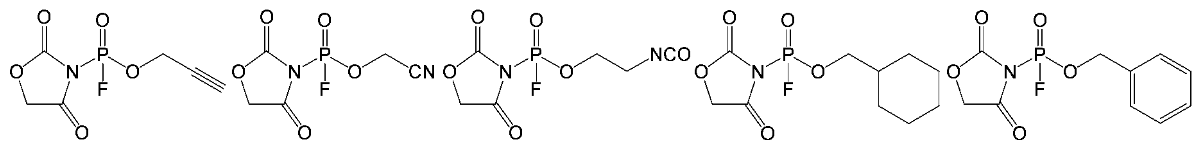

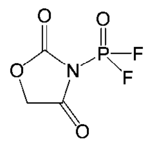 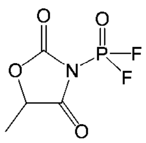 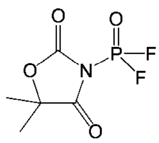 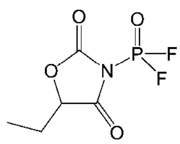 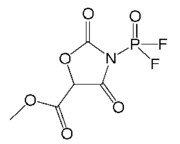 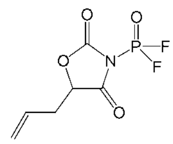

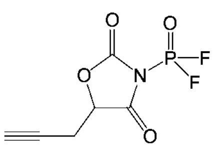 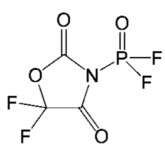 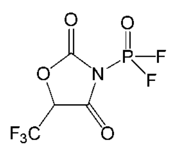 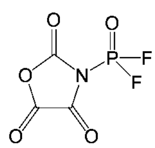 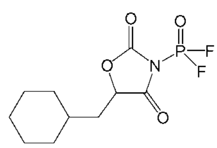 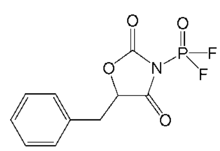

Examples of the compound represented by the general formula (2) include the following compounds.

[Chem. 54]

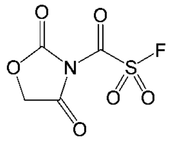
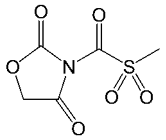
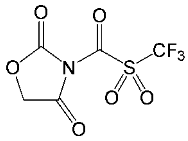
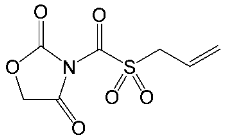
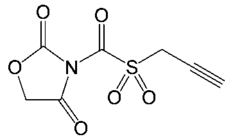

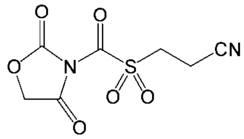
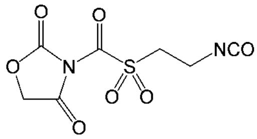
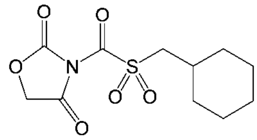
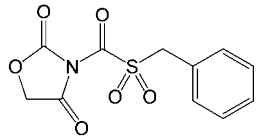

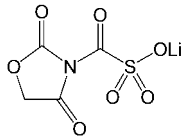
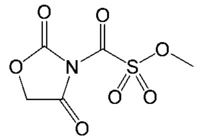
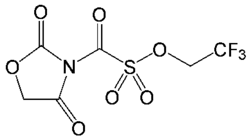
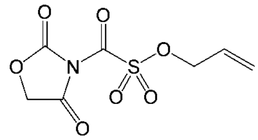

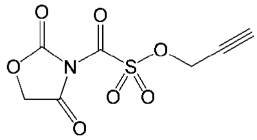
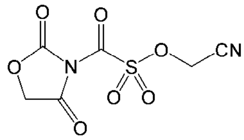
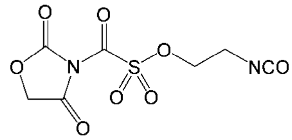
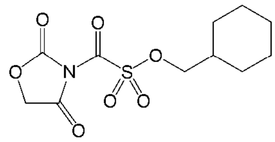

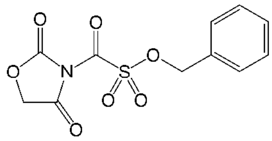
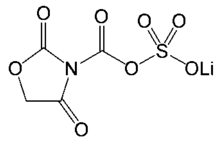
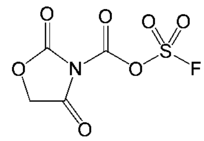
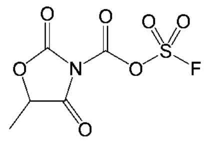

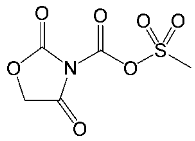
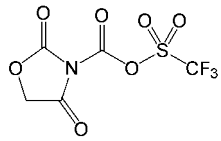
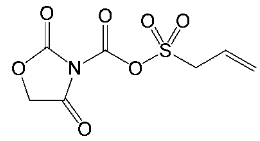
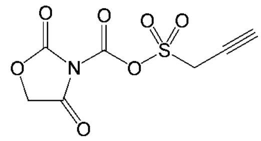

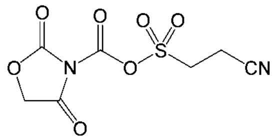
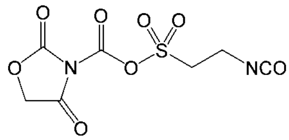
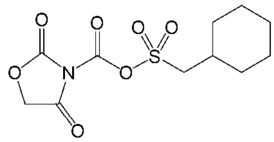
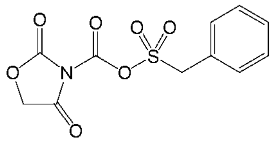

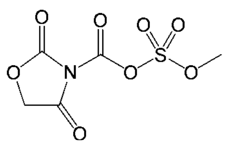
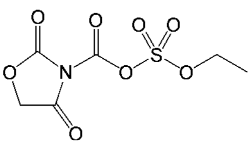
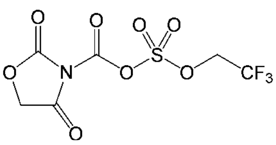
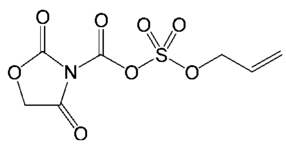

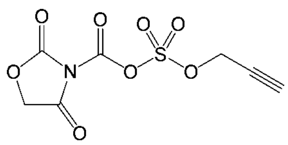
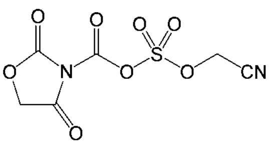
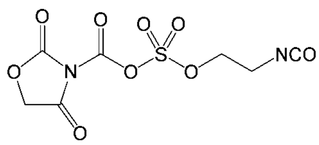

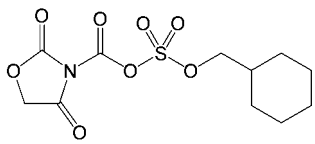
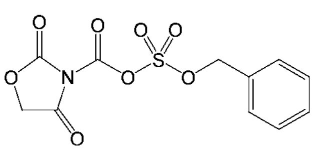

[Chem. 55]

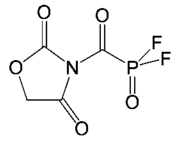
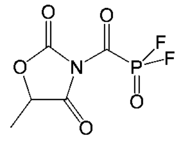
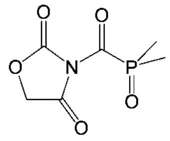
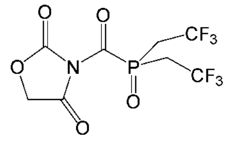
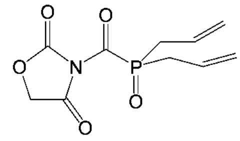

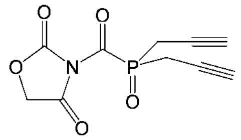
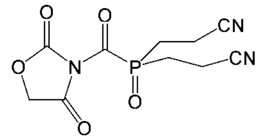
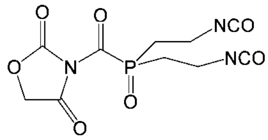

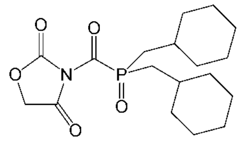
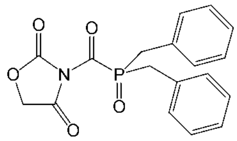

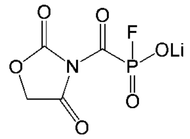
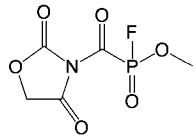
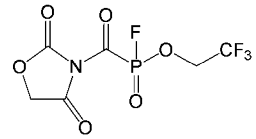
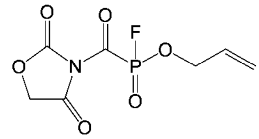
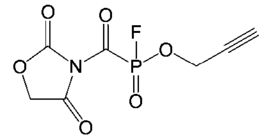

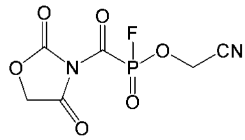
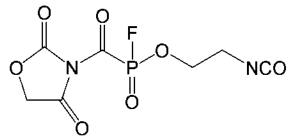
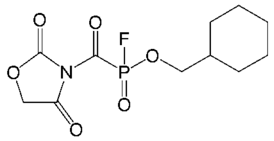
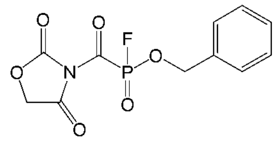

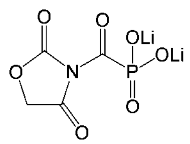
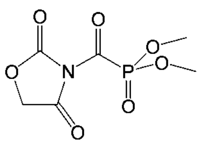
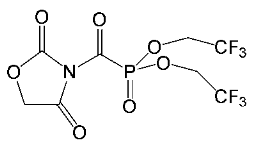
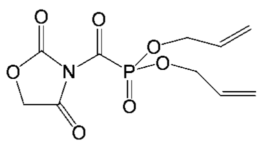

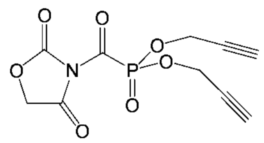
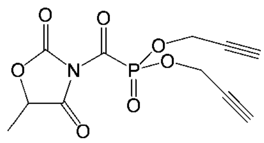
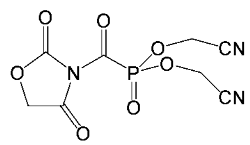

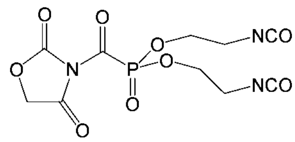
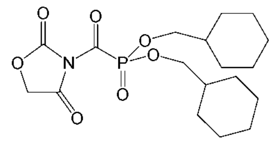
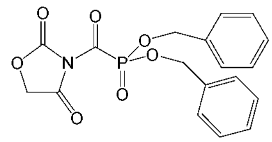

[Chem. 56]

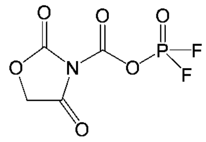 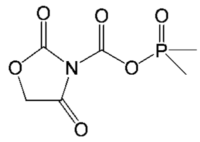 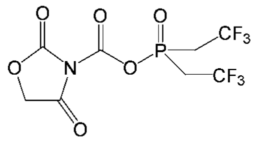 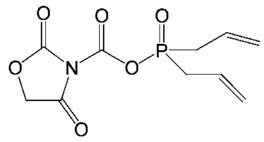

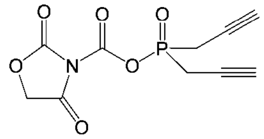 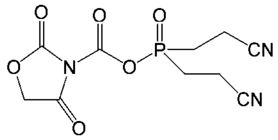 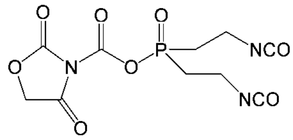 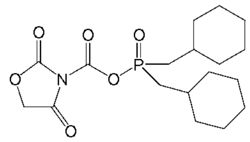

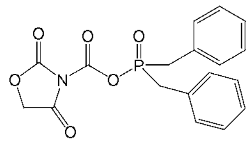 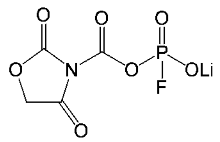 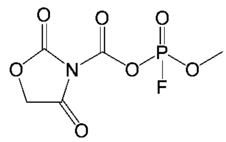 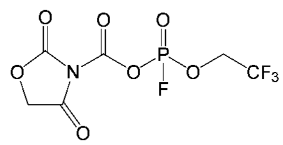

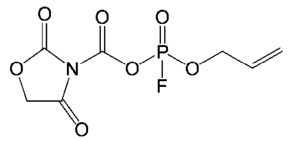 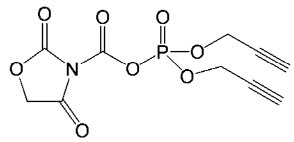 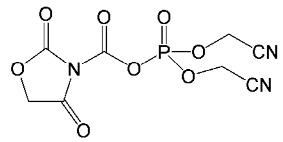 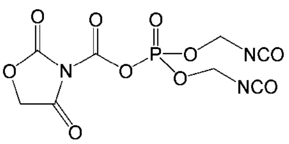

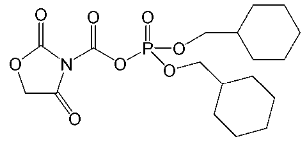 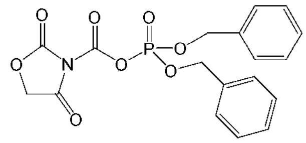

[Chem. 57]

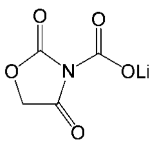 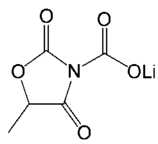 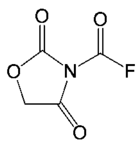 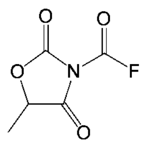 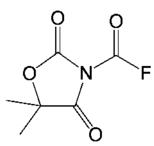

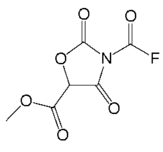 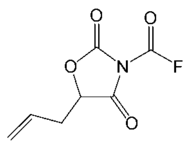 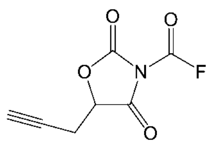 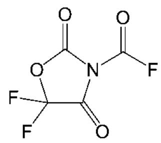

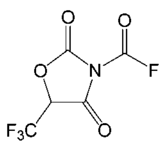 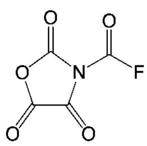 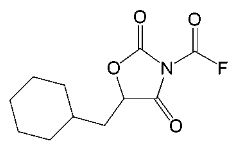 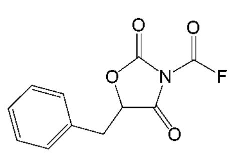

Examples of the compound represented by the general formula (3) include the following compounds.

[Chem. 58]

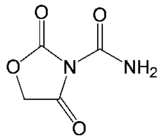
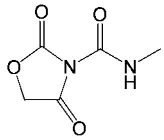
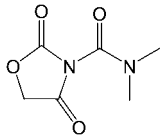
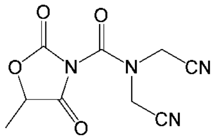

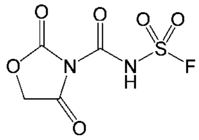
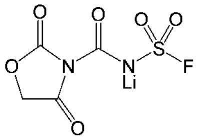
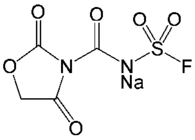
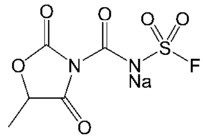
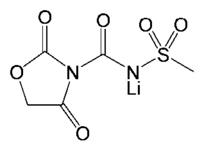

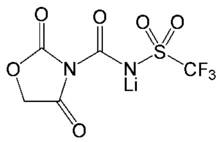
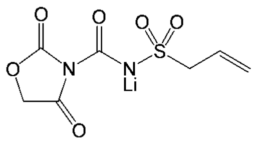
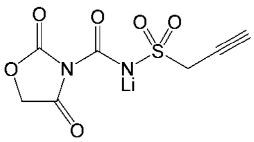

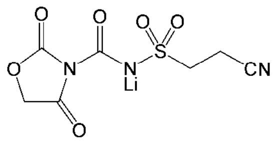
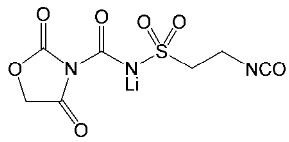
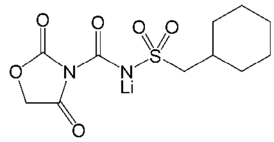
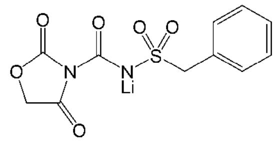

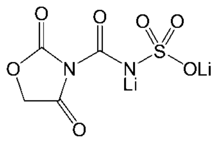
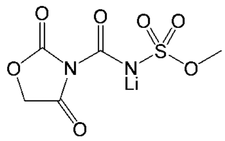
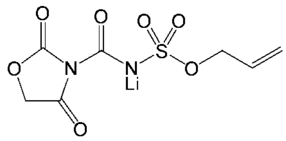
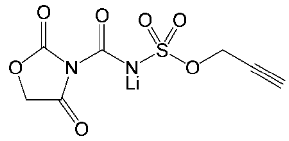

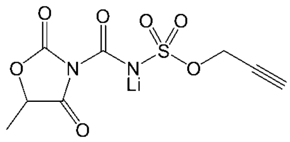
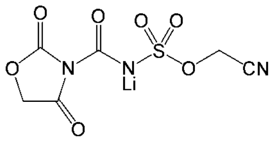
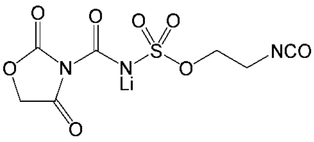
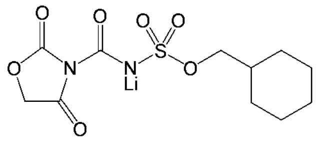

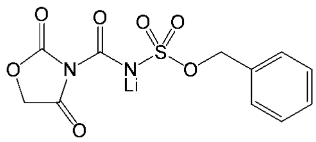
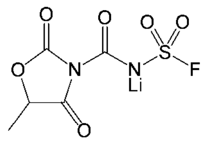
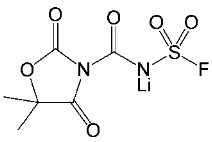
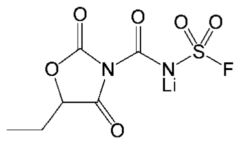

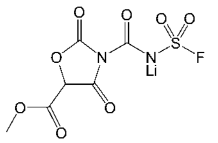
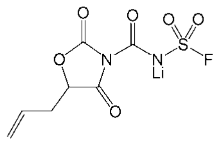
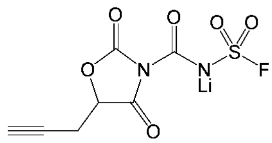
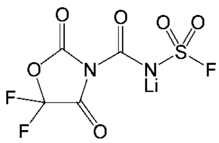

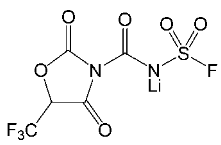
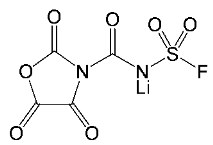
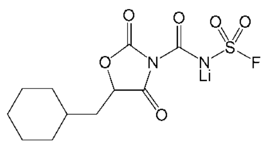
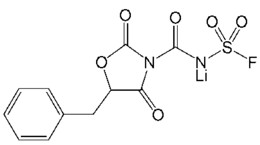

[Chem. 59]

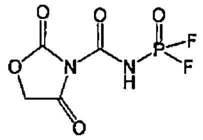
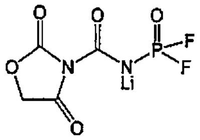
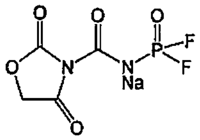
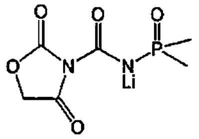
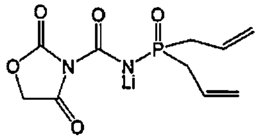

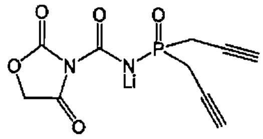
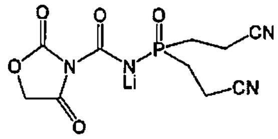
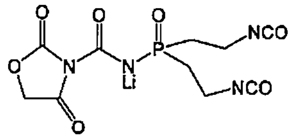

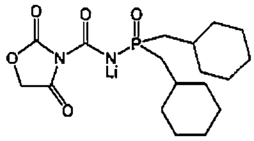
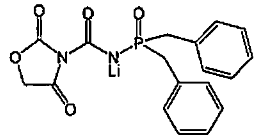

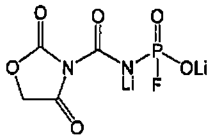
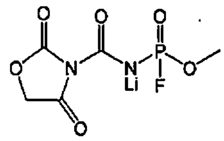
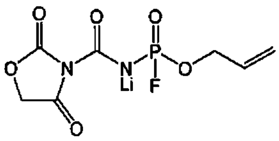
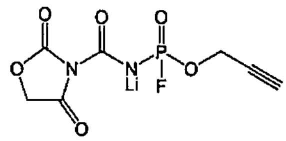

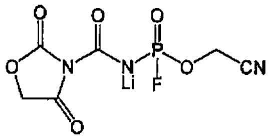
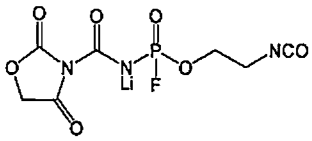
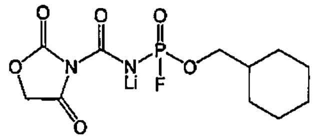

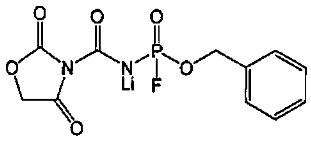
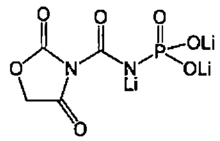
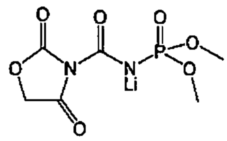
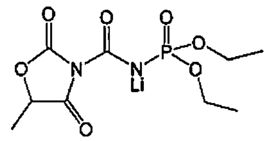

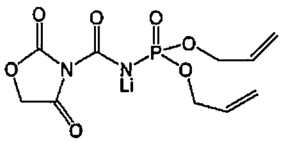
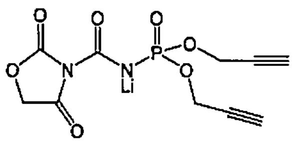
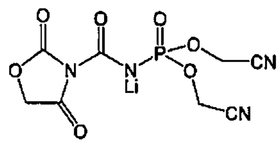

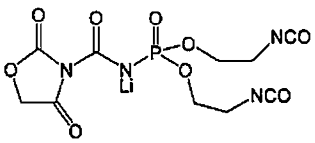
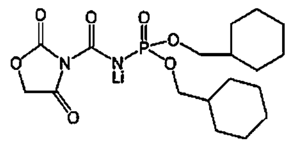
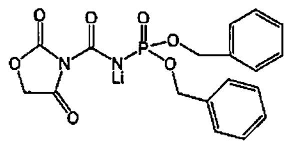

Examples of the compound represented by the general formula (4) include the following compounds.

[Chem. 60]

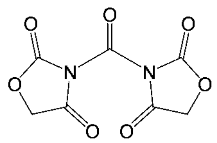 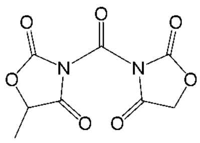 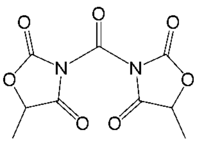 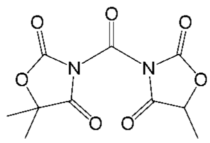 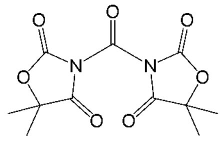

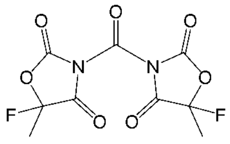 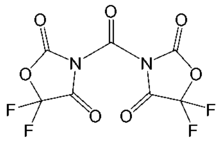 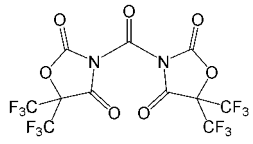 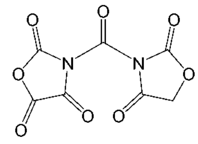 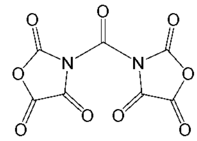

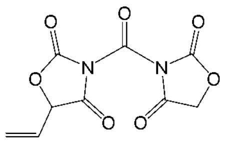 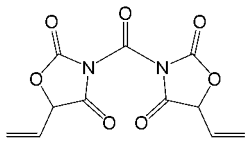 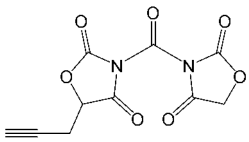 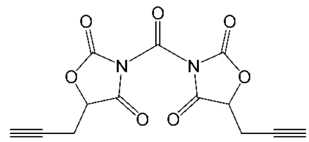

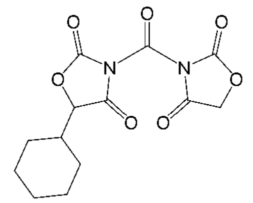 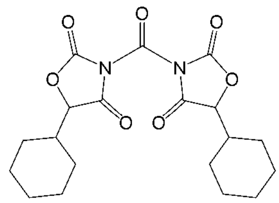 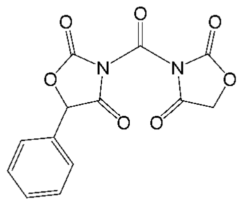 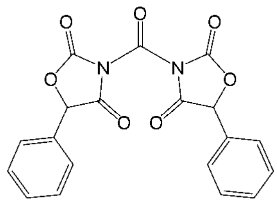

Examples of the compound represented by the general formula (5) include the following compounds.

[Chem. 61]

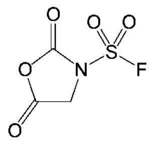 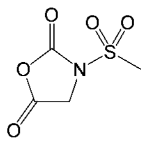 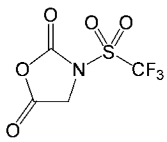 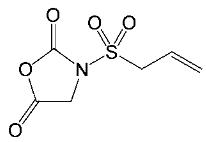

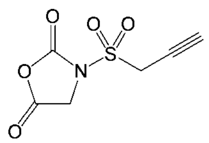 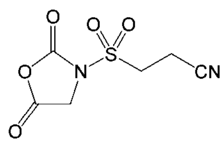 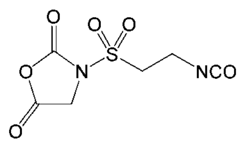 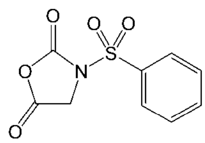

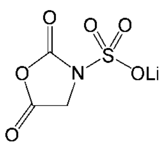 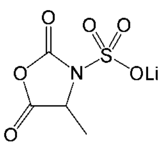 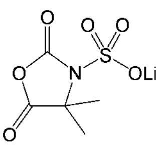 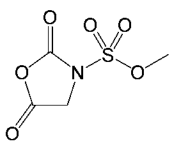 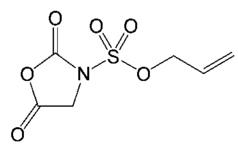

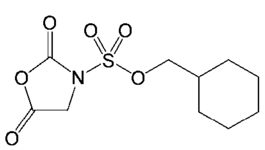 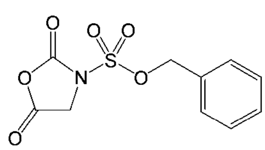 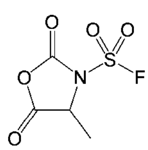 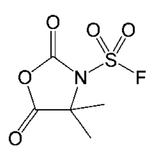 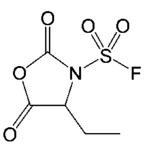

[Chem. 62]

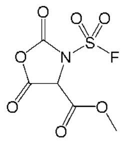 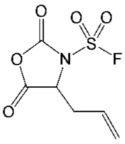 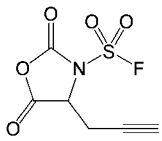 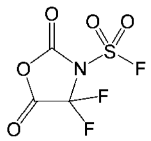

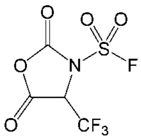 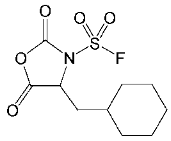 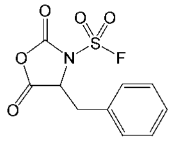

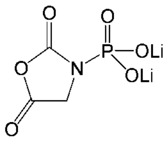 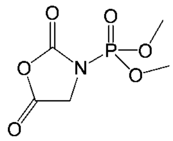 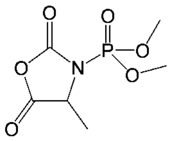 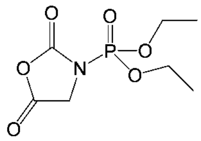 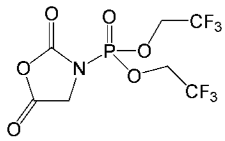

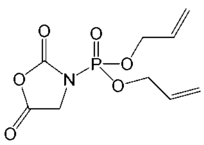 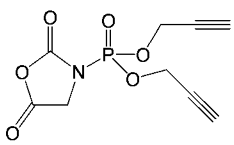 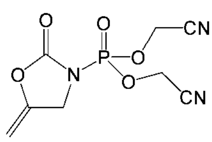 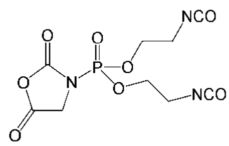

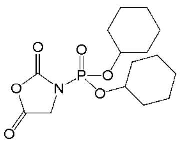 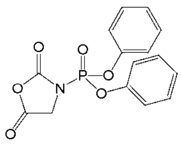 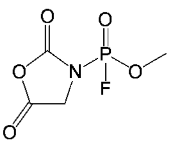 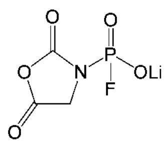 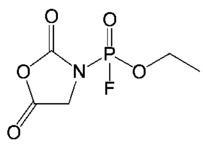

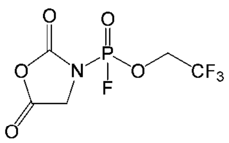 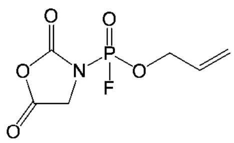 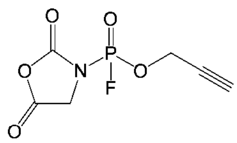

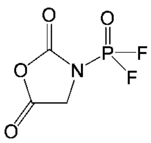 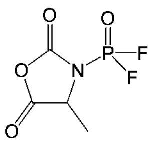 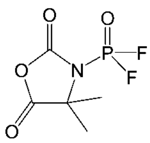

Examples of the compound represented by the general formula (6) include the following compounds.

[Chem. 63]

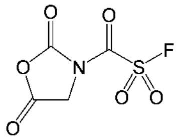
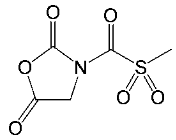
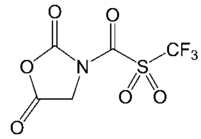
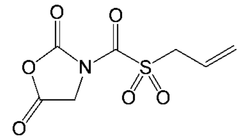
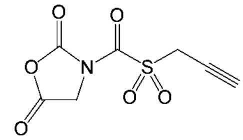

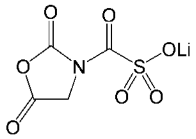
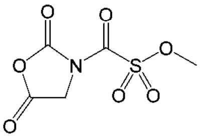
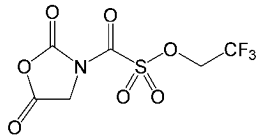
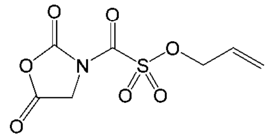

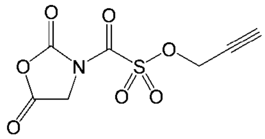
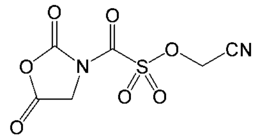
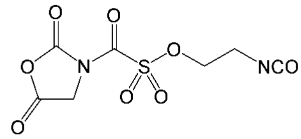

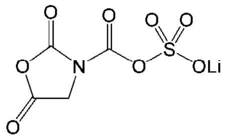
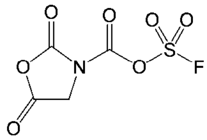
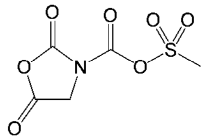
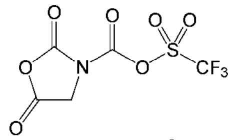

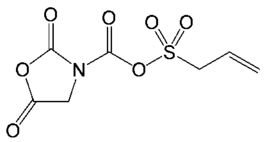
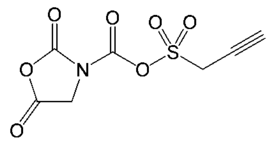
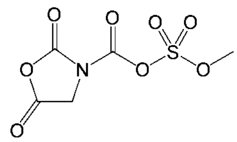
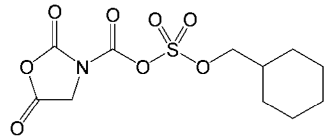

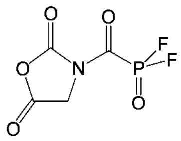
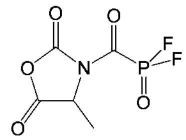
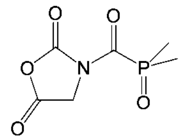
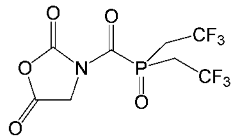

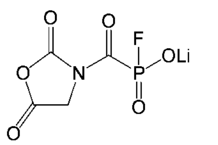
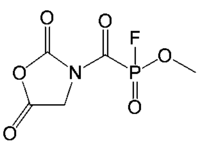
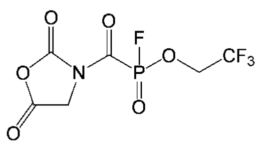
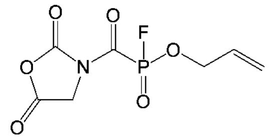

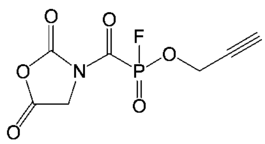
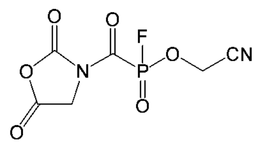
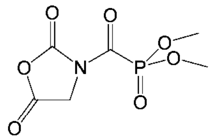
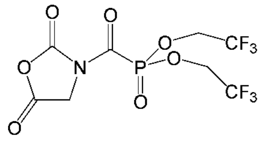

[Chem. 64]

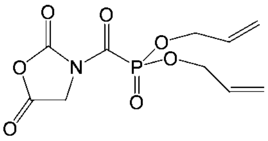 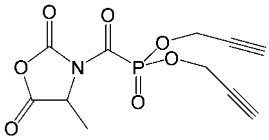 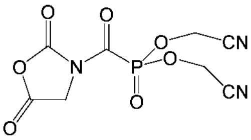 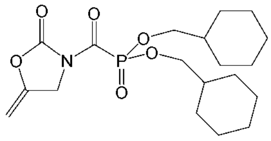

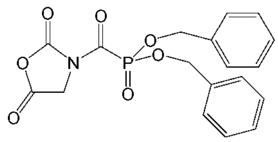 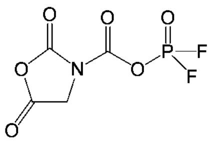 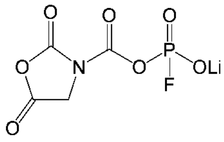

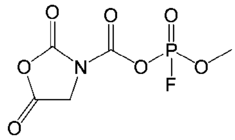 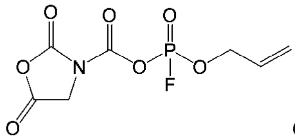 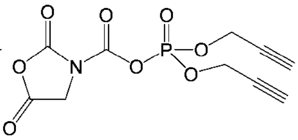

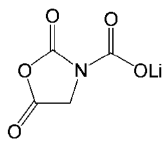 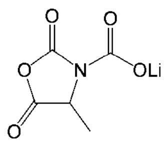 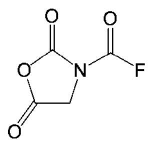 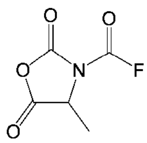

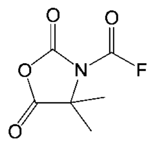 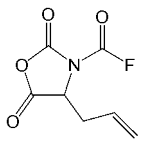 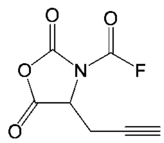 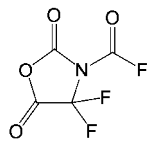

Examples of the compound represented by the general formula (7) include the following compounds.

[Chem. 65]

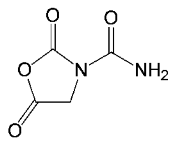 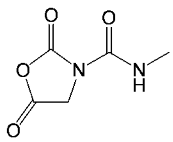 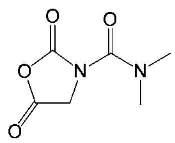 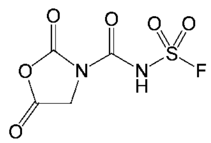 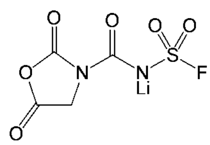

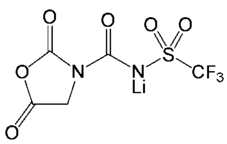 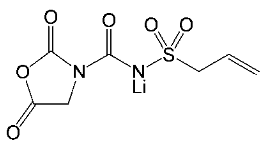 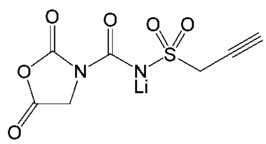 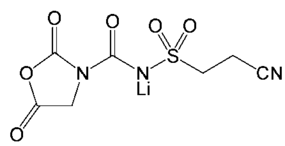

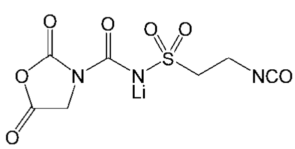 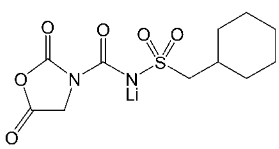 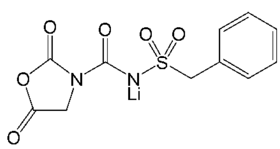 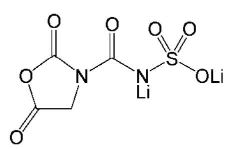

[Chem. 66]

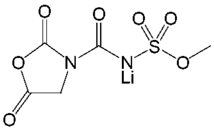
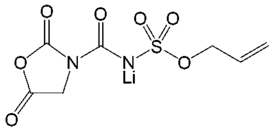
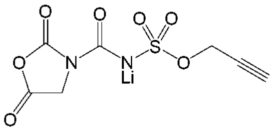
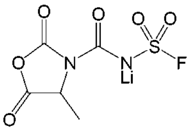
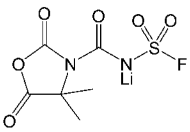
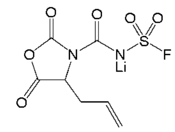
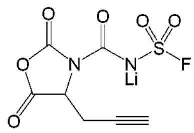
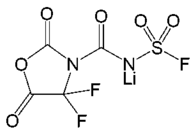
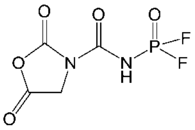
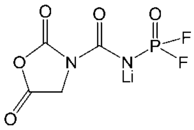
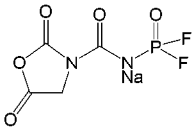
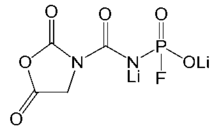
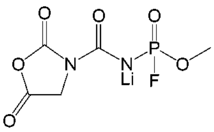
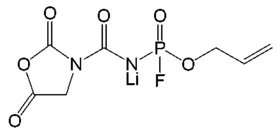
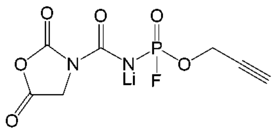
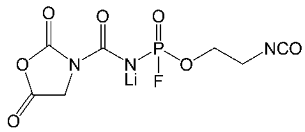
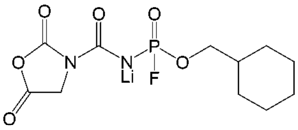
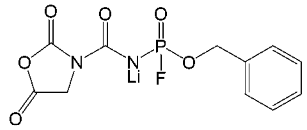
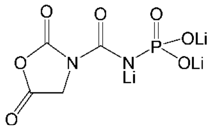
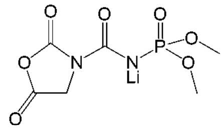
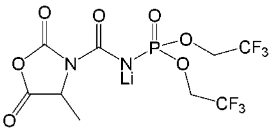
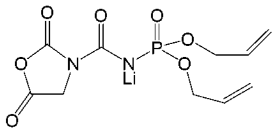
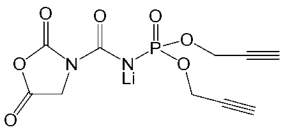
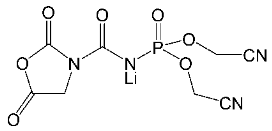

Examples of the compound represented by the general formula (8) include the following compounds.

[Chem. 67]

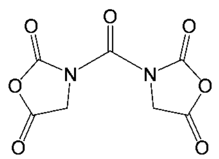
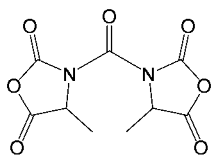
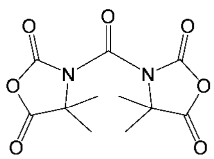
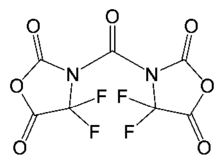
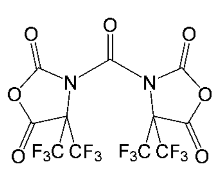
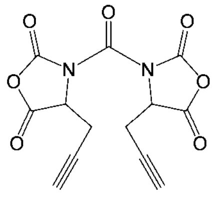
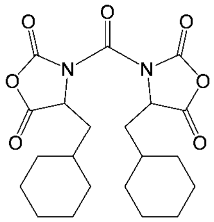
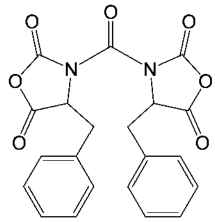

The compounds represented by the general formulae (1) to (8), which are the components (III) in the present disclosure, can be synthesized using a known synthesis reaction once the chemical structures thereof are understood. In the present disclosure, as described later, specifically, synthesis reactions are clarified through synthesis examples.

The compounds represented by the general formulae (1) to (8) may be used singly or in any combination of two or more thereof in any ratio.

The component (III) is preferably at least one selected from the group consisting of the compounds represented by the general formulae (1) to (4) from the viewpoint of (i) the effect of improving the discharge capacity retention rate after a cycle test, (ii) the effect of improving (suppressing) the initial resistance, (iii) the effect of improving the discharge capacity retention rate after a cycle test and the initial resistance in a well-balanced manner, or (iv) the effect of improving (suppressing) the gas generation amount during a cycle test.

Moreover, in a lithium ion battery in which cations are mainly lithium, from the viewpoint of (i) or (ii) above, at least

one selected from the group consisting of the compounds represented by the general formulae (1) to (7) is preferable, and at least one selected from the group consisting of the compounds represented by the general formulae (1) to (4) is particularly preferable.

Moreover, in a sodium ion battery in which cations are mainly sodium, from the viewpoint of (i) above, at least one selected from the group consisting of the compounds represented by the general formulae (1) to (4) and (8) is preferable, and at least one selected from the group consisting of the compounds represented by the general formulae (1) to (4) is particularly preferable.

The component (III) of the present disclosure may be, for example, at 0.009 to 11.5% by mass, preferably 0.05 to 8.5% by mass, and further preferably 0.09 to 5% by mass with respect to the total amount of the nonaqueous electrolyte solution. Within this range, the discharge capacity retention rate characteristic in a cycle test is easily improved when a nonaqueous electrolyte solution battery is formed.

<Other Additive>

The nonaqueous electrolyte solution of the present disclosure contains the above components (I), (II), and (III) as basic constituent components, but as long as the gist of the present disclosure is not impaired, another additive which is generally used in this technical field (hereinafter also referred to as "other additive") may be added to the nonaqueous electrolyte solution of the present disclosure at any ratio.

Examples of such another additive include cyclohexylbenzene, cyclohexylfluorobenzene, biphenyl, 2-fluorobiphenyl, t-butylbenzene, t-amylbenzene, 2-fluorotoluene, fluorobenzene, vinylene carbonate, an oligomer of vinylene carbonate (having a number average molecular weight in terms of polystyrene of 170 to 5000), vinylethylene carbonate, difluoroanisole, fluoroethylene carbonate, 1,6-diisocyanatohexane, ethynylethylene carbonate, trans-difluoroethylene carbonate, methylpropargyl carbonate, ethylpropargyl carbonate, dipropargyl carbonate, maleic anhydride, succinic anhydride, 1,3-propanesultone, 1,3-propenesultone, 1,4-butanesultone, dimethylvinylene carbonate, 1,3,2-dioxathiolane-2,2-dioxide, 4-propyl-1,3,2-dioxathiolane-2,2-dioxide, methylenemethane disulfonate, dimethylenemethane disulfonate, trimethylene methane disulfonate, methyl methanesulfonate, methanesulfonyl fluoride, ethenesulfonyl fluoride, phenyl difluorophosphate, 1,2-ethanedisulfonic anhydride, tris(trimethylsilyl)borate, succinonitrile, (ethoxy)pentafluorocyclotriphosphazene, tetrafluoro(picolinato)phosphate, difluoro(picolinato)borate, and 1,3-dimethyl-1,3-divinyl-1,3-di(1,1,1,3,3,3-hexafluoroisopropyl)disiloxane.

Moreover, examples of the other additive include lithium difluorophosphate, sodium difluorophosphate, lithium fluorosulfate, sodium fluorosulfate, lithium trifluoromethanesulfonate, sodium trifluoromethanesulfonate, lithium nonafluorobutanesulfonate, sodium nonafluorobutanesulfonate, lithium difluorobis(oxalato)phosphate, sodium difluorobis(oxalato)phosphate, lithium difluorooxalatoborate, sodium difluorooxalatoborate, lithium bis(oxalato)borate, sodium bis(oxalato)borate, lithium tetrafluorooxalatophosphate, sodium tetrafluorooxalatophosphate, lithium tris(oxalato)phosphate, sodium tris(oxalato)phosphate, lithium difluoro(malonato)borate, sodium difluoro(malonato)borate, lithium tetrafluoro(malonato)phosphate, sodium tetrafluoro(malonato)phosphate, lithium monofluorophosphate, sodium monofluorophosphate, lithium bis(difluorophosphoryl)imide, sodium bis(difluorophosphoryl)imide, lithium (difluorophosphoryl)(fluorosulfonyl)imide, sodium (difluorophosphoryl)(fluorosulfonyl)imide, lithium bis(trifluoromethanesulfonyl)imide, sodium bis(trifluoromethanesulfonyl)imide, lithium bis(pentafluoroethanesulfonyl)imide, sodium bis(pentafluoroethanesulfonyl)imide, lithium (trifluoromethanesulfonyl)(pentafluoroethanesulfonyl)imide, sodium (trifluoromethanesulfonyl)(pentafluoroethanesulfonyl)imide, lithium (trifluoromethanesulfonyl)(fluorosulfonyl)imide, sodium (trifluoromethanesulfonyl)(fluorosulfonyl)imide, lithium (pentafluoroethanesulfonyl)(fluorosulfonyl)imide, sodium (pentafluoroethanesulfonyl)(fluorosulfonyl)imide, lithium tris(trifluoromethanesulfonyl)methide, sodium tris(trifluoromethanesulfonyl)methide, carboxylates such as lithium acrylate, sodium acrylate, lithium methacrylate, and sodium methacrylate, sulfate ester salts such as lithium methyl sulfate, sodium methyl sulfate, lithium ethyl sulfate, and sodium ethyl sulfate, lithium nitrate, lithium nitrite, sodium nitrate, and sodium nitrite.

In the present disclosure, the concentration of the other additive may be, for example, 0.01% by mass to 10% by mass with respect to the total amount of the nonaqueous electrolyte solution.

Moreover, the nonaqueous electrolyte solution of the present disclosure may further contain a compound represented by the following general formula (9) in order to improve the cycle capacity retention rate and the gas generation during a cycle test.

[Chem. 68]

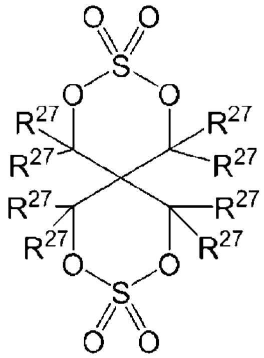

(9)

In the general formula (9), $R^{27}$'s each independently represent a hydrogen atom, a halogen atom, or an alkyl group having 1 to 12 carbon atoms, and an oxygen atom may be included in any carbon atom-carbon atom bond in the alkyl group. Moreover, any hydrogen atom of the alkyl group may be substituted with a fluorine atom. The range of the alkyl group includes the range of the alkyl group having 1 to 10 carbon atoms explained above and alkyl groups having up to 12 carbon atoms.

Suitable examples of such a compound include the compound 1-1 or 1-2 below.

The compounds of the general formula (9) can all be easily produced or prepared by those skilled in the art by a known preparation method. Specifically, the compounds represented by the general formula (9) can be prepared, for example, by a method disclosed in a document such as Can. J. Chem., 79, 1040-1048 (2001).

[Chem. 69]

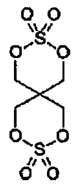

Compound 1-1

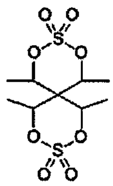

Compound 1-2

The concentration of such a compound may be, for example, 0.01 to 10% by mass, and preferably 0.1 to 5% by mass, with respect to the total amount of the nonaqueous electrolyte solution of the present disclosure.

Moreover, the nonaqueous electrolyte solution of the present disclosure may further contain a compound represented by the following general formula (10) in order to improve the cycle capacity retention rate and the gas generation during a cycle test.

[Chem. 70]

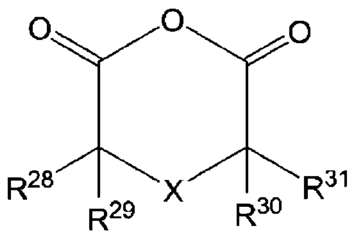

(10)

In the general formula (10), $R^{28}$ to $R^{31}$ each independently represent a hydrogen atom, a halogen atom, an alkyl group, an alkenyl group, or an aryl group. X represents an oxygen atom, a sulfur atom, or an $SO_2$ group.

The compound of the formula (10) is preferably a compound represented by the following formula from the viewpoint of the initial resistance.

[Chem. 71]

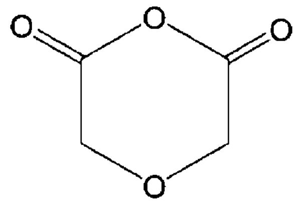

The compounds of the general formula (10) can all be easily produced or prepared by those skilled in the art by a known preparation method. For example, the compound represented by the formula above is a known compound and is commercially available from Tokyo Chemical Industry Co., Ltd., for example.

The concentration of such a compound may be, for example, 0.01 to 10% by mass, and preferably 0.1 to 5% by mass, with respect to the total amount of the nonaqueous electrolyte solution of the present disclosure.

In addition, the nonaqueous electrolyte solution of the present disclosure can also contain a polymer, and as in the case of being used in a nonaqueous electrolyte solution battery referred to as a polymer battery, the nonaqueous electrolyte solution can be quasi-solidified with a gelling agent or a cross-linked polymer before use. The polymer solid electrolyte includes one containing a nonaqueous organic solvent as a plasticizer.

Nonaqueous Electrolyte Solution Battery

The nonaqueous electrolyte solution battery of the present disclosure includes, at least, the nonaqueous electrolyte solution of the present disclosure, a negative electrode, and a positive electrode. Furthermore, a separator, an exterior body, and the like are preferably included.

[Negative Electrode]

Although the negative electrode is not particularly limited, it is preferable to use a material capable of reversibly intercalating and deintercalating alkali metal ions such as lithium ions and sodium ions or alkaline earth metal ions.

[Negative Electrode Active Material]

For example, in the case of a lithium ion secondary battery in which cations are mainly lithium, the negative electrode active material constituting the negative electrode is one capable of doping and dedoping lithium ions. Examples of the negative electrode active material include a material containing at least one selected from a carbon material in which the d value of the lattice plane (002 plane) in X-ray diffraction is 0.340 nm or less, a carbon material in which the d value of the lattice plane (002 plane) in X-ray diffraction exceeds 0.340 nm, oxides of one or more metals selected from Si, Sn, and Al, one or more metals selected from Si, Sn, and Al, alloys containing these metals, alloys of the metals or the alloys and lithium, and lithium titanium oxide. One type of these negative electrode active materials can be used alone, or two or more types thereof can be used in combination. Moreover, as the negative electrode active material, lithium metal, metal nitrides, tin compounds, conductive polymers, and the like may also be used.

For example, in the case of a sodium ion secondary battery in which the cations are mainly sodium, as the negative electrode active material constituting the negative electrode, sodium metal, an alloy of sodium metal and another metal such as tin, an intermetallic compound, various carbon materials such as hard carbon, a metal oxide such as titanium oxide, a metal nitride, (elemental) tin, a tin compound, activated carbon, a conductive polymer, and the like are used. In addition to these, (elemental) phosphorus such as red phosphorus and black phosphorus, phosphorus compounds such as Co-P, Cu-P, Sn-P, Ge-P, and Mo-P, (elemental) antimony, antimony compounds such as Sb/C and Bi-Sb, and the like are used. One type of these negative electrode active materials may be used alone, or two or more types thereof may be used in combination.

[Negative Electrode Current Collector]

The negative electrode has a negative electrode current collector. As the negative electrode current collector, for example, copper, stainless steel, nickel, titanium, or alloys thereof can be used.

[Negative Electrode Active Material Layer]

In the negative electrode, for example, a negative electrode active material layer is formed on at least one surface

of the negative electrode current collector. The negative electrode active material layer includes, for example, the negative electrode active material described above, a binder, and, when necessary, an electrically conductive agent.

Examples of the binder include polytetrafluoroethylene, polyvinylidene fluoride, tetrafluoroethylene-perfluoroalkyl vinyl ether copolymers, styrene-butadiene rubber (hereinafter also referred to as "SBR"), carboxymethyl cellulose, methylcellulose, cellulose acetate phthalate, hydroxypropyl methylcellulose, and polyvinyl alcohol.

As the electrically conductive agent, for example, carbon materials such as acetylene black, Ketjen black, furnace black, and carbon fibers can be used.

[Positive Electrode]

Although the positive electrode is not particularly limited, it is preferable to use a material capable of reversibly intercalating and deintercalating alkali metal ions such as lithium ions and sodium ions or alkaline earth metal ions.

[Positive Electrode Active Material]

For example, when the cation is lithium, lithium-containing transition metal oxide composites such as $LiCoO_2$, $LiNiO_2$, $LiMnO_2$, and $LiMn_2O_4$, lithium-containing transition metal oxide composites including a mixture of a plurality of transition metals in the lithium-containing transition metal oxide composites such as Co, Mn, and Ni, lithium-containing transition metal oxide composites in which a part of the transition metals in the lithium-containing transition metal oxide composites is substituted with a metal other than the transition metals, phosphate compounds of transition metals such as $LiFePO_4$, $LiCoPO_4$, and $LiMnPO_4$ referred to as olivine, oxides such as $TiO_2$, $V_2O_5$, and $MoO_3$, sulfides such as $TiS_2$ and FeS, conductive polymers such as polyacetylene, polyparaphenylene, polyaniline, and polypyrrole, activated carbon, radical-generating polymers, carbon materials, and the like are used as a positive electrode material.

In particular, one containing at least one of (A) a lithium-transition metal oxide composite containing nickel or one or more metals selected from the group consisting of manganese, cobalt, and aluminum in addition to nickel and having a layered structure, (B) a lithium-manganese oxide composite having a spinel structure and containing nickel, (C) a lithium-containing olivine-type phosphate salt containing nickel, and (D) an excessive lithium layered transition metal oxide having a layered rocksalt structure and containing nickel, which are described below, may be used.

((A) Lithium-Transition Metal Oxide Composite)

Positive Electrode Active Material (A)

Suitable examples of the lithium-transition metal oxide composite containing nickel or one or more metals selected from the group consisting of manganese, cobalt, and aluminum in addition to nickel and having a layered structure include lithium-nickel oxide composites, lithium-nickel-cobalt oxide composites, lithium-nickel-cobalt-aluminum oxide composites, lithium-nickel-manganese oxide composites, and lithium-nickel-manganese-cobalt oxide composites. Moreover, those in which a part of the transition metal atoms that are main components of these lithium-transition metal oxide composites has been substituted with other elements such as Al, Ti, V, Cr, Fe, Cu, Zn, Mg, Ga, Zr, Si, B, Ba, Y, and Sn may also be used.

As specific examples of the lithium-nickel oxide composite, $LiNiO_2$, $LiNiO_2$ to which different elements such as Mg, Zr, Al, and Ti are added, and one in which particle surfaces of $LiNiO_2$ particle powder are partly coated with aluminum oxide may be used.

The lithium-nickel-cobalt oxide composite and the lithium-nickel-cobalt-aluminum oxide composite are represented by the following general formula [1-1].

$$Li_aNi_{1-b-c}Co_bM^1{}_cO_2 \qquad [1\text{-}1]$$

In the formula [1-1], $M^1$ is at least one element selected from the group consisting of Al, Fe, Mg, Zr, Ti, and B. a satisfies $0.9 \le a \le 1.2$, and b and c satisfy the conditions $0.1 \le b \le 0.3$ and $0 \le c \le 0.1$.

These oxide composites can be prepared, for example, according to the production method described in JP2009-137834A or the like. Suitable specific examples thereof include $LiNi_{0.8}Co_{0.2}O_2$, $LiNi_{0.85}Co_{0.10}Al_{0.05}O_2$, $LiNi_{0.87}Co_{0.10}Al_{0.03}O_2$, and $LiNi_{0.6}Co_{0.3}Al_{0.1}O_2$.

Specific examples of the lithium-nickel-manganese oxide composite include $LiNi_{0.5}Mn_{0.5}O_2$ and $LiCO_{0.5}Mn_{0.5}O_2$.

Examples of the lithium-nickel-manganese-cobalt oxide composite include a lithium-containing oxide composite represented by the following general formula [1-2].

$Li_dNi_eMn_fCo_gM^2_h$ [1-2]

In the formula [1-2], $M^2$ is at least one element selected from the group consisting of Al, Fe, Mg, Zr, Ti, B, and Sn. d satisfies $0.9 \leq d \leq 1.2$, and e, f, g, and h satisfy the conditions $e + f + g + h = 1, 0 < e \leq 0.9, 0 < f \leq 0.5, 0 < g \leq 0.5$, and $h \geq 0$.

The lithium-nickel-manganese-cobalt oxide composite preferably contains manganese within the range shown in the general formula [1-2] in order to improve the structural stability and improve the safety at a high temperature in a lithium battery and, in particular, more preferably further contains cobalt within the range shown in the general formula [1-2] in order to improve the high rate characteristic of the lithium ion battery.

Specifically, suitable examples thereof include $Li[Ni_{1/3}Mn_{1/3}Co_{1/3}]O_2$ (hereinafter sometimes referred to as "NCM111"), $Li[Ni_{0.45}Mn_{0.35}Co_{0.2}]O_2$, $Li[Ni_{0.5}Mn_{0.3}Co_{0.2}]O_2$, $Li[Ni_{0.6}Mn_{0.2}Co_{0.2}]O_2$ (hereinafter sometimes referred to as "NCM622"), $Li[Ni_{0.8}Mn_{0.1}Co_{0.1}]O_2$ (hereinafter sometimes referred to as "NCM811"), $Li[Ni_{0.49}Mn_{0.3}Co_{0.2}Zr_{0.01}]O_2$, and $Li[Ni_{0.49}Mn_{0.3}Co_{0.2}Mg_{0.01}]O_2$, which have a charging/discharging region at 4.3 V or more.

((B) Lithium-Manganese Oxide Composite Having Spinel Structure)

Positive Electrode Active Material (B)

Examples of the lithium-manganese oxide composite having a spinel structure include a spinel-type lithium-manganese oxide composite represented by the general formula [1-3].

$Li_j(Mn_{2-k}M^3_k)O_4$ [1-3]

In the formula [1-3], $M^3$ contains Ni and may contain at least one metal element selected from the group consisting of Co, Fe, Mg, Cr, Cu, Al, and Ti in addition thereto. j satisfies $1.05 \leq j \leq 1.15$, and k satisfies $0 < k \leq 0.20$.

Specific examples thereof include $LiMn_{1.9}Ni_{0.1}O_4$ and $LiMn_{1.5}Ni_{0.5}O_4$.

((C) Lithium-Containing Olivine-Type Phosphate)

Positive Electrode Active Material (C)

Examples of the lithium-containing olivine-type phosphate include those represented by the following general formula [1-4].

$LiFe_{1-n}M^4_nPO_4$ [1-4]

In the formula [1-4], $M^4$ contains Ni and may contain at least one selected from Co, Mn, Cu, Zn, Nb, Mg, Al, Ti, W, Zr, and Cd in addition thereto, and n satisfies $0 < n \leq 1$.

Specifically, suitable examples thereof include $LiNiPO_4$.

((D) Excessive Lithium Layered Transition Metal Oxide)

Positive Electrode Active Material (D)

Examples of the excessive lithium layered transition metal oxide having a layered rocksalt structure include those represented by the following general formula [1-5].

$xLiM^5O_2 \cdot (1-x)Li_2M^6O_3$ [1-5]

In the formula [1-5], x is a number satisfying $0 < x < 1$, and $M^5$ is at least one metal element having an average oxidation number of $3^+$. $M^6$ is at least one metal element having an average oxidation number of $4^+$. In the formula [1-5], $M^5$ is preferably one or more metal elements selected from trivalent Mn, Ni, Co, Fe, V, and Cr, but the average oxidation number may be set to 3 with equal amounts of divalent and tetravalent metals.

Moreover, in the formula [1-5], $M^6$ is preferably one or more metal elements selected from Mn, Zr, and Ti. Here, nickel is always contained in either $M^5$ or $M^6$. Specifically, suitable examples thereof include $0.5[LiNi_{0.5}Mn_{0.5}O_2] \cdot 0.5[Li_2MnO_3]$, $0.5[LiNi_{1/3}Co_{1/3}Mn_{1/3}O_2] \cdot 0.5[Li_2MnO_3]$, $0.5[LiNi_{0.375}Co_{0.25}Mn_{0.375}O_2] \cdot 0.5[Li_2MnO_3]$, $0.5[LiNi_{0.375}Co_{0.125}Fe_{0.125}Mn_{0.375}O_2] \cdot 0.5[Li_2MnO_3]$, and $0.45[LiNi_{0.375}Co_{0.25}Mn_{0.375}O_2] \cdot 0.10[Li_2TiO_3] \cdot 0.45[Li_2MnO_3]$.

The positive electrode active material (D) represented by the general formula [1-5] is known to exhibit a high capacity in high-voltage charge at 4.4 V or higher (in terms of Li) (for example, U.S. patent 7,135,252).

These positive electrode active materials can be prepared, for example, according to the production methods

described in JP2008-270201A, WO2013/118661, and JP2013-030284A.

For example, when the cation is sodium, sodium-containing transition metal oxide composites such as $NaCrO_2$, $NaFe_{0.5}Co_{0.5}O_2$, $NaFe_{0.4}Mn_{0.3}Ni_{0.3}O_2$, $NaNi_{0.5}Ti_{0.3}Mn_{0.2}O_2$, $NaNi_{1/3}Ti_{1/3}Mn_{1/3}O_2$, $NaNi_{0.33}Ti_{0.33}Mn_{0.16}Mg_{0.17}O_2$, $Na_{2/3}Ni_{1/3}Ti_{1/6}Mn_{1/2}O_2$, and $Na_{2/3}Ni_{1/3}Mn_{2/3}O_2$, sodium-containing transition metal oxide composites including a mixture of a plurality of transition metals in the sodium-containing transition metal oxide composites such as Co, Mn, and Ni, sodium-containing transition metal oxide composites in which a part of the transition metals in the sodium-containing transition metal oxide composites is substituted with a metal other than the transition metals, polyanion type compounds such as $NaFePO_4$, $NaVPO_4F$, $Na_3V_2(PO_4)_3$, and $Na_2Fe_2(SO_4)_3$, sodium salts of Prussian Blue analogues represented by a compositional formula $Na_aM_b[Fe(CN)_6]_c$ (M represents Cr, Mn, Fe, Co, Ni, Cu, or Zn, $0 \leq a \leq 2$, $0.5 \leq b \leq 1.5$, and $0.5 \leq c \leq 1.5$), oxides such as $TiO_2$, $V_2O_5$, and $MoO_3$, sulfides such as $TiS_2$ and FeS, conductive polymers such as polyacetylene, polyparaphenylene, polyaniline, and polypyrrole, activated carbon, radical-generating polymers, carbon materials, and the like are used as a positive electrode material (positive electrode active material).

[Positive Electrode Current Collector]

The positive electrode has a positive electrode current collector. As the positive electrode current collector, for example, aluminum, stainless steel, nickel, titanium, or alloys thereof can be used.

[Positive Electrode Active Material Layer]

In the positive electrode, for example, a positive electrode active material layer is formed on at least one surface of the positive electrode current collector. The positive electrode active material layer includes, for example, the positive electrode active material described above, a binder, and, when necessary, an electrically conductive agent.

Examples of the binder include polytetrafluoroethylene, polyvinylidene fluoride, tetrafluoroethylene-perfluoroalkyl vinyl ether copolymers, styrene-butadiene rubber (hereinafter also referred to as "SBR"), carboxymethyl cellulose, methylcellulose, cellulose acetate phthalate, hydroxypropyl methylcellulose, and polyvinyl alcohol.

As the electrically conductive agent, for example, carbon materials such as acetylene black, Ketjen black, furnace black, carbon fibers, graphite (granular graphite and flake graphite), and fluorinated graphite can be used. For the positive electrode, acetylene black or Ketjen black with low crystallinity or the like is preferably used.

[Production Methods of Electrodes (Positive Electrode and Negative Electrode)]

For example, an electrode can be obtained by dispersing and kneading predetermined blending amounts of an active material, a binder, and, if necessary, an electrically conductive agent in a solvent such as N-methyl-2-pyrrolidone (hereinafter also referred to as "NMP") and water, applying the obtained paste on a current collector and drying to form an active material layer. The obtained electrode is preferably compressed by a method such as roll pressing to adjust the electrode to an appropriate density.

[Separator]

The nonaqueous electrolyte solution battery of the present disclosure can have a separator. As a separator for preventing contact between the positive electrode and the negative electrode, for example, a nonwoven fabric or a porous sheet made of polyolefin such as polypropylene and polyethylene, cellulose, paper, glass fibers, or the like is used. These films are preferably microporous so that the electrolyte solution easily permeates and so that the ions easily pass through.

Examples of the polyolefin separator include a film that electrically insulates the positive electrode and the negative electrode and that is permeable to lithium ions such as microporous polymer films including a porous polyolefin film. As a specific example of the porous polyolefin film, for example, a porous polyethylene film alone or a multilayer film obtained by stacking a porous polyethylene film and a porous polypropylene film may be used. Another example is a film obtained by combining a porous polyethylene film and a polypropylene film or the like.

The nonaqueous electrolyte solution of the present disclosure may be impregnated and held in the separator. The impregnation method is not particularly restricted and may be performed by a known method. Specifically, a battery having a positive electrode, a separator, and a negative electrode can be impregnated by injecting the electrolyte solution at the end.

[Exterior Body]

As the exterior body of the nonaqueous electrolyte solution battery of the present disclosure, for example, a coin-shaped, cylindrical, or square-shaped metal can or a laminated exterior body can be suitably used. Suitable examples of

the metal can material include nickel-plated steel plates, stainless steel plates, nickel-plated stainless steel plates, aluminum or alloys thereof, nickel, and titanium. As the laminated exterior body, for example, an aluminum laminated film, a SUS laminated film, or a silica-coated laminated film of polypropylene, polyethylene, or the like can be used.

The configuration of the nonaqueous electrolyte solution battery according to the present embodiment is not particularly restricted but can be, for example, a configuration in which an electrode element including a positive electrode and a negative electrode arranged to face each other and a nonaqueous electrolyte solution are included in an exterior body. The shape of the nonaqueous electrolyte solution battery is not particularly limited, but an electrochemical device having a shape such as a coin shape, a cylindrical shape, a square shape, and an aluminum laminate sheet shape is assembled with the above elements.

The nonaqueous electrolyte solution battery including the nonaqueous electrolyte solution of the present disclosure can be produced, for example, by impregnating a separator with the nonaqueous electrolyte solution described above, disposing the separator between the positive electrode described above and the negative electrode described above, and assembling the cell.

## EXAMPLES

Hereinafter, the present disclosure will be more specifically explained with Synthesis Examples, Examples, and Comparative Examples, but the scope of the present disclosure is not limited at all by the Synthesis Examples, the Examples, and the Comparative Examples.

Synthesis Examples of Component (III):

Synthesis Example 1-1 (Synthesis Example of Compound (1a) Represented by General Formula (1))

[Chem. 72]

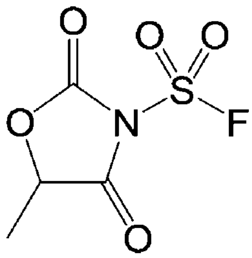

(1a)

Lithium hydride (0.1 g, 13 mmol) was added to 5-methyloxazolidine-2,4-dione (1.2 g, 10 mmol) in 50 ml of acetonitrile, and then the mixture solution was ice-cooled. Subsequently, sulfuryl fluoride (1.2 g, 12 mmol) was added to the reaction solution, and the mixture was heated to room temperature and then stirred for 20 hours. The obtained suspension was filtered, and the filtrate was concentrated under reduced pressure to obtain a target compound (1a) as a white solid (1.5 g, yield 73%).

$^{1}$H NMR($CD_3CN$)σ1.56, 5.18ppm
$^{19}$F NMR($CD_3CN$)σ40.8ppm

Synthesis Example 1-2 (Synthesis Example of Compound (1b) Represented by General Formula (1)

[Chem. 73]

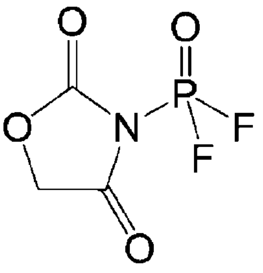

(1b)

Phosphoryl chloride (1.6 g, 10 mmol) was added to oxazolidine-2,4-dione (1.0 g, 10 mmol) in 50 ml of acetonitrile, and then the reaction solution was ice-cooled. Subsequently, sodium hydride (0.3 g, 13 mmol) was added little by little to the reaction solution, and the mixture was heated to room temperature and then stirred for one hour. The obtained suspension was filtered, and the filtrate was concentrated under reduced pressure to obtain N-dichlorophosphoryloxazolidine-2,4-dione as an intermediate. Next, after 50 ml of acetonitrile was added, potassium fluoride (1.4 g, 24 mmol) was added, and the mixture was stirred at room temperature for 23 hours. The obtained suspension was filtered, and the filtrate was concentrated under reduced pressure to obtain a target compound (1b) (1.2 g, yield 66%).

$^{1}$H NMR($CD_3CN$)σ4.88ppm
$^{19}$F NMR($CD_3CN$)σ-72.0, -69.1ppm

Synthesis Example 1-3 (Synthesis Example of Compound (1c) Represented by General Formula (1))

[Chem. 74]

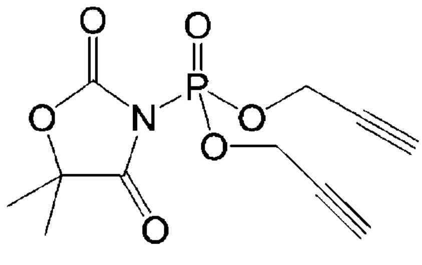

(1c)

Phosphoryl chloride (1.6 g, 10 mmol) was added to 5,5-dimethyloxazolidine-2,4-dione (1.3 g, 10 mmol) in 50 ml of acetonitrile, and then the reaction solution was ice-cooled. Subsequently, sodium hydride (0.3 g, 13 mmol) was added little by little to the reaction solution, and the mixture was heated to room temperature and then stirred for one hour. The obtained suspension was filtered, and the filtrate was concentrated under reduced pressure to obtain N-dichlorophosphoryl-5,5-dimethyloxazolidine-2,4-dione as an intermediate. Next, 50 ml of 1,2-dichloroethane and propargyl alcohol (1.4 g, 25 mmol) were added, and then the mixture solution was ice-cooled. Subsequently, triethylamine (2.4 g, 24 mmol) was slowly added dropwise so that the internal temperature of the reaction solution did not exceed 5°C, and the mixture was heated to room temperature and then stirred for two hours. The reaction solution was washed twice with 20 ml of 1 M hydrochloric acid solution, and the obtained organic layer was concentrated under reduced pressure to obtain a target compound (1c) (1.9 g, yield 66%).
$^{1}$H NMR($CD_3CN$)σ1.58, 2.99, 4.92ppm

Synthesis Example 1-4 (Synthesis Example of Compound (1d) Represented by General Formula (1))

[Chem. 75]

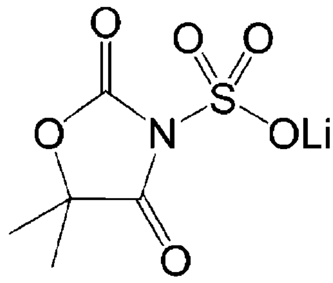

(1d)

Lithium hydride (0.1 g, 13 mmol) was added to 5,5-dimethyloxazolidine-2,4-dione (1.3 g, 10 mmol) in 50 ml of ethyl methyl carbonate, and the mixture was stirred for 15 hours. To the suspension, sulfur trioxide (0.9 g, 11 mmol) was added to the reaction solution, and the mixture was stirred at room temperature for 30 minutes. The obtained suspension was filtered to obtain a target compound (1d) as a filtrate (2.0 g, yield 92%).

$^{1}H$ NMR($CD_3CN$)σ1.55ppm

Synthesis Example 1-5 (Synthesis Example of Compound (1e) Represented by General Formula (1))

[Chem. 76]

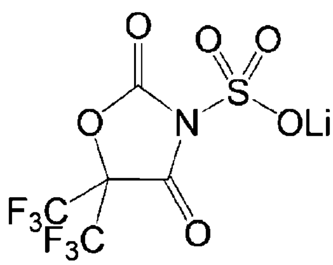

(1e)

Lithium hydride (0.1 g, 13 mmol) was added to 5,5-bis(trifluoromethyl)oxazolidine-2,4-dione (2.4 g, 10 mmol) in 50 ml of ethyl methyl carbonate, and the mixture was stirred for 15 hours. To the suspension, sulfur trioxide (0.9 g, 11 mmol) was added to the reaction solution, and the mixture was stirred at room temperature for 30 minutes. The obtained suspension was filtered to obtain a target compound (1e) as a filtrate (2.9 g, yield 89%).

$^{19}F$ NMR($CD_3CN$)σ-72.8ppm

Synthesis Example 1-6 (Synthesis Example of Compound (1f) Represented by General Formula (1))

[Chem. 77]

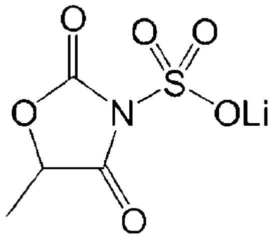

(1f)

Lithium hydride (0.1 g, 13 mmol) was added to 5-methyloxazolidine-2,4-dione (1.2 g, 10 mmol) in 50 ml of ethyl methyl carbonate, and the mixture was stirred for 17 hours. To the suspension, sulfur trioxide (0.9 g, 11 mmol) was added to the reaction solution, and the mixture was stirred at room temperature for 30 minutes. The obtained suspension was filtered to obtain a target compound (1f) as a filtrate (1.9 g, yield 91%).

$^1$H NMR($CD_3CN$)σ1.49, 4.83ppm

Synthesis Example 1-7 (Synthesis Example of Compound (1g) Represented by General Formula (1))

[Chem. 78]

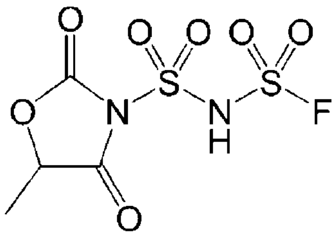

(1g)

Bis(fluorosulfonyl)imide (1.9 g, 10 mmol) was added to 5-methyloxazolidine-2,4-dione (1.2 g, 10 mmol) in 50 ml of acetonitrile, and then the reaction solution was ice-cooled. Subsequently, lithium hydride (0.1 g, 13 mmol) was added little by little to the reaction solution, and the mixture was heated to 50°C and then stirred for 18 hours. The obtained suspension was filtered, and the filtrate was concentrated under reduced pressure to obtain a target compound (1g) (2.3 g, yield 80%).

$^1$H NMR($CD_3CN$)σ1.50, 4.90ppm
$^{19}$F NMR($CD_3CN$)σ51.8ppm

Synthesis Example 1-8 (Synthesis Example of Compound (1h) Represented by General Formula (1))

[Chem. 79]

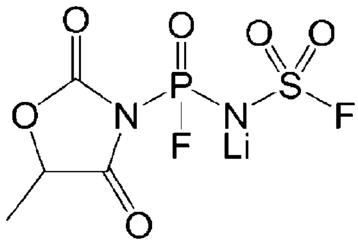

(1h)

Lithium (difluorophosphoryl)(fluorosulfonyl)imide (1.9 g, 10 mmol) was added to 5-methyloxazolidine-2,4-dione (1.2 g, 10 mmol) in 50 ml of acetonitrile, and then the reaction solution was ice-cooled. Subsequently, lithium hydride (0.1 g, 13 mmol) was added little by little to the reaction solution, and the mixture was heated to 50°C and then stirred for 18 hours. The obtained suspension was filtered, and the filtrate was concentrated under reduced pressure to obtain a target compound (1h) (2.2 g, yield 74%).

$^1$H NMR($CD_3CN$)σ1.48, 4.92ppm
$^{19}$F NMR($CD_3CN$)σ52.2, -72.1, -69.2ppm

Synthesis Example 1-9 (Synthesis Example of Compound (1i) Represented by General Formula (1))

[Chem. 80]

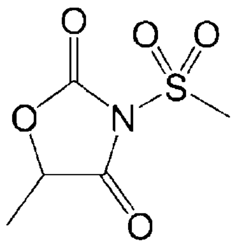

(1i)

Methanesulfonyl chloride (1.26 g, 11 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 15 hours. The obtained suspension was filtered, and the filtrate was concentrated under reduced pressure to obtain a target compound (1i) as a white solid (1.7 g, yield 84%).
$^1$H NMR($CD_3CN$)σ1.56, 3.12, 5.21ppm

Synthesis Example 1-10 (Synthesis Example of Compound (1j) Represented by General Formula (1))

[Chem. 81]

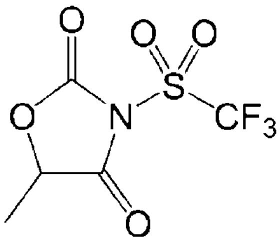

(1 j)

Trifluoromethanesulfonyl chloride (1.85 g, 11 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 15 hours. The obtained suspension was filtered, and the filtrate was concentrated under reduced pressure to obtain a target compound (1j) as a white solid (2.0 g, yield 78%).

$^{1}H$ NMR($CD_3CN$)σ1.47, 4.92ppm
$^{19}F$ NMR($CD_3CN$)σ-80.1ppm

<u>Synthesis Example 1-11</u> (Synthesis Example of Compound (1k) Represented by General Formula (1))

[Chem. 82]

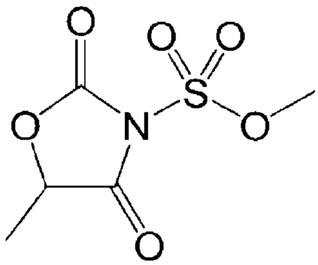

(1 k)

Methyl chlorosulfonate (1.43 g, 11 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 15 hours. The obtained suspension was filtered, and the filtrate was concentrated under reduced pressure to obtain a target compound (1k) as a white solid (1.6 g, yield 73%).

$^{1}H$ NMR($CD_3CN$)σ1.53, 4.21, 5.17ppm

<u>Synthesis Example 1-12</u> (Synthesis Example of Compound (11) Represented by General Formula (1))

[Chem. 83]

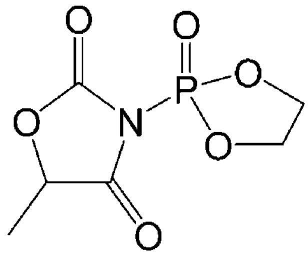

(1 l)

2-Chloro-2-oxo-1,3,2-dioxaphosphorane (1.56 g, 11 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 20 hours. The obtained suspension was filtered, and the filtrate was concentrated under reduced pressure to obtain a target compound (1l) as a white solid (2.0 g, yield 87%).

$^{1}H$ NMR($CD_3CN$)σ1.51, 4.15, 4.79ppm

Synthesis Example 1-13 (Synthesis Example of Compound (1m) Represented by General Formula (1))

[Chem. 84]

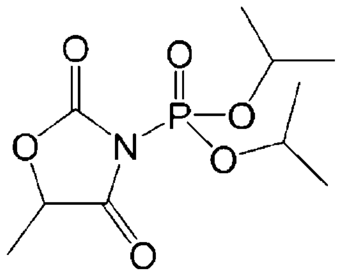

(1 m)

Diisopropyl chlorophosphate (2.2 g, 11 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 14 hours. The obtained suspension was filtered, and the filtrate was concentrated under reduced pressure to obtain a target compound (1 m) as a white solid (2.7 g, yield 92%).

$^{1}H$ NMR($CD_3CN$)σ1.43, 1.59, 4.15, 4.71ppm

Synthesis Example 1-14 (Synthesis Example of Compound (1n) Represented by General Formula (1))

[Chem. 85]

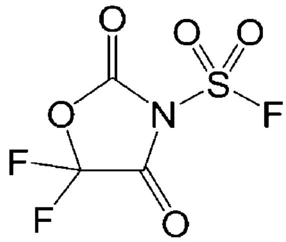

(1 n)

Oxalyl chloride (1.5 g, 12 mmol) was added to fluorosulfonyl isocyanate (1.3 g, 10 mmol) in 50 ml of acetonitrile, and then the mixture was heated to 90°C, refluxed and stirred for 21 hours. Sodium fluoride (1.3 g, 31 mmol) was added to the obtained reaction solution, and then the mixture was heated to 50°C and then stirred for 13 hours. The obtained suspension was filtered, and the filtrate was concentrated under reduced pressure to obtain a target compound (1n) as a white solid (1.5 g, yield 68%).

$^{1}$H NMR($CD_3CN$)σ1.43, 1.59, 4.15, 4.71ppm
$^{19}$F NMR($CD_3CN$)σ49.5, -88.2ppm

<u>Synthesis Example 1-15</u> (Synthesis Example of Compound (1o) Represented by General Formula (1))

[Chem. 86]

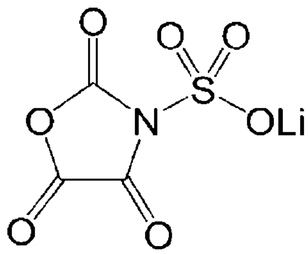

(1 o)

Lithium hydride (0.1 g, 13 mmol) was added to oxazolidine-2,4,5-trione (1.2 g, 10 mmol) in 50 ml of ethyl methyl carbonate, and the mixture was stirred for 15 hours. To the suspension, sulfur trioxide (0.9 g, 11 mmol) was added to the reaction solution, and the mixture was stirred at room temperature for 30 minutes. The obtained suspension was filtered to obtain a target compound (1o) as a filtrate (1.8 g, yield 86%).

<u>Synthesis Example 2-1</u> (Synthesis Example of Compound (2a) Represented by General Formula (2))

[Chem. 87]

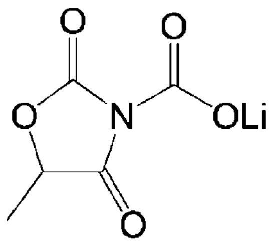

(2a)

After methyl chloroformate (1.1 g, 1.2 mmol) was added to 5-methyloxazolidine-2,4-dione (1.2 g, 10 mmol) in 50 ml of acetonitrile, sodium hydride (0.3 g, 13 mmol) was added little by little at room temperature, and the mixture was stirred for one hour. The obtained suspension was filtered, and the filtrate was concentrated under reduced pressure to obtain N-methoxycarbonyl-5-methyloxazolidine-2,4-dione as an intermediate. Next, 50 ml of acetonitrile and 1.3 g (10 mmol) of lithium iodide were added, and the mixture was heated to 50°C and stirred for 20 hours. The reaction solution was concentrated under reduced pressure to obtain (2a) (1.3 g, yield 79%).

$^1H$ NMR($CD_3CN$)σ1.57, 5.11ppm

<u>Synthesis Example 2-2</u> (Synthesis Example of Compound (2b) Represented by General Formula (2)

[Chem. 88]

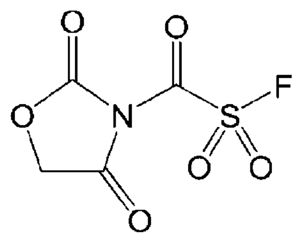

(2 b)

Triphosgene (1.5 g, 5.1 mmol) was added to oxazolidine-2,4-dione (1.2 g, 10 mmol) in 50 ml of acetonitrile, and then the mixture solution was ice-cooled. Subsequently, sodium hydride (0.3 g, 12 mmol) was added to the reaction solution, and the mixture was heated to room temperature and then stirred for 19 hours. The obtained suspension was filtered, and the filtrate was concentrated under reduced pressure to obtain N-chlorocarbonyl-oxazolidine-2,4-dione as an intermediate. Next, after adding 50 ml of acetonitrile and ice cooling, sodium hydride (0.3 g, 13 mmol) was added, and hydrogen sulfide (0.3 g, 10 mmol) was added to the reaction solution. The mixture was heated to room temperature and then stirred for 12 hours. The obtained suspension was filtered, and the filtrate was concentrated under reduced pressure to obtain N-thiolcarbonyl-oxazolidine-2,4-dione as an intermediate. Next, after adding 30 ml of water, potassium peroxymonosulfate (3.8 g, 25 mmol) and potassium chloride (0.7 g, 10 mmol) were added, and the mixture was stirred at room temperature for two hours. Thereafter, sodium fluoride (0.4 g, 10 mmol) was added, and the mixture was stirred at room temperature for 24 hours. The obtained reaction solution was washed with 30 ml of ethyl acetate added thereto, and the organic layer was dried with anhydrous sodium sulfate added thereto and filtered. The obtained filtrate was concentrated under reduced pressure to obtain a target compound (2b) (0.4 g, yield 19%).

$^1H$ NMR($CD_3CN$)σ5.04ppm
$^{19}F$ NMR($CD_3CN$)σ49.3ppm

<u>Synthesis Example 2-3</u> (Synthesis Example of Compound (2c) Represented by General Formula (2))

[Chem. 89]

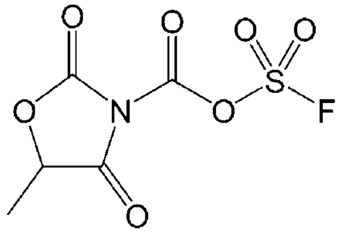

(2c)

Triphosgene (1.5 g, 5.1 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 19 hours. The obtained suspension was filtered, and the filtrate was concentrated under reduced pressure to obtain N-chlorocarbonyl-5-methyloxazolidine-2,4-dione as an intermediate. Next, after 50 ml of acetonitrile was added, lithium fluorosulfate (1.1 g, 10 mmol) was added, and the mixture was stirred at room temperature for 23 hours. The obtained reaction solution was concentrated under reduced pressure to obtain a target compound (2c) (1.6 g, yield 66%).

$^{1}H$ NMR($CD_3CN$)$\sigma$1.65, 5.37ppm
$^{19}F$ NMR($CD_3CN$)$\sigma$47.3ppm

<u>Synthesis Example 2-4</u> (Synthesis Example of Compound (2d) Represented by General Formula (2))

[Chem. 90]

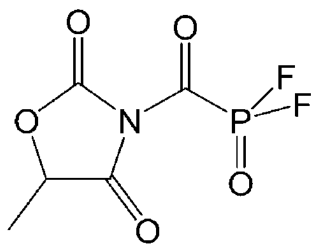

(2d)

Triphosgene (1.5 g, 5.1 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 19 hours. The obtained suspension was filtered, and the filtrate was concentrated under reduced pressure to obtain N-chlorocarbonyl-5-methyloxazolidine-2,4-dione as an intermediate. Next, after 50 ml of acetonitrile was added, dichlorohydroxyphosphine (1.2 g, 10 mmol) was added, and the mixture was stirred at room temperature for 23 hours. Thereafter, sodium fluoride (0.8 g, 20 mmol) was added, and the mixture was stirred at room temperature for 25 hours. The obtained reaction solution was concentrated under reduced pressure to obtain a target compound (2d) (0.9 g, yield 40%).

$^{1}H$ NMR($CD_3CN$)$\sigma$1.61, 5.21ppm
$^{19}F$ NMR($CD_3CN$)$\sigma$-71.5, -68.6ppm

<u>Synthesis Example 2-5</u> (Synthesis Example of Compound (2e) Represented by General Formula (2))

[Chem. 91]

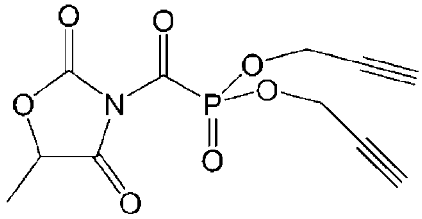

(2 e)

Triphosgene (1.5 g, 5.1 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 19 hours. The obtained suspension was filtered, and the filtrate was concentrated under reduced pressure to obtain N-chlorocarbonyl-5-methyloxazolidine-2,4-dione as an intermediate. Next, after 50 ml of acetonitrile was added, 2-propynylphosphite (2.0 g, 10 mmol) was added, and the mixture was stirred at room temperature for 23 hours. The obtained reaction solution was concentrated under reduced pressure to obtain a target compound (2e) (1.5 g, yield 48%).

$^{1}H$ NMR($CD_3CN$)σ1.57, 5.11ppm

Synthesis Example 2-6 (Synthesis Example of Compound (2f) Represented by General Formula (2))

[Chem. 92]

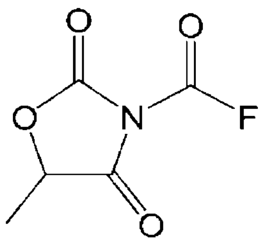

(2 f)

Triphosgene (1.5 g, 5.1 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 20 hours. Sodium fluoride (0.6 g, 14 mmol) was added to the obtained suspension, and then the mixture was stirred at room temperature for 15 hours. The obtained suspension was filtered, and the filtrate was concentrated under reduced pressure to obtain a target compound (2f) as a white solid (1.0 g, yield 60%).

$^{1}H$ NMR($CD_3CN$)σ1.51, 4.95ppm
$^{19}F$ NMR($CD_3CN$)σ-68.2ppm

Synthesis Example 3-1 (Synthesis Example of Compound (3a) Represented by General Formula (3))

[Chem. 93]

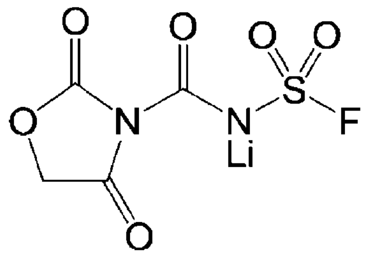

(3a)

After fluorosulfonyl isocyanate (1.4 g, 11 mmol) was added to oxazolidine-2,4-dione (1.0 g, 10 mmol) in 50 ml of acetonitrile, lithium hydride (0.1 g, 13 mmol) was added little by little at room temperature, and the mixture was stirred for three hours. The obtained suspension was filtered, and the filtrate was concentrated under reduced pressure to obtain a target compound (3a) (2.1 g, yield 91%).

$^{1}H$ NMR($CD_3CN$)σ4.76ppm
$^{19}F$ NMR($CD_3CN$)σ49.5ppm

Synthesis Example 3-2 (Synthesis Example of Compound (3b) Represented by General Formula (3))

[Chem. 94]

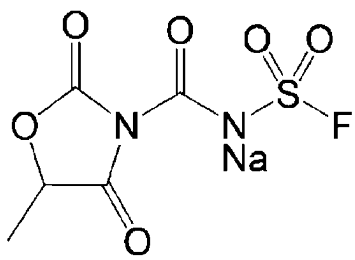

(3b)

After fluorosulfonyl isocyanate (1.4 g, 11 mmol) was added to 5-methyloxazolidine-2,4-dione (1.2 g, 10 mmol) in 50 ml of acetonitrile, sodium hydride (0.3 g, 13 mmol) was added little by little at room temperature, and the mixture was

stirred for three hours. The obtained suspension was filtered, and the filtrate was concentrated under reduced pressure to obtain a target compound (3b) (2.4 g, yield 88%).

$^1$H NMR($CD_3CN$)σ1.55, 5.09ppm
$^{19}$F NMR($CD_3CN$)σ49.7ppm

<u>Synthesis Example 3-3</u> (Synthesis Example of Compound (3c) Represented by General Formula (3)

[Chem. 95]

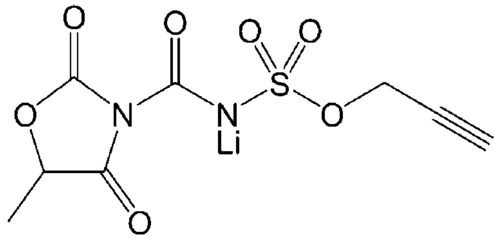

(3c)

After fluorosulfonyl isocyanate (1.4 g, 11 mmol) was added to 5-methyloxazolidine-2,4-dione (1.2 g, 10 mmol) in 50 ml of acetonitrile, lithium hydride (0.2 g, 25 mmol) was added little by little at room temperature, and the mixture was stirred for three hours. Next, propargyl alcohol (0.7 g, 12 mmol) was added, and then the mixture was heated to 50°C and stirred for five hours. The obtained suspension was filtered, and the filtrate was concentrated under reduced pressure to obtain a target compound (3c) (2.5 g, yield 85%).

$^1$H NMR($CD_3CN$)σ1.55, 3.65, 5.09ppm

<u>Synthesis Example 3-4</u> (Synthesis Example of Compound (3d) Represented by General Formula (3))

[Chem. 96]

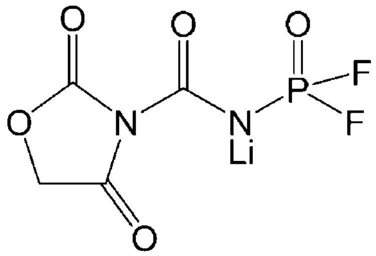

(3d)

After difluorophosphoryl isocyanate (1.4 g, 11 mmol) was added to oxazolidine-2,4-dione (1.0 g, 10 mmol) in 50 ml of acetonitrile, lithium hydride (0.1 g, 13 mmol) was added little by little at room temperature, and the mixture was stirred for three hours. The obtained suspension was filtered, and the filtrate was concentrated under reduced pressure to obtain a target compound (3d) (2.1 g, yield 91%).

$^1$H NMR($CD_3CN$)σ4.76ppm
$^{19}$F NMR($CD_3CN$)σ-74.7, -71.8ppm

<u>Synthesis Example 3-5</u> (Synthesis Example of Compound (3e) Represented by General Formula (3))

[Chem. 97]

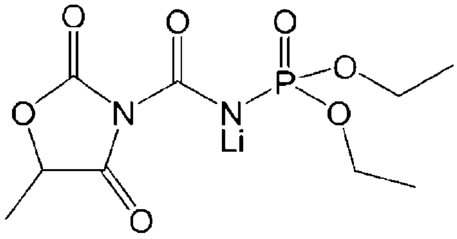

(3 e)

After diethylphosphoryl isocyanate (2.0 g, 11 mmol) was added to 5-methyloxazolidine-2,4-dione (1.2 g, 10 mmol) in 50 ml of acetonitrile, lithium hydride (0.1 g, 13 mmol) was added little by little at room temperature, and the mixture was stirred for three hours. The obtained suspension was filtered, and the filtrate was concentrated under reduced pressure to obtain a target compound (3e) (2.6 g, yield 83%).

$^1H$ NMR($CD_3CN$)σ1.55, 1.59, 4.27, 5.09ppm

Synthesis Example 3-6 (Synthesis Example of Compound (3f) Represented by General Formula (3))

[Chem. 98]

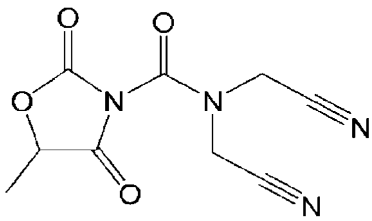

(3 f)

Triphosgene (1.5 g, 5.1 mmol) was added to 5-methyloxazolidine-2,4-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for five hours. To the obtained suspension, iminodiacetonitrile (1.0 g, 10 mmol) was added little by little under ice cooling. The mixture solution was heated to room temperature and then stirred for 17 hours. The obtained suspension was filtered, and the filtrate was concentrated under reduced pressure to obtain a target compound (3f) as a white solid (1.5 g, yield 61%).

$^1H$ NMR($CD_3CN$)σ1.54, 4.22, 5.08ppm

Synthesis Example 4-1 (Synthesis Example of Compound (4a) Represented by General Formula (4))

[Chem. 99]

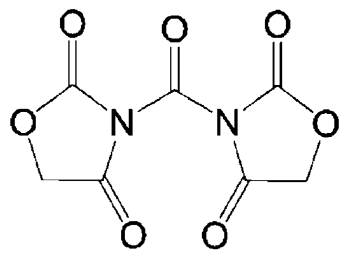

(4a)

After triphosgene (1.5 g, 5.1 mmol) was added to oxazolidine-2,4-dione (1.0 g, 10 mmol) in 50 ml of acetonitrile, sodium hydride (0.3 g, 13 mmol) was added little by little at room temperature, and the mixture was stirred for 21 hours. The obtained suspension was filtered, and the filtrate was concentrated under reduced pressure to obtain a target compound (4a) (1.0 g, yield 89%).

$^1$H NMR($CD_3CN$)σ4.84ppm

Synthesis Example 4-2 (Synthesis Example of Compound (4b) Represented by General Formula (4))

[Chem. 100]

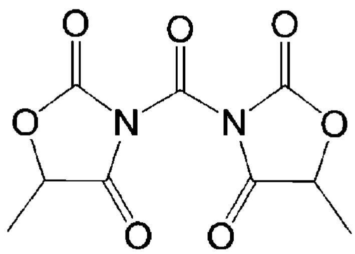

(4b)

After triphosgene (1.5 g, 5.1 mmol) was added to 5-methyloxazolidine-2,4-dione (1.2 g, 10 mmol) in 50 ml of acetonitrile, sodium hydride (0.3 g, 13 mmol) was added little by little at room temperature, and the mixture was stirred for 21 hours. The obtained suspension was filtered, and the filtrate was concentrated under reduced pressure to obtain a target compound (4b) (1.1 g, yield 90%).

$^1$H NMR($CD_3CN$)σ1.57, 5.11ppm

Synthesis Example 4-3 (Synthesis Example of Compound (4c) Represented by General Formula (4))

[Chem. 101]

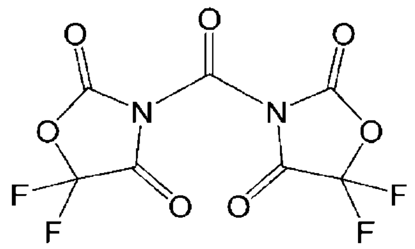

(4c)

A compound (4c) was synthesized in the same manner as in the synthesis example of the compound (4a) except that 10 mmol of 5,5-difluorooxazolidine-2,4-dione was used instead of 10 mmol of oxazolidine-2,4-dione. (1.3 g, yield 85%)
$^{19}F$ NMR($CD_3CN$)σ-85.2ppm

Synthesis Example 4-4 (Synthesis Example of Compound (4d) Represented by General Formula (4))

[Chem. 102]

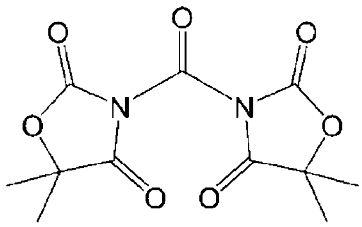

(4d)

A compound (4d) was synthesized in the same manner as in the synthesis example of the compound (4a) except that 10 mmol of 5,5-dimethyloxazolidine-2,4-dione was used instead of 10 mmol of oxazolidine-2,4-dione. (1.3 g, yield 85%)
$^{1}H$ NMR($CD_3CN$)σ1.56ppm

Synthesis Example 5-1 (Synthesis Example of Compound (5a) Represented by General Formula (5))

[Chem. 103]

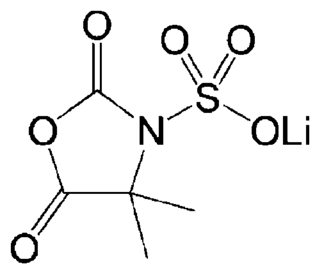

(5a)

Lithium hydride (0.1 g, 13 mmol) was added to 4,4-dimethyloxazolidine-2,5-dione (1.3 g, 10 mmol) in 50 ml of ethyl methyl carbonate, and the mixture was stirred for 15 hours. To the suspension, sulfur trioxide (0.9 g, 11 mmol) was

added to the reaction solution, and the mixture was stirred at room temperature for 30 minutes. The obtained suspension was filtered to obtain a target compound (5a) as a filtrate (1.9 g, yield 88%).

$^{1}H$ NMR($CD_3CN$)σ1.61ppm

Synthesis Example 5-2 (Synthesis Example of Compound (5b) Represented by General Formula (5))

[Chem. 104]

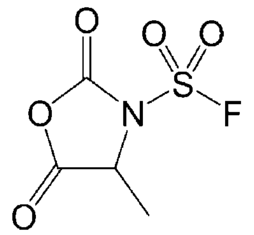

(5 b)

Lithium hydride (0.1 g, 13 mmol) was added to 4-methyloxazolidine-2,5-dione (1.2 g, 10 mmol) in 50 ml of acetonitrile, and then the mixture solution was ice-cooled. Subsequently, sulfuryl fluoride (1.2 g, 12 mmol) was added to the reaction solution, and the mixture was heated to room temperature and then stirred for 20 hours. The obtained suspension was filtered, and the filtrate was concentrated under reduced pressure to obtain a target compound (5b) as a white solid (1.4 g, yield 68%).

$^{1}H$ NMR($CD_3CN$)σ1.57, 5.21ppm
$^{19}F$ NMR($CD_3CN$)σ41.0ppm

Synthesis Example 5-3 (Synthesis Example of Compound (5c) Represented by General Formula (5))

[Chem. 105]

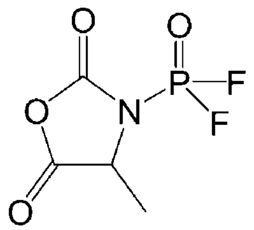

(5 c)

Phosphoryl chloride (1.6 g, 10 mmol) was added to 4-methyloxazolidine-2,5-dione (1.2 g, 10 mmol) in 50 ml of acetonitrile, and then the reaction solution was ice-cooled. Subsequently, sodium hydride (0.3 g, 13 mmol) was added little by little to the reaction solution, and the mixture was heated to room temperature and then stirred for one hour. The obtained suspension was filtered, and the filtrate was concentrated under reduced pressure to obtain N-dichlorophosphoryl-4-methyloxazolidine-2,5-dione as an intermediate. Next, after 50 ml of acetonitrile was added, potassium fluoride (1.4 g, 24 mmol) was added, and the mixture was stirred at room temperature for 23 hours. The obtained suspension was filtered, and the filtrate was concentrated under reduced pressure to obtain a target compound (5c) (1.3 g, yield 66%).

$^{1}H$ NMR($CD_3CN$)σ1.53, 4.96ppm
$^{19}F$ NMR($CD_3CN$)σ-71.8, -68.9ppm

Synthesis Example 5-4 (Synthesis Example of Compound (5d) Represented by General Formula (5))

[Chem. 106]

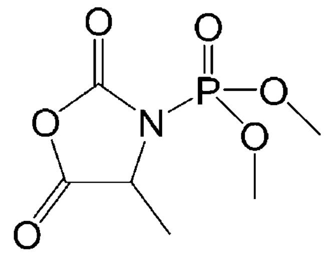

(5 d)

Dimethyl chlorophosphate (1.6 g, 11 mmol) was added to 4-methyloxazolidine-2,5-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 14 hours. The obtained suspension was filtered, and the filtrate was concentrated under reduced pressure to obtain a target compound (1 m) as a white solid (2.0 g, yield 86%).

$^1$H NMR($CD_3CN$)σ1.59, 4.29, 4.79ppm

Synthesis Example 6-1 (Synthesis Example of Compound (6a) Represented by General Formula (6))

[Chem. 107]

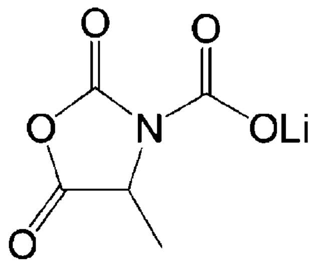

(6a)

After methyl chloroformate (1.1 g, 1.2 mmol) was added to 4-methyloxazolidine-2,5-dione (1.2 g, 10 mmol) in 50 ml of acetonitrile, sodium hydride (0.3 g, 13 mmol) was added little by little at room temperature, and the mixture was stirred for one hour. The obtained suspension was filtered, and the filtrate was concentrated under reduced pressure to obtain N-methoxycarbonyl-4-methyloxazolidine-2,5-dione as an intermediate. Next, 50 ml of acetonitrile and lithium iodide (1.3 g, 10 mmol) were added, and the mixture was heated to 50°C and stirred for 20 hours. The reaction solution was concentrated under reduced pressure to obtain a target compound (6a) (1.2 g, yield 70%).

$^1$H NMR($CD_3CN$)σ1.63, 5.28ppm

Synthesis Example 6-2 (Synthesis Example of Compound (6b) Represented by General Formula (6))

[Chem. 108]

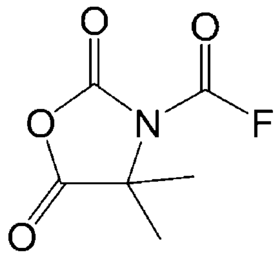

(6 b)

Triphosgene (1.5 g, 5.1 mmol) was added to 4,4-dimethyloxazolidine-2,5-dione (1.3 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 20 hours. Sodium fluoride (0.6 g, 14 mmol) was added to the obtained suspension, and then the mixture was stirred at room temperature for 15 hours. The obtained suspension was filtered, and the filtrate was concentrated under reduced pressure to obtain a target compound (6b) as a white solid (1.0 g, yield 57%).

$^{1}H$ NMR($CD_3CN$)σ1.51ppm
$^{19}F$ NMR($CD_3CN$)σ-62.3ppm
To

Synthesis Example 6-3 (Synthesis Example of Compound (6c) Represented by General Formula (6))

[Chem. 109]

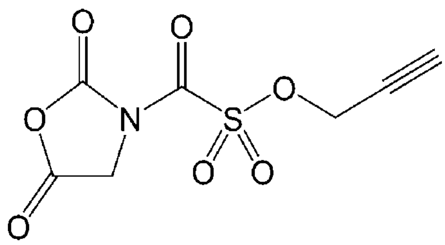

(6 c)

Triphosgene (1.5 g, 5.1 mmol) was added to oxazolidine-2,5-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 19 hours. The obtained suspension was filtered, and the filtrate was concentrated under reduced pressure to obtain N-chlorocarbonyl-oxazolidine-2,5-dione as an intermediate. Next, after 50 ml of acetonitrile was added, the mixture was ice-cooled, and sodium hydride (0.3 g, 13 mmol) was added. Hydrogen sulfide (0.3 g, 10 mmol) was added to the reaction solution, and the mixture was heated to room temperature and then stirred for 12 hours. The obtained suspension was filtered, and the filtrate was concentrated under reduced pressure to obtain N-thiolcarbonyl-oxazolidine-2,5-dione as an intermediate. Next, after 30 ml of water was added, potassium peroxymonosulfate (3.8 g, 25 mmol) and potassium chloride (0.7 g, 10 mmol) were added, and the mixture was stirred at room temperature for three hours. The obtained reaction solution was washed with 30 ml of ethyl acetate added thereto, and the organic layer was dried with anhydrous sodium sulfate added thereto and filtered. The obtained filtrate was concentrated under reduced pressure to obtain N-chlorosulfonylcarbonyl-oxazolidine-2,5-dione as an intermediate. Thereafter, after adding 50 ml of acetonitrile and ice cooling, propargyl alcohol (0.6 g, 10 mmol) was added, and sodium hydride (0.3 g, 13 mmol) was added. The mixture was heated to room temperature and then stirred for 20 hours. The obtained suspension was filtered, and the filtrate was concentrated under reduced pressure to obtain a target compound (6c) (0.3 g, yield 12%).

$^{1}H$ NMR($CD_3CN$)σ2.96, 4.57, 4.94ppm

Synthesis Example 6-4 (Synthesis Example of Compound (6d) Represented by General Formula (6))

[Chem. 110]

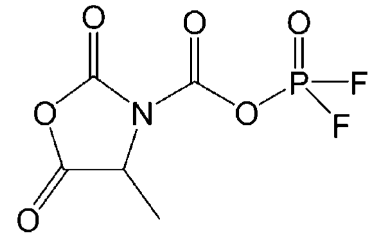

(6 d)

Triphosgene (1.5 g, 5.1 mmol) was added to 4-methyloxazolidine-2,5-dione (1.2 g, 10 mmol) in 50 ml of acetonitrile, and then the mixture solution was ice-cooled. Subsequently, sodium hydride (0.3 g, 13 mmol) was added to the reaction solution, and the mixture was heated to room temperature and then stirred for 19 hours. The obtained suspension was filtered, and the filtrate was concentrated under reduced pressure to obtain N-chlorocarbonyl-4-methyloxazolidine-2,5-dione as an intermediate. Next, after adding 50 ml of acetonitrile and ice cooling, difluorophosphoric acid (1.0 g, 10 mmol) and sodium hydride (0.3 g, 13 mmol) were added to the reaction solution, and the mixture was heated to room temperature and then stirred for 18 hours. The obtained suspension was filtered and concentrated under reduced pressure to obtain a target compound (6d) (1.1 g, yield 45%).

$^{1}H$ NMR($CD_3CN$)σ1.65, 5.31ppm
$^{19}F$ NMR($CD_3CN$)σ-73.2, -70.3ppm

Synthesis Example 7-1 (Synthesis Example of Compound (7a) Represented by General Formula (7))

[Chem. 111]

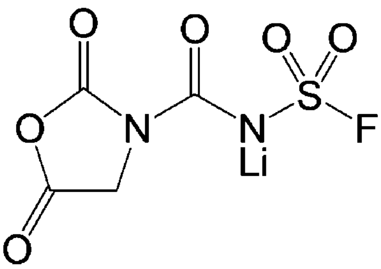

(7a)

After fluorosulfonyl isocyanate (1.4 g, 11 mmol) was added to oxazolidine-2,5-dione (1.0 g, 10 mmol) in 50 ml of acetonitrile, lithium hydride (0.1 g, 13 mmol) was added little by little at room temperature, and the mixture was stirred for three hours. The obtained suspension was filtered, and the filtrate was concentrated under reduced pressure to obtain a target compound (7a) (2.0 g, yield 87%).

$^{1}H$ NMR($CD_3CN$)σ4.82ppm
$^{19}F$ NMR($CD_3CN$)σ50.4ppm

Synthesis Example 7-2 (Synthesis Example of Compound (7b) Represented by General Formula (7))

[Chem. 112]

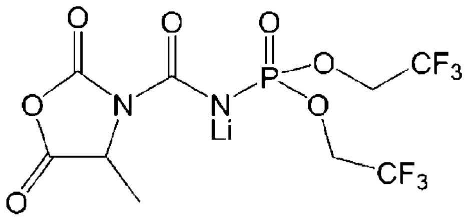

(7 b)

After bis(2,2,2-trifluoroethyl)phosphoryl isocyanate (3.1 g, 11 mmol) was added to 4-methyloxazolidine-2,5-dione (1.2 g, 10 mmol) in 50 ml of acetonitrile, lithium hydride (0.1 g, 13 mmol) was added little by little at room temperature, and the mixture was stirred for three hours. The obtained suspension was filtered, and the filtrate was concentrated under reduced pressure to obtain a target compound (7b) (3.0 g, yield 71%).

$^{1}$H NMR($CD_3CN$)σ1.55, 4.27, 5.09ppm
$^{19}$F NMR($CD_3CN$)σ-81.4ppm

Synthesis Example 7-3 (Synthesis Example of Compound (7c) Represented by General Formula (7))

[Chem. 113]

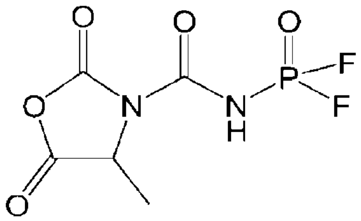

(7 c)

Difluorophosphoryl isocyanate (1.4 g, 11 mmol) was added to 4-methyloxazolidine-2,5-dione (1.2 g, 10 mmol) in 50 ml of acetonitrile, and the mixture was stirred for three hours. The obtained suspension was filtered, and the filtrate was concentrated under reduced pressure to obtain a target compound (7c) (2.0 g, yield 86%).

$^{1}$H NMR($CD_3CN$)σ1.61, 5.23ppm
$^{19}$F NMR($CD_3CN$)σ-74.3, -71.4ppm

Synthesis Example 8-1 (Synthesis Example of Compound (8a) Represented by General Formula (8))

[Chem. 114]

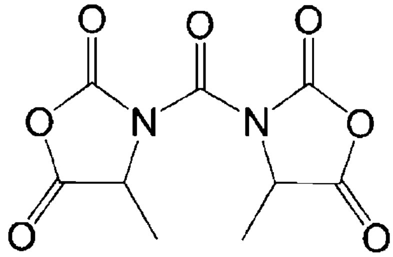

(8a)

After triphosgene (1.5 g, 5.1 mmol) was added to 4-methyloxazolidine-2,5-dione (1.2 g, 10 mmol) in 50 ml of acetonitrile, sodium hydride (0.3 g, 13 mmol) was added little by little at room temperature, and the mixture was stirred for 21 hours. The obtained suspension was filtered, and the filtrate was concentrated under reduced pressure to obtain a target compound (8a) (1.1 g, yield 82%).

$^{1}H$ NMR($CD_3CN$)σ1.62, 5.22ppm

Synthesis Example 8-2 (Synthesis Example of Compound (8b) Represented by General Formula (8))

[Chem. 115]

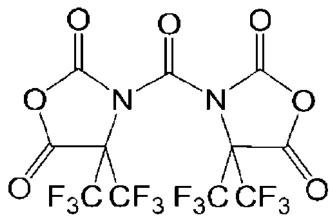

(8b)

A compound (8b) was synthesized in the same manner as in the synthesis example of the compound (8a) except that 10 mmol of 4,4-ditrifluoromethyloxazolidine-2,5-dione was used instead of 10 mmol of 4-methyloxazolidine-2,5-dione (1.2 g, yield 78%).

$^{19}F$ NMR($CD_3CN$)σ-83.2ppm

Comparative Example Compound:

In the following Examples and Comparative Examples, the following compounds were adopted as control compounds.

[Chem. 116]

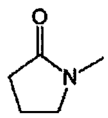

Comparative Example Compound 1

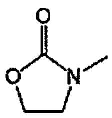

Comparative Example Compound 2

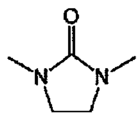

Comparative Example Compound 3

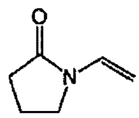

Comparative Example Compound 4

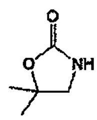

Comparative Example Compound 5

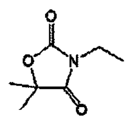

Comparative Example Compound 6

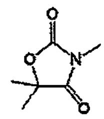

Comparative Example Compound 7

Here, Comparative Example Compound 1 corresponds to the compound used in Example 1 of PTL 1 or Example

23 of PTL 2, Comparative Example Compound 2 corresponds to the compound used in Example 2 of PTL 1 or Example 24 of PTL 2, Comparative Example Compound 3 corresponds to the compound used in Example 3 of PTL 1 or Example 25 of PTL 2, Comparative Example Compound 4 corresponds to the compound used in Example 4 of PTL 1 or Example 26 of PTL 2, Comparative Example Compound 5 corresponds to the compound used in Examples 1 to 3 of PTL 3, Comparative Example Compound 6 corresponds to the compound used in Example 5 of PTL 3, and Comparative Example Compound 7 corresponds to the compound used in Example 4 of PTL 3.

[Preparation of Nonaqueous Electrolyte Solutions of Examples and Comparative Examples]

<Comparative Example 1-1> (Preparation of Comparative Nonaqueous Electrolyte Solution 0 as Base)

As a nonaqueous organic solvent, a solvent obtained by mixing EC, DMC, and EMC at a volume ratio of EC: DMC: EMC = 2.5: 3.5: 4 was used, and $LiPF_6$ as a solute was dissolved in the solvent in such a manner that the content in the nonaqueous electrolyte solution became 1.00 mol/L. Thus, a comparative nonaqueous electrolyte solution 0 was prepared.

<Examples 1-1 to 1-15>

Nonaqueous electrolyte solutions according to the Examples were prepared by the same procedures as those of the comparative nonaqueous electrolyte solution 0 except that the compounds shown in Table 1 were dissolved as the component (III) at the contents shown in Table 1.

<Comparative Examples 1-2 to 1-8>

Comparative nonaqueous electrolyte solutions 1 to 7 were prepared in the same manner as in Example 1-1 except that Comparative Example Compounds 1 to 7 shown in Table 1 were dissolved instead of the component (III) at the contents shown in Table 1.

<Preparation of Nonaqueous Electrolyte Solution Secondary Battery>

(Production of NCM811 Positive Electrode)

In 92.0% by mass of $LiNi_{0.8}Mn_{0.1}Co_{0.1}O_2$ powder, 3.5% by mass of polyvinylidene fluoride (hereinafter also referred to as "PVDF") as a binder and 4.5% by mass of acetylene black as a conductive material were mixed, and 45% by mass of N-methyl-2-pyrrolidone (hereinafter also referred to as "NMP") with respect to the total mass of the $LiNi_{0.8}Mn_{0.1}Co_{0.1}O_2$ powder, the binder, and the conductive material was added to produce a positive electrode mixture paste. The paste was applied onto both sides of an aluminum foil (A1085), dried, pressed and then punched into 4 cm × 5 cm to obtain an NCM811 positive electrode for testing in which a positive electrode active material layer was formed on a current collector.

(Production of Graphite Negative Electrode)

In 97% by mass of natural graphite powder, 2% by mass of styrene-butadiene rubber (hereinafter also referred to as "SBR"), 1% by mass of sodium carboxymethyl cellulose (hereinafter also referred to as "CMC"), and water were mixed to produce a negative electrode mixture paste. The paste was applied onto a copper foil, dried, pressed and then punched into 4.5 cm × 5.5 cm to obtain a graphite negative electrode for testing in which a negative electrode active material layer was formed on a current collector.

(Production of Silicon-Containing Graphite (also referred to as "Si-Graphite") Negative Electrode)

In 85% by mass of artificial graphite powder, 7% by mass of nanosilicon, 3% by mass of a conductive material (HS-100 manufactured by Denka Company Limited), 2% by mass of carbon nanofiber (VGCF manufactured by Showa Denko K.K.), 2% by mass of SBR, 1% by mass of CMC, and water were mixed to produce a negative electrode mixture paste. The paste was applied onto a copper foil, dried, pressed and then punched into 4.5 cm × 5.5 cm to obtain a silicon-containing graphite negative electrode for testing in which a negative electrode active material layer was formed on a current collector.

(Production of Nonaqueous Electrolyte Solution Battery)

Under an argon atmosphere at a dew point of -50°C or lower, a terminal was welded to the above NCM811 positive electrode, and both sides of the welded product were then sandwiched between two polyethylene separators (5 cm × 6 cm). Further, the outside of the sandwiched product was sandwiched between two graphite negative electrodes or silicon-containing graphite negative electrodes to which a terminal had been welded in advance in such a manner that the surface of the negative electrode active material faced the surface of the positive electrode active material. The resultant product was put in an aluminum laminated bag having an opening on one side, and after the nonaqueous electrolyte solution prepared above was vacuum-injected into the bag, the opening was sealed with heat. In this manner, the aluminum laminated nonaqueous electrolyte solution batteries according to the Examples and the Comparative Examples shown in the tables below were produced.

[Evaluation Conditions]

(Initial Charge and Discharge)

Each nonaqueous electrolyte solution battery (cell) produced as described above was left to stand at an ambient temperature of 25°C for 12 hours (impregnation time: 12 hours) and then conditioned at an ambient temperature of 25°C under the following conditions. That is, as initial charge and discharge, a charge and discharge cycle including constant-current constant-voltage charging at an upper limit charge voltage of 4.3 V and at 0.1 C rate (9 mA), discharging at 0.2 C rate constant current to a discharge end voltage of 2.7 V, subsequent constant-current constant-voltage charging at an upper limit charge voltage of 4.3 V and at 0.2 C rate, and discharging at 0.2 C rate constant current to a discharge end voltage of 2.7 V was repeated three times.

(Initial Resistance Measurement)

The cell subjected to the conditioning was charged to 4.3 V at 25°C and 0.2 C rate, and then impedance measurement was performed in an environment at -20°C to measure the resistance value.

(Cycle Test)

The cell after the measurement of the initial resistance was subjected to a charge and discharge test at an ambient temperature of 25°C to evaluate the cycle characteristic. A charge and discharge cycle was repeated at a current value of 90 mA by a constant-current constant-voltage method at an upper limit charge voltage of 4.3 V and a lower limit discharge voltage of 2.7 V. Then, the discharge capacity retention rate at the 400th cycle in the charge and discharge test at an ambient temperature of 25°C was calculated as follows to evaluate the degree of deterioration of the cell. The "discharge capacity retention rate after cycles" expressed as the discharge capacity retention rate at the 400th cycle was determined using the following equation. The discharge capacity at the first cycle in the charge and discharge test at the ambient temperature of 25°C was defined as the initial discharge capacity.

Discharge capacity retention rate (%) after cycles = (discharge capacity at 400th cycle/initial discharge capacity) × 100

(Evaluation of Gas Generation Amount)

Before and after the evaluation of the cycle characteristic, the volume of the cell was measured by Archimedes' method using silicone oil (silicone oil KF54 manufactured by Shin-Etsu Chemical Co., Ltd.), and the gas generation amount V (unit: $cm^3$) (gas generation amount V = volume V2 of cell after evaluation of cycle characteristic - volume V1 of cell before evaluation of cycle characteristic) was determined. Based on the gas generation amount V, the "cycle test gas generation amount" was evaluated.

For the nonaqueous electrolyte solutions 1-1, 1-2-1 to 1-2-5, and 1-3 to 1-15 and the comparative nonaqueous electrolyte solutions 0 to 7 prepared as described above, the initial resistance and the capacity retention rates in the cycle test were evaluated as described above, and the results are shown in Table 1 below. Here, these values are relative values, and the initial resistance and the capacity retention rate in the cycle test of the case using the comparative nonaqueous electrolyte solution 0 were regarded as 100 for the evaluation.

[Table 1]

Table 1

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 1-1 | Graphite | Nonaqueous electrolyte solution 1-1 | (1a) | 1.0 | 78.8 | 107.2 |
| Example 1-2-1 | | Nonaqueous electrolyte solution 1-2-1 | (1b) | 0.01 | 96.2 | 103.3 |
| Example 1-2-2 | | Nonaqueous electrolyte solution 1-2-2 | | 0.1 | 91.4 | 105.5 |
| Example 1-2-3 | | Nonaqueous electrolyte solution 1-2-3 | | 1.0 | 79.1 | 113.5 |
| Example 1-2-4 | | Nonaqueous electrolyte solution 1-2-4 | | 5.0 | 82.2 | 110.8 |
| Example 1-2-5 | | Nonaqueous electrolyte solution 1-2-5 | | 11.0 | 86.8 | 106.4 |
| Example 1-3 | | Nonaqueous electrolyte solution 1-3 | (1c) | 1.0 | 92.7 | 112.6 |
| Example 1-4 | | Nonaqueous electrolyte solution 1-4 | (1d) | | 77.6 | 114.1 |
| Example 1-5 | | Nonaqueous electrolyte solution 1-5 | (1e) | | 81.3 | 112.9 |
| Example 1-6 | | Nonaqueous electrolyte solution 1-6 | (1f) | 0.5 | 81.4 | 115.2 |
| Example 1-7 | | Nonaqueous electrolyte solution 1-7 | (1g) | 1.0 | 82.4 | 110.4 |
| Example 1-8 | | Nonaqueous electrolyte solution 1-8 | (1h) | 1.0 | 82.8 | 111.6 |
| Example 1-9 | | Nonaqueous electrolyte solution 1-9 | (1i) | 1.0 | 88.3 | 108.2 |
| Example 1-10 | | Nonaqueous electrolyte solution 1-10 | (1j) | 1.0 | 86.4 | 107.2 |
| Example 1-11 | | Nonaqueous electrolyte solution 1-11 | (1k) | 1.0 | 82.3 | 108.8 |
| Example 1-12 | | Nonaqueous electrolyte solution 1-12 | (1l) | 1.0 | 88.9 | 106.7 |
| Example 1-13 | | Nonaqueous electrolyte solution 1-13 | (1m) | 1.0 | 89.1 | 107.6 |
| Example 1-14 | | Nonaqueous electrolyte solution 1-14 | (1n) | 1.0 | 81.3 | 108.9 |

(continued)

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 1-15 | | Nonaqueous electrolyte solution 1-15 | (1o) | 1.0 | 82.7 | 109.3 |
| Comparative Example 1-1 | | Comparative nonaqueous electrolyte solution 0 | - | - | 100 | 100 |
| Comparative Example 1-2 | | Comparative nonaqueous electrolyte solution 1 | 1 | 1.0 | 102.1 | 99.7 |
| Comparative Example 1-3 | | Comparative nonaqueous electrolyte solution 2 | 2 | | 101.2 | 100.5 |
| Comparative Example 1-4 | | Comparative nonaqueous electrolyte solution 3 | 3 | | 104.7 | 98.7 |
| Comparative Example 1-5 | | Comparative nonaqueous electrolyte solution 4 | 4 | | 103.5 | 100.2 |
| Comparative Example 1-6 | | Comparative nonaqueous electrolyte solution 5 | 5 | | 99.5 | 97.9 |
| Comparative Example 1-7 | | Comparative nonaqueous electrolyte solution 6 | 6 | | 98.4 | 103.2 |
| Comparative Example 1-8 | | Comparative nonaqueous electrolyte solution 7 | 7 | | 98.3 | 102.9 |

From the results shown in Table 1, it is understood that when the compound represented by the general formula (1) is contained as the component (III) of the present disclosure, the nonaqueous electrolyte solution battery using graphite as the negative electrode active material exhibits excellent cycle characteristic and also has excellent initial resistance characteristic as compared with the cases using the additives disclosed in the related art.

<Comparative Example 2-1> (Preparation of Comparative Nonaqueous Electrolyte Solution 0 as Base)

This is the same as the comparative nonaqueous electrolyte solution 0 prepared in Comparative Example 1-1.

<Examples 2-1 to 2-6>

Nonaqueous electrolyte solutions according to the Examples were prepared by the same procedures as those of the comparative nonaqueous electrolyte solution 0 except that the compounds shown in Table 2 were dissolved as the component (III) at the contents shown in Table 2.

<Comparative Examples 2-2 to 2-8>

The comparative nonaqueous electrolyte solutions according to Comparative Examples 2-2 to 2-8 are the same as the comparative nonaqueous electrolyte solutions 1 to 7 prepared in Comparative Examples 1-2 to 1-8.

For the nonaqueous electrolyte solutions 2-1 to 2-6 and the comparative nonaqueous electrolyte solutions 0 to 7 prepared as described above, the initial resistance and the capacity retention rates in the cycle test were evaluated as described above, and the results are shown in Table 2 below. Here, these values are relative values, and the initial resistance and the capacity retention rate in the cycle test of the case using the comparative nonaqueous electrolyte solution 0 were regarded as 100 for the evaluation.

[Table 2]

Table 2

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 2-1 | Graphite | Nonaqueous electrolyte solution 2-1 | (2a) | 1.0 | 89.4 | 113.5 |
| Example 2-2 | | Nonaqueous electrolyte solution 2-2 | (2b) | | 84.3 | 107.3 |
| Example 2-3 | | Nonaqueous electrolyte solution 2-3 | (2c) | | 85.2 | 109.2 |
| Example 2-4 | | Nonaqueous electrolyte solution 2-4 | (2d) | | 86.2 | 108.8 |
| Example 2-5 | | Nonaqueous electrolyte solution 2-5 | (2e) | | 90.1 | 110.6 |
| Example 2-6 | | Nonaqueous electrolyte solution 2-6 | (2f) | | 88.6 | 111.1 |
| Comparative Example 2-1 | | Comparative nonaqueous electrolyte solution 0 | - | - | 100 | 100 |
| Comparative Example 2-2 | | Comparative nonaqueous electrolyte solution 1 | 1 | 1.0 | 102.1 | 99.7 |
| Comparative Example 2-3 | | Comparative nonaqueous electrolyte solution 2 | 2 | | 101.2 | 100.5 |
| Comparative Example 2-4 | | Comparative nonaqueous electrolyte solution 3 | 3 | | 104.7 | 98.7 |
| Comparative Example 2-5 | | Comparative nonaqueous electrolyte solution 4 | 4 | | 103.5 | 100.2 |
| Comparative Example 2-6 | | Comparative nonaqueous electrolyte solution 5 | 5 | | 99.5 | 97.9 |
| Comparative Example 2-7 | | Comparative nonaqueous electrolyte solution 6 | 6 | | 98.4 | 103.2 |
| Comparative Example 2-8 | | Comparative nonaqueous electrolyte solution 7 | 7 | | 98.3 | 102.9 |

From the results shown in Table 2, it is understood that when the compound represented by the general formula (2) is contained as the component (III) of the present disclosure, the nonaqueous electrolyte solution battery using graphite

as the negative electrode active material exhibits excellent cycle characteristic and also has excellent initial resistance characteristic as compared with the cases using the additives disclosed in the related art.

<Comparative Example 3-1> (Preparation of Comparative Nonaqueous Electrolyte Solution 0 as Base)

This is the same as the comparative nonaqueous electrolyte solution 0 prepared in Comparative Example 1-1.

<Examples 3-1-1 to 3-1-5 and 3-2 to 3-6>

Nonaqueous electrolyte solutions according to the Examples were prepared by the same procedures as those of the comparative nonaqueous electrolyte solution 0 except that the compounds shown in Table 3 were dissolved as the component (III) at the contents shown in Table 3.

<Comparative Examples 3-2 to 3-8>

The comparative nonaqueous electrolyte solutions according to Comparative Examples 3-2 to 3-8 are the same as the comparative nonaqueous electrolyte solutions 1 to 7 prepared in Comparative Examples 1-2 to 1-8.

For the nonaqueous electrolyte solutions 3-1-1 to 3-1-5 and 3-2 to 3-6 and the comparative nonaqueous electrolyte solutions 0 to 7 prepared as described above, the initial resistance and the capacity retention rates in the cycle test were evaluated as described above, and the results are shown in Table 3 below. Here, these values are relative values, and the initial resistance and the capacity retention rate in the cycle test of the case using the comparative nonaqueous electrolyte solution 0 were regarded as 100 for the evaluation.

[Table 3]

Table 3

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 3-1-1 | | Nonaqueous electrolyte solution 3-1-1 | (3a) | 0.01 | 94.4 | 104.4 |
| Example 3-1-2 | | Nonaqueous electrolyte solution 3-1-2 | | 0.1 | 88.8 | 108.8 |
| Example 3-1-3 | | Nonaqueous electrolyte solution 3-1-3 | | 1.0 | 76.7 | 110.9 |
| Example 3-1-4 | | Nonaqueous electrolyte solution 3-1-4 | | 5.0 | 80.6 | 109.4 |
| Example 3-1-5 | | Nonaqueous electrolyte solution 3-1-5 | | 11.0 | 84.6 | 108.8 |

(continued)

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 3-2 | Graphite | Nonaqueous electrolyte solution 3-2 | (3b) | 1.0 | 88.2 | 107.3 |
| Example 3-3 | | Nonaqueous electrolyte solution 3-3 | (3c) | | 85.2 | 110.1 |
| Example 3-4 | | Nonaqueous electrolyte solution 3-4 | (3d) | | 84.2 | 109.9 |
| Example 3-5 | | Nonaqueous electrolyte solution 3-5 | (3e) | | 86.2 | 108.1 |
| Example 3-6 | | Nonaqueous electrolyte solution 3-6 | (3f) | | 88.1 | 107.9 |
| Comparative Example 3-1 | | Comparative nonaqueous electrolyte solution 0 | - | - | 100 | 100 |
| Comparative Example 3-2 | | Comparative nonaqueous electrolyte solution 1 | 1 | 1.0 | 102.1 | 99.7 |
| Comparative Example 3-3 | | Comparative nonaqueous electrolyte solution 2 | 2 | | 101.2 | 100.5 |
| Comparative Example 3-4 | | Comparative nonaqueous electrolyte solution 3 | 3 | | 104.7 | 98.7 |
| Comparative Example 3-5 | | Comparative nonaqueous electrolyte solution 4 | 4 | | 103.5 | 100.2 |
| Comparative Example 3-6 | | Comparative nonaqueous electrolyte solution 5 | 5 | | 99.5 | 97.9 |
| Comparative Example 3-7 | | Comparative nonaqueous electrolyte solution 6 | 6 | | 98.4 | 103.2 |
| Comparative Example 3-8 | | Comparative nonaqueous electrolyte solution 7 | 7 | | 98.3 | 102.9 |

From the results shown in Table 3, it is understood that when the compound represented by the general formula (3) is contained as the component (III) of the present disclosure, the nonaqueous electrolyte solution battery using graphite as the negative electrode active material exhibits excellent cycle characteristic and also has excellent initial resistance characteristic as compared with the cases using the additives disclosed in the related art.

<Comparative Example 4-1> (Preparation of Comparative Nonaqueous Electrolyte Solution 0 as Base)

This is the same as the comparative nonaqueous electrolyte solution 0 prepared in Comparative Example 1-1.

<Examples 4-1, 4-2-1 to 4-2-5, 4-3, and 4-4>

Nonaqueous electrolyte solutions according to the Examples were prepared by the same procedures as those of the comparative nonaqueous electrolyte solution 0 except that the compounds shown in Table 4 were dissolved as the component (III) at the contents shown in Table 4.

<Comparative Examples 4-2 to 4-8>

The comparative nonaqueous electrolyte solutions according to Comparative Examples 4-2 to 4-8 are the same as the comparative nonaqueous electrolyte solutions 1 to 7 prepared in Comparative Examples 1-2 to 1-8.

For the nonaqueous electrolyte solutions 4-1, 4-2-1 to 4-2-5, 4-3, and 4-4 and the comparative nonaqueous electrolyte solutions 0 to 7 prepared as described above, the initial resistance and the capacity retention rates in the cycle test were evaluated as described above, and the results are shown in Table 4 below. Here, these values are relative values, and the initial resistance and the capacity retention rate in the cycle test of the case using the comparative nonaqueous electrolyte solution 0 were regarded as 100 for the evaluation.

[Table 4]

Table 4

<table>
<tr><th rowspan="2"></th><th rowspan="2">Negative Electrode Active Material</th><th rowspan="2">Nonaqueous Electrolyte Solution</th><th colspan="2">Component (III) or Comparative Example Compound</th><th rowspan="2">Initial Resistance (relative value)</th><th rowspan="2">Cycle Discharge Capacity Retention Rate (relative value)</th></tr>
<tr><th>Type</th><th>Content [% by mass]</th></tr>
<tr><td>Example 4-1</td><td rowspan="9">Graphite</td><td>Nonaqueous electrolyte solution 4-1</td><td>(4a)</td><td>1.0</td><td>92.3</td><td>113.4</td></tr>
<tr><td>Example 4-2-1</td><td>Nonaqueous electrolyte solution 4-2-1</td><td rowspan="5">(4b)</td><td>0.01</td><td>96.8</td><td>105.1</td></tr>
<tr><td>Example 4-2-2</td><td>Nonaqueous electrolyte solution 4-2-2</td><td>0.1</td><td>92.4</td><td>108.2</td></tr>
<tr><td>Example 4-2-3</td><td>Nonaqueous electrolyte solution 4-2-3</td><td>1.0</td><td>89.1</td><td>116.8</td></tr>
<tr><td>Example 4-2-4</td><td>Nonaqueous electrolyte solution 4-2-4</td><td>5.0</td><td>90.8</td><td>113.1</td></tr>
<tr><td>Example 4-2-5</td><td>Nonaqueous electrolyte solution 4-2-5</td><td>11.0</td><td>94.9</td><td>109.2</td></tr>
<tr><td>Example 4-3</td><td>Nonaqueous electrolyte solution 4-3</td><td>(4c)</td><td rowspan="2">1.0</td><td>88.0</td><td>109.9</td></tr>
<tr><td>Example 4-4</td><td>Nonaqueous electrolyte solution 4-4</td><td>(4d)</td><td>88.2</td><td>111.6</td></tr>
<tr><td>Comparative Example 4-1</td><td>Comparative nonaqueous electrolyte solution 0</td><td>-</td><td>-</td><td>100</td><td>100</td></tr>
</table>

(continued)

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Comparative Example 4-2 | | Comparative nonaqueous electrolyte solution 1 | 1 | 1.0 | 102.1 | 99.7 |
| Comparative Example 4-3 | | Comparative nonaqueous electrolyte solution 2 | 2 | | 101.2 | 100.5 |
| Comparative Example 4-4 | | Comparative nonaqueous electrolyte solution 3 | 3 | | 104.7 | 98.7 |
| Comparative Example 4-5 | | Comparative nonaqueous electrolyte solution 4 | 4 | | 103.5 | 100.2 |
| Comparative Example 4-6 | | Comparative nonaqueous electrolyte solution 5 | 5 | | 99.5 | 97.9 |
| Comparative Example 4-7 | | Comparative nonaqueous electrolyte solution 6 | 6 | | 98.4 | 103.2 |
| Comparative Example 4-8 | | Comparative nonaqueous electrolyte solution 7 | 7 | | 98.3 | 102.9 |

From the results shown in Table 4, it is understood that when the compound represented by the general formula (4) is contained as the component (III) of the present disclosure, the nonaqueous electrolyte solution battery using graphite as the negative electrode active material exhibits excellent cycle characteristic and also has excellent initial resistance characteristic as compared with the cases using the additives disclosed in the related art.

<Comparative Example 5-1> (Preparation of Comparative Nonaqueous Electrolyte Solution 0 as Base)

This is the same as the comparative nonaqueous electrolyte solution 0 prepared in Comparative Example 1-1.

<Examples 5-1-1 to 5-1-5 and 5-2 to 5-4>

Nonaqueous electrolyte solutions according to the Examples were prepared by the same procedures as those of the comparative nonaqueous electrolyte solution 0 except that the compounds shown in Table 5 were dissolved as the component (III) at the contents shown in Table 5.

<Comparative Examples 5-2 to 5-8>

The comparative nonaqueous electrolyte solutions according to Comparative Examples 5-2 to 5-8 are the same as the comparative nonaqueous electrolyte solutions 1 to 7 prepared in Comparative Examples 1-2 to 1-8.

For the nonaqueous electrolyte solutions 5-1-1 to 5-1-5 and 5-2 to 5-4 and the comparative nonaqueous electrolyte solutions 0 to 7 prepared as described above, the initial resistance and the capacity retention rates in the cycle test were evaluated as described above, and the results are shown in Table 5 below. Here, these values are relative values, and the initial resistance and the capacity retention rate in the cycle test of the case using the comparative nonaqueous electrolyte solution 0 were regarded as 100 for the evaluation.

[Table 5]

Table 5

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 5-1-1 | Graphite | Nonaqueous electrolyte solution 5-1-1 | (5a) | 0.01 | 95.8 | 102.3 |
| Example 5-1-2 | | Nonaqueous electrolyte solution 5-1-2 | | 0.1 | 90.4 | 103.5 |
| Example 5-1-3 | | Nonaqueous electrolyte solution 5-1-3 | | 1.0 | 82.1 | 107.6 |
| Example 5-1-4 | | Nonaqueous electrolyte solution 5-1-4 | | 5.0 | 85.9 | 104.9 |
| Example 5-1-5 | | Nonaqueous electrolyte solution 5-1-5 | | 11.0 | 96.7 | 104.1 |
| Example 5-2 | | Nonaqueous electrolyte solution 5-2 | (5b) | 1.0 | 81.6 | 106.9 |
| Example 5-3 | | Nonaqueous electrolyte solution 5-3 | (5c) | | 83.8 | 106.3 |
| Example 5-4 | | Nonaqueous electrolyte solution 5-4 | (5d) | | 88.9 | 105.4 |
| Comparative Example 5-1 | | Comparative nonaqueous electrolyte solution 0 | - | - | 100 | 100 |
| Comparative Example 5-2 | | Comparative nonaqueous electrolyte solution 1 | 1 | 1.0 | 102.1 | 99.7 |
| Comparative Example 5-3 | | Comparative nonaqueous electrolyte solution 2 | 2 | | 101.2 | 100.5 |
| Comparative Example 5-4 | | Comparative nonaqueous electrolyte solution 3 | 3 | | 104.7 | 98.7 |
| Comparative Example 5-5 | | Comparative nonaqueous electrolyte solution 4 | 4 | | 103.5 | 100.2 |
| Comparative Example 5-6 | | Comparative nonaqueous electrolyte solution 5 | 5 | | 99.5 | 97.9 |
| Comparative Example 5-7 | | Comparative nonaqueous electrolyte solution 6 | 6 | | 98.4 | 103.2 |
| Comparative Example 5-8 | | Comparative nonaqueous electrolyte solution 7 | 7 | | 98.3 | 102.9 |

From the results shown in Table 5, it is understood that when the compound represented by the general formula (5) is contained as the component (III) of the present disclosure, the nonaqueous electrolyte solution battery using graphite as the negative electrode active material exhibits excellent cycle characteristic and also has excellent initial resistance characteristic as compared with the cases using the additives disclosed in the related art.

<Comparative Example 6-1> (Preparation of Comparative Nonaqueous Electrolyte Solution 0 as Base)

This is the same as the comparative nonaqueous electrolyte solution 0 prepared in Comparative Example 1-1.

<Examples 6-1 to 6-4>

Nonaqueous electrolyte solutions according to the Examples were prepared by the same procedures as those of the comparative nonaqueous electrolyte solution 0 except that the compounds shown in Table 6 were dissolved as the component (III) at the contents shown in Table 6.

<Comparative Examples 6-2 to 6-8>

The comparative nonaqueous electrolyte solutions according to Comparative Examples 6-2 to 6-8 are the same as the comparative nonaqueous electrolyte solutions 1 to 7 prepared in Comparative Examples 1-2 to 1-8.

For the nonaqueous electrolyte solutions 6-1 to 6-4 and the comparative nonaqueous electrolyte solutions 0 to 7 prepared as described above, the initial resistance and the capacity retention rates in the cycle test were evaluated as described above, and the results are shown in Table 6 below. Here, these values are relative values, and the initial resistance and the capacity retention rate in the cycle test of the case using the comparative nonaqueous electrolyte solution 0 were regarded as 100 for the evaluation.

[Table 6]

Table 6

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 6-1 | | Nonaqueous electrolyte solution 6-1 | (6a) | 1.0 | 92.3 | 107.6 |
| Example 6-2 | | Nonaqueous electrolyte solution 6-2 | (6b) | | 93.2 | 107.2 |
| Example 6-3 | | Nonaqueous electrolyte solution 6-3 | (6c) | | 92.1 | 108.1 |
| Example 6-4 | | Nonaqueous electrolyte solution 6-4 | (6d) | | 89.2 | 108.0 |
| Comparative Example 6-1 | | Comparative nonaqueous electrolyte solution 0 | - | - | 100 | 100 |

(continued)

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Comparative Example 6-2 | Graphite | Comparative nonaqueous electrolyte solution 1 | 1 | 1.0 | 102.1 | 99.7 |
| Comparative Example 6-3 | | Comparative nonaqueous electrolyte solution 2 | 2 | | 101.2 | 100.5 |
| Comparative Example 6-4 | | Comparative nonaqueous electrolyte solution 3 | 3 | | 104.7 | 98.7 |
| Comparative Example 6-5 | | Comparative nonaqueous electrolyte solution 4 | 4 | | 103.5 | 100.2 |
| Comparative Example 6-6 | | Comparative nonaqueous electrolyte solution 5 | 5 | | 99.5 | 97.9 |
| Comparative Example 6-7 | | Comparative nonaqueous electrolyte solution 6 | 6 | | 98.4 | 103.2 |
| Comparative Example 6-8 | | Comparative nonaqueous electrolyte solution 7 | 7 | | 98.3 | 102.9 |

From the results shown in Table 6, it is understood that when the compound represented by the general formula (6) is contained as the component (III) of the present disclosure, the nonaqueous electrolyte solution battery using graphite as the negative electrode active material exhibits excellent cycle characteristic and also has excellent initial resistance characteristic as compared with the cases using the additives disclosed in the related art.

<Comparative Example 7-1> (Preparation of Comparative Nonaqueous Electrolyte Solution 0 as Base)

This is the same as the comparative nonaqueous electrolyte solution 0 prepared in Comparative Example 1-1.

<Examples 7-1 to 7-3>

Nonaqueous electrolyte solutions according to the Examples were prepared by the same procedures as those of the comparative nonaqueous electrolyte solution 0 except that the compounds shown in Table 7 were dissolved as the component (III) at the contents shown in Table 7.

<Comparative Examples 7-2 to 7-8>

The comparative nonaqueous electrolyte solutions according to Comparative Examples 7-2 to 7-8 are the same as the comparative nonaqueous electrolyte solutions 1 to 7 prepared in Comparative Examples 1-2 to 1-8.

For the nonaqueous electrolyte solutions 7-1 to 7-3 and the comparative nonaqueous electrolyte solutions 0 to 7 prepared as described above, the initial resistance and the capacity retention rates in the cycle test were evaluated as described above, and the results are shown in Table 7 below. Here, these values are relative values, and the initial resistance and the capacity retention rate in the cycle test of the case using the comparative nonaqueous electrolyte solution 0 were regarded as 100 for the evaluation.

[Table 7]

Table 7

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 7-1 | Graphite | Nonaqueous electrolyte solution 7-1 | (7a) | 1.0 | 85.4 | 104.9 |
| Example 7-2 | | Nonaqueous electrolyte solution 7-2 | (7b) | | 89.6 | 105.2 |
| Example 7-3 | | Nonaqueous electrolyte solution 7-3 | (7c) | | 87.2 | 104.7 |
| Comparative Example 7-1 | | Comparative nonaqueous electrolyte solution 0 | - | - | 100 | 100 |
| Comparative Example 7-2 | | Comparative nonaqueous electrolyte solution 1 | 1 | 1.0 | 102.1 | 99.7 |
| Comparative Example 7-3 | | Comparative nonaqueous electrolyte solution 2 | 2 | | 101.2 | 100.5 |
| Comparative Example 7-4 | | Comparative nonaqueous electrolyte solution 3 | 3 | | 104.7 | 98.7 |
| Comparative Example 7-5 | | Comparative nonaqueous electrolyte solution 4 | 4 | | 103.5 | 100.2 |
| Comparative Example 7-6 | | Comparative nonaqueous electrolyte solution 5 | 5 | | 99.5 | 97.9 |
| Comparative Example 7-7 | | Comparative nonaqueous electrolyte solution 6 | 6 | | 98.4 | 103.2 |
| Comparative Example 7-8 | | Comparative nonaqueous electrolyte solution 7 | 7 | | 98.3 | 102.9 |

From the results shown in Table 7, it is understood that when the compound represented by the general formula (7) is contained as the component (III) of the present disclosure, the nonaqueous electrolyte solution battery using graphite as the negative electrode active material exhibits excellent cycle characteristic and also has excellent initial resistance characteristic as compared with the cases using the additives disclosed in the related art.

<Comparative Example 8-1> (Preparation of Comparative Nonaqueous Electrolyte Solution 0 as Base)

This is the same as the comparative nonaqueous electrolyte solution 0 prepared in Comparative Example 1-1.

<Examples 8-1-1 to 8-1-5 and 8-2>

Nonaqueous electrolyte solutions according to the Examples were prepared by the same procedures as those of the comparative nonaqueous electrolyte solution 0 except that the compounds shown in Table 8 were dissolved as the component (III) at the contents shown in Table 8.

<Comparative Examples 8-2 to 8-8>

The comparative nonaqueous electrolyte solutions according to Comparative Examples 8-2 to 8-8 are the same as the comparative nonaqueous electrolyte solutions 1 to 7 prepared in Comparative Examples 1-2 to 1-8.

For the nonaqueous electrolyte solutions 8-1-1 to 8-1-5 and 8-2 and the comparative nonaqueous electrolyte solutions 0 to 7 prepared as described above, the initial resistance and the capacity retention rates in the cycle test were evaluated as described above, and the results are shown in Table 8 below. Here, these values are relative values, and the initial resistance and the capacity retention rate in the cycle test of the case using the comparative nonaqueous electrolyte solution 0 were regarded as 100 for the evaluation.

[Table 8]

Table 8

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 8-1-1 | | Nonaqueous electrolyte solution 8-1-1 | (8a) | 0.01 | 98.2 | 103.4 |
| Example 8-1-2 | | Nonaqueous electrolyte solution 8-1-2 | | 0.1 | 95.6 | 105.7 |
| Example 8-1-3 | | Nonaqueous electrolyte solution 8-1-3 | | 1.0 | 92.3 | 110.3 |
| Example 8-1-4 | | Nonaqueous electrolyte solution 8-1-4 | | 5.0 | 94.1 | 109.4 |
| Example 8-1-5 | | Nonaqueous electrolyte solution 8-1-5 | | 11.0 | 97.5 | 106.7 |
| Example 8-2 | | Nonaqueous electrolyte solution 8-2 | (8b) | 1.0 | 90.1 | 107.6 |

(continued)

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Comparative Example 8-1 | Graphite | Comparative nonaqueous electrolyte solution 0 | - | - | 100 | 100 |
| Comparative Example 8-2 | | Comparative nonaqueous electrolyte solution 1 | 1 | 1.0 | 102.1 | 99.7 |
| Comparative Example 8-3 | | Comparative nonaqueous electrolyte solution 2 | 2 | | 101.2 | 100.5 |
| Comparative Example 8-4 | | Comparative nonaqueous electrolyte solution 3 | 3 | | 104.7 | 98.7 |
| Comparative Example 8-5 | | Comparative nonaqueous electrolyte solution 4 | 4 | | 103.5 | 100.2 |
| Comparative Example 8-6 | | Comparative nonaqueous electrolyte solution 5 | 5 | | 99.5 | 97.9 |
| Comparative Example 8-7 | | Comparative nonaqueous electrolyte solution 6 | 6 | | 98.4 | 103.2 |
| Comparative Example 8-8 | | Comparative nonaqueous electrolyte solution 7 | 7 | | 98.3 | 102.9 |

From the results shown in Table 8, it is understood that when the compound represented by the general formula (8) is contained as the component (III) of the present disclosure, the nonaqueous electrolyte solution battery using graphite as the negative electrode active material exhibits excellent cycle characteristic and also has excellent initial resistance characteristic as compared with the cases using the additives disclosed in the related art.

<Comparative Example 9-1> (Preparation of Comparative Nonaqueous Electrolyte Solution 9-0 as Base)

EC, DMC, and EMC were used as nonaqueous organic solvents, and FEC was used as another additive. These materials were mixed at a volume ratio of EC: FEC: DMC: EMC = 3: 0.2: 3: 3.8. Next, $LiPF_6$ as a solute was added and dissolved in such a manner that the content in the nonaqueous electrolyte solution became 1.00 mol/L to prepare a comparative nonaqueous electrolyte solution 9-0.

<Examples 9-1 to 9-19>

Nonaqueous electrolyte solutions according to the Examples were prepared by the same procedures as those of the comparative nonaqueous electrolyte solution 9-0 except that the compounds shown in Table 9 were dissolved as the component (III) at the contents shown in Table 9.

<Comparative Examples 9-2 to 9-8>

Comparative nonaqueous electrolyte solutions 9-1 to 9-7 were prepared in the same manner as in Example 9-1 except that Comparative Example Compounds 1 to 7 shown in Table 9 were dissolved instead of the component (III) at the contents shown in Table 9.

For the nonaqueous electrolyte solutions 9-1 to 9-19 and the comparative nonaqueous electrolyte solutions 9-0 to 9-7 prepared as described above, the initial resistance and the capacity retention rates in the cycle test were evaluated as described above using nonaqueous electrolyte solution batteries using Si-graphite instead of graphite as the negative electrode active material, and the results are shown in Table 9 below. Here, these values are relative values, and the initial resistance and the capacity retention rate in the cycle test of the case using the comparative nonaqueous electrolyte

solution 0 were regarded as 100 for the evaluation.

[Table 9]

Table 9

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 9-1 | | Nonaqueous electrolyte solution 9-1 | (1a) | 1.0 | 70.6 | 126.8 |
| Example 9-2 | | Nonaqueous electrolyte solution 9-2 | (1b) | | 77.4 | 125.9 |
| Example 9-3 | | Nonaqueous electrolyte solution 9-3 | (1c) | | 81.8 | 131.3 |
| Example 9-4 | | Nonaqueous electrolyte solution 9-4 | (1d) | | 73.2 | 128.6 |
| Example 9-5 | | Nonaqueous electrolyte solution 9-5 | (1f) | 0.5 | 75.6 | 130.7 |

(continued)

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Example 9-6 | Si-Graphite | Nonaqueous electrolyte solution 9-6 | (1k) | 1.0 | 82.2 | 124.9 |
| Example 9-7 | | Nonaqueous electrolyte solution 9-7 | (2a) | | 76.7 | 131.2 |
| Example 9-8 | | Nonaqueous electrolyte solution 9-8 | (2f) | | 80.1 | 127.1 |
| Example 9-9 | | Nonaqueous electrolyte solution 9-9 | (3a) | | 76.8 | 130.5 |
| Example 9-10 | | Nonaqueous electrolyte solution 9-10 | (3d) | | 79.2 | 124.2 |
| Example 9-11 | | Nonaqueous electrolyte solution 9-11 | (4a) | | 81.5 | 133.6 |
| Example 9-12 | | Nonaqueous electrolyte solution 9-12 | (4b) | | 79.9 | 134.8 |
| Example 9-13 | | Nonaqueous electrolyte solution 9-13 | (5a) | | 79.8 | 125.4 |
| Example 9-14 | | Nonaqueous electrolyte solution 9-14 | (5c) | | 81.6 | 127.0 |
| Example 9-15 | | Nonaqueous electrolyte solution 9-15 | (6a) | | 79.7 | 128.9 |
| Example 9-16 | | Nonaqueous electrolyte solution 9-16 | (6c) | | 83.3 | 127.7 |
| Example 9-17 | | Nonaqueous electrolyte solution 9-17 | (7a) | | 78.2 | 125.3 |
| Example 9-18 | | Nonaqueous electrolyte solution 9-18 | (7c) | | 82.0 | 124.1 |
| Example 9-19 | | Nonaqueous electrolyte solution 9-19 | (8a) | | 80.8 | 127.3 |
| Comparative Example 9-1 | | Comparative nonaqueous electrolyte solution 9-0 | - | - | 100 | 100 |

(continued)

| | Negative Electrode Active Material | Nonaqueous Electrolyte Solution | Component (III) or Comparative Example Compound | | Initial Resistance (relative value) | Cycle Discharge Capacity Retention Rate (relative value) |
|---|---|---|---|---|---|---|
| | | | Type | Content [% by mass] | | |
| Comparative Example 9-2 | | Comparative nonaqueous electrolyte solution 9-1 | 1 | 1.0 | 105.6 | 97.2 |
| Comparative Example 9-3 | | Comparative nonaqueous electrolyte solution 9-2 | 2 | | 103.5 | 99.8 |
| Comparative Example 9-4 | | Comparative nonaqueous electrolyte solution 9-3 | 3 | | 110.4 | 96.8 |
| Comparative Example 9-5 | | Comparative nonaqueous electrolyte solution 9-4 | 4 | | 107.6 | 99.3 |
| Comparative Example 9-6 | | Comparative nonaqueous electrolyte solution 9-5 | 5 | | 99.2 | 98.1 |
| Comparative Example 9-7 | | Comparative nonaqueous electrolyte solution 9-6 | 6 | | 84.2 | 123.4 |
| Comparative Example 9-8 | | Comparative nonaqueous electrolyte solution 9-7 | 7 | | 84.4 | 122.1 |

From the results shown in Table 9, it is understood that when the component (III) of the present disclosure is contained, the nonaqueous electrolyte solution battery using Si-graphite as the negative electrode active material exhibits excellent cycle characteristic and also has excellent initial resistance characteristic as compared with the cases using the additives disclosed in the related art.

<Comparative Example 10-1> (Preparation of Comparative Nonaqueous Electrolyte Solution 10-0 as Base)

This is the same as the comparative nonaqueous electrolyte solution 9-0 prepared in Comparative Example 9-1.

<Examples 10-1 to 10-19>

Nonaqueous electrolyte solutions according to the Examples were prepared by the same procedures as those of the comparative nonaqueous electrolyte solution 10-0 except that the compounds shown in Table 10 were dissolved as the component (III) at the contents shown in Table 10.

For the nonaqueous electrolyte solutions 10-1 to 10-19 and the comparative nonaqueous electrolyte solution 10-0 prepared as described above, the amounts of gas generated in the cycle test were evaluated as described above, and the results are shown in Table 10 below. Here, the values are relative values, and the gas generation amount in the cycle test of the case using the comparative nonaqueous electrolyte solution 10-0 was regarded as 100 for the evaluation.

[Table 10]

Table 10

| | Negative Electrode Active Material | Nonaqueous Electrolyte Solution | Component (III) or Comparative Example Compound | | Cycle Test Gas Generation Amount (relative value) |
|---|---|---|---|---|---|
| | | | Type | Content [% by mass] | |
| Example 10-1 | Graphite | Nonaqueous electrolyte solution 10-1 | (1a) | 1.0 | 62.3 |
| Example 10-2 | | Nonaqueous electrolyte solution 10-2 | (1b) | | 53.4 |
| Example 10-3 | | Nonaqueous electrolyte solution 10-3 | (1c) | | 54.7 |
| Example 10-4 | | Nonaqueous electrolyte solution 10-4 | (1d) | | 51.8 |
| Example 10-5 | | Nonaqueous electrolyte solution 10-5 | (1f) | 0.5 | 58.2 |
| Example 10-6 | | Nonaqueous electrolyte solution 10-6 | (1k) | 1.0 | 65.6 |
| Example 10-7 | | Nonaqueous electrolyte solution 10-7 | (2a) | | 72.3 |
| Example 10-8 | | Nonaqueous electrolyte solution 10-8 | (2f) | | 71.1 |
| Example 10-9 | | Nonaqueous electrolyte solution 10-9 | (3a) | | 65.7 |
| Example 10-10 | | Nonaqueous electrolyte solution 10-10 | (3d) | | 61.6 |
| Example 10-11 | | Nonaqueous electrolyte solution 10-11 | (4a) | | 70.6 |
| Example 10-12 | | Nonaqueous electrolyte solution 10-12 | (4b) | | 71.4 |
| Example 10-13 | | Nonaqueous electrolyte solution 10-13 | (5a) | | 84.5 |
| Example 10-14 | | Nonaqueous electrolyte solution 10-14 | (5c) | | 81.2 |
| Example 10-15 | | Nonaqueous electrolyte solution 10-15 | (6a) | | 90.4 |
| Example 10-16 | | Nonaqueous electrolyte solution 10-16 | (6c) | | 82.8 |
| Example 10-17 | | Nonaqueous electrolyte solution 10-17 | (7a) | | 81.9 |
| Example 10-18 | | Nonaqueous electrolyte solution 10-18 | (7c) | | 85.0 |
| Example 10-19 | | Nonaqueous electrolyte solution 10-19 | (8a) | | 89.7 |
| Comparative Example 10-1 | | Comparative nonaqueous electrolyte solution 10-0 | - | - | 100 |

From the results shown in Table 10, it is understood that when the component (III) of the present disclosure is contained, the gas generation amount in the cycle test is suppressed in the nonaqueous electrolyte solution battery using graphite as the negative electrode active material.

<Comparative Example 11-1> (Preparation of Comparative Nonaqueous Electrolyte Solution 8 as Base)

As a nonaqueous organic solvent, a solvent obtained by mixing EC, DMC, and EMC at a volume ratio of EC: DMC: EMC = 3: 3: 4 was used, and $LiPF_6$ as a solute was dissolved in the solvent in such a manner that the content in the nonaqueous electrolyte solution became 1.00 mol/L. Further, VC and DFOB as other additives were dissolved at the contents shown in Table 11 below to prepare a comparative nonaqueous electrolyte solution 8.

<Examples 11-1 to 11-14 and Comparative Examples 11-2 to 11-8>

Nonaqueous electrolyte solutions according to the Examples and the Comparative Examples were prepared by dissolving the components (III) or the comparative example compounds shown in Table 11 in the comparative nonaqueous electrolyte solution 8 at the contents shown in Table 11.

For the nonaqueous electrolyte solutions 11-1 to 11-14 and the comparative nonaqueous electrolyte solutions 8 to 15 prepared as described above, the initial resistance and the capacity retention rates in the cycle test were evaluated as described above, and the results are shown in Table 11 below. Here, these values are relative values, and the initial resistance value and the capacity retention rate in the cycle test of the case using the comparative nonaqueous electrolyte solution 8 were regarded as 100 for the evaluation.

[Table 11]

Table 11

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Example 11-1 | | Nonaqueous electrolyte solution 11-1 | (1a) | 1.0 | | | | | 81.6 | 105.3 |
| Example 11-2 | | Nonaqueous electrolyte solution 11-2 | (1b) | 1.0 | | | | | 82.3 | 110.8 |
| Example 11-3 | | Nonaqueous electrolyte solution 11-3 | (1c) | 1.0 | | | | | 95.3 | 109.8 |
| Example 11-4 | | Nonaqueous electrolyte solution 11-4 | (1d) | 1.0 | | | | | 81.4 | 110.4 |
| Example 11-5 | | Nonaqueous electrolyte solution 11-5 | (1f) | 0.5 | | | | | 82.1 | 111.2 |
| Example 11-6 | | Nonaqueous electrolyte solution 11-6 | (2a) | 1.0 | | | | | 92.7 | 109.9 |
| Example 11-7 | | Nonaqueous electrolyte solution 11-7 | (3a) | 1.0 | | | | | 80.9 | 107.8 |
| Example 11-8 | | Nonaqueous electrolyte solution 11-8 | (3b) | 1.0 | | | | | 92.3 | 105.6 |
| Example 11-9 | | Nonaqueous electrolyte solution 11-9 | (4a) | 1.0 | | | | | 95.8 | 110.7 |
| Example 11-10 | | Nonaqueous electrolyte solution 11-10 | (4b) | 1.0 | | | | | 92.0 | 111.1 |
| Example 11-11 | | Nonaqueous electrolyte solution 11-11 | (5a) | 1.0 | | | | | 92.7 | 109.9 |
| Example 11-12 | | Nonaqueous electrolyte solution 11-12 | (6a) | 1.0 | | | | | 85.5 | 109.1 |
| Example 11-13 | | Nonaqueous electrolyte solution 11-13 | (7a) | 1.0 | | | | | 82.9 | 106.5 |
| Example 11-14 | | Nonaqueous electrolyte solution 11-14 | (8a) | 1.0 | | | | | 96.8 | 107.6 |
| Comparative Example 11-1 | Graphite | Comparative nonaqueous electrolyte solution 8 | - | - | VC | 1.0 | DFOB | 1.0 | 100 | 100 |

(continued)

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Comparative Example 11-2 | | Comparative nonaqueous electrolyte solution 9 | 1 | 1.0 | | | | | 104.6 | 97.5 |
| Comparative Example 11-3 | | Comparative nonaqueous electrolyte solution 10 | 2 | | | | | | 103.7 | 98.3 |
| Comparative Example 11-4 | | Comparative nonaqueous electrolyte solution 11 | 3 | | | | | | 107.2 | 96.5 |
| Comparative Example 11-5 | | Comparative nonaqueous electrolyte solution 12 | 4 | | | | | | 106 | 98.0 |
| Comparative Example 11-6 | | Comparative nonaqueous electrolyte solution 13 | 5 | | | | | | 102 | 95.7 |
| Comparative Example 11-7 | | Comparative nonaqueous electrolyte solution 14 | 6 | | | | | | 99.8 | 101.2 |
| Comparative Example 11-8 | | Comparative nonaqueous electrolyte solution 15 | 7 | | | | | | 100.2 | 100.1 |

From the results shown in Table 11, it is understood that when the component (III) of the present disclosure is contained, the nonaqueous electrolyte solution battery using graphite as the negative electrode active material and using a nonaqueous electrolyte solution containing another additive exhibits excellent cycle characteristic as well as excellent initial resistance characteristic as compared with the cases using the comparative example compounds.

<Comparative Example 12-1> (Preparation of Comparative Nonaqueous Electrolyte Solution 16 as Base)

A comparative nonaqueous electrolyte solution 16 was prepared by the same procedures as those of the comparative nonaqueous electrolyte solution 8 except that VC and BOB were dissolved as other additives at the contents shown in Table 12 below.

<Examples 12-1 to 12-14 and Comparative Examples 12-2 to 12-8>

Nonaqueous electrolyte solutions according to the Examples and the Comparative Examples were prepared by dissolving the components (III) or the comparative example compounds shown in Table 12 in the comparative nonaqueous electrolyte solution 16 at the contents shown in Table 12.

For the nonaqueous electrolyte solutions 12-1 to 12-14 and the comparative nonaqueous electrolyte solutions 16 to 23 prepared as described above, the initial resistance and the capacity retention rates in the cycle test were evaluated as described above, and the results are shown in Table 12 below. Here, these values are relative values, and the initial resistance value and the capacity retention rate in the cycle test of the case using the comparative nonaqueous electrolyte solution 16 were regarded as 100 for the evaluation.

[Table 12]

Table 12

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Example 12-1 | Graphite | Nonaqueous electrolyte solution 12-1 | (1a) | 1.0 | | | | | 81.2 | 105.1 |
| Example 12-2 | | Nonaqueous electrolyte solution 12-2 | (1b) | 1.0 | | | | | 82.1 | 110.4 |
| Example 12-3 | | Nonaqueous electrolyte solution 12-3 | (1c) | 1.0 | | | | | 94.8 | 109.2 |
| Example 12-4 | | Nonaqueous electrolyte solution 12-4 | (1d) | 1.0 | | | | | 83.6 | 110.0 |
| Example 12-5 | | Nonaqueous electrolyte solution 12-5 | (1f) | 0.5 | | | | | 83.7 | 108.9 |
| Example 12-6 | | Nonaqueous electrolyte solution 12-6 | (2a) | 1.0 | | | | | 92.8 | 109.6 |
| Example 12-7 | | Nonaqueous electrolyte solution 12-7 | (3a) | 1.0 | | | | | 80.3 | 106.9 |
| Example 12-8 | | Nonaqueous electrolyte solution 12-8 | (3b) | 1.0 | | | | | 92.1 | 105.2 |
| Example 12-9 | | Nonaqueous electrolyte solution 12-9 | (4a) | 1.0 | | | | | 95.7 | 110.1 |
| Example 12-10 | | Nonaqueous electrolyte solution 12-10 | (4b) | 1.0 | | | | | 91.6 | 110.6 |
| Example 12-11 | | Nonaqueous electrolyte solution 12-11 | (5a) | 1.0 | | | | | 81.9 | 104.0 |
| Example 12-12 | | Nonaqueous electrolyte solution 12-12 | (6a) | 1.0 | | | | | 85.1 | 107.4 |
| Example 12-13 | | Nonaqueous electrolyte solution 12-13 | (7a) | 1.0 | | | | | 82.4 | 108.3 |
| Example 12-14 | | Nonaqueous electrolyte solution 12-14 | (8a) | 1.0 | | | | | 96.5 | 106.8 |
| Comparative Example 12-1 | | Comparative nonaqueous electrolyte solution 16 | - | - | VC | 1.0 | BOB | 1.0 | 100 | 100 |

(continued)

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Comparative Example 12-2 | | Comparative nonaqueous electrolyte solution 17 | 1 | 1.0 | | | | | 104.2 | 97.1 |
| Comparative Example 12-3 | | Comparative nonaqueous electrolyte solution 18 | 2 | | | | | | 103.7 | 98.1 |
| Comparative Example 12-4 | | Comparative nonaqueous electrolyte solution 19 | 3 | | | | | | 107.2 | 96.0 |
| Comparative Example 12-5 | | Comparative nonaqueous electrolyte solution 20 | 4 | | | | | | 105.3 | 97.3 |
| Comparative Example 12-6 | | Comparative nonaqueous electrolyte solution 21 | 5 | | | | | | 101.2 | 95.4 |
| Comparative Example 12-7 | | Comparative nonaqueous electrolyte solution 22 | 6 | | | | | | 99.3 | 100.4 |
| Comparative Example 12-8 | | Comparative nonaqueous electrolyte solution 23 | 7 | | | | | | 99.2 | 99.5 |

From the results shown in Table 12, it is understood that when the component (III) of the present disclosure is contained, the nonaqueous electrolyte solution battery using graphite as the negative electrode active material and using a nonaqueous electrolyte solution containing another additive exhibits excellent cycle characteristic as well as excellent initial resistance characteristic as compared with the cases using the comparative example compounds.

<Comparative Example 13-1> (Preparation of Comparative Nonaqueous Electrolyte Solution 24 as Base)

A comparative nonaqueous electrolyte solution 24 was prepared by the same procedures as those of the comparative nonaqueous electrolyte solution 8 except that VC and TFOP were dissolved as other additives at the contents shown in Table 13 below.

<Examples 13-1 to 13-14 and Comparative Examples 13-2 to 13-8>

Nonaqueous electrolyte solutions according to the Examples and the Comparative Examples were prepared by dissolving the components (III) or the comparative example compounds shown in Table 13 in the comparative nonaqueous electrolyte solution 24 at the contents shown in Table 13.

For the nonaqueous electrolyte solutions 13-1 to 13-14 and the comparative nonaqueous electrolyte solutions 24 to 31 prepared as described above, the initial resistance and the capacity retention rates in the cycle test were evaluated as described above, and the results are shown in Table 13 below. Here, these values are relative values, and the initial resistance and the capacity retention rate in the cycle test of the case using the comparative nonaqueous electrolyte solution 24 were regarded as 100 for the evaluation.

[Table 13]

Table 13

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Example 13-1 | Graphite | Nonaqueous electrolyte solution 13-1 | (1a) | 1.0 | VC | 1.0 | TFOP | 1.0 | 81.4 | 105.7 |
| Example 13-2 | | Nonaqueous electrolyte solution 13-2 | (1b) | 1.0 | | | | | 82.4 | 110.6 |
| Example 13-3 | | Nonaqueous electrolyte solution 13-3 | (1c) | 1.0 | | | | | 95.3 | 109.5 |
| Example 13-4 | | Nonaqueous electrolyte solution 13-4 | (1d) | 1.0 | | | | | 83.8 | 110.4 |
| Example 13-5 | | Nonaqueous electrolyte solution 13-5 | (1f) | 0.5 | | | | | 85.1 | 109.9 |
| Example 13-6 | | Nonaqueous electrolyte solution 13-6 | (2a) | 1.0 | | | | | 93.2 | 110.3 |
| Example 13-7 | | Nonaqueous electrolyte solution 13-7 | (3a) | 1.0 | | | | | 80.1 | 107.2 |
| Example 13-8 | | Nonaqueous electrolyte solution 13-8 | (3b) | 1.0 | | | | | 92.3 | 105.4 |
| Example 13-9 | | Nonaqueous electrolyte solution 13-9 | (4a) | 1.0 | | | | | 95.7 | 110.5 |
| Example 13-10 | | Nonaqueous electrolyte solution 13-10 | (4b) | 1.0 | | | | | 92.3 | 111.2 |
| Example 13-11 | | Nonaqueous electrolyte solution 13-11 | (5a) | 1.0 | | | | | 82.5 | 104.5 |
| Example 13-12 | | Nonaqueous electrolyte solution 13-12 | (6a) | 1.0 | | | | | 85.5 | 107.6 |
| Example 13-13 | | Nonaqueous electrolyte solution 13-13 | (7a) | 1.0 | | | | | 82.9 | 108.7 |
| Example 13-14 | | Nonaqueous electrolyte solution 13-14 | (8a) | 1.0 | | | | | 96.8 | 107.0 |
| Comparative Example 13-1 | | Comparative nonaqueous electrolyte solution 24 | - | - | | | | | 100 | 100 |

(continued)

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Comparative Example 13-2 | | Comparative nonaqueous electrolyte solution 25 | 1 | 1.0 | | | | | 104.5 | 97.6 |
| Comparative Example 13-3 | | Comparative nonaqueous electrolyte solution 26 | 2 | | | | | | 103.9 | 98.5 |
| Comparative Example 13-4 | | Comparative nonaqueous electrolyte solution 27 | 3 | | | | | | 108.1 | 96.1 |
| Comparative Example 13-5 | | Comparative nonaqueous electrolyte solution 28 | 4 | | | | | | 105.9 | 97.8 |
| Comparative Example 13-6 | | Comparative nonaqueous electrolyte solution 29 | 5 | | | | | | 101.5 | 95.9 |
| Comparative Example 13-7 | | Comparative nonaqueous electrolyte solution 30 | 6 | | | | | | 99.9 | 100.5 |
| Comparative Example 13-8 | | Comparative nonaqueous electrolyte solution 31 | 7 | | | | | | 99.6 | 100.1 |

From the results shown in Table 13, it is understood that when the component (III) of the present disclosure is contained, the nonaqueous electrolyte solution battery using graphite as the negative electrode active material and using a nonaqueous electrolyte solution containing another additive exhibits excellent cycle characteristic as well as excellent initial resistance characteristic as compared with the cases using the comparative example compounds.

<Comparative Example 14-1> (Preparation of Comparative Nonaqueous Electrolyte Solution 32 as Base)

A comparative nonaqueous electrolyte solution 32 was prepared by the same procedures as those of the comparative nonaqueous electrolyte solution 8 except that VC and DFBOP were dissolved as other additives at the contents shown in Table 14 below.

<Examples 14-1 to 14-14 and Comparative Examples 14-2 to 14-8>

Nonaqueous electrolyte solutions according to the Examples and the Comparative Examples were prepared by dissolving the components (III) or the comparative example compounds shown in Table 14 in the comparative nonaqueous electrolyte solution 32 at the contents shown in Table 14.

For the nonaqueous electrolyte solutions 14-1 to 14-14 and the comparative nonaqueous electrolyte solutions 32 to 39 prepared as described above, the initial resistance and the capacity retention rates in the cycle test were evaluated as described above, and the results are shown in Table 14 below. Here, these values are relative values, and the initial resistance and the capacity retention rate in the cycle test of the case using the comparative nonaqueous electrolyte solution 32 were regarded as 100 for the evaluation.

[Table 14]

Table 14

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Example 14-1 | Graphite | Nonaqueous electrolyte solution 14-1 | (1a) | 1.0 | VC | 1.0 | DFBOP | 1.0 | 81.8 | 105.3 |
| Example 14-2 | | Nonaqueous electrolyte solution 14-2 | (1b) | 1.0 | | | | | 83.0 | 110.2 |
| Example 14-3 | | Nonaqueous electrolyte solution 14-3 | (1c) | 1.0 | | | | | 95.8 | 109.2 |
| Example 14-4 | | Nonaqueous electrolyte solution 14-4 | (1d) | 1.0 | | | | | 84.2 | 110.2 |
| Example 14-5 | | Nonaqueous electrolyte solution 14-5 | (1f) | 0.5 | | | | | 85.1 | 109.7 |
| Example 14-6 | | Nonaqueous electrolyte solution 14-6 | (2a) | 1.0 | | | | | 93.7 | 109.2 |
| Example 14-7 | | Nonaqueous electrolyte solution 14-7 | (3a) | 1.0 | | | | | 80.2 | 107.3 |
| Example 14-8 | | Nonaqueous electrolyte solution 14-8 | (3b) | 1.0 | | | | | 92.7 | 105.1 |
| Example 14-9 | | Nonaqueous electrolyte solution 14-9 | (4a) | 1.0 | | | | | 96.8 | 110.0 |
| Example 14-10 | | Nonaqueous electrolyte solution 14-10 | (4b) | 1.0 | | | | | 93.0 | 109.9 |
| Example 14-11 | | Nonaqueous electrolyte solution 14-11 | (5a) | 1.0 | | | | | 83.8 | 103.8 |
| Example 14-12 | | Nonaqueous electrolyte solution 14-12 | (6a) | 1.0 | | | | | 87.1 | 106.1 |
| Example 14-13 | | Nonaqueous electrolyte solution 14-13 | (7a) | 1.0 | | | | | 84.1 | 107.9 |
| Example 14-14 | | Nonaqueous electrolyte solution 14-14 | (8a) | 1.0 | | | | | 97.5 | 106.3 |
| Comparative Example 14-1 | | Comparative nonaqueous electrolyte solution 32 | - | - | | | | | 100 | 100 |

(continued)

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Comparative Example 14-2 | | Comparative nonaqueous electrolyte solution 33 | 1 | 1.0 | | | | | 105.2 | 97.4 |
| Comparative Example 14-3 | | Comparative nonaqueous electrolyte solution 34 | 2 | | | | | | 104.6 | 98.2 |
| Comparative Example 14-4 | | Comparative nonaqueous electrolyte solution 35 | 3 | | | | | | 109.1 | 95.6 |
| Comparative Example 14-5 | | Comparative nonaqueous electrolyte solution 36 | 4 | | | | | | 107.1 | 97.1 |
| Comparative Example 14-6 | | Comparative nonaqueous electrolyte solution 37 | 5 | | | | | | 102.5 | 95.3 |
| Comparative Example 14-7 | | Comparative nonaqueous electrolyte solution 38 | 6 | | | | | | 100.3 | 99.9 |
| Comparative Example 14-8 | | Comparative nonaqueous electrolyte solution 39 | 7 | | | | | | 99.9 | 98.9 |

From the results shown in Table 14, it is understood that when the component (III) of the present disclosure is contained, the nonaqueous electrolyte solution battery using graphite as the negative electrode active material and using a nonaqueous electrolyte solution containing another additive exhibits excellent cycle characteristic as well as excellent initial resistance characteristic as compared with the cases using the comparative example compounds.

<Comparative Example 15-1> (Preparation of Comparative Nonaqueous Electrolyte Solution 40 as Base)

A comparative nonaqueous electrolyte solution 40 was prepared by the same procedures as those of the comparative nonaqueous electrolyte solution 8 except that DFP, FS, and VC were dissolved as other additives at the contents shown in Table 15 below.

<Examples 15-1 to 15-14 and Comparative Examples 15-2 to 15-8>

Nonaqueous electrolyte solutions according to the Examples and the Comparative Examples were prepared by dissolving the components (III) or the comparative example compounds shown in Table 15 in the comparative nonaqueous electrolyte solution 40 at the contents shown in Table 15.

For the nonaqueous electrolyte solutions 15-1 to 15-14 and the comparative nonaqueous electrolyte solutions 40 to 47 prepared as described above, the initial resistance and the capacity retention rates in the cycle test were evaluated as described above, and the results are shown in Table 15 below. Here, these values are relative values, and the initial resistance and the capacity retention rate in the cycle test of the case using the comparative nonaqueous electrolyte solution 40 were regarded as 100 for the evaluation.

[Table 15]

Table 15

| | Negative Electrode Active Material | Nonaqueous Electrolyte Solution | Component (III) or Comparative Example Compound | | Other Additive (I) | | Other Additive (II) | | Other Additive (III) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [% by mass] | Type | Content [% by mass] | Type | Content [% by mass] | | |
| Example 15-1 | Graphite | Nonaqueous electrolyte solution 15-1 | (1a) | 1.0 | | | | | | | 82.4 | 104.8 |
| Example 15-2 | | Nonaqueous electrolyte solution 15-2 | (1b) | 1.0 | | | | | | | 83.6 | 109.6 |
| Example 15-3 | | Nonaqueous electrolyte solution 15-3 | (1c) | 1.0 | | | | | | | 96.5 | 108.5 |
| Example 15-4 | | Nonaqueous electrolyte solution 15-4 | (1d) | 1.0 | | | | | | | 84.3 | 109.6 |
| Example 15-5 | | Nonaqueous electrolyte solution 15-5 | (1f) | 0.5 | | | | | | | 85.8 | 110.0 |
| Example 15-6 | | Nonaqueous electrolyte solution 15-6 | (2a) | 1.0 | | | | | | | 94.6 | 108.7 |
| Example 15-7 | | Nonaqueous electrolyte solution 15-7 | (3a) | 1.0 | | | | | | | 81.2 | 106.3 |
| Example 15-8 | | Nonaqueous electrolyte solution 15-8 | (3b) | 1.0 | | | | | | | 93.7 | 104.1 |
| Example 15-9 | | Nonaqueous electrolyte solution 15-9 | (4a) | 1.0 | | | | | | | 97.8 | 109.4 |
| Example 15-10 | | Nonaqueous electrolyte solution 15-10 | (4b) | 1.0 | | | | | | | 93.9 | 109.0 |
| Example 15-11 | | Nonaqueous electrolyte solution 15-11 | (5a) | 1.0 | | | | | | | 85.8 | 103.0 |
| Example 15-12 | | Nonaqueous electrolyte solution 15-12 | (6a) | 1.0 | | | | | | | 90.1 | 103.6 |

(continued)

| | Negative Electrode Active Material | Nonaqueous Electrolyte Solution | Component (III) or Comparative Example Compound | | Other Additive (I) | | Other Additive (II) | | Other Additive (III) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [% by mass] | Type | Content [% by mass] | Type | Content [% by mass] | | |
| Example 15-13 | | Nonaqueous electrolyte solution 15-13 | (7a) | 1.0 | | | | | | | 84.9 | 107.2 |
| Example 15-14 | | Nonaqueous electrolyte solution 15-14 | (8a) | 1.0 | | | | | | | 98.6 | 104.2 |
| Comparative Example 15-1 | | Comparative nonaqueous electrolyte solution 40 | - | - | DFP | 0.45 | VC | 1.0 | FS | 1.0 | 100 | 100 |
| Comparative Example 15-2 | | Comparative nonaqueous electrolyte solution 41 | 1 | | | | | | | | 106.0 | 96.8 |
| Comparative Example 15-3 | | Comparative nonaqueous electrolyte solution 42 | 2 | | | | | | | | 105.8 | 97.1 |
| Comparative Example 15-4 | | Comparative nonaqueous electrolyte solution 43 | 3 | | | | | | | | 111.1 | 94.3 |
| Comparative Example 15-5 | | Comparative nonaqueous electrolyte solution 44 | 4 | 1.0 | | | | | | | 108.9 | 95.6 |
| Comparative Example 15-6 | | Comparative nonaqueous electrolyte solution 45 | 5 | | | | | | | | 105.4 | 94.9 |
| Comparative Example 15-7 | | Comparative nonaqueous electrolyte solution 46 | 6 | | | | | | | | 102.5 | 99.2 |
| Comparative Example 15-8 | | Comparative nonaqueous electrolyte solution 47 | 7 | | | | | | | | 103.1 | 98.2 |

From the results shown in Table 15, it is understood that when the component (III) of the present disclosure is contained, the nonaqueous electrolyte solution battery using graphite as the negative electrode active material and using a nonaqueous electrolyte solution containing another additive exhibits excellent cycle characteristic as well as excellent initial resistance characteristic as compared with the cases using the comparative example compounds.

<Comparative Example 16-1> (Preparation of Comparative Nonaqueous Electrolyte Solution 48 as Base)

A comparative nonaqueous electrolyte solution 48 was prepared by the same procedures as those of the comparative nonaqueous electrolyte solution 8 except that DFP, VC, and DFPFSI were dissolved as other additives at the contents shown in Table 16 below.

<Examples 16-1 to 16-14 and Comparative Examples 16-2 to 16-8>

Nonaqueous electrolyte solutions according to the Examples and the Comparative Examples were prepared by dissolving the components (III) or the comparative example compounds shown in Table 16 in the comparative nonaqueous electrolyte solution 48 at the contents shown in Table 16.

For the nonaqueous electrolyte solutions 16-1 to 16-14 and the comparative nonaqueous electrolyte solutions 48 to 55 prepared as described above, the initial resistance and the capacity retention rates in the cycle test were evaluated as described above, and the results are shown in Table 16 below. Here, these values are relative values, and the initial resistance and the capacity retention rate in the cycle test of the case using the comparative nonaqueous electrolyte solution 48 were regarded as 100 for the evaluation.

[Table 16]

Table 16

| | Negative Electrode Active Material | Nonaqueous Electrolyte Solution | Component (III) or Comparative Example Compound | | Other Additive (I) | | Other Additive (II) | | Other Additive (III) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [% by mass] | Type | Content [% by mass] | Type | Content [% by mass] | | |
| Example 16-1 | | Nonaqueous electrolyte solution 16-1 | (1a) | 1.0 | | | | | | | 82.7 | 105.1 |
| Example 16-2 | | Nonaqueous electrolyte solution 16-2 | (1b) | 1.0 | | | | | | | 84.0 | 109.8 |
| Example 16-3 | | Nonaqueous electrolyte solution 16-3 | (1c) | 1.0 | | | | | | | 96.9 | 108.9 |
| Example 16-4 | | Nonaqueous electrolyte solution 16-4 | (1d) | 1.0 | | | | | | | 84.4 | 110.0 |
| Example 16-5 | | Nonaqueous electrolyte solution 16-5 | (1f) | 0.5 | | | | | | | 85.6 | 110.7 |
| Example 16-6 | | Nonaqueous electrolyte solution 16-6 | (2a) | 1.0 | | | | | | | 95.3 | 108.7 |
| Example 16-7 | | Nonaqueous electrolyte solution 16-7 | (3a) | 1.0 | | | | | | | 81.9 | 106.4 |
| Example 16-8 | | Nonaqueous electrolyte solution 16-8 | (3b) | 1.0 | | | | | | | 94.7 | 104.2 |
| Example 16-9 | | Nonaqueous electrolyte solution 16-9 | (4a) | 1.0 | | | | | | | 98.2 | 109.4 |
| Example 16-10 | | Nonaqueous electrolyte solution 16-10 | (4b) | 1.0 | | | | | | | 94.6 | 109.3 |
| Example 16-11 | | Nonaqueous electrolyte solution 16-11 | (5a) | 1.0 | | | | | | | 87.1 | 103.2 |
| Example 16-12 | | Nonaqueous electrolyte solution 16-12 | (6a) | 1.0 | | | | | | | 90.6 | 103.9 |

(continued)

| | Negative Electrode Active Material | Nonaqueous Electrolyte Solution | Component (III) or Comparative Example Compound | | Other Additive (I) | | Other Additive (II) | | Other Additive (III) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [% by mass] | Type | Content [% by mass] | Type | Content [% by mass] | | |
| Example 16-13 | Graphite | Nonaqueous electrolyte solution 16-13 | (7a) | 1.0 | DFP | 0.45 | VC | 1.0 | DFPFSI | 1.0 | 85.9 | 107.1 |
| Example 16-14 | | Nonaqueous electrolyte solution 16-14 | (8a) | 1.0 | | | | | | | 98.9 | 104.1 |
| Comparative Example 16-1 | | Comparative nonaqueous electrolyte solution 48 | - | - | | | | | | | 100 | 100 |
| Comparative Example 16-2 | | Comparative nonaqueous electrolyte solution 49 | 1 | 1.0 | | | | | | | 107.8 | 96.8 |
| Comparative Example 16-3 | | Comparative nonaqueous electrolyte solution 50 | 2 | | | | | | | | 106.8 | 97.1 |
| Comparative Example 16-4 | | Comparative nonaqueous electrolyte solution 51 | 3 | | | | | | | | 111.0 | 94.5 |
| Comparative Example 16-5 | | Comparative nonaqueous electrolyte solution 52 | 4 | | | | | | | | 109.2 | 96.9 |
| Comparative Example 16-6 | | Comparative nonaqueous electrolyte solution 53 | 5 | | | | | | | | 105.9 | 95.0 |
| Comparative Example 16-7 | | Comparative nonaqueous electrolyte solution 54 | 6 | | | | | | | | 103.4 | 99.1 |
| Comparative Example 16-8 | | Comparative nonaqueous electrolyte solution 55 | 7 | | | | | | | | 102.6 | 98.9 |

From the results shown in Table 16, it is understood that when the component (III) of the present disclosure is contained, the nonaqueous electrolyte solution battery using graphite as the negative electrode active material and using a nonaqueous electrolyte solution containing another additive exhibits excellent cycle characteristic as well as excellent initial resistance characteristic as compared with the cases using the comparative example compounds.

<Comparative Example 17-1> (Preparation of Comparative Nonaqueous Electrolyte Solution 56 as Base)

A comparative nonaqueous electrolyte solution 56 was prepared by the same procedures as those of the comparative nonaqueous electrolyte solution 8 except that FSI as an additional solute and DTD and VC as other additives were dissolved at the contents shown in Table 17 below.

<Examples 17-1 to 17-14 and Comparative Examples 17-2 to 17-8>

Nonaqueous electrolyte solutions according to the Examples and the Comparative Examples were prepared by dissolving the components (III) or the comparative example compounds shown in Table 17 in the comparative nonaqueous electrolyte solution 56 at the contents shown in Table 17.

For the nonaqueous electrolyte solutions 17-1 to 17-14 and the comparative nonaqueous electrolyte solutions 56 to 63 prepared as described above, the initial resistance and the capacity retention rates in the cycle test were evaluated as described above, and the results are shown in Table 17 below. Here, these values are relative values, and the initial resistance and the capacity retention rate in the cycle test of the case using the comparative nonaqueous electrolyte solution 56 were regarded as 100 for the evaluation.

[Table 17]

Table 17

| | Negative Electrode Active Material | Nonaqueous Electrolyte Solution | Component (III) or Comparative Example Compound | | Additional Solute | | Other Additive (I) | | Other Additive (II) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [mol/L] | Type | Content [% by mass] | Type | Content [% by mass] | | |
| Example 17-1 | | Nonaqueous electrolyte solution 17-1 | (1a) | 1.0 | | | | | | | 82.6 | 105.4 |
| Example 17-2 | | Nonaqueous electrolyte solution 17-2 | (1b) | 1.0 | | | | | | | 83.8 | 110.6 |
| Example 17-3 | | Nonaqueous electrolyte solution 17-3 | (1c) | 1.0 | | | | | | | 96.9 | 109.4 |
| Example 17-4 | | Nonaqueous electrolyte solution 17-4 | (1d) | 1.0 | | | | | | | 84.7 | 110.3 |
| Example 17-5 | | Nonaqueous electrolyte solution 17-5 | (1f) | 0.5 | | | | | | | 86.2 | 110.1 |
| Example 17-6 | | Nonaqueous electrolyte solution 17-6 | (2a) | 1.0 | | | | | | | 95.3 | 108.1 |
| Example 17-7 | | Nonaqueous electrolyte solution 17-7 | (3a) | 1.0 | | | | | | | 81.8 | 106.5 |
| Example 17-8 | | Nonaqueous electrolyte solution 17-8 | (3b) | 1.0 | | | | | | | 94.6 | 104.1 |
| Example 17-9 | | Nonaqueous electrolyte solution 17-9 | (4a) | 1.0 | | | | | | | 98.5 | 109.5 |
| Example 17-10 | | Nonaqueous electrolyte solution 17-10 | (4b) | 1.0 | | | | | | | 94.6 | 109.7 |
| Example 17-11 | | Nonaqueous electrolyte solution 17-11 | (5a) | 1.0 | | | | | | | 87.3 | 103.2 |
| Example 17-12 | | Nonaqueous electrolyte solution 17-12 | (6a) | 1.0 | | | | | | | 90.9 | 103.6 |

(continued)

| | Negative Electrode Active Material | Nonaqueous Electrolyte Solution | Component (III) or Comparative Example Compound | | Additional Solute | | Other Additive (I) | | Other Additive (II) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [mol/L] | Type | Content [% by mass] | Type | Content [% by mass] | | |
| Example 17-13 | | Nonaqueous electrolyte solution 17-13 | (7a) | 1.0 | | | | | | | 87.2 | 106.9 |
| Example 17-14 | | Nonaqueous electrolyte solution 17-14 | (8a) | 1.0 | | | | | | | 98.6 | 103.8 |
| Comparative Example 17-1 | Graphite | Comparative nonaqueous electrolyte solution 56 | - | - | FSI | 0.1 | VC | 1.0 | DTD | 1.0 | 100 | 100 |
| Comparative Example 17-2 | | Comparative nonaqueous electrolyte solution 57 | 1 | | | | | | | | 107.8 | 97.1 |
| Comparative Example 17-3 | | Comparative nonaqueous electrolyte solution 58 | 2 | | | | | | | | 106.8 | 96.9 |
| Comparative Example 17-4 | | Comparative nonaqueous electrolyte solution 59 | 3 | | | | | | | | 110.6 | 94.3 |
| Comparative Example 17-5 | | Comparative nonaqueous electrolyte solution 60 | 4 | 1.0 | | | | | | | 109.2 | 97.1 |
| Comparative Example 17-6 | | Comparative nonaqueous electrolyte solution 61 | 5 | | | | | | | | 106.3 | 95.3 |
| Comparative Example 17-7 | | Comparative nonaqueous electrolyte solution 62 | 6 | | | | | | | | 104.1 | 99.0 |
| Comparative Example 17-8 | | Comparative nonaqueous electrolyte solution 63 | 7 | | | | | | | | 103.5 | 98.9 |

From the results shown in Table 17, it is understood that when the component (III) of the present disclosure is contained, the nonaqueous electrolyte solution battery using graphite as the negative electrode active material and using a nonaqueous electrolyte solution containing an additional solute and another additive exhibits excellent cycle characteristic as well as excellent initial resistance characteristic as compared with the cases using the comparative example compounds.

<Comparative Example 18-1> (Preparation of Comparative Nonaqueous Electrolyte Solution 64 as Base)

A comparative nonaqueous electrolyte solution 64 was prepared by the same procedures as those of the comparative nonaqueous electrolyte solution 8 except that FSI as an additional solute and VC and TDUTO as other additives were dissolved at the contents shown in Table 18 below.

<Examples 18-1 to 18-14 and Comparative Examples 18-2 to 18-8>

Nonaqueous electrolyte solutions according to the Examples and the Comparative Examples were prepared by dissolving the components (III) or the comparative example compounds shown in Table 18 in the comparative nonaqueous electrolyte solution 64 at the contents shown in Table 18.

For the nonaqueous electrolyte solutions 18-1 to 18-14 and the comparative nonaqueous electrolyte solutions 64 to 71 prepared as described above, the initial resistance and the capacity retention rates in the cycle test were evaluated as described above, and the results are shown in Table 18 below. Here, these values are relative values, and the initial resistance and the capacity retention rate in the cycle test of the case using the comparative nonaqueous electrolyte solution 64 were regarded as 100 for the evaluation.

[Table 18]

Table 18

| | Negative Electrode Active Material | Nonaqueous Electrolyte Solution | Component (III) or Comparative Example Compound | | Additional Solute | | Other Additive (I) | | Other Additive (II) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [mol/L] | Type | Content [% by mass] | Type | Content [% by mass] | | |
| Example 18-1 | | Nonaqueous electrolyte solution 18-1 | (1a) | 1.0 | | | | | | | 82.3 | 105.2 |
| Example 18-2 | | Nonaqueous electrolyte solution 18-2 | (1b) | 1.0 | | | | | | | 83.7 | 109.8 |
| Example 18-3 | | Nonaqueous electrolyte solution 18-3 | (1c) | 1.0 | | | | | | | 96.6 | 109.0 |
| Example 18-4 | | Nonaqueous electrolyte solution 18-4 | (1d) | 1.0 | | | | | | | 84.1 | 110.1 |
| Example 18-5 | | Nonaqueous electrolyte solution 18-5 | (1f) | 0.5 | | | | | | | 85.2 | 111.2 |
| Example 18-6 | | Nonaqueous electrolyte solution 18-6 | (2a) | 1.0 | | | | | | | 94.9 | 107.6 |
| Example 18-7 | | Nonaqueous electrolyte solution 18-7 | (3a) | 1.0 | | | | | | | 81.5 | 106.4 |
| Example 18-8 | | Nonaqueous electrolyte solution 18-8 | (3b) | 1.0 | | | | | | | 94.2 | 103.7 |
| Example 18-9 | | Nonaqueous electrolyte solution 18-9 | (4a) | 1.0 | | | | | | | 98.0 | 109.5 |
| Example 18-10 | | Nonaqueous electrolyte solution 18-10 | (4b) | 1.0 | | | | | | | 94.1 | 109.8 |
| Example 18-11 | | Nonaqueous electrolyte solution 18-11 | (5a) | 1.0 | | | | | | | 87.1 | 103.0 |
| Example 18-12 | | Nonaqueous electrolyte solution 18-12 | (6a) | 1.0 | | | | | | | 90.8 | 103.3 |

(continued)

| | Negative Electrode Active Material | Nonaqueous Electrolyte Solution | Component (III) or Comparative Example Compound | | Additional Solute | | Other Additive (I) | | Other Additive (II) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [mol/L] | Type | Content [% by mass] | Type | Content [% by mass] | | |
| Example 18-13 | Graphite | Nonaqueous electrolyte solution 18-13 | (7a) | 1.0 | FSI | 0.1 | VC | 1.0 | TDUTO | 1.0 | 87.3 | 106.8 |
| Example 18-14 | | Nonaqueous electrolyte solution 18-14 | (8a) | 1.0 | | | | | | | 98.5 | 103.5 |
| Comparative Example 18-1 | | Comparative nonaqueous electrolyte solution 64 | - | - | | | | | | | 100 | 100 |
| Comparative Example 18-2 | | Comparative nonaqueous electrolyte solution 65 | 1 | 1.0 | | | | | | | 107.8 | 96.8 |
| Comparative Example 18-3 | | Comparative nonaqueous electrolyte solution 66 | 2 | | | | | | | | 106.8 | 96.5 |
| Comparative Example 18-4 | | Comparative nonaqueous electrolyte solution 67 | 3 | | | | | | | | 110.2 | 94.3 |
| Comparative Example 18-5 | | Comparative nonaqueous electrolyte solution 68 | 4 | | | | | | | | 108.8 | 97.2 |
| Comparative Example 18-6 | | Comparative nonaqueous electrolyte solution 69 | 5 | | | | | | | | 106.0 | 95.8 |
| Comparative Example 18-7 | | Comparative nonaqueous electrolyte solution 70 | 6 | | | | | | | | 104.1 | 98.6 |
| Comparative Example 18-8 | | Comparative nonaqueous electrolyte solution 71 | 7 | | | | | | | | 103.3 | 99.1 |

From the results shown in Table 18, it is understood that when the component (III) of the present disclosure is contained, the nonaqueous electrolyte solution battery using graphite as the negative electrode active material and using a nonaqueous electrolyte solution containing an additional solute and another additive exhibits excellent cycle characteristic as well as excellent initial resistance characteristic as compared with the cases using the comparative example compounds.

<Comparative Example 19-1> (Preparation of Comparative Nonaqueous Electrolyte Solution 72 as Base)

EC, DMC, and EMC were used as nonaqueous organic solvents, and FEC was used as another additive. These materials were mixed at a volume ratio of EC: FEC: DMC: EMC = 3: 0.2: 3: 3.8. Next, $LiPF_6$ as a solute was added and dissolved in such a manner that the content in the nonaqueous electrolyte solution became 1.00 mol/L, and VC and DFOB were dissolved as other additives at the contents shown in Table 19 below to prepare a comparative nonaqueous electrolyte solution 72.

<Examples 19-1 to 19-14 and Comparative Examples 19-2 to 19-8>

Nonaqueous electrolyte solutions according to the Examples and the Comparative Examples were prepared by dissolving the components (III) or the comparative example compounds shown in Table 19 in the comparative nonaqueous electrolyte solution 72 at the contents shown in Table 19.

For the nonaqueous electrolyte solutions 19-1 to 19-14 and the comparative nonaqueous electrolyte solutions 72 to 79 prepared as described above, the initial resistance and the capacity retention rates in the cycle test were evaluated as described above using nonaqueous electrolyte solution batteries using Si-graphite as the negative electrode active material, and the results are shown in Table 19 below. Here, these values are relative values, and the initial resistance and the capacity retention rate in the cycle test of the case using the comparative nonaqueous electrolyte solution 72 were regarded as 100 for the evaluation.

[Table 19]

Table 19

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Example 19-1 | | Nonaqueous electrolyte solution 19-1 | (1a) | 1.0 | | | | | 72.9 | 126.1 |
| Example 19-2 | | Nonaqueous electrolyte solution 19-2 | (1b) | 1.0 | | | | | 79.6 | 124.7 |
| Example 19-3 | | Nonaqueous electrolyte solution 19-3 | (1c) | 1.0 | | | | | 83.8 | 130.8 |
| Example 19-4 | | Nonaqueous electrolyte solution 19-4 | (1d) | 1.0 | | | | | 74.8 | 128.2 |
| Example 19-5 | | Nonaqueous electrolyte solution 19-5 | (1f) | 0.5 | | | | | 75.3 | 130.1 |
| Example 19-6 | | Nonaqueous electrolyte solution 19-6 | (2a) | 1.0 | | | | | 78.5 | 130.0 |
| Example 19-7 | | Nonaqueous electrolyte solution 19-7 | (3a) | 1.0 | | | | | 78.4 | 129.6 |
| Example 19-8 | | Nonaqueous electrolyte solution 19-8 | (3b) | 1.0 | | | | | 81.2 | 124.1 |
| Example 19-9 | | Nonaqueous electrolyte solution 19-9 | (4a) | 1.0 | | | | | 84.0 | 132.8 |
| Example 19-10 | | Nonaqueous electrolyte solution 19-10 | (4b) | 1.0 | | | | | 82.0 | 134.3 |
| Example 19-11 | | Nonaqueous electrolyte solution 19-11 | (5a) | 1.0 | | | | | 81.7 | 124.7 |
| Example 19-12 | | Nonaqueous electrolyte solution 19-12 | (6a) | 1.0 | | | | | 81.6 | 128.0 |
| Example 19-13 | | Nonaqueous electrolyte solution 19-13 | (7a) | 1.0 | | | | | 79.9 | 124.9 |
| Example 19-14 | | Nonaqueous electrolyte solution 19-14 | (8a) | 1.0 | | | | | 82.4 | 126.2 |
| Comparative Example 19-1 | Si-Graphite | Comparative nonaqueous electrolyte solution 72 | - | - | VC | 1.0 | DFOB | 1.0 | 100 | 100 |

(continued)

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Comparative Example 19-2 | | Comparative nonaqueous electrolyte solution 73 | 1 | 1.0 | | | | | 107.9 | 96.0 |
| Comparative Example 19-3 | | Comparative nonaqueous electrolyte solution 74 | 2 | | | | | | 105.7 | 98.7 |
| Comparative Example 19-4 | | Comparative nonaqueous electrolyte solution 75 | 3 | | | | | | 112.3 | 95.7 |
| Comparative Example 19-5 | | Comparative nonaqueous electrolyte solution 76 | 4 | | | | | | 109.6 | 98.1 |
| Comparative Example 19-6 | | Comparative nonaqueous electrolyte solution 77 | 5 | | | | | | 101.4 | 96.9 |
| Comparative Example 19-7 | | Comparative nonaqueous electrolyte solution 78 | 6 | | | | | | 86.2 | 122.6 |
| Comparative Example 19-8 | | Comparative nonaqueous electrolyte solution 79 | 7 | | | | | | 86.1 | 121.1 |

From the results shown in Table 19, it is understood that when the component (III) of the present disclosure is contained, the nonaqueous electrolyte solution battery using Si-graphite as the negative electrode active material and using a nonaqueous electrolyte solution containing another additive exhibits excellent cycle characteristic as well as excellent initial resistance characteristic as compared with the cases using the comparative example compounds.

<Comparative Example 20-1> (Preparation of Comparative Nonaqueous Electrolyte Solution 80 as Base)

EC, DMC, and EMC were used as nonaqueous organic solvents, and FEC was used as another additive. These materials were mixed at a volume ratio of EC: FEC: DMC: EMC = 3: 0.2: 3: 3.8. Next, $LiPF_6$ as a solute was added and dissolved in such a manner that the content in the nonaqueous electrolyte solution became 1.00 mol/L, and VC and BOB were dissolved as other additives at the contents shown in Table 20 below to prepare a comparative nonaqueous electrolyte solution 80.

<Examples 20-1 to 20-14 and Comparative Examples 20-2 to 20-8>

Nonaqueous electrolyte solutions according to the Examples and the Comparative Examples were prepared by dissolving the components (III) or the comparative example compounds shown in Table 20 in the comparative nonaqueous electrolyte solution 80 at the contents shown in Table 20.

For the nonaqueous electrolyte solutions 20-1 to 20-14 and the comparative nonaqueous electrolyte solutions 80 to 87 prepared as described above, the initial resistance and the capacity retention rates in the cycle test were evaluated as described above using nonaqueous electrolyte solution batteries using Si-graphite as the negative electrode active material, and the results are shown in Table 20 below. Here, these values are relative values, and the initial resistance and the capacity retention rate in the cycle test of the case using the comparative nonaqueous electrolyte solution 80 were regarded as 100 for the evaluation.

[Table 20]

Table 20

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Example 20-1 | Si-Graphite | Nonaqueous electrolyte solution 20-1 | (1a) | 1.0 | VC | 1.0 | BOB | 1.0 | 72.0 | 125.7 |
| Example 20-2 | | Nonaqueous electrolyte solution 20-2 | (1b) | 1.0 | | | | | 78.8 | 124.0 |
| Example 20-3 | | Nonaqueous electrolyte solution 20-3 | (1c) | 1.0 | | | | | 83.7 | 130.0 |
| Example 20-4 | | Nonaqueous electrolyte solution 20-4 | (1d) | 1.0 | | | | | 74.1 | 127.8 |
| Example 20-5 | | Nonaqueous electrolyte solution 20-5 | (1f) | 0.5 | | | | | 75.4 | 128.1 |
| Example 20-6 | | Nonaqueous electrolyte solution 20-6 | (2a) | 1.0 | | | | | 77.6 | 129.7 |
| Example 20-7 | | Nonaqueous electrolyte solution 20-7 | (3a) | 1.0 | | | | | 77.6 | 129.5 |
| Example 20-8 | | Nonaqueous electrolyte solution 20-8 | (3b) | 1.0 | | | | | 80.3 | 123.8 |
| Example 20-9 | | Nonaqueous electrolyte solution 20-9 | (4a) | 1.0 | | | | | 83.0 | 132.5 |
| Example 20-10 | | Nonaqueous electrolyte solution 20-10 | (4b) | 1.0 | | | | | 81.1 | 134.1 |
| Example 20-11 | | Nonaqueous electrolyte solution 20-11 | (5a) | 1.0 | | | | | 81.0 | 124.5 |
| Example 20-12 | | Nonaqueous electrolyte solution 20-12 | (6a) | 1.0 | | | | | 81.3 | 127.1 |
| Example 20-13 | | Nonaqueous electrolyte solution 20-13 | (7a) | 1.0 | | | | | 79.3 | 124.6 |
| Example 20-14 | | Nonaqueous electrolyte solution 20-14 | (8a) | 1.0 | | | | | 82.0 | 125.8 |
| Comparative Example 20-1 | | Comparative nonaqueous electrolyte solution 80 | - | - | | | | | 100 | 100 |

(continued)

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Comparative Example 20-2 | | Comparative nonaqueous electrolyte solution 81 | 1 | 1.0 | | | | | 107.5 | 95.7 |
| Comparative Example 20-3 | | Comparative nonaqueous electrolyte solution 82 | 2 | | | | | | 105.2 | 97.9 |
| Comparative Example 20-4 | | Comparative nonaqueous electrolyte solution 83 | 3 | | | | | | 111.7 | 95.0 |
| Comparative Example 20-5 | | Comparative nonaqueous electrolyte solution 84 | 4 | | | | | | 108.7 | 97.3 |
| Comparative Example 20-6 | | Comparative nonaqueous electrolyte solution 85 | 5 | | | | | | 100.9 | 96.3 |
| Comparative Example 20-7 | | Comparative nonaqueous electrolyte solution 86 | 6 | | | | | | 85.3 | 121.9 |
| Comparative Example 20-8 | | Comparative nonaqueous electrolyte solution 87 | 7 | | | | | | 85.9 | 120.9 |

From the results shown in Table 20, it is understood that when the component (III) of the present disclosure is contained, the nonaqueous electrolyte solution battery using Si-graphite as the negative electrode active material and using a nonaqueous electrolyte solution containing another additive exhibits excellent cycle characteristic as well as excellent initial resistance characteristic as compared with the cases using the comparative example compounds.

<Comparative Example 21-1> (Preparation of Comparative Nonaqueous Electrolyte Solution 88 as Base)

EC, DMC, and EMC were used as nonaqueous organic solvents, and FEC was used as another additive. These materials were mixed at a volume ratio of EC: FEC: DMC: EMC = 3: 0.2: 3: 3.8. Next, $LiPF_6$ as a solute was added and dissolved in such a manner that the content in the nonaqueous electrolyte solution became 1.00 mol/L, and VC and TFOP were dissolved as other additives at the contents shown in Table 21 below to prepare a comparative nonaqueous electrolyte solution 88.

<Examples 21-1 to 21-14 and Comparative Examples 21-2 to 21-8>

Nonaqueous electrolyte solutions according to the Examples and the Comparative Examples were prepared by dissolving the components (III) or the comparative example compounds shown in Table 21 in the comparative nonaqueous electrolyte solution 88 at the contents shown in Table 21.

For the nonaqueous electrolyte solutions 21-1 to 21-14 and the comparative nonaqueous electrolyte solutions 88 to 95 prepared as described above, the initial resistance and the capacity retention rates in the cycle test were evaluated as described above using nonaqueous electrolyte solution batteries using Si-graphite as the negative electrode active material, and the results are shown in Table 21 below. Here, these values are relative values, and the initial resistance and the capacity retention rate in the cycle test of the case using the comparative nonaqueous electrolyte solution 88 were regarded as 100 for the evaluation.

[Table 21]

Table 21

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Example 21-1 | | Nonaqueous electrolyte solution 21-1 | (1a) | 1.0 | | | | | 72.6 | 126.5 |
| Example 21-2 | | Nonaqueous electrolyte solution 21-2 | (1b) | 1.0 | | | | | 79.1 | 124.9 |
| Example 21-3 | | Nonaqueous electrolyte solution 21-3 | (1c) | 1.0 | | | | | 84.4 | 130.6 |
| Example 21-4 | | Nonaqueous electrolyte solution 21-4 | (1d) | 1.0 | | | | | 74.8 | 127.8 |
| Example 21-5 | | Nonaqueous electrolyte solution 21-5 | (1f) | 0.5 | | | | | 74.9 | 129.1 |
| Example 21-6 | | Nonaqueous electrolyte solution 21-6 | (2a) | 1.0 | | | | | 78.1 | 130.6 |
| Example 21-7 | | Nonaqueous electrolyte solution 21-7 | (3a) | 1.0 | | | | | 78.3 | 130.0 |
| Example 21-8 | | Nonaqueous electrolyte solution 21-8 | (3b) | 1.0 | | | | | 80.6 | 124.9 |
| Example 21-9 | | Nonaqueous electrolyte solution 21-9 | (4a) | 1.0 | | | | | 83.8 | 133.3 |
| Example 21-10 | | Nonaqueous electrolyte solution 21-10 | (4b) | 1.0 | | | | | 81.7 | 134.9 |
| Example 21-11 | | Nonaqueous electrolyte solution 21-11 | (5a) | 1.0 | | | | | 81.9 | 124.9 |
| Example 21-12 | | Nonaqueous electrolyte solution 21-12 | (6a) | 1.0 | | | | | 82.0 | 127.8 |
| Example 21-13 | | Nonaqueous electrolyte solution 21-13 | (7a) | 1.0 | | | | | 80.4 | 124.7 |
| Example 21-14 | | Nonaqueous electrolyte solution 21-14 | (8a) | 1.0 | | | | | 82.5 | 126.6 |
| Comparative Example 21-1 | Si-Graphite | Comparative nonaqueous electrolyte solution 88 | - | - | VC | 1.0 | TFOP | 1.0 | 100 | 100 |

(continued)

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Comparative Example 21-2 | | Comparative nonaqueous electrolyte solution 89 | 1 | 1.0 | | | | | 108.4 | 95.8 |
| Comparative Example 21-3 | | Comparative nonaqueous electrolyte solution 90 | 2 | | | | | | 105.9 | 98.9 |
| Comparative Example 21-4 | | Comparative nonaqueous electrolyte solution 91 | 3 | | | | | | 112.7 | 95.4 |
| Comparative Example 21-5 | | Comparative nonaqueous electrolyte solution 92 | 4 | | | | | | 109.7 | 97.4 |
| Comparative Example 21-6 | | Comparative nonaqueous electrolyte solution 93 | 5 | | | | | | 101.9 | 96.6 |
| Comparative Example 21-7 | | Comparative nonaqueous electrolyte solution 94 | 6 | | | | | | 85.3 | 122.9 |
| Comparative Example 21-8 | | Comparative nonaqueous electrolyte solution 95 | 7 | | | | | | 86.2 | 121.9 |

From the results shown in Table 21, it is understood that when the component (III) of the present disclosure is contained, the nonaqueous electrolyte solution battery using Si-graphite as the negative electrode active material and using a nonaqueous electrolyte solution containing another additive exhibits excellent cycle characteristic as well as excellent initial resistance characteristic as compared with the cases using the comparative example compounds.

<Comparative Example 22-1> (Preparation of Comparative Nonaqueous Electrolyte Solution 96 as Base)

EC, DMC, and EMC were used as nonaqueous organic solvents, and FEC was used as another additive. These materials were mixed at a volume ratio of EC: FEC: DMC: EMC = 3: 0.2: 3: 3.8. Next, $LiPF_6$ as a solute was added and dissolved in such a manner that the content in the nonaqueous electrolyte solution became 1.00 mol/L, and VC and DFBOP were dissolved as other additives at the contents shown in Table 22 below to prepare a comparative nonaqueous electrolyte solution 96.

<Examples 22-1 to 22-14 and Comparative Examples 22-2 to 22-8>

Nonaqueous electrolyte solutions according to the Examples and the Comparative Examples were prepared by dissolving the components (III) or the comparative example compounds shown in Table 22 in the comparative nonaqueous electrolyte solution 96 at the contents shown in Table 22.

For the nonaqueous electrolyte solutions 22-1 to 22-14 and the comparative nonaqueous electrolyte solutions 96 to 103 prepared as described above, the initial resistance and the capacity retention rates in the cycle test were evaluated as described above using nonaqueous electrolyte solution batteries using Si-graphite as the negative electrode active material, and the results are shown in Table 22 below. Here, these values are relative values, and the initial resistance and the capacity retention rate in the cycle test of the case using the comparative nonaqueous electrolyte solution 96 were regarded as 100 for the evaluation.

[Table 22]

Table 22

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Example 22-1 | | Nonaqueous electrolyte solution 22-1 | (1a) | 1.0 | | | | | 73.5 | 126.1 |
| Example 22-2 | | Nonaqueous electrolyte solution 22-2 | (1b) | 1.0 | | | | | 79.7 | 123.7 |
| Example 22-3 | | Nonaqueous electrolyte solution 22-3 | (1c) | 1.0 | | | | | 84.7 | 130.4 |
| Example 22-4 | | Nonaqueous electrolyte solution 22-4 | (1d) | 1.0 | | | | | 74.9 | 127.5 |
| Example 22-5 | | Nonaqueous electrolyte solution 22-5 | (1f) | 0.5 | | | | | 75.4 | 129.3 |
| Example 22-6 | | Nonaqueous electrolyte solution 22-6 | (2a) | 1.0 | | | | | 78.7 | 129.4 |
| Example 22-7 | | Nonaqueous electrolyte solution 22-7 | (3a) | 1.0 | | | | | 78.5 | 128.6 |
| Example 22-8 | | Nonaqueous electrolyte solution 22-8 | (3b) | 1.0 | | | | | 81.3 | 124.4 |
| Example 22-9 | | Nonaqueous electrolyte solution 22-9 | (4a) | 1.0 | | | | | 84.1 | 132.1 |
| Example 22-10 | | Nonaqueous electrolyte solution 22-10 | (4b) | 1.0 | | | | | 82.7 | 133.8 |
| Example 22-11 | | Nonaqueous electrolyte solution 22-11 | (5a) | 1.0 | | | | | 82.8 | 123.6 |
| Example 22-12 | | Nonaqueous electrolyte solution 22-12 | (6a) | 1.0 | | | | | 82.3 | 127.1 |
| Example 22-13 | | Nonaqueous electrolyte solution 22-13 | (7a) | 1.0 | | | | | 81.3 | 124.3 |
| Example 22-14 | | Nonaqueous electrolyte solution 22-14 | (8a) | 1.0 | | | | | 83.2 | 126.2 |
| Comparative Example 22-1 | Si-Graphite | Comparative nonaqueous electrolyte solution 96 | - | - | VC | 1.0 | DFBOP | 1.0 | 100 | 99 |

(continued)

| | Negative Electrode Active Material | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|---|
| | | | Type | Content [% by mass] | Type | Content [% by mass] | Type | Content [% by mass] | | |
| Comparative Example 22-2 | | Comparative nonaqueous electrolyte solution 97 | 1 | 1.0 | | | | | 108.9 | 95.7 |
| Comparative Example 22-3 | | Comparative nonaqueous electrolyte solution 98 | 2 | | | | | | 106.5 | 98.5 |
| Comparative Example 22-4 | | Comparative nonaqueous electrolyte solution 99 | 3 | | | | | | 113.1 | 95.3 |
| Comparative Example 22-5 | | Comparative nonaqueous electrolyte solution 100 | 4 | | | | | | 110.0 | 96.1 |
| Comparative Example 22-6 | | Comparative nonaqueous electrolyte solution 101 | 5 | | | | | | 102.2 | 95.9 |
| Comparative Example 22-7 | | Comparative nonaqueous electrolyte solution 102 | 6 | | | | | | 85.9 | 121.3 |
| Comparative Example 22-8 | | Comparative nonaqueous electrolyte solution 103 | 7 | | | | | | 87.0 | 120.3 |

From the results shown in Table 22, it is understood that when the component (III) of the present disclosure is contained, the nonaqueous electrolyte solution battery using Si-graphite as the negative electrode active material and using a nonaqueous electrolyte solution containing another additive exhibits excellent cycle characteristic as well as excellent initial resistance characteristic as compared with the cases using the comparative example compounds.

<Comparative Example 23-1> (Preparation of Comparative Nonaqueous Electrolyte Solution 104 as Base)

EC, DMC, and EMC were used as nonaqueous organic solvents, and FEC was used as another additive. These materials were mixed at a volume ratio of EC: FEC: DMC: EMC = 3: 0.2: 3: 3.8. Next, $LiPF_6$ as a solute was added and dissolved in such a manner that the content in the nonaqueous electrolyte solution became 1.00 mol/L, and DFP, VC, and FS were dissolved as other additives at the contents shown in Table 23 below to prepare a comparative nonaqueous electrolyte solution 104.

<Examples 23-1 to 23-14 and Comparative Examples 23-2 to 23-8>

Nonaqueous electrolyte solutions according to the Examples and the Comparative Examples were prepared by dissolving the components (III) or the comparative example compounds shown in Table 23 in the comparative nonaqueous electrolyte solution 104 at the contents shown in Table 23.

For the nonaqueous electrolyte solutions 23-1 to 23-14 and the comparative nonaqueous electrolyte solutions 104 to 111 prepared as described above, the initial resistance and the capacity retention rates in the cycle test were evaluated as described above using nonaqueous electrolyte solution batteries using Si-graphite as the negative electrode active material, and the results are shown in Table 23 below. Here, these values are relative values, and the initial resistance and the capacity retention rate in the cycle test of the case using the comparative nonaqueous electrolyte solution 104 were regarded as 100 for the evaluation.

[Table 23]

Table 23

| | Negative Electrode Active Material | Nonaqueous Electrolyte Solution | Component (III) or Comparative Example Compound | | Other Additive (I) | | Other Additive (II) | | Other Additive (III) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [% by mass] | Type | Content [% by mass] | Type | Content [% by mass] | | |
| Example 23-1 | | Nonaqueous electrolyte solution 23-1 | (1a) | 1.0 | | | | | | | 76.5 | 127.3 |
| Example 23-2 | | Nonaqueous electrolyte solution 23-2 | (1b) | 1.0 | | | | | | | 81.1 | 125.0 |
| Example 23-3 | | Nonaqueous electrolyte solution 23-3 | (1c) | 1.0 | | | | | | | 87.7 | 130.6 |
| Example 23-4 | | Nonaqueous electrolyte solution 23-4 | (1d) | 1.0 | | | | | | | 75.4 | 129.1 |
| Example 23-5 | | Nonaqueous electrolyte solution 23-5 | (1f) | 0.5 | | | | | | | 77.2 | 129.7 |
| Example 23-6 | | Nonaqueous electrolyte solution 23-6 | (2a) | 1.0 | | | | | | | 78.9 | 129.8 |
| Example 23-7 | | Nonaqueous electrolyte solution 23-7 | (3a) | 1.0 | | | | | | | 81.4 | 129.9 |
| Example 23-8 | | Nonaqueous electrolyte solution 23-8 | (3b) | 1.0 | | | | | | | 84.6 | 125.4 |
| Example 23-9 | | Nonaqueous electrolyte solution 23-9 | (4a) | 1.0 | | | | | | | 87.0 | 133.4 |
| Example 23-10 | | Nonaqueous electrolyte solution 23-10 | (4b) | 1.0 | | | | | | | 83.5 | 134.6 |
| Example 23-11 | | Nonaqueous electrolyte solution 23-11 | (5a) | 1.0 | | | | | | | 84.5 | 124.5 |
| Example 23-12 | | Nonaqueous electrolyte solution 23-12 | (6a) | 1.0 | | | | | | | 85.5 | 128.0 |

(continued)

| | Negative Electrode Active Material | Nonaqueous Electrolyte Solution | Component (III) or Comparative Example Compound | | Other Additive (I) | | Other Additive (II) | | Other Additive (III) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [% by mass] | Type | Content [% by mass] | Type | Content [% by mass] | | |
| Example 23-13 | Si-Graphite | Nonaqueous electrolyte solution 23-13 | (7a) | 1.0 | DFP | 0.45 | VC | 1.0 | FS | 1.0 | 82.5 | 125.7 |
| Example 23-14 | | Nonaqueous electrolyte solution 23-14 | (8a) | 1.0 | | | | | | | 86.1 | 123.1 |
| Comparative Example 23-1 | | Comparative nonaqueous electrolyte solution 104 | - | - | | | | | | | 100 | 100 |
| Comparative Example 23-2 | | Comparative nonaqueous electrolyte solution 105 | 1 | 1.0 | | | | | | | 110.1 | 96.0 |
| Comparative Example 23-3 | | Comparative nonaqueous electrolyte solution 106 | 2 | | | | | | | | 108.2 | 100.1 |
| Comparative Example 23-4 | | Comparative nonaqueous electrolyte solution 107 | 3 | | | | | | | | 115.7 | 96.7 |
| Comparative Example 23-5 | | Comparative nonaqueous electrolyte solution 108 | 4 | | | | | | | | 113.3 | 97.6 |
| Comparative Example 23-6 | | Comparative nonaqueous electrolyte solution 109 | 5 | | | | | | | | 103.9 | 96.8 |
| Comparative Example 23-7 | | Comparative nonaqueous electrolyte solution 110 | 6 | | | | | | | | 86.0 | 121.9 |
| Comparative Example 23-8 | | Comparative nonaqueous electrolyte solution 111 | 7 | | | | | | | | 89.1 | 120.9 |

From the results shown in Table 23, it is understood that when the component (III) of the present disclosure is contained, the nonaqueous electrolyte solution battery using Si-graphite as the negative electrode active material and using a nonaqueous electrolyte solution containing another additive exhibits excellent cycle characteristic and exhibits improved initial resistance characteristic as a whole as compared with the cases using the comparative example compounds.

<Comparative Example 24-1> (Preparation of Comparative Nonaqueous Electrolyte Solution 112 as Base)

EC, DMC, and EMC were used as nonaqueous organic solvents, and FEC was used as another additive. These materials were mixed at a volume ratio of EC: FEC: DMC: EMC = 3: 0.2: 3: 3.8. Next, $LiPF_6$ as a solute was added and dissolved in such a manner that the content in the nonaqueous electrolyte solution became 1.00 mol/L, and DFP, VC, and DFPFSI were dissolved as other additives at the contents shown in Table 24 below to prepare a comparative nonaqueous electrolyte solution 112.

<Examples 24-1 to 24-14 and Comparative Examples 24-2 to 24-8>

Nonaqueous electrolyte solutions according to the Examples and the Comparative Examples were prepared by dissolving the components (III) or the comparative example compounds shown in Table 23 in the comparative nonaqueous electrolyte solution 112 at the contents shown in Table 24.

For the nonaqueous electrolyte solutions 24-1 to 24-14 and the comparative nonaqueous electrolyte solutions 112 to 119 prepared as described above, the initial resistance and the capacity retention rates in the cycle test were evaluated as described above using nonaqueous electrolyte solution batteries using Si-graphite as the negative electrode active material, and the results are shown in Table 24 below. Here, these values are relative values, and the initial resistance and the capacity retention rate in the cycle test of the case using the comparative nonaqueous electrolyte solution 112 were regarded as 100 for the evaluation.

[Table 24]

Table 24

| | Negative Electrode Active Material | Nonaqueous Electrolyte Solution | Component (III) or Comparative Example Compound | | Other Additive (I) | | Other Additive (II) | | Other Additive (III) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [% by mass] | Type | Content [% by mass] | Type | Content [% by mass] | | |
| Example 24-1 | | Nonaqueous electrolyte solution 24-1 | (1a) | 1.0 | | | | | | | 77.6 | 128.9 |
| Example 24-2 | | Nonaqueous electrolyte solution 24-2 | (1b) | 1.0 | | | | | | | 83.0 | 136.2 |
| Example 24-3 | | Nonaqueous electrolyte solution 24-3 | (1c) | 1.0 | | | | | | | 87.7 | 132.2 |
| Example 24-4 | | Nonaqueous electrolyte solution 24-4 | (1d) | 1.0 | | | | | | | 77.4 | 130.0 |
| Example 24-5 | | Nonaqueous electrolyte solution 24-5 | (1f) | 0.5 | | | | | | | 79.2 | 131.3 |
| Example 24-6 | | Nonaqueous electrolyte solution 24-6 | (2a) | 1.0 | | | | | | | 80.5 | 131.7 |
| Example 24-7 | | Nonaqueous electrolyte solution 24-7 | (3a) | 1.0 | | | | | | | 81.5 | 131.5 |
| Example 24-8 | | Nonaqueous electrolyte solution 24-8 | (3b) | 1.0 | | | | | | | 86.1 | 127.0 |
| Example 24-9 | | Nonaqueous electrolyte solution 24-9 | (4a) | 1.0 | | | | | | | 87.5 | 133.5 |
| Example 24-10 | | Nonaqueous electrolyte solution 24-10 | (4b) | 1.0 | | | | | | | 84.5 | 136.0 |
| Example 24-11 | | Nonaqueous electrolyte solution 24-11 | (5a) | 1.0 | | | | | | | 85.8 | 126.1 |
| Example 24-12 | | Nonaqueous electrolyte solution 24-12 | (6a) | 1.0 | | | | | | | 87.5 | 129.2 |

(continued)

| | Negative Electrode Active Material | Nonaqueous Electrolyte Solution | Component (III) or Comparative Example Compound | | Other Additive (I) | | Other Additive (II) | | Other Additive (III) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [% by mass] | Type | Content [% by mass] | Type | Content [% by mass] | | |
| Example 24-13 | Si-Graphite | Nonaqueous electrolyte solution 24-13 | (7a) | 1.0 | DFP | 0.45 | VC | 1.0 | DFPFSI | 1.0 | 83.3 | 125.8 |
| Example 24-14 | | Nonaqueous electrolyte solution 24-14 | (8a) | 1.0 | | | | | | | 84.3 | 127.8 |
| Comparative Example 24-1 | | Comparative nonaqueous electrolyte solution 112 | - | - | | | | | | | 100 | 100 |
| Comparative Example 24-2 | | Comparative nonaqueous electrolyte solution 113 | 1 | 1.0 | | | | | | | 111.4 | 97.0 |
| Comparative Example 24-3 | | Comparative nonaqueous electrolyte solution 114 | 2 | | | | | | | | 109.9 | 101.1 |
| Comparative Example 24-4 | | Comparative nonaqueous electrolyte solution 115 | 3 | | | | | | | | 117.3 | 98.1 |
| Comparative Example 24-5 | | Comparative nonaqueous electrolyte solution 116 | 4 | | | | | | | | 114.7 | 98.2 |
| Comparative Example 24-6 | | Comparative nonaqueous electrolyte solution 117 | 5 | | | | | | | | 105.7 | 97.7 |
| Comparative Example 24-7 | | Comparative nonaqueous electrolyte solution 118 | 6 | | | | | | | | 87.2 | 123.1 |
| Comparative Example 24-8 | | Comparative nonaqueous electrolyte solution 119 | 7 | | | | | | | | 90.3 | 121.7 |

From the results shown in Table 24, it is understood that when the component (III) of the present disclosure is contained, the nonaqueous electrolyte solution battery using Si-graphite as the negative electrode active material and using a nonaqueous electrolyte solution containing another additive exhibits excellent cycle characteristic and exhibits improved initial resistance characteristic as a whole as compared with the cases using the comparative example compounds.

<Comparative Example 25-1> (Preparation of Comparative Nonaqueous Electrolyte Solution 120 as Base)

EC, DMC, and EMC were used as nonaqueous organic solvents, and FEC was used as another additive. These materials were mixed at a volume ratio of EC: FEC: DMC: EMC = 3: 0.2: 3: 3.8. Next, $LiPF_6$ as a solute was added and dissolved in such a manner that the content in the nonaqueous electrolyte solution became 1.00 mol/L, and FSI as an additional solute and VC and DTD as other additives were dissolved at the contents shown in Table 25 below to prepare a comparative nonaqueous electrolyte solution 120.

<Examples 25-1 to 25-14 and Comparative Examples 25-2 to 25-8>

Nonaqueous electrolyte solutions according to the Examples and the Comparative Examples were prepared by dissolving the components (III) or the comparative example compounds shown in Table 25 in the comparative nonaqueous electrolyte solution 120 at the contents shown in Table 25.

For the nonaqueous electrolyte solutions 25-1 to 25-14 and the comparative nonaqueous electrolyte solutions 120 to 127 prepared as described above, the initial resistance and the capacity retention rates in the cycle test were evaluated as described above using nonaqueous electrolyte solution batteries using Si-graphite as the negative electrode active material, and the results are shown in Table 25 below. Here, these values are relative values, and the initial resistance and the capacity retention rate in the cycle test of the case using the comparative nonaqueous electrolyte solution 120 were regarded as 100 for the evaluation.

[Table 25]

Table 25

| | Negative Electrode Active Material | Nonaqueous Electrolyte Solution | Component (III) or Comparative Example Compound | | Additional Solute | | Other Additive (I) | | Other Additive (II) | | Initial Resistance (relative value) | Cycle Discharge Capacity Retention Rate (relative value) |
|---|---|---|---|---|---|---|---|---|---|---|---|---|
| | | | Type | Content [% by mass] | Type | Conten t [mol/L] | Type | Content [% by mass] | Type | Content [% by mass] | | |
| Example 25-1 | | Nonaqueous electrolyte solution 25-1 | (1a) | 1.0 | | | | | | | 75.6 | 127.8 |
| Example 25-2 | | Nonaqueous electrolyte solution 25-2 | (1b) | 1.0 | | | | | | | 81.4 | 126.0 |
| Example 25-3 | | Nonaqueous electrolyte solution 25-3 | (1c) | 1.0 | | | | | | | 86.0 | 131.6 |
| Example 25-4 | | Nonaqueous electrolyte solution 25-4 | (1d) | 1.0 | | | | | | | 76.3 | 128.9 |
| Example 25-5 | | Nonaqueous electrolyte solution 25-5 | (1f) | 0.5 | | | | | | | 78.1 | 130.5 |
| Example 25-6 | | Nonaqueous electrolyte solution 25-6 | (2a) | 1.0 | | | | | | | 78.7 | 130.6 |
| Example 25-7 | | Nonaqueous electrolyte solution 25-7 | (3a) | 1.0 | | | | | | | 80.8 | 131.4 |
| Example 25-8 | | Nonaqueous electrolyte solution 25-8 | (3b) | 1.0 | | | | | | | 85.0 | 125.9 |
| Example 25-9 | | Nonaqueous electrolyte solution 25-9 | (4a) | 1.0 | | | | | | | 87.2 | 133.1 |
| Example 25-10 | | Nonaqueous electrolyte solution 25-10 | (4b) | 1.0 | | | | | | | 83.5 | 135.6 |
| Example 25-11 | | Nonaqueous electrolyte solution 25-11 | (5a) | 1.0 | | | | | | | 85.5 | 125.8 |
| Example 25-12 | | Nonaqueous electrolyte solution 25-12 | (6a) | 1.0 | | | | | | | 86.2 | 129.2 |

(continued)

| | Negative Electrode Active Material | Nonaqueous Electrolyte Solution | Component (III) or Comparative Example Compound | | Additional Solute | | Other Additive (I) | | Other Additive (II) | | Initial Resistance (relative value) | Cycle Discharge Capacity Retention Rate (relative value) |
|---|---|---|---|---|---|---|---|---|---|---|---|---|
| | | | Type | Content [% by mass] | Type | Conten t [mol/L] | Type | Content [% by mass] | Type | Content [% by mass] | | |
| Example 25-13 | Si-Graphite | Nonaqueous electrolyte solution 25-13 | (7a) | 1.0 | FSI | 0.1 | VC | 1.0 | DTD | 1.0 | 82.5 | 125.7 |
| Example 25-14 | | Nonaqueous electrolyte solution 25-14 | (8a) | 1.0 | | | | | | | 82.7 | 126.7 |
| Comparative Example 25-1 | | Comparative nonaqueous electrolyte solution 120 | - | - | | | | | | | 100 | 100 |
| Comparative Example 25-2 | | Comparative nonaqueous electrolyte solution 121 | 1 | 1.0 | | | | | | | 109.2 | 96.8 |
| Comparative Example 25-3 | | Comparative nonaqueous electrolyte solution 122 | 2 | | | | | | | | 107.9 | 100.1 |
| Comparative Example 25-4 | | Comparative nonaqueous electrolyte solution 123 | 3 | | | | | | | | 116.4 | 97.8 |
| Comparative Example 25-5 | | Comparative nonaqueous electrolyte solution 124 | 4 | | | | | | | | 114.1 | 97.7 |
| Comparative Example 25-6 | | Comparative nonaqueous electrolyte solution 125 | 5 | | | | | | | | 103.8 | 97.0 |
| Comparative Example 25-7 | | Comparative nonaqueous electrolyte solution 126 | 6 | | | | | | | | 85.0 | 122.6 |
| Comparative Example 25-8 | | Comparative nonaqueous electrolyte solution 127 | 7 | | | | | | | | 89.2 | 121.0 |

From the results shown in Table 25, it is understood that when the component (III) of the present disclosure is contained, the nonaqueous electrolyte solution battery using Si-graphite as the negative electrode active material and using a nonaqueous electrolyte solution containing an additional solute and another additive exhibits excellent cycle characteristic and exhibits improved initial resistance characteristic as a whole as compared with the cases using the comparative example compounds.

<Comparative Example 26-1> (Preparation of Comparative Nonaqueous Electrolyte Solution 128 as Base)

PC, DMC, and EMC were used as nonaqueous organic solvents, and FEC was used as another additive. These materials were mixed at a volume ratio of PC: FEC: DMC: EMC = 2: 0.5: 4: 3.5. Next, $LiPF_6$ as a solute was added and dissolved in such a manner that the content in the nonaqueous electrolyte solution became 1.00 mol/L, and $LiBF_4$ as an additional solute and VC and TDUTO as other additives were dissolved at the contents shown in Table 26 below to prepare a comparative nonaqueous electrolyte solution 128.

<Examples 26-1 to 26-14 and Comparative Examples 26-2 to 26-8>

Nonaqueous electrolyte solutions according to the Examples and the Comparative Examples were prepared by dissolving the components (III) or the comparative example compounds shown in Table 26 in the comparative nonaqueous electrolyte solution 128 at the contents shown in Table 26.

For the nonaqueous electrolyte solutions 26-1 to 26-14 and the comparative nonaqueous electrolyte solutions 128 to 135 prepared as described above, the initial resistance and the capacity retention rates in the cycle test were evaluated as described above using nonaqueous electrolyte solution batteries using Si-graphite as the negative electrode active material, and the results are shown in Table 26 below. Here, these values are relative values, and the initial resistance and the capacity retention rate in the cycle test of the case using the comparative nonaqueous electrolyte solution 128 were regarded as 100 for the evaluation.

[Table 26]

Table 26

| | Negative Electrode Active Material | Nonaqueous Electrolyte Solution | Component (III) or Comparative Example Compound | | Additional Solute | | Other Additive (I) | | Other Additive (II) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [mol/L] | Type | Content [% by mass] | Type | Content [% by mass] | | |
| Example 26-1 | | Nonaqueous electrolyte solution 26-1 | (1a) | 1.0 | | | | | | | 74.7 | 128.7 |
| Example 26-2 | | Nonaqueous electrolyte solution 26-2 | (1b) | 1.0 | | | | | | | 80.6 | 126.9 |
| Example 26-3 | | Nonaqueous electrolyte solution 26-3 | (1c) | 1.0 | | | | | | | 85.9 | 132.2 |
| Example 26-4 | | Nonaqueous electrolyte solution 26-4 | (1d) | 1.0 | | | | | | | 74.7 | 129.5 |
| Example 26-5 | | Nonaqueous electrolyte solution 26-5 | (1f) | 0.5 | | | | | | | 76.1 | 130.6 |
| Example 26-6 | | Nonaqueous electrolyte solution 26-6 | (2a) | 1.0 | | | | | | | 77.0 | 131.2 |
| Example 26-7 | | Nonaqueous electrolyte solution 26-7 | (3a) | 1.0 | | | | | | | 79.9 | 131.6 |
| Example 26-8 | | Nonaqueous electrolyte solution 26-8 | (3b) | 1.0 | | | | | | | 83.8 | 126.1 |
| Example 26-9 | | Nonaqueous electrolyte solution 26-9 | (4a) | 1.0 | | | | | | | 87.0 | 133.9 |
| Example 26-10 | | Nonaqueous electrolyte solution 26-10 | (4b) | 1.0 | | | | | | | 82.5 | 135.6 |
| Example 26-11 | | Nonaqueous electrolyte solution 26-11 | (5a) | 1.0 | | | | | | | 85.2 | 126.5 |
| Example 26-12 | | Nonaqueous electrolyte solution 26-12 | (6a) | 1.0 | | | | | | | 86.0 | 129.3 |

(continued)

| | Negative Electrode Active Material | Nonaqueous Electrolyte Solution | Component (III) or Comparative Example Compound | | Additional Solute | | Other Additive (I) | | Other Additive (II) | | Initial Resistance (relative value) | Cycle Discharge Capacity Retention Rate (relative value) |
|---|---|---|---|---|---|---|---|---|---|---|---|---|
| | | | Type | Content [% by mass] | Type | Content [mol/L] | Type | Content [% by mass] | Type | Content [% by mass] | | |
| Example 26-13 | | Nonaqueous electrolyte solution 26-13 | (7a) | 1.0 | | | | | | | 82.1 | 126.6 |
| Example 26-14 | | Nonaqueous electrolyte solution 26-14 | (8a) | 1.0 | | | | | | | 81.3 | 127.2 |
| Comparative Example 26-1 | Si-Graphite | Comparative nonaqueous electrolyte solution 128 | - | - | $LiBF_4$ | 0.02 | VC | 1.0 | TDUTO | 1.0 | 100 | 100 |
| Comparative Example 26-2 | | Comparative nonaqueous electrolyte solution 129 | 1 | | | | | | | | 108.1 | 97.6 |
| Comparative Example 26-3 | | Comparative nonaqueous electrolyte solution 130 | 2 | | | | | | | | 107.7 | 100.7 |
| Comparative Example 26-4 | | Comparative nonaqueous electrolyte solution 131 | 3 | | | | | | | | 115.8 | 98.2 |
| Comparative Example 26-5 | | Comparative nonaqueous electrolyte solution 132 | 4 | 1.0 | | | | | | | 114.1 | 98.7 |
| Comparative Example 26-6 | | Comparative nonaqueous electrolyte solution 133 | 5 | | | | | | | | 103.5 | 97.0 |
| Comparative Example 26-7 | | Comparative nonaqueous electrolyte solution 134 | 6 | | | | | | | | 84.6 | 123.5 |
| Comparative Example 26-8 | | Comparative nonaqueous electrolyte solution 135 | 7 | | | | | | | | 87.4 | 121.1 |

From the results shown in Table 26, it is understood that when the component (III) of the present disclosure is contained, the nonaqueous electrolyte solution battery using Si-graphite as the negative electrode active material and using a nonaqueous electrolyte solution containing an additional solute and another additive exhibits excellent cycle characteristic and exhibits improved initial resistance characteristic as a whole as compared with the cases using the comparative example compounds.

(Production of Sodium Ion Battery Positive Electrode: $NaNi_{0.5}Ti_{0.3}Mn_{0.2}O_2$ Positive Electrode)

By mixing 90% by mass of $NaNi_{0.5}Ti_{0.3}Mn_{0.2}O_2$ as a positive electrode active material, 5% by mass of acetylene black as a conductive agent, and 5% by mass of PVDF as a binder and further adding NMP as a solvent in such a manner that the content thereof became 50% by mass with respect to the total mass of the positive electrode active material, the conductive agent, and the binder, a slurry solution was prepared. The slurry solution was applied onto both sides of an aluminum foil (A1085) serving as a positive electrode current collector, dried at 150°C for 12 hours and then punched into 4 cm $\times$ 5 cm to obtain an $NaNi_{0.5}Ti_{0.3}Mn_{0.2}O_2$ positive electrode for testing in which a positive electrode active material layer was formed on the current collector.

(Production of Hard Carbon Negative Electrode)

By mixing 90% by mass of hard carbon powder (manufactured by KUREHA CORPORATION, Carbotron P) and 10% by mass of PVDF as a binder and further adding NMP as a solvent in such a manner that the content thereof became 50% by mass with respect to the total mass of the negative electrode active material and the binder, a slurry solution was prepared. The slurry solution was applied onto an aluminum foil serving as a negative electrode current collector, dried at 150°C for 12 hours and then punched into 4.5 cm $\times$ 5.5 cm to obtain a hard carbon negative electrode for testing in which a negative electrode active material layer was formed on the current collector.

(Production of Nonaqueous Electrolyte Solution Battery)

Under an argon atmosphere at a dew point of -50°C or lower, a terminal was welded to the above $NaNi_{0.5}Ti_{0.3}Mn_{0.2}O_2$ positive electrode, and both sides of the welded product were then sandwiched between two polyethylene separators (5 cm $\times$ 6 cm). Further, the outside of the sandwiched product was sandwiched between two hard carbon negative electrodes to which a terminal had been welded in advance in such a manner that the surface of the negative electrode active material faced the surface of the positive electrode active material. The resultant product was put in an aluminum laminated bag having an opening on one side, and after the nonaqueous electrolyte solution described below was vacuum-injected into the bag, the opening was sealed with heat. In this manner, the aluminum laminated nonaqueous electrolyte solution batteries according to the Examples and the Comparative Examples shown in the tables below were produced.

[Evaluation Conditions]

(Initial Charge and Discharge)

Each nonaqueous electrolyte solution battery (cell) produced as described above was left to stand at an ambient temperature of 25°C for 12 hours (impregnation time: 12 hours) and then conditioned at an ambient temperature of 25°C under the following conditions. That is, as initial charge and discharge, a charge and discharge cycle including constant-current constant-voltage charging at an upper limit charge voltage of 4.1 V and at 0.1 C rate (9 mA), discharging at 0.2 C rate constant current to a discharge end voltage of 1.5 V, subsequent constant-current constant-voltage charging at an upper limit charge voltage of 4.1 V and at 0.2 C rate, and discharging at 0.2 C rate constant current to a discharge end voltage of 1.5 V was repeated three times.

(Initial Resistance Measurement)

The cell subjected to the conditioning was charged to 4.3 V at 25°C and 0.2 C rate, and then impedance measurement was performed in an environment at -20°C to measure the resistance value.

(Cycle Test)

The cell after the measurement of the initial resistance was subjected to a charge and discharge test at an ambient temperature of 25°C to evaluate the cycle characteristic. A charge and discharge cycle was repeated at a current

value of 90 mA by a constant-current constant-voltage method at an upper limit charge voltage of 4.1 V and a lower limit discharge voltage of 1.5 V. Then, the discharge capacity retention rate at the 400th cycle in the charge and discharge test at an ambient temperature of 25°C was calculated as follows to evaluate the degree of deterioration of the cell. The "discharge capacity retention rate after cycles" expressed as the discharge capacity retention rate at the 400th cycle was determined using the following equation. The discharge capacity at the first cycle in the charge and discharge test at the ambient temperature of 25°C was defined as the initial discharge capacity.

Discharge capacity retention rate (%) after cycles = (discharge capacity at 400th cycle/initial discharge capacity) × 100

<Comparative Example 27-1> (Preparation of Comparative Nonaqueous Electrolyte Solution 136 as Base)

EC, PC, and EMC were used as nonaqueous organic solvents, and FEC was used as another additive. These materials were mixed at a volume ratio of EC: PC: FEC: EMC = 1: 2: 0.2: 6. Next, $NaPF_6$ as a solute was added and dissolved in such a manner that the content in the nonaqueous electrolyte solution became 1.00 mol/L to prepare a comparative nonaqueous electrolyte solution 136.

<Examples 27-1 to 27-14 and Comparative Examples 27-2 to 27-8>

Nonaqueous electrolyte solutions according to the Examples and the Comparative Examples were prepared by dissolving the components (III) or the comparative example compounds shown in Table 27 in the comparative nonaqueous electrolyte solution 136 at the contents shown in Table 27.

For the nonaqueous electrolyte solutions 27-1 to 27-14 and the comparative nonaqueous electrolyte solutions 136 to 143 prepared as described above, the initial resistance and the capacity retention rates in the cycle test were evaluated as described above, and the results are shown in Table 27 below. Here, these values are relative values, and the initial resistance and the capacity retention rate in the cycle test of the case using the comparative nonaqueous electrolyte solution 136 were regarded as 100 for the evaluation.

[Table 27]

Table 27

| | Nonaqueous Electrolyte Solution | Component (III) or Comparative Example Compound | | Initial Resistance (relative value) | Cycle Discharge Capacity Retention Rate (relative value) |
|---|---|---|---|---|---|
| | | Type | Content [% by mass] | | |
| Example 27-1 | Nonaqueous electrolyte solution 27-1 | (1a) | 1.0 | 82.2 | 109.2 |
| Example 27-2 | Nonaqueous electrolyte solution 27-2 | (1b) | 1.0 | 79.8 | 113.7 |
| Example 27-3 | Nonaqueous electrolyte solution 27-3 | (1c) | 1.0 | 93.8 | 115.6 |
| Example 27-4 | Nonaqueous electrolyte solution 27-4 | (1d) | 1.0 | 83.5 | 116.3 |
| Example 27-5 | Nonaqueous electrolyte solution 27-5 | (1f) | 0.5 | 84.9 | 115.5 |
| Example 27-6 | Nonaqueous electrolyte solution 27-6 | (2a) | 1.0 | 90.1 | 116.1 |
| Example 27-7 | Nonaqueous electrolyte solution 27-7 | (3a) | 1.0 | 78.9 | 113.1 |

(continued)

| | Nonaqueous Electrolyte Solution | Component (III) or Comparative Example Compound | | Initial Resistance (relative value) | Cycle Discharge Capacity Retention Rate (relative value) |
|---|---|---|---|---|---|
| | | Type | Content [% by mass] | | |
| Example 27-8 | Nonaqueous electrolyte solution 27-8 | (3b) | 1.0 | 88.2 | 109.5 |
| Example 27-9 | Nonaqueous electrolyte solution 27-9 | (4a) | 1.0 | 93.7 | 114.6 |
| Example 27-10 | Nonaqueous electrolyte solution 27-10 | (4b) | 1.0 | 92.0 | 117.7 |
| Example 27-11 | Nonaqueous electrolyte solution 27-11 | (5a) | 1.0 | 82.9 | 107.8 |
| Example 27-12 | Nonaqueous electrolyte solution 27-12 | (6a) | 1.0 | 93.4 | 109.3 |
| Example 27-13 | Nonaqueous electrolyte solution 27-13 | (7a) | 1.0 | 88.5 | 107.1 |
| Example 27-14 | Nonaqueous electrolyte solution 27-14 | (8a) | 1.0 | 92.5 | 113.0 |
| Comparative Example 27-1 | Comparative nonaqueous electrolyte solution 136 | - | | 100 | 100 |
| Comparative Example 27-2 | Comparative nonaqueous electrolyte solution 137 | 1 | 1.0 | 103.4 | 98.2 |
| Comparative Example 27-3 | Comparative nonaqueous electrolyte solution 138 | 2 | | 103.9 | 100.2 |
| Comparative Example 27-4 | Comparative nonaqueous electrolyte solution 139 | 3 | | 106.1 | 97.4 |
| Comparative Example 27-5 | Comparative nonaqueous electrolyte solution 140 | 4 | | 105.4 | 99.2 |
| Comparative Example 27-6 | Comparative nonaqueous electrolyte solution 141 | 5 | | 101.7 | 97.0 |
| Comparative Example 27-7 | Comparative nonaqueous electrolyte solution 142 | 6 | | 101.4 | 103.2 |
| Comparative Example 27-8 | Comparative nonaqueous electrolyte solution 143 | 7 | | 101.6 | 101.6 |

From the results shown in Table 27, it is understood that when the component (III) of the present disclosure is contained, the nonaqueous electrolyte solution battery using hard carbon as the negative electrode active material exhibits excellent cycle characteristic and excellent initial resistance characteristic as compared with the cases using the comparative example compounds.

<Comparative Example 28-1> (Preparation of Comparative Nonaqueous Electrolyte Solution 144 as Base)

As a nonaqueous organic solvent, a solvent obtained by mixing PC, DMC, and EMC at a volume ratio of PC: DMC: EMC = 3: 4: 3 was used, and $NaPF_6$ as a solute was dissolved in the solvent in such a manner that the content in the nonaqueous electrolyte solution became 1.00 mol/L. Further, VC and Na-DFOB shown below as other additives were dissolved at the contents shown in Table 28 below to prepare a comparative nonaqueous electrolyte solution 144.

[Chem. 117]

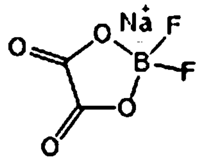

Na-DFOB

<Examples 28-1 to 28-14 and Comparative Examples 28-2 to 28-8>

Nonaqueous electrolyte solutions according to the Examples and the Comparative Examples were prepared by dissolving the components (III) or the comparative example compounds shown in Table 28 in the comparative nonaqueous electrolyte solution 144 at the contents shown in Table 28.

For the nonaqueous electrolyte solutions 28-1 to 28-14 and the comparative nonaqueous electrolyte solutions 144 to 151 prepared as described above, the initial resistance and the capacity retention rates in the cycle test were evaluated as described above, and the results are shown in Table 28 below. Here, these values are relative values, and the initial resistance and the capacity retention rate in the cycle test of the case using the comparative nonaqueous electrolyte solution 144 were regarded as 100 for the evaluation.

[Table 28]

Table 28

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 28-1 | Nonaqueous electrolyte solution 28-1 | (1a) | 1.0 | | | | | 83.6 | 108.7 |
| Example 28-2 | Nonaqueous electrolyte solution 28-2 | (1b) | 1.0 | | | | | 80.6 | 113.6 |
| Example 28-3 | Nonaqueous electrolyte solution 28-3 | (1c) | 1.0 | | | | | 94.0 | 114.9 |
| Example 28-4 | Nonaqueous electrolyte solution 28-4 | (1d) | 1.0 | | | | | 84.7 | 115.2 |
| Example 28-5 | Nonaqueous electrolyte solution 28-5 | (1f) | 0.5 | | | | | 85.1 | 114.5 |
| Example 28-6 | Nonaqueous electrolyte solution 28-6 | (2a) | 1.0 | | | | | 91.5 | 116.0 |
| Example 28-7 | Nonaqueous electrolyte solution 28-7 | (3a) | 1.0 | | | | | 79.0 | 112.3 |

(continued)

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 28-8 | Nonaqueous electrolyte solution 28-8 | (3b) | 1.0 | | | | | 88.5 | 109.0 |
| Example 28-9 | Nonaqueous electrolyte solution 28-9 | (4a) | 1.0 | | | | | 94.0 | 113.6 |
| Example 28-10 | Nonaqueous electrolyte solution 28-10 | (4b) | 1.0 | | | | | 93.1 | 116.9 |
| Example 28-11 | Nonaqueous electrolyte solution 28-11 | (5a) | 1.0 | | | | | 83.7 | 106.8 |
| Example 28-12 | Nonaqueous electrolyte solution 28-12 | (6a) | 1.0 | | | | | 94.5 | 108.9 |
| Example 28-13 | Nonaqueous electrolyte solution 28-13 | (7a) | 1.0 | | | | | 89.8 | 106.2 |
| Example 28-14 | Nonaqueous electrolyte solution 28-14 | (8a) | 1.0 | | | | | 93.2 | 112.0 |
| Comparative Example 28-1 | Comparative nonaqueous electrolyte solution 144 | - | - | VC | 1.0 | Na-DFOB | 1.0 | 100 | 100 |

(continued)

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Comparative Example 28-2 | Comparative nonaqueous electrolyte solution 145 | 1 | 1.0 | | | | | 104.8 | 97.0 |
| Comparative Example 28-3 | Comparative nonaqueous electrolyte solution 146 | 2 | | | | | | 104.5 | 100.2 |
| Comparative Example 28-4 | Comparative nonaqueous electrolyte solution 147 | 3 | | | | | | 106.5 | 96.7 |
| Comparative Example 28-5 | Comparative nonaqueous electrolyte solution 148 | 4 | | | | | | 106.1 | 97.9 |
| Comparative Example 28-6 | Comparative nonaqueous electrolyte solution 149 | 5 | | | | | | 102.5 | 96.5 |
| Comparative Example 28-7 | Comparative nonaqueous electrolyte solution 150 | 6 | | | | | | 102.2 | 103.0 |
| Comparative Example 28-8 | Comparative nonaqueous electrolyte solution 151 | 7 | | | | | | 101.8 | 101.0 |

From the results shown in Table 28, it is understood that when the component (III) of the present disclosure is contained, the nonaqueous electrolyte solution battery using hard carbon as the negative electrode active material and using a nonaqueous electrolyte solution containing another additive exhibits excellent cycle characteristic and excellent initial resistance characteristic as compared with the cases using the comparative example compounds.

<Comparative Example 29-1> (Preparation of Comparative Nonaqueous Electrolyte Solution 152 as Base)

A comparative nonaqueous electrolyte solution 152 was prepared by the same procedures as those of Comparative Example 28-1 except that Na-TFOP shown below was dissolved as another additive instead of 1.0% by mass of Na-DFOB at the content shown in Table 29 below.

[Chem. 118]

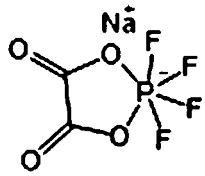

Na-TFOP

<Examples 29-1 to 29-14 and Comparative Examples 29-2 to 29-8>

Nonaqueous electrolyte solutions according to the Examples and the Comparative Examples were prepared by dissolving the components (III) or the comparative example compounds shown in Table 29 in the comparative nonaqueous electrolyte solution 152 at the contents shown in Table 29.

For the nonaqueous electrolyte solutions 29-1 to 29-14 and the comparative nonaqueous electrolyte solutions 152 to 159 prepared as described above, the initial resistance and the capacity retention rates in the cycle test were evaluated as described above, and the results are shown in Table 29 below. Here, these values are relative values, and the initial resistance and the capacity retention rate in the cycle test of the case using the comparative nonaqueous electrolyte solution 152 were regarded as 100 for the evaluation.

[Table 29]

Table 29

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 29-1 | Nonaqueous electrolyte solution 29-1 | (1a) | 1.0 | | | | | 82.5 | 108.8 |
| Example 29-2 | Nonaqueous electrolyte solution 29-2 | (1b) | 1.0 | | | | | 78.9 | 113.8 |
| Example 29-3 | Nonaqueous electrolyte solution 29-3 | (1c) | 1.0 | | | | | 92.2 | 116.1 |
| Example 29-4 | Nonaqueous electrolyte solution 29-4 | (1d) | 1.0 | | | | | 83.6 | 116.3 |
| Example 29-5 | Nonaqueous electrolyte solution 29-5 | (1f) | 0.5 | | | | | 84.1 | 117.3 |
| Example 29-6 | Nonaqueous electrolyte solution 29-6 | (2a) | 1.0 | | | | | 90.6 | 117.2 |

(continued)

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 29-7 | Nonaqueous electrolyte solution 29-7 | (3a) | 1.0 | | | | | 77.5 | 113.3 |
| Example 29-8 | Nonaqueous electrolyte solution 29-8 | (3b) | 1.0 | | | | | 87.8 | 109.6 |
| Example 29-9 | Nonaqueous electrolyte solution 29-9 | (4a) | 1.0 | | | | | 92.5 | 114.8 |
| Example 29-10 | Nonaqueous electrolyte solution 29-10 | (4b) | 1.0 | | | | | 92.6 | 116.9 |
| Example 29-11 | Nonaqueous electrolyte solution 29-11 | (5a) | 1.0 | | | | | 83.5 | 106.9 |
| Example 29-12 | Nonaqueous electrolyte solution 29-12 | (6a) | 1.0 | | | | | 94.1 | 109.4 |
| Example 29-13 | Nonaqueous electrolyte solution 29-13 | (7a) | 1.0 | | | | | 89.6 | 107.2 |
| Example 29-14 | Nonaqueous electrolyte solution 29-14 | (8a) | 1.0 | | | | | 92.0 | 112.3 |
| Comparative Example 29-1 | Comparative nonaqueous electrolyte solution 152 | - | - | VC | 1.0 | Na-TFOP | 1.0 | 100 | 100 |

(continued)

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Comparative Example 29-2 | Comparative nonaqueous electrolyte solution 153 | 1 | 1.0 | | | | | 103.8 | 97.7 |
| Comparative Example 29-3 | Comparative nonaqueous electrolyte solution 154 | 2 | 1.0 | | | | | 102.7 | 100.9 |
| Comparative Example 29-4 | Comparative nonaqueous electrolyte solution 155 | 3 | 1.0 | | | | | 105.7 | 97.5 |
| Comparative Example 29-5 | Comparative nonaqueous electrolyte solution 156 | 4 | 1.0 | | | | | 105.6 | 99.0 |
| Comparative Example 29-6 | Comparative nonaqueous electrolyte solution 157 | 5 | 1.0 | | | | | 101.8 | 97.6 |
| Comparative Example 29-7 | Comparative nonaqueous electrolyte solution 158 | 6 | 1.0 | | | | | 101.8 | 103.5 |
| Comparative Example 29-8 | Comparative nonaqueous electrolyte solution 159 | 7 | 1.0 | | | | | 101.4 | 102.0 |

From the results shown in Table 29, it is understood that when the component (III) of the present disclosure is contained, the nonaqueous electrolyte solution battery using hard carbon as the negative electrode active material and using a nonaqueous electrolyte solution containing another additive exhibits excellent cycle characteristic and excellent initial resistance characteristic as compared with the cases using the comparative example compounds.

<Comparative Example 30-1> (Preparation of Comparative Nonaqueous Electrolyte Solution 160 as Base)

A comparative nonaqueous electrolyte solution 160 was prepared by the same procedures as those of Comparative Example 28-1 except that Na-DFBOP shown below was dissolved as another additive instead of 1.0% by mass of Na-DFOB at the content shown in Table 30 below.

[Chem. 119]

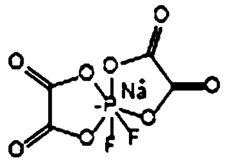

Na-DFBOP

<Examples 30-1 to 30-14 and Comparative Examples 30-2 to 30-8>

Nonaqueous electrolyte solutions according to the Examples and the Comparative Examples were prepared by dissolving the components (III) or the comparative example compounds shown in Table 30 in the comparative nonaqueous electrolyte solution 160 at the contents shown in Table 30.

For the nonaqueous electrolyte solutions 30-1 to 30-14 and the comparative nonaqueous electrolyte solutions 160 to 167 prepared as described above, the initial resistance and the capacity retention rates in the cycle test were evaluated as described above, and the results are shown in Table 30 below. Here, these values are relative values, and the initial resistance and the capacity retention rate in the cycle test of the case using the comparative nonaqueous electrolyte solution 160 were regarded as 100 for the evaluation.

[Table 30]

Table 30

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 30-1 | Nonaqueous electrolyte solution 30-1 | (1a) | 1.0 | | | | | 82.4 | 109.2 |
| Example 30-2 | Nonaqueous electrolyte solution 30-2 | (1b) | 1.0 | | | | | 78.5 | 114.4 |
| Example 30-3 | Nonaqueous electrolyte solution 30-3 | (1c) | 1.0 | | | | | 91.5 | 116.2 |
| Example 30-4 | Nonaqueous electrolyte solution 30-4 | (1d) | 1.0 | | | | | 82.8 | 117.2 |
| Example 30-5 | Nonaqueous electrolyte solution 30-5 | (1f) | 0.5 | | | | | 83.9 | 117.1 |
| Example 30-6 | Nonaqueous electrolyte solution 30-6 | (2a) | 1.0 | | | | | 90.0 | 118.2 |
| Example 30-7 | Nonaqueous electrolyte solution 30-7 | (3a) | 1.0 | | | | | 77.4 | 114.4 |
| Example 30-8 | Nonaqueous electrolyte solution 30-8 | (3b) | 1.0 | | | | | 87.2 | 110.6 |
| Example 30-9 | Nonaqueous electrolyte solution 30-9 | (4a) | 1.0 | | | | | 91.7 | 115.1 |
| Example 30-10 | Nonaqueous electrolyte solution 30-10 | (4b) | 1.0 | | | | | 92.0 | 117.0 |
| Example 30-11 | Nonaqueous electrolyte solution 30-11 | (5a) | 1.0 | | | | | 83.2 | 107.3 |
| Example 30-12 | Nonaqueous electrolyte solution 30-12 | (6a) | 1.0 | | | | | 93.5 | 109.6 |
| Example 30-13 | Nonaqueous electrolyte solution 30-13 | (7a) | 1.0 | | | | | 89.0 | 108.6 |
| Example 30-14 | Nonaqueous electrolyte solution 30-14 | (8a) | 1.0 | | | | | 91.3 | 112.5 |
| Comparative Example 30-1 | Comparative nonaqueous electrolyte solution 160 | - | - | VC | 1.0 | Na-DFBOP | 1.0 | 100 | 100 |

(continued)

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Comparative Example 30-2 | Comparative nonaqueous electrolyte solution 161 | 1 | 1.0 | | | | | 103.1 | 97.9 |
| Comparative Example 30-3 | Comparative nonaqueous electrolyte solution 162 | 2 | | | | | | 101.9 | 101.9 |
| Comparative Example 30-4 | Comparative nonaqueous electrolyte solution 163 | 3 | | | | | | 105.0 | 98.3 |
| Comparative Example 30-5 | Comparative nonaqueous electrolyte solution 164 | 4 | | | | | | 105.3 | 100.3 |
| Comparative Example 30-6 | Comparative nonaqueous electrolyte solution 165 | 5 | | | | | | 101.0 | 97.6 |
| Comparative Example 30-7 | Comparative nonaqueous electrolyte solution 166 | 6 | | | | | | 101.4 | 104.3 |
| Comparative Example 30-8 | Comparative nonaqueous electrolyte solution 167 | 7 | | | | | | 100.5 | 102.3 |

From the results shown in Table 30, it is understood that when the component (III) of the present disclosure is contained, the nonaqueous electrolyte solution battery using hard carbon as the negative electrode active material and using a nonaqueous electrolyte solution containing another additive exhibits excellent cycle characteristic and excellent initial resistance characteristic as compared with the cases using the comparative example compounds.

<Comparative Example 31-1> (Preparation of Comparative Nonaqueous Electrolyte Solution 168 as Base)

A comparative nonaqueous electrolyte solution 168 was prepared by the same procedures as those of Comparative Example 28-1 except that Na-DFP represented by the formula below was dissolved as another additive instead of 1.0% by mass of Na-DFOB at the content shown in Table 31 below.

[Chem. 120]

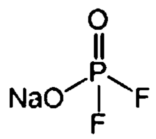

Na－DFP

<Examples 31-1 to 31-14 and Comparative Examples 31-2 to 31-8>

Nonaqueous electrolyte solutions according to the Examples and the Comparative Examples were prepared by dissolving the components (III) or the comparative example compounds shown in Table 31 in the comparative nonaqueous electrolyte solution 168 at the contents shown in Table 31.

For the nonaqueous electrolyte solutions 31-1 to 31-14 and the comparative nonaqueous electrolyte solutions 168 to 175 prepared as described above, the initial resistance and the capacity retention rates in the cycle test were evaluated as described above, and the results are shown in Table 31 below. Here, these values are relative values, and the initial resistance and the capacity retention rate in the cycle test of the case using the comparative nonaqueous electrolyte solution 168 were regarded as 100 for the evaluation.

[Table 31]

Table 31

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 31-1 | Nonaqueous electrolyte solution 31-1 | (1a) | 1.0 | | | | | 83.3 | 108.1 |
| Example 31-2 | Nonaqueous electrolyte solution 31-2 | (1b) | 1.0 | | | | | 80.1 | 113.0 |
| Example 31-3 | Nonaqueous electrolyte solution 31-3 | (1c) | 1.0 | | | | | 93.3 | 116.1 |

(continued)

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 31-4 | Nonaqueous electrolyte solution 31-4 | (1d) | 1.0 | | | | | 84.3 | 116.6 |
| Example 31-5 | Nonaqueous electrolyte solution 31-5 | (1f) | 0.5 | | | | | 86.1 | 114.9 |
| Example 31-6 | Nonaqueous electrolyte solution 31-6 | (2a) | 1.0 | | | | | 90.6 | 116.7 |
| Example 31-7 | Nonaqueous electrolyte solution 31-7 | (3a) | 1.0 | | | | | 78.6 | 113.7 |
| Example 31-8 | Nonaqueous electrolyte solution 31-8 | (3b) | 1.0 | | | | | 88.5 | 109.3 |
| Example 31-9 | Nonaqueous electrolyte solution 31-9 | (4a) | 1.0 | | | | | 93.6 | 113.8 |
| Example 31-10 | Nonaqueous electrolyte solution 31-10 | (4b) | 1.0 | | | | | 92.7 | 115.6 |
| Example 31-11 | Nonaqueous electrolyte solution 31-11 | (5a) | 1.0 | | | | | 85.0 | 106.9 |
| Example 31-12 | Nonaqueous electrolyte solution 31-12 | (6a) | 1.0 | | | | | 95.4 | 109.4 |
| Example 31-13 | Nonaqueous electrolyte solution 31-13 | (7a) | 1.0 | | | | | 90.2 | 107.4 |
| Example 31-14 | Nonaqueous electrolyte solution 31-14 | (8a) | 1.0 | | | | | 91.7 | 112.0 |
| Comparative Example 31-1 | Comparative nonaqueous electrolyte solution 168 | - | - | Na-DFP | 0.50 | VC | 1.0 | 100 | 100 |

(continued)

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Comparative Example 31-2 | Comparative nonaqueous electrolyte solution 169 | 1 | 1.0 | | | | | 105.3 | 97.1 |
| Comparative Example 31-3 | Comparative nonaqueous electrolyte solution 170 | 2 | | | | | | 102.8 | 101.2 |
| Comparative Example 31-4 | Comparative nonaqueous electrolyte solution 171 | 3 | | | | | | 105.2 | 97.3 |
| Comparative Example 31-5 | Comparative nonaqueous electrolyte solution 172 | 4 | | | | | | 107.4 | 99.6 |
| Comparative Example 31-6 | Comparative nonaqueous electrolyte solution 173 | 5 | | | | | | 102.4 | 97.6 |
| Comparative Example 31-7 | Comparative nonaqueous electrolyte solution 174 | 6 | | | | | | 102.0 | 102.9 |
| Comparative Example 31-8 | Comparative nonaqueous electrolyte solution 175 | 7 | | | | | | 102.2 | 101.8 |

From the results shown in Table 31, it is understood that when the component (III) of the present disclosure is contained, the nonaqueous electrolyte solution battery using hard carbon as the negative electrode active material and using a nonaqueous electrolyte solution containing another additive exhibits excellent cycle characteristic and excellent initial resistance characteristic as compared with the cases using the comparative example compounds.

<Comparative Example 32-1> (Preparation of Comparative Nonaqueous Electrolyte Solution 176 as Base)

A comparative nonaqueous electrolyte solution 176 was prepared by the same procedures as those of Comparative Example 28-1 except that Na-FS represented by the formula below was dissolved as another additive instead of 1.0% by mass of Na-DFOB at the content shown in Table 32 below.

[Chem. 121]

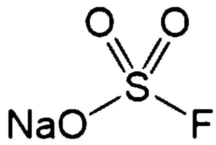

Na－FS

<Examples 32-1 to 32-14 and Comparative Examples 32-2 to 32-8>

Nonaqueous electrolyte solutions according to the Examples and the Comparative Examples were prepared by dissolving the components (III) or the comparative example compounds shown in Table 32 in the comparative nonaqueous electrolyte solution 176 at the contents shown in Table 32.

For the nonaqueous electrolyte solutions 32-1 to 32-14 and the comparative nonaqueous electrolyte solutions 176 to 183 prepared as described above, the initial resistance and the capacity retention rates in the cycle test were evaluated as described above, and the results are shown in Table 32 below. Here, these values are relative values, and the initial resistance and the capacity retention rate in the cycle test of the case using the comparative nonaqueous electrolyte solution 176 were regarded as 100 for the evaluation.

[Table 32]

Table 32

| | Nonaqueous Electrolyte Solution | Component (III) or Comparative Example Compound | | Other Additive (I) | | Other Additive (II) | | **Initial** Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 32-1 | Nonaqueous electrolyte solution 32-1 | (1a) | 1.0 | | | | | 84.5 | 106.7 |
| Example 32-2 | Nonaqueous electrolyte solution 32-2 | (1b) | 1.0 | | | | | 81.2 | 111.7 |
| Example 32-3 | Nonaqueous electrolyte solution 32-3 | (1c) | 1.0 | | | | | 94.8 | 115.0 |
| Example 32-4 | Nonaqueous electrolyte solution 32-4 | (1d) | 1.0 | | | | | 85.8 | 116.5 |
| Example 32-5 | Nonaqueous electrolyte solution 32-5 | (1f) | 0.5 | | | | | 86.3 | 115.9 |
| Example 32-6 | Nonaqueous electrolyte solution 32-6 | (2a) | 1.0 | | | | | 90.7 | 116.4 |
| Example 32-7 | Nonaqueous electrolyte solution 32-7 | (3a) | 1.0 | | | | | 79.7 | 112.6 |

(continued)

| | Nonaqueous Electrolyte Solution | Component (III) or Comparative Example Compound | | Other Additive (I) | | Other Additive (II) | | **Initial** Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 32-8 | Nonaqueous electrolyte solution 32-8 | (3b) | 1.0 | | | | | 89.9 | 109.3 |
| Example 32-9 | Nonaqueous electrolyte solution 32-9 | (4a) | 1.0 | | | | | 95.1 | 112.8 |
| Example 32-10 | Nonaqueous electrolyte solution 32-10 | (4b) | 1.0 | | | | | 93.5 | 115.4 |
| Example 32-11 | Nonaqueous electrolyte solution 32-11 | (5a) | 1.0 | | | | | 85.3 | 106.3 |
| Example 32-12 | Nonaqueous electrolyte solution 32-12 | (6a) | 1.0 | | | | | 96.7 | 108.6 |
| Example 32-13 | Nonaqueous electrolyte solution 32-13 | (7a) | 1.0 | | | | | 90.3 | 106.9 |
| Example 32-14 | Nonaqueous electrolyte solution 32-14 | (8a) | 1.0 | | | | | 92.6 | 111.3 |
| Comparative Example 32-1 | Comparative nonaqueous electrolyte solution 176 | - | - | VC | 1.0 | Na-FS | 1.0 | 100 | 100 |

(continued)

| | Nonaqueous Electrolyte Solution | Component (III) or Comparative Example Compound | | Other Additive (I) | | Other Additive (II) | | **Initial** Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Comparative Example 32-2 | Comparative nonaqueous electrolyte solution 177 | 1 | 1.0 | | | | | 105.3 | 96.9 |
| Comparative Example 32-3 | Comparative nonaqueous electrolyte solution 178 | 2 | | | | | | 103.1 | 100.8 |
| Comparative Example 32-4 | Comparative nonaqueous electrolyte solution 179 | 3 | | | | | | 106.0 | 97.3 |
| Comparative Example 32-5 | Comparative nonaqueous electrolyte solution 180 | 4 | | | | | | 108.7 | 99.5 |
| Comparative Example 32-6 | Comparative nonaqueous electrolyte solution 181 | 5 | | | | | | 102.8 | 96.4 |
| Comparative Example 32-7 | Comparative nonaqueous electrolyte solution 182 | 6 | | | | | | 103.2 | 102.3 |
| Comparative Example 32-8 | Comparative nonaqueous electrolyte solution 183 | 7 | | | | | | 103.3 | 101.7 |

From the results shown in Table 32, it is understood that when the component (III) of the present disclosure is contained, the nonaqueous electrolyte solution battery using hard carbon as the negative electrode active material and using a nonaqueous electrolyte solution containing another additive exhibits excellent cycle characteristic and excellent initial resistance characteristic as compared with the cases using the comparative example compounds.

<Comparative Example 33-1> (Preparation of Comparative Nonaqueous Electrolyte Solution 184 as Base)

A comparative nonaqueous electrolyte solution 184 was prepared by the same procedures as those of Comparative Example 28-1 except that DTD was dissolved as another additive instead of 1.0% by mass of Na-DFOB at the content shown in Table 33 below.

<Examples 33-1 to 33-14 and Comparative Examples 33-2 to 33-8>

Nonaqueous electrolyte solutions according to the Examples and the Comparative Examples were prepared by dissolving the components (III) or the comparative example compounds shown in Table 33 in the comparative nonaqueous electrolyte solution 184 at the contents shown in Table 33.

For the nonaqueous electrolyte solutions 33-1 to 33-14 and the comparative nonaqueous electrolyte solutions

184 to 191 prepared as described above, the initial resistance and the capacity retention rates in the cycle test were evaluated as described above, and the results are shown in Table 33 below. Here, these values are relative values, and the initial resistance and the capacity retention rate in the cycle test of the case using the comparative nonaqueous electrolyte solution 184 were regarded as 100 for the evaluation.

[Table 33]

Table 33

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 33-1 | Nonaqueous electrolyte solution 33-1 | (1a) | 1.0 | | | | | 83.5 | 108.1 |
| Example 33-2 | Nonaqueous electrolyte solution 33-2 | (1b) | 1.0 | | | | | 80.6 | 111.8 |
| Example 33-3 | Nonaqueous electrolyte solution 33-3 | (1c) | 1.0 | | | | | 94.4 | 115.7 |
| Example 33-4 | Nonaqueous electrolyte solution 33-4 | (1d) | 1.0 | | | | | 84.6 | 117.0 |
| Example 33-5 | Nonaqueous electrolyte solution 33-5 | (1f) | 0.5 | | | | | 85.1 | 116.3 |
| Example 33-6 | Nonaqueous electrolyte solution 33-6 | (2a) | 1.0 | | | | | 89.3 | 117.5 |
| Example 33-7 | Nonaqueous electrolyte solution 33-7 | (3a) | 1.0 | | | | | 79.2 | 113.7 |
| Example 33-8 | Nonaqueous electrolyte solution 33-8 | (3b) | 1.0 | | | | | 88.7 | 110.6 |
| Example 33-9 | Nonaqueous electrolyte solution 33-9 | (4a) | 1.0 | | | | | 93.8 | 112.9 |
| Example 33-10 | Nonaqueous electrolyte solution 33-10 | (4b) | 1.0 | | | | | 92.8 | 116.6 |
| Example 33-11 | Nonaqueous electrolyte solution 33-11 | (5a) | 1.0 | | | | | 84.6 | 107.3 |

(continued)

| | Nonaqueous Electrolyte Solution | Component (III) or Comparative Example Compound | | Other Additive (I) | | Other Additive (II) | | Initial Resistance (relative value) | Cycle Discharge Capacity Retention Rate (relative value) |
|---|---|---|---|---|---|---|---|---|---|
| | | Type | Content [% by mass] | Type | Content [% by mass] | Type | Content [% by mass] | | |
| Example 33-12 | Nonaqueous electrolyte solution 33-12 | (6a) | 1.0 | VC | 1.0 | DTD | 1.0 | 96.0 | 109.7 |
| Example 33-13 | Nonaqueous electrolyte solution 33-13 | (7a) | 1.0 | VC | 1.0 | DTD | 1.0 | 89.5 | 107.3 |
| Example 33-14 | Nonaqueous electrolyte solution 33-14 | (8a) | 1.0 | VC | 1.0 | DTD | 1.0 | 91.8 | 112.6 |
| Comparative Example 33-1 | Comparative nonaqueous electrolyte solution 184 | - | - | VC | 1.0 | DTD | 1.0 | 100 | 100 |
| Comparative Example 33-2 | Comparative nonaqueous electrolyte solution 185 | 1 | 1.0 | VC | 1.0 | DTD | 1.0 | 105.0 | 97.1 |
| Comparative Example 33-3 | Comparative nonaqueous electrolyte solution 186 | 2 | 1.0 | VC | 1.0 | DTD | 1.0 | 102.6 | 100.9 |
| Comparative Example 33-4 | Comparative nonaqueous electrolyte solution 187 | 3 | 1.0 | VC | 1.0 | DTD | 1.0 | 104.5 | 97.3 |
| Comparative Example 33-5 | Comparative nonaqueous electrolyte solution 188 | 4 | 1.0 | VC | 1.0 | DTD | 1.0 | 108.0 | 100.0 |
| Comparative Example 33-6 | Comparative nonaqueous electrolyte solution 189 | 5 | 1.0 | VC | 1.0 | DTD | 1.0 | 102.6 | 97.7 |
| Comparative Example 33-7 | Comparative nonaqueous electrolyte solution 190 | 6 | 1.0 | VC | 1.0 | DTD | 1.0 | 101.9 | 102.4 |
| Comparative Example 33-8 | Comparative nonaqueous electrolyte solution 191 | 7 | 1.0 | VC | 1.0 | DTD | 1.0 | 103.2 | 103.0 |

From the results shown in Table 33, it is understood that when the component (III) of the present disclosure is contained, the nonaqueous electrolyte solution battery using hard carbon as the negative electrode active material and using a nonaqueous electrolyte solution containing another additive exhibits excellent cycle characteristic and excellent initial resistance characteristic as compared with the cases using the comparative example compounds.

## Claims

1. A nonaqueous electrolyte solution comprising:

(I) a solute;
(II) a nonaqueous organic solvent; and
(III) at least one selected from the group consisting of a compound represented by the following general formula (1), a compound represented by the general formula (2), a compound represented by the general formula (3), a compound represented by the general formula (4), a compound represented by the general formula (5), a compound represented by the general formula (6), a compound represented by the general formula (7), and a compound represented by the general formula (8):

[Chem. 1]

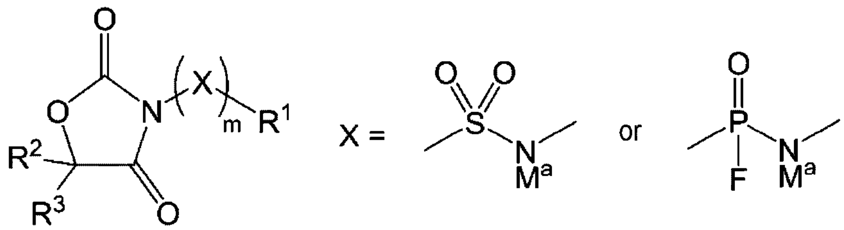

(1)

[wherein in the general formula (1),
m represents an integer of 0 or 1,
$M^a$ represents a hydrogen atom or $(M_1^{A+})_a$, wherein $M_1^{A+}$ represents a metal cation or an onium cation, A represents the valence of the cation, and a represents a number satisfying $A \times a = 1$,
$R^1$ represents $-S(=O)_2R^a$ or $-P(=O)(R^b)(R^c)$,
$R^a$, $R^b$, and $R^c$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_2^{B+})_b$, wherein $M_2^{B+}$ represents a proton, a metal cation, or an onium cation, B represents the valence of the cation, and b represents a number satisfying $B \times b = 1$, wherein $R^b$ and $R^c$ may together form a 5-membered ring or a 6-membered ring together with the phosphorus atom, and
$R^2$ and $R^3$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^2$ and $R^3$ may be integrated to form a carbonyl group]

[Chem. 2]

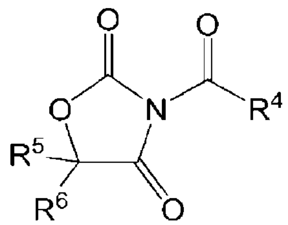

(2)

[wherein in the general formula (2),
$R^4$ represents a halogen atom, $-(O)_{x1}-S(=O)_2R^d$, $-(O)_{y1}-P(=O)(R^e)(R^f)$, or $-O^-(M_3^{C+})_c$, wherein $M_3^{C+}$ represents a proton, a metal cation, or an onium cation, C represents the valence of the cation, c represents a number satisfying $C \times c = 1$, and x1 and y1 each represent an integer of 0 or 1,
$R^d$, $R^e$, and $R^f$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_4^{D+})_d$, wherein $M_4^{D+}$ represents a proton, a metal cation, or an onium cation, D represents the valence of the cation, and d represents a number satisfying $D \times d = 1$, and
$R^5$ and $R^6$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^5$ and $R^6$ may be integrated to form a carbonyl group]

[Chem. 3]

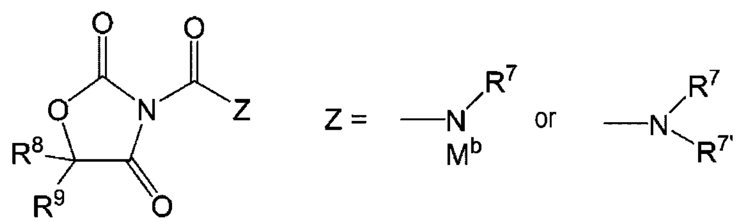

(3)

[wherein in the general formula (3),
$M^b$ represents a hydrogen atom or $(M_5^{E+})_e$, wherein $M_5^{E+}$ represents a metal cation or an onium cation, E represents the valence of the cation, and e represents a number satisfying $E \times e = 1$,
$R^7$ and $R^{7'}$ each independently represent a hydrogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon

group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), $-S(=O)_2R^g$, or $-P(=O)(R^h)(R^i)$,

$R^g$, $R^h$, and $R^i$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_6^{F+})_f$, wherein $M_6^{F+}$ represents a proton, a metal cation, or an onium cation, F represents the valence of the cation, and f represents a number satisfying $F \times f = 1$, and

$R^8$ and $R^9$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^8$ and $R^9$ may be integrated to form a carbonyl group]

[Chem. 4]

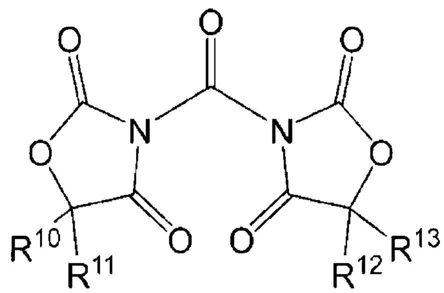

(4)

[wherein in the general formula (4),

$R^{10}$, $R^{11}$, $R^{12}$, and $R^{13}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{10}$ and $R^{11}$ or $R^{12}$ and $R^{13}$ may be each independently integrated to form a carbonyl group]

[Chem. 5]

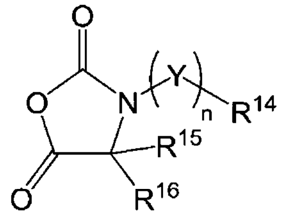 Y = 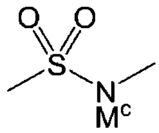 or 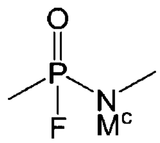 (5)

[wherein in the general formula (5),

n represents an integer of 0 or 1,

$M^c$ represents a hydrogen atom or $(M_7{}^{G+})_g$, wherein $M_7{}^{G+}$ represents a metal cation or an onium cation, G represents the valence of the cation, and g represents a number satisfying G × g = 1,

$R^{14}$ represents $-S(=O)_2R^j$ or $-P(=O)(R^k)(R^l)$, wherein $R^j$, $R^k$, and $R^l$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_8{}^{H+})_h$, wherein $M_8{}^{H+}$ represents a proton, a metal cation, or an onium cation, H represents the valence of the cation, and h represents a number satisfying H × h = 1, and

$R^{15}$ and $R^{16}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{15}$ and $R^{16}$ may be integrated to form a carbonyl group]

[Chem. 6]

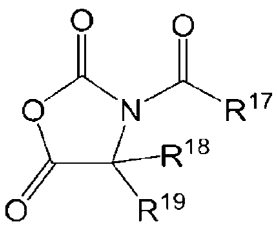 (6)

[wherein in the general formula (6),

$R^{17}$ represents a halogen atom, $-(O)_{x2}-S(=O)_2R^m$, $-(O)_{y2}-P(=O)(R^n)(R^o)$, or $-O^-(M_9{}^{I+})_i$, wherein $M_9{}^{I+}$ represents a proton, a metal cation, or an onium cation, I represents the valence of the cation, and i represents a number satisfying I × i = 1,

x2 and y2 each represent an integer of 0 or 1,

$R^m$, $R^n$, and $R^o$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon

atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_{10}^{J+})_j$, wherein $M_{10}^{J+}$ represents a proton, a metal cation, or an onium cation, J represents the valence of the cation, and j represents a number satisfying $J \times j = 1$, and

$R^{18}$ and $R^{19}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{18}$ and $R^{19}$ may be integrated to form a carbonyl group]

[Chem. 7]

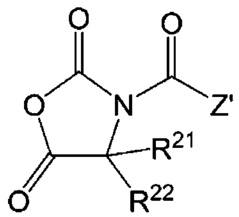

Z' =

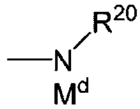

or

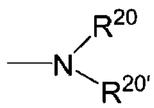

(7)

[wherein in the general formula (7),

$M^d$ represents a hydrogen atom or $(M_{11}^{K+})_k$, wherein $M_{11}^{K+}$ represents a metal cation or an onium cation, K represents the valence of the cation, and k represents a number satisfying $K \times k = 1$,

$R^{20}$ and $R^{20'}$ each independently represent a hydrogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), $-S(=O)_2R^p$, or $-P(=O)(R^q)(R^r)$,

$R^p$, $R^q$, and $R^r$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_{12}^{L+})_1$, wherein $M_{12}^{L+}$ represents a proton, a metal cation, or an onium cation, L represents the valence of the cation, and 1 represents a number satisfying $L \times 1 = 1$, and

$R^{21}$ and $R^{22}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon

atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{21}$ and $R^{22}$ may be integrated to form a carbonyl group]

[Chem. 8]

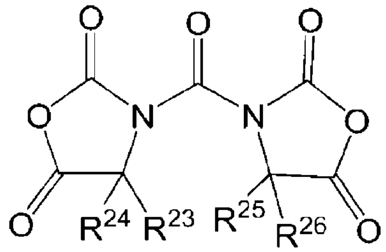

(8)

[wherein in the general formula (8),
$R^{23}$, $R^{24}$, $R^{25}$, and $R^{26}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{23}$ and $R^{24}$ or $R^{25}$ and $R^{26}$ may be each independently integrated to form a carbonyl group.]

2. The nonaqueous electrolyte solution according to claim 1, wherein the concentration of the (III) is 0.009 to 11.5% by mass with respect to the total amount of the nonaqueous electrolyte solution.

3. The nonaqueous electrolyte solution according to claim 1, wherein the (III) is at least one selected from the group consisting of the compound represented by the general formula (1), the compound represented by the general formula (2), the compound represented by the general formula (3), the compound represented by the general formula (4), the compound represented by the general formula (5), the compound represented by the general formula (6), and the compound represented by the general formula (7).

4. The nonaqueous electrolyte solution according to claim 1, wherein the (III) is at least one selected from the group consisting of the compound represented by the general formula (1), the compound represented by the general formula (2), the compound represented by the general formula (3), and the compound represented by the general formula (4).

5. The nonaqueous electrolyte solution according to claim 1, wherein the compound represented by the general formula (1) is at least one selected from the group consisting of the following compounds.

[Chem. 9]

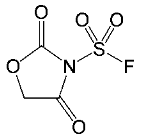 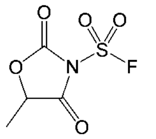 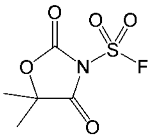 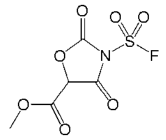 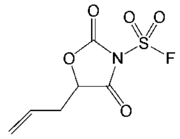 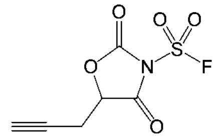

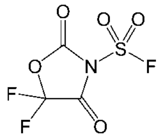 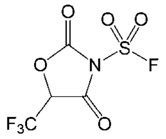 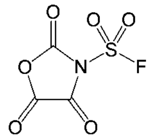 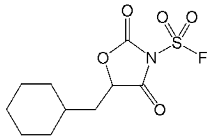 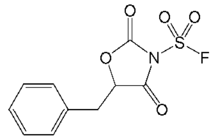

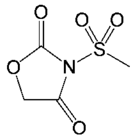 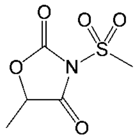 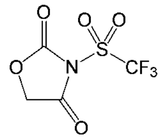 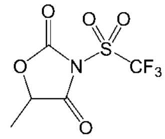 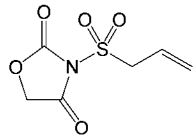

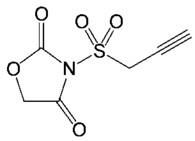 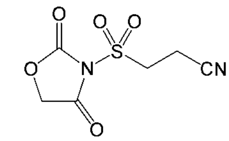 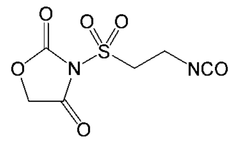 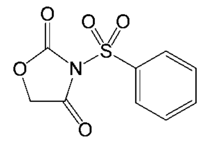

[Chem. 10]

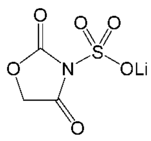 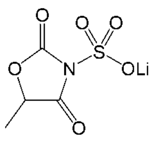 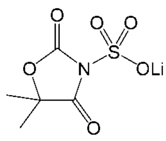 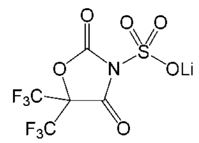 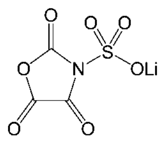

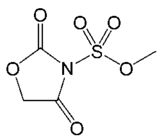 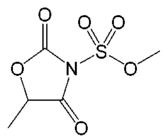 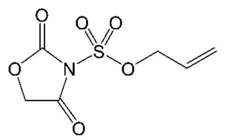 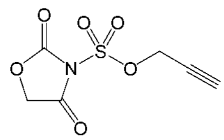 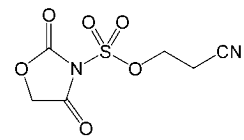

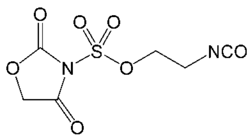 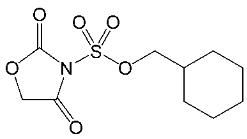 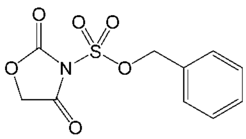 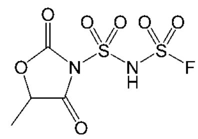 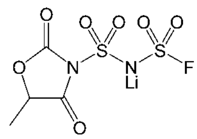

[Chem. 11]

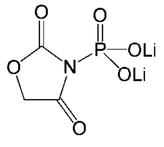
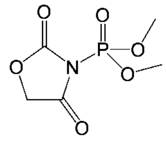
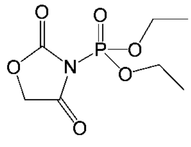
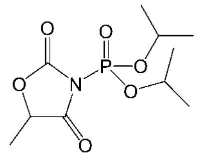
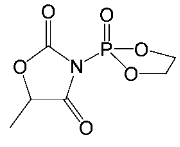
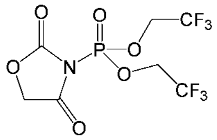
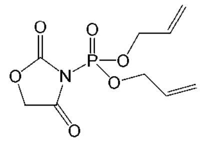
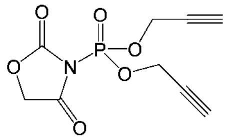
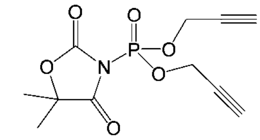
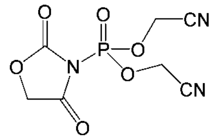
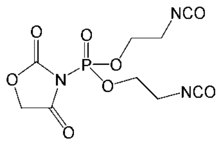
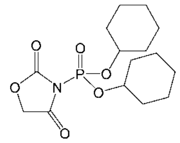
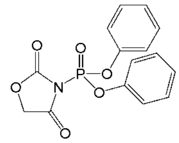
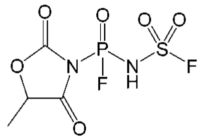
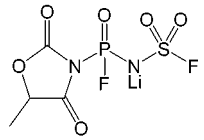
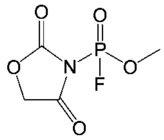
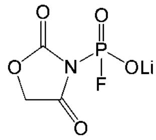
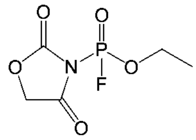
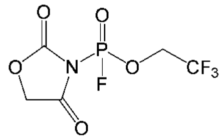
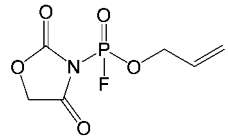
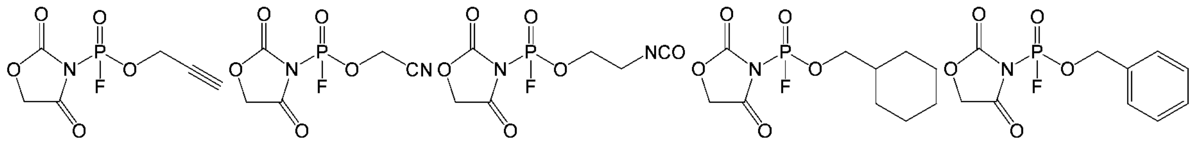
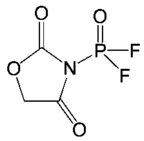
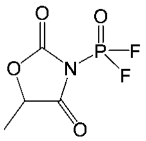
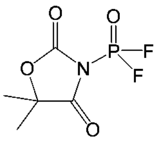
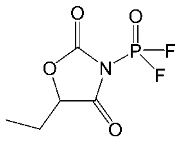
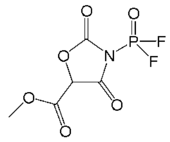
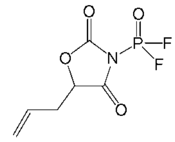
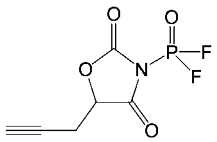
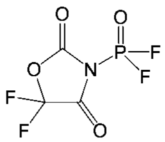
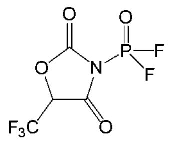
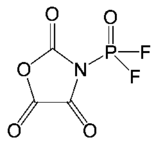
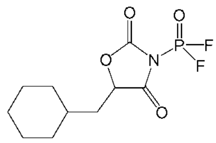
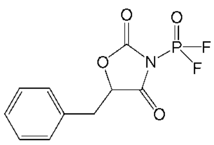

**6.** The nonaqueous electrolyte solution according to claim 1, wherein the compound represented by the general formula (2) is at least one selected from the group consisting of the following compounds.

[Chem. 12]

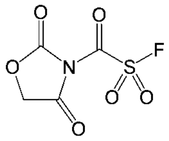
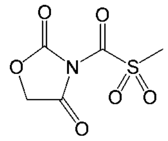
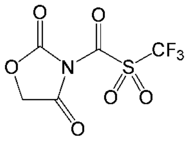
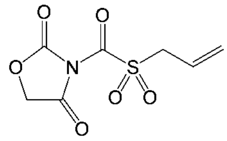
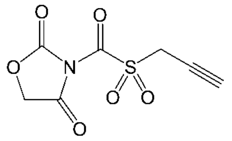

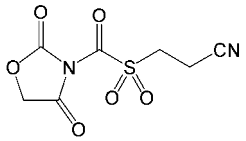
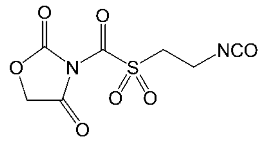
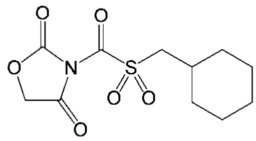
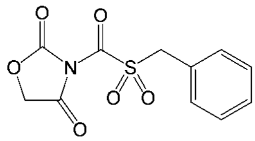

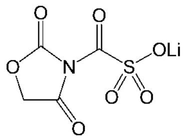
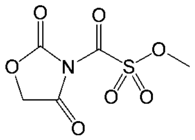
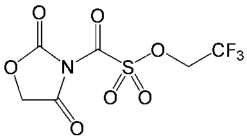
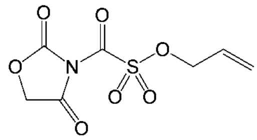

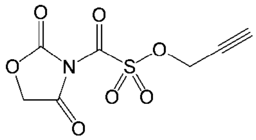
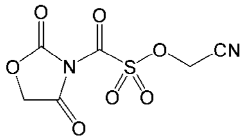
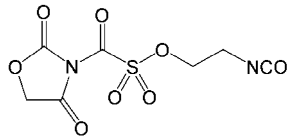
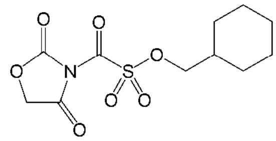

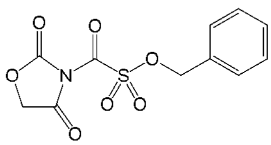
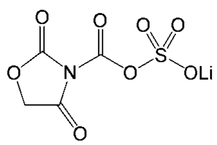
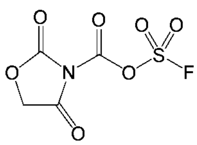
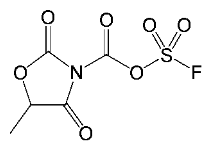

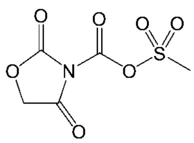
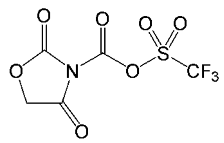
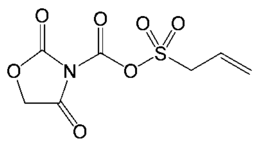
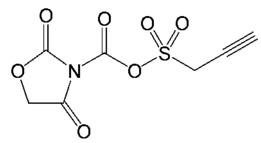

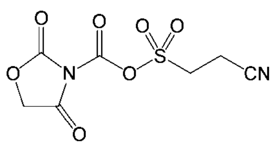
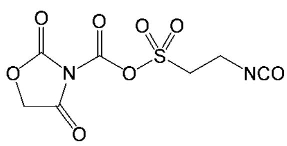
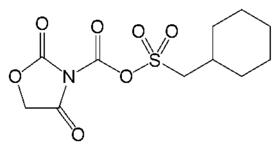
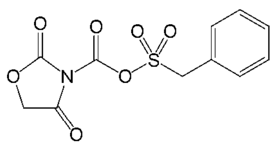

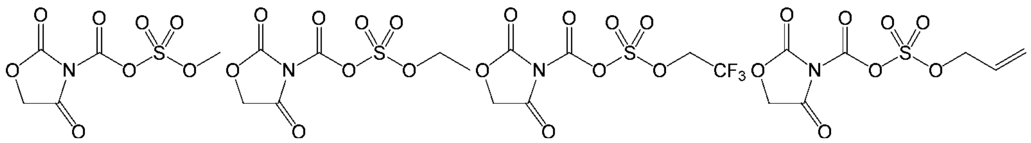

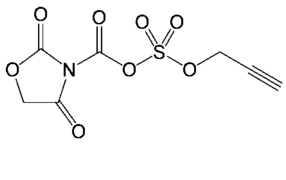
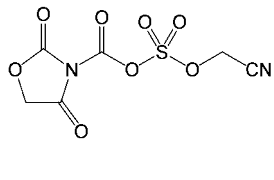
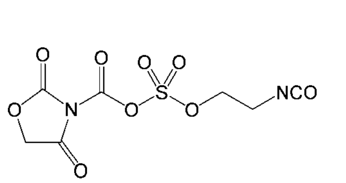

[Chem. 13]

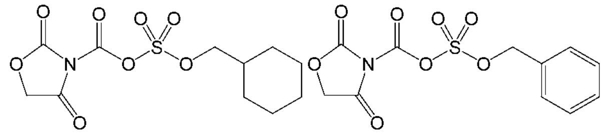
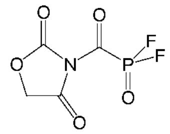
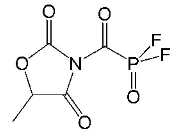
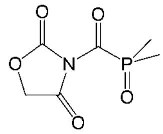
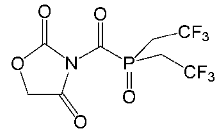
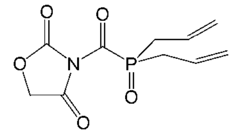
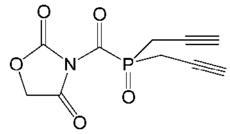
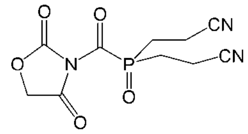
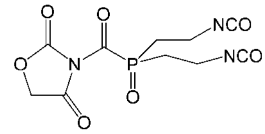
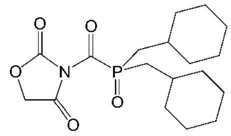
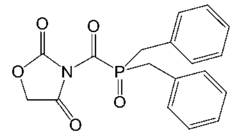
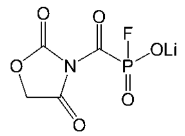
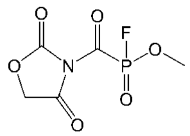
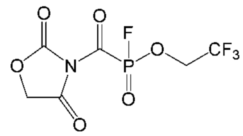
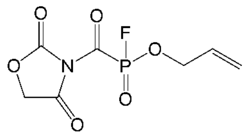
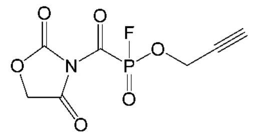
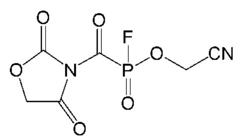
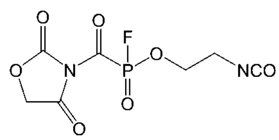
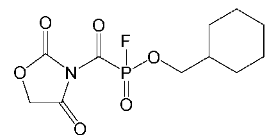
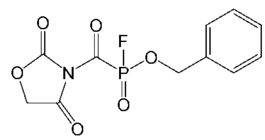
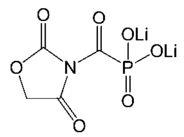
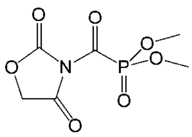
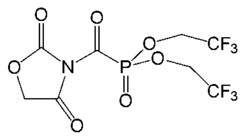
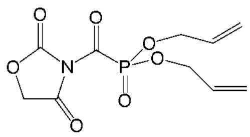
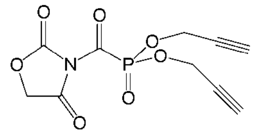
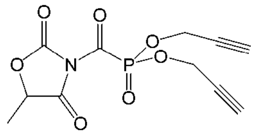
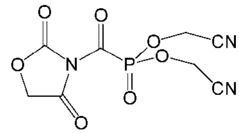
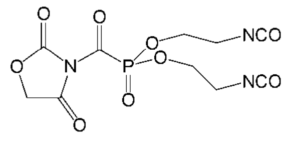
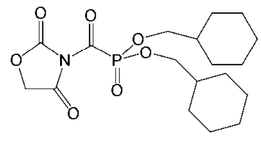
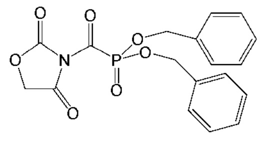
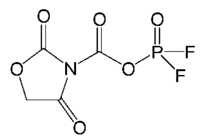
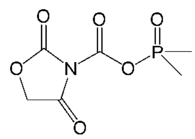
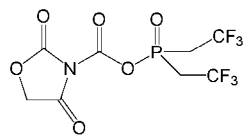
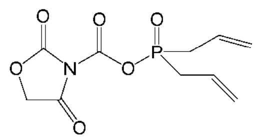

[Chem. 14]

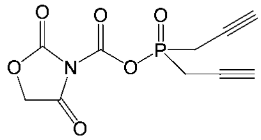 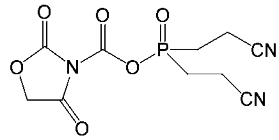 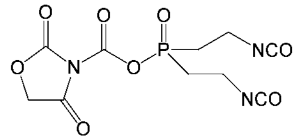 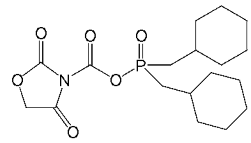

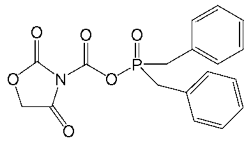 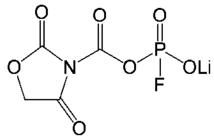 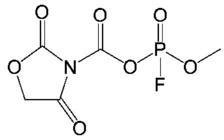 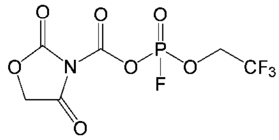

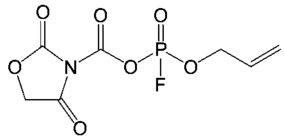 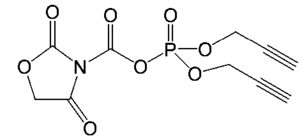 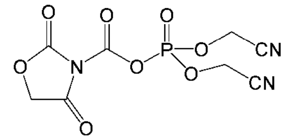 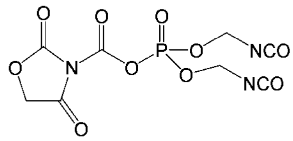

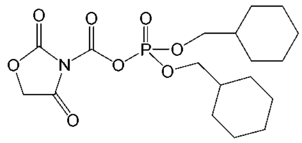 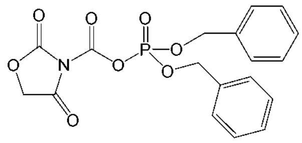

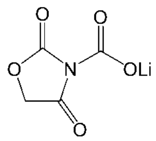 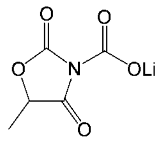 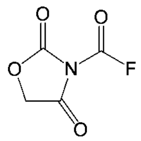 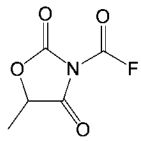 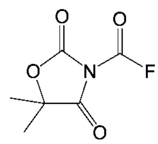

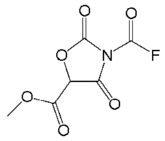 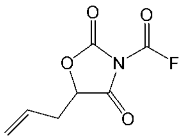 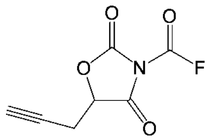 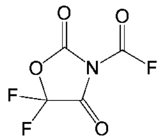

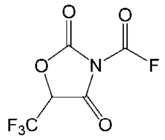 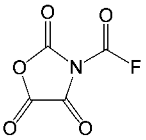 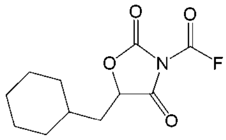 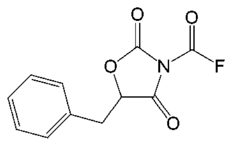

**7.** The nonaqueous electrolyte solution according to claim 1, wherein the compound represented by the general formula (3) is at least one selected from the group consisting of the following compounds.

[Chem. 15]

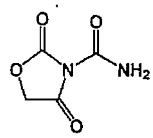 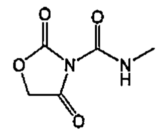 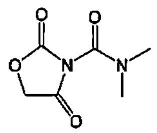 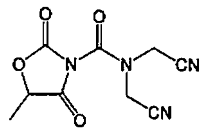

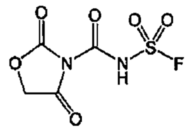 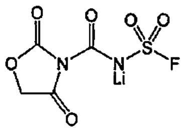 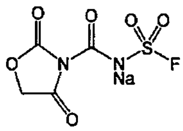 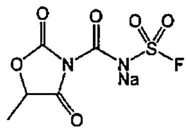 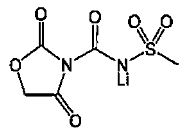

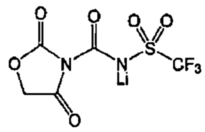 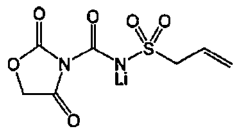 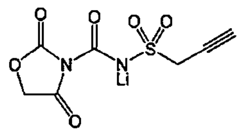

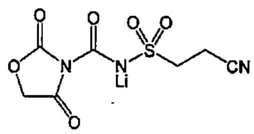 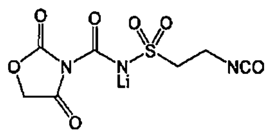 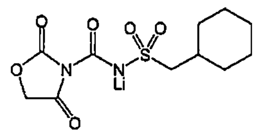 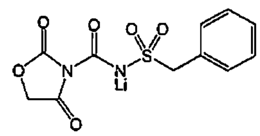

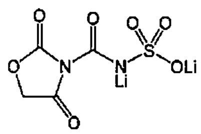 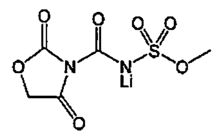 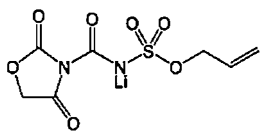 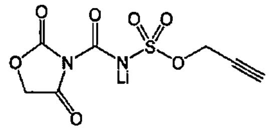

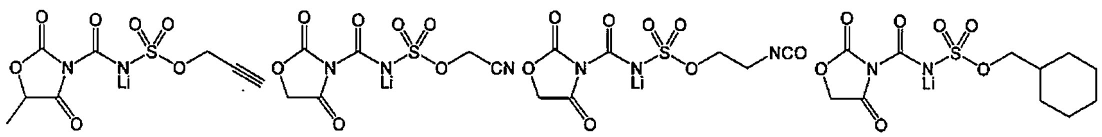

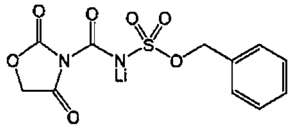 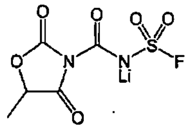 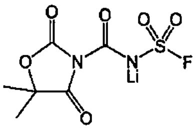 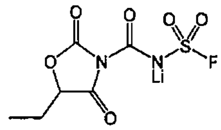

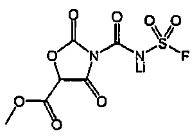 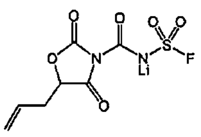 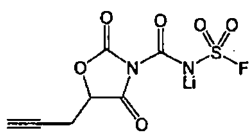 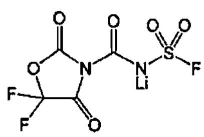

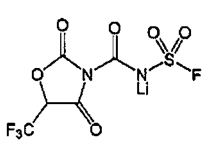 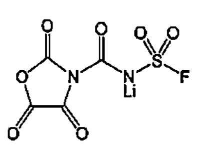 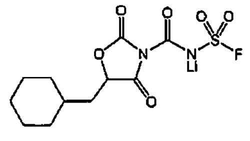 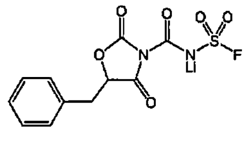

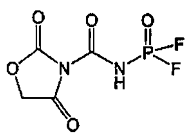 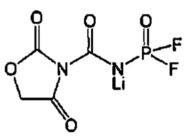 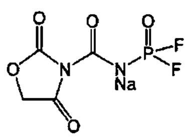 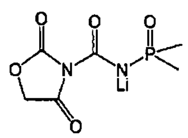 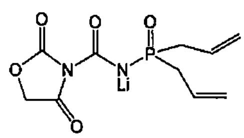

[Chem. 16]

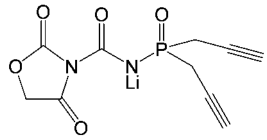
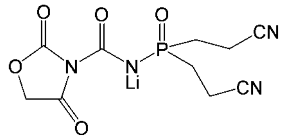
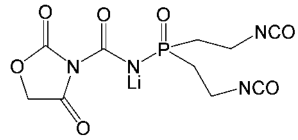
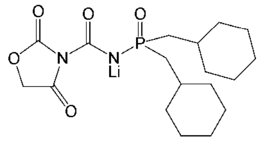
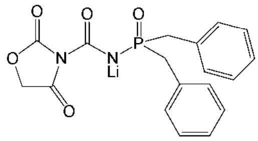
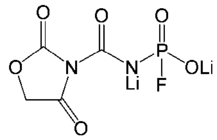
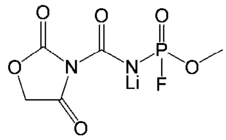
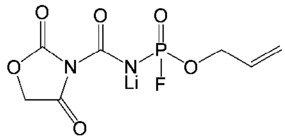
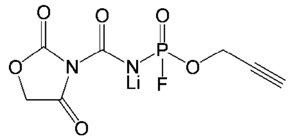
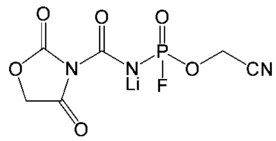
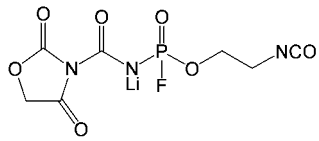
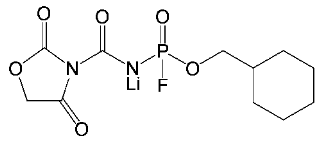
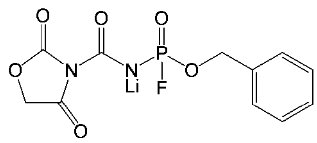
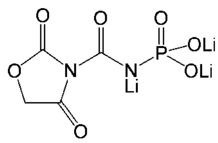
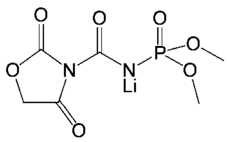
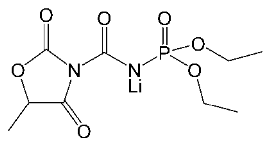
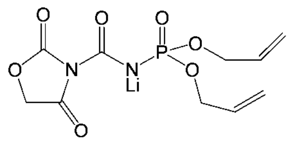
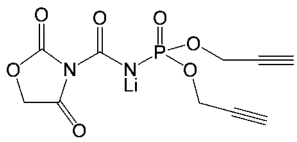
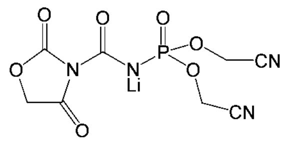
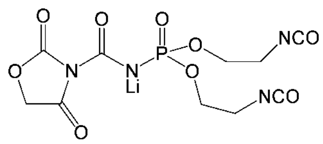
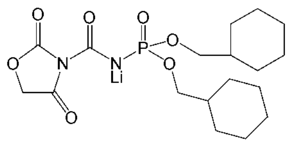
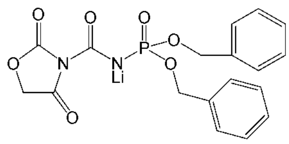

**8.** The nonaqueous electrolyte solution according to claim 1, wherein the compound represented by the general formula (4) is at least one selected from the group consisting of the following compounds.

[Chem. 17]

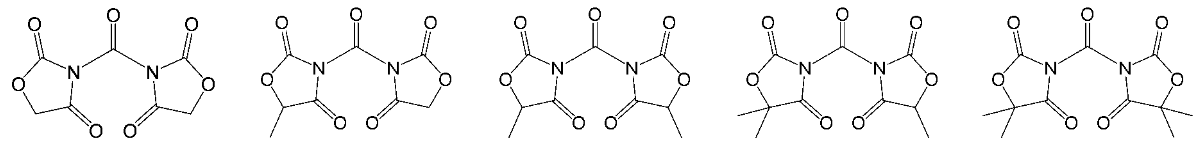

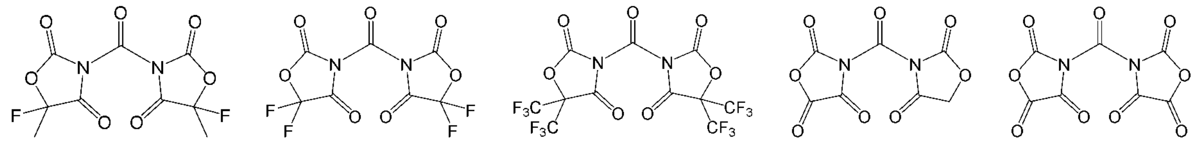

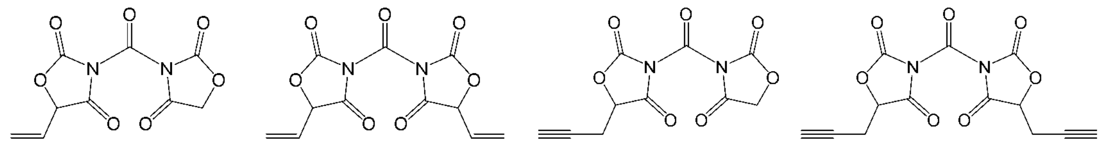

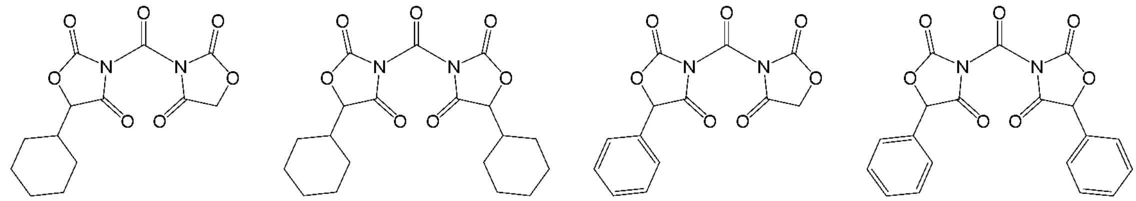

**9.** The nonaqueous electrolyte solution according to claim 1, wherein the compound represented by the general formula (5) is at least one selected from the group consisting of the following compounds.

[Chem. 18]

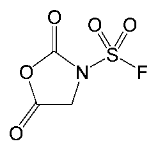
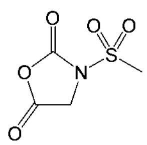
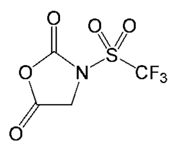
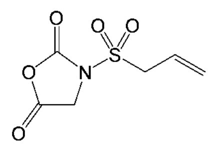

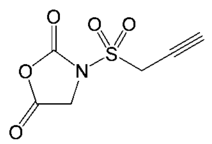
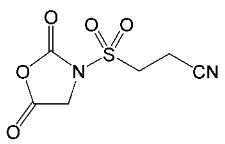
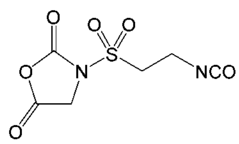
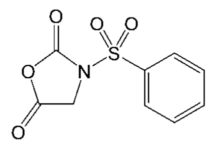

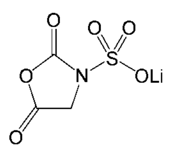
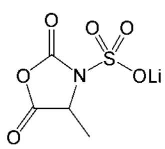
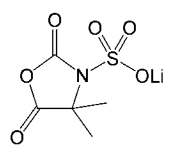
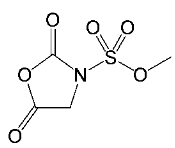
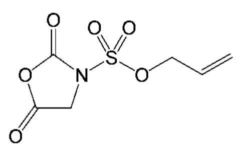

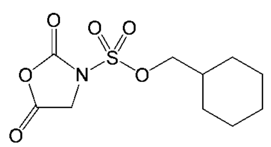
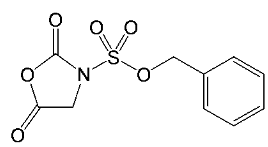
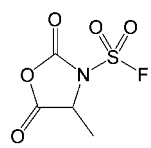
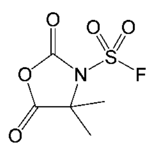
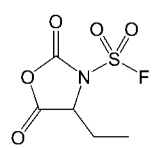

[Chem. 19]

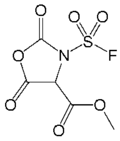
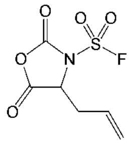
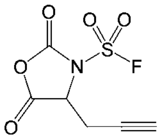
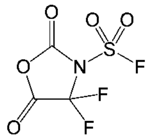
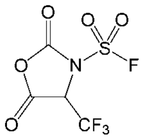
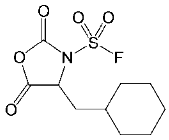
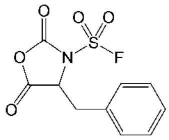
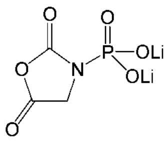
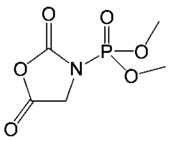
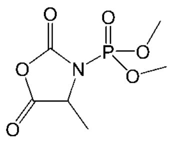
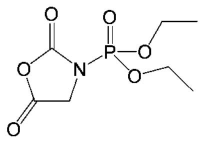
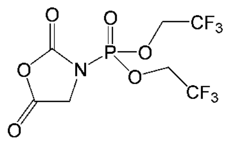
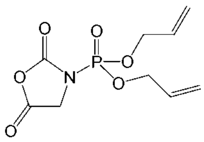
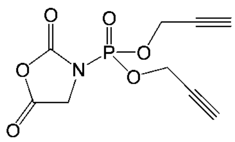
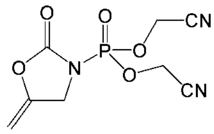
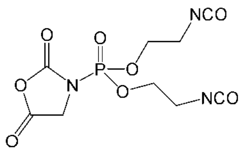
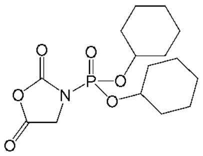
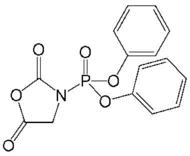
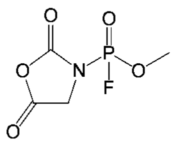
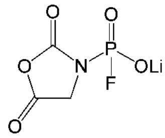
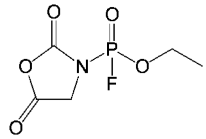
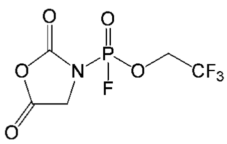
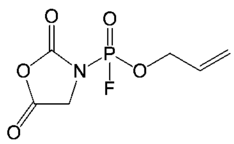
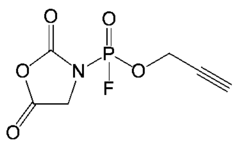
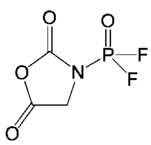
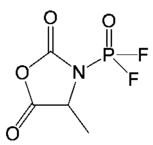
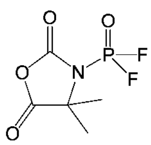

**10.** The nonaqueous electrolyte solution according to claim 1, wherein the compound represented by the general formula (6) is at least one selected from the group consisting of the following compounds.

[Chem. 20]

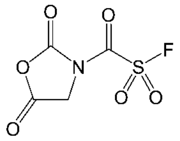
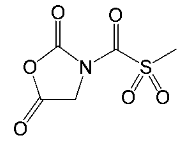
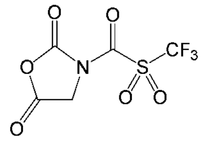
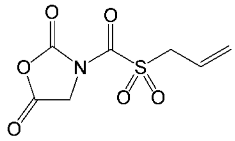
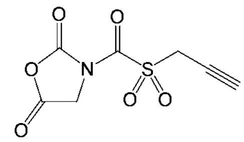

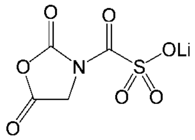
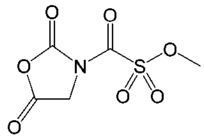
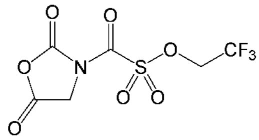
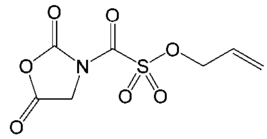

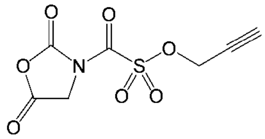
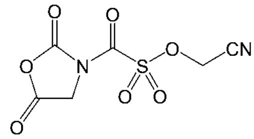
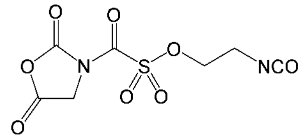

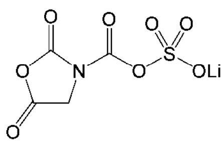
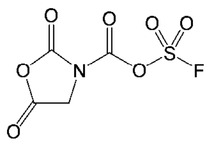
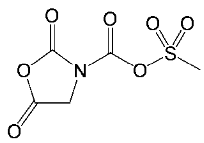
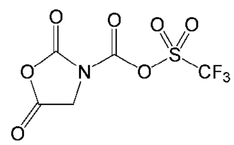

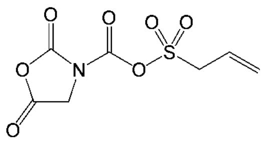
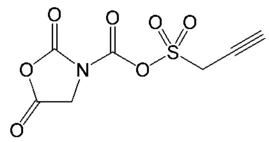
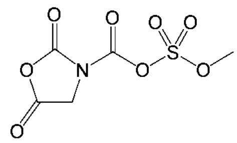
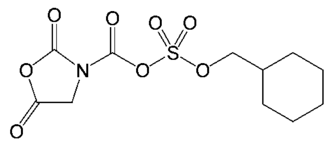

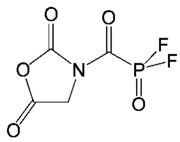
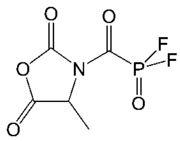
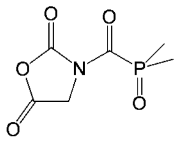
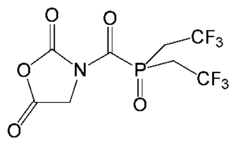

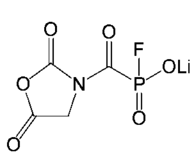
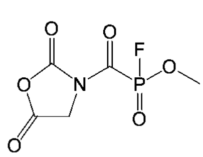
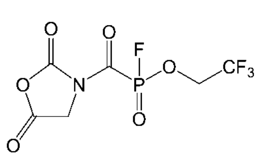
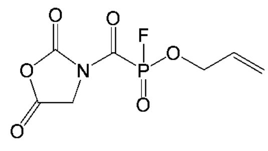

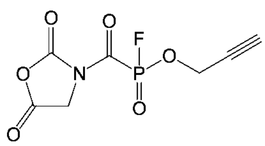
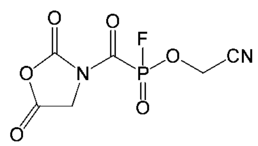
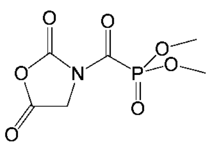
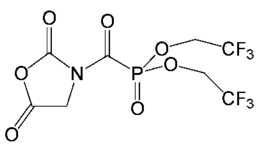

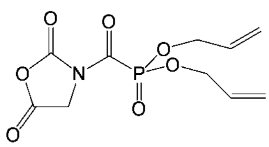
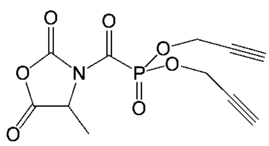
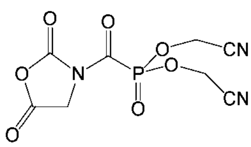
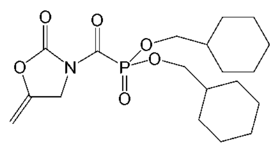

[Chem. 21]

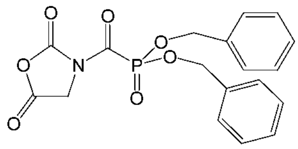 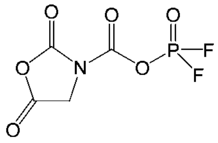 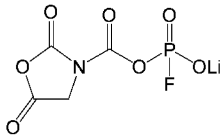

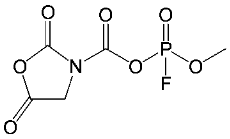 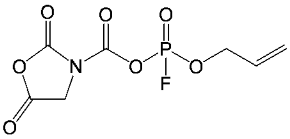 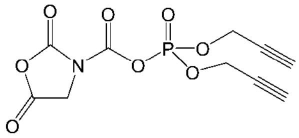

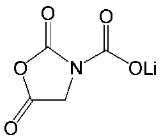 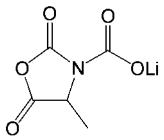 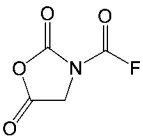 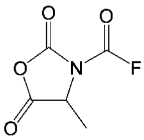

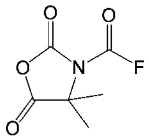 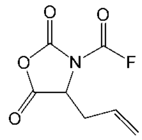 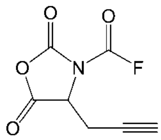 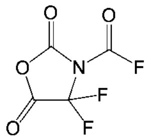

**11.** The nonaqueous electrolyte solution according to claim 1, wherein the compound represented by the general formula (7) is at least one selected from the group consisting of the following compounds.

[Chem. 22]

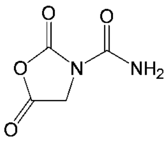 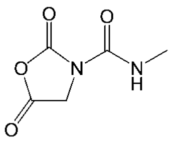 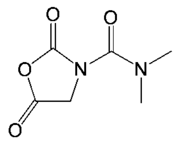 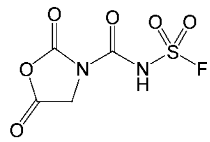 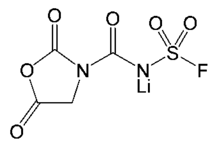

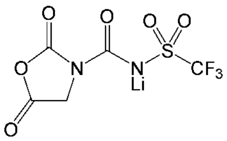 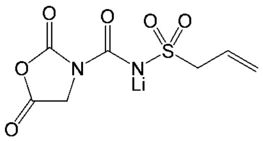 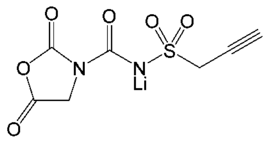 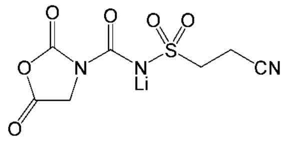

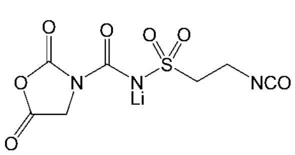 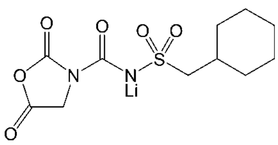 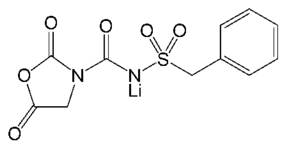 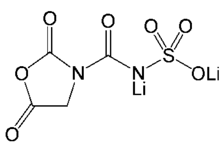

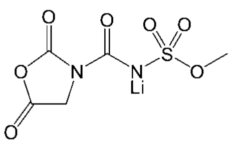 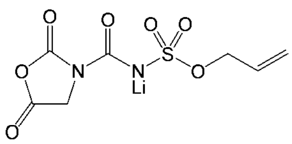 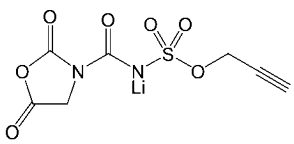 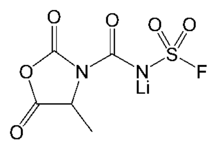

[Chem. 23]

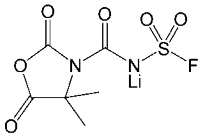
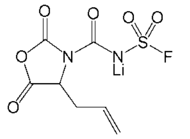
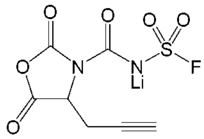
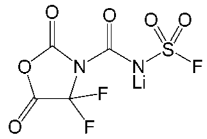

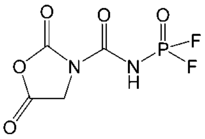
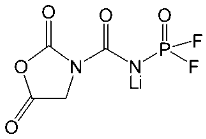
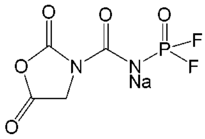
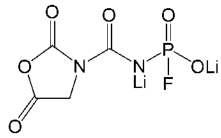

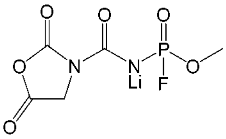
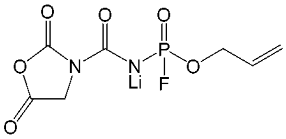
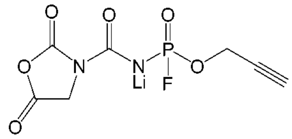
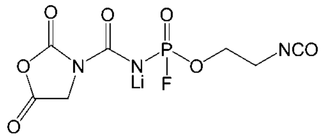

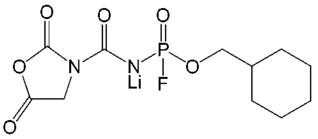
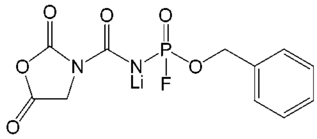
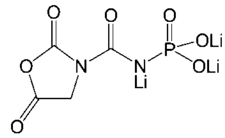
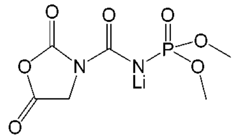

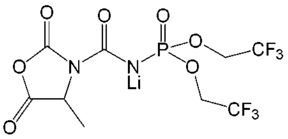
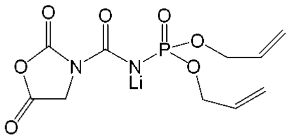
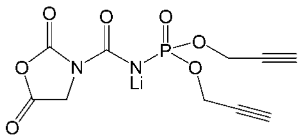
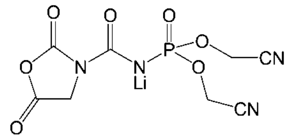

**12.** The nonaqueous electrolyte solution according to claim 1, wherein the compound represented by the general formula (8) is at least one selected from the group consisting of the following compounds.

[Chem. 24]

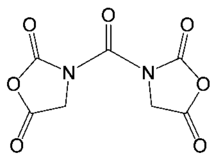
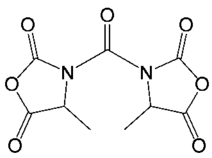
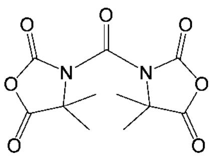
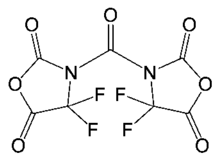

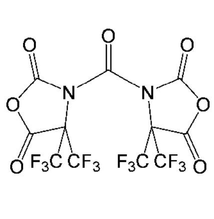
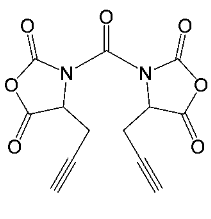
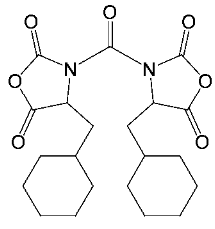
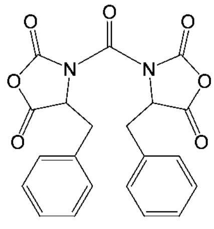

**13.** The nonaqueous electrolyte solution according to claim 1, wherein the (I) is at least one selected from the group consisting of $LiPF_6$, $LiBF_4$, $LiSbF_6$, $LiAsF_6$, $LiClO_4$, $LiN(SO_2F)_2$, $LiAlO_2$, $LiAlCl_4$, LiCl, and LiI or at least one selected from the group consisting of $NaPF_6$, $NaBF_4$, $NaSbF_6$, $NaAsF_6$, $NaClO_4$, $NaN(SO_2F)_2$, $NaAlO_2$, $NaAlCl_4$, NaCl, and NaI.

**14.** The nonaqueous electrolyte solution according to claim 1, wherein the (II) contains at least one selected from the group consisting of a cyclic ester, a chain ester, a cyclic ether, a chain ether, a sulfone compound, a sulfoxide compound, and an ionic liquid.

**15.** The nonaqueous electrolyte solution according to claim 14, wherein the cyclic ester contains a cyclic carbonate.

**16.** The nonaqueous electrolyte solution according to claim 15, wherein the cyclic carbonate contains at least one selected from the group consisting of ethylene carbonate and propylene carbonate.

**17.** The nonaqueous electrolyte solution according to claim 14, wherein the chain ester contains a chain carbonate.

**18.** The nonaqueous electrolyte solution according to claim 17, wherein the chain carbonate contains at least one selected from the group consisting of ethyl methyl carbonate, dimethyl carbonate, diethyl carbonate, and methyl propyl carbonate.

**19.** The nonaqueous electrolyte solution according to claim 1, further comprising at least one selected from the group consisting of cyclohexylbenzene, cyclohexylfluorobenzene, biphenyl, 2-fluorobiphenyl, t-butylbenzene, t-amylbenzene, 2-fluorotoluene, fluorobenzene, vinylene carbonate, an oligomer of vinylene carbonate (having a number average molecular weight in terms of polystyrene of 170 to 5000), vinylethylene carbonate, difluoroanisole, fluoroethylene carbonate, 1,6-diisocyanatohexane, ethynylethylene carbonate, trans-difluoroethylene carbonate, methyl propargyl carbonate, ethyl propargyl carbonate, dipropargyl carbonate, maleic anhydride, succinic anhydride, 1,3-propanesultone, 1,3-propenesultone, 1,4-butanesultone, dimethylvinylene carbonate, 1,3,2-dioxathiolane-2,2-dioxide, 4-propyl-1,3,2-dioxathiolane-2,2-dioxide, methylene methanedisulfonate, dimethylene methanedisulfonate, trimethylene methanedisulfonate, methyl methanesulfonate, methanesulfonyl fluoride, ethenesulfonyl fluoride, phenyl difluorophosphate, 1,2-ethanedisulfonic anhydride, tris(trimethylsilyl)borate, succinonitrile, (ethoxy)pentafluorocyclotriphosphazene, tetrafluoro(picolinato)phosphate, difluoro(picolinato)borate, 1,3-dimethyl-1,3-divinyl-1,3-di(1,1,1,3,3,3-hexafluoroisopropyl)disiloxane, lithium difluorophosphate, sodium difluorophosphate, lithium fluorosulfate, sodium fluorosulfate, lithium trifluoromethanesulfonate, sodium trifluoromethanesulfonate, lithium nonafluorobutanesulfonate, sodium nonafluorobutanesulfonate, lithium difluorobis(oxalato)phosphate, sodium difluorobis(oxalato)phosphate, lithium difluorooxalatoborate, sodium difluorooxalatoborate, lithium bis(oxalato)borate, sodium bis(oxalato)borate, lithium tetrafluorooxalatophosphate, sodium tetrafluorooxalatophosphate, lithium tris(oxalato)phosphate, sodium tris(oxalato)phosphate, lithium difluoro(malonato)borate, sodium difluoro(malonato)borate, lithium tetrafluoro(malonato)phosphate, sodium tetrafluoro(malonato)phosphate, lithium monofluorophosphate, sodium monofluorophosphate, lithium bis(difluorophosphoryl)imide, sodium bis(difluorophosphoryl)imide, lithium (difluorophosphoryl)(fluorosulfonyl)imide, sodium (difluorophosphoryl)(fluorosulfonyl)imide, lithium bis(trifluoromethanesulfonyl)imide, sodium bis(trifluoromethanesulfonyl)imide, lithium bis(pentafluoroethanesulfonyl)imide, sodium bis(pentafluoroethanesulfonyl)imide, lithium (trifluoromethanesulfonyl)(pentafluoroethanesulfonyl)imide, sodium (trifluoromethanesulfonyl)(pentafluoroethanesulfonyl)imide, lithium (trifluoromethanesulfonyl)(fluorosulfonyl)imide, sodium (trifluoromethanesulfonyl)(fluorosulfonyl)imide, lithium (pentafluoroethanesulfonyl)(fluorosulfonyl)imide, sodium (pentafluoroethanesulfonyl)(fluorosulfonyl)imide, lithium tris(trifluoromethanesulfonyl)methide, sodium tris(trifluoromethanesulfonyl)methide, lithium acrylate, sodium acrylate, lithium methacrylate, sodium methacrylate, lithium methyl sulfate, sodium methyl sulfate, lithium ethyl sulfate, sodium ethyl sulfate, lithium nitrate, lithium nitrite, sodium nitrate, and sodium nitrite.

**20.** The nonaqueous electrolyte solution according to claim 1, further comprising a compound represented by the following general formula (9):

[Chem. 25]

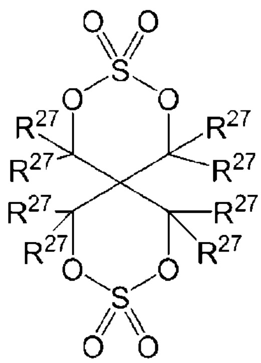

(9)

[wherein in the general formula (9), $R^{27}$ each independently represents a hydrogen atom, a halogen atom, or an alkyl group having 1 to 10 carbon atoms, an oxygen atom may be included in any carbon atom-carbon atom bond in the alkyl group, and any hydrogen atom of the alkyl group may be substituted with a fluorine atom].

**21.** The nonaqueous electrolyte solution according to claim 20, wherein the compound represented by the general formula (9) is at least one selected from the group consisting of the following compounds.

[Chem. 26]

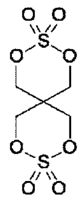

Compound 1-1

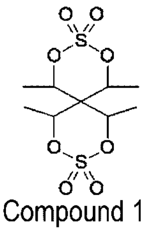

Compound 1-2

**22.** The nonaqueous electrolyte solution according to claim 1, further comprising a compound represented by the following general formula (10):

[Chem. 27]

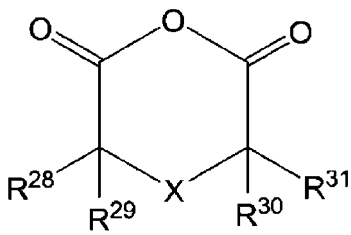

(10)

[wherein in the general formula (10), $R^{28}$ to $R^{31}$ each independently represent a hydrogen atom, a halogen atom, an alkyl group, an alkenyl group, or an aryl group, and X represents an oxygen atom, a sulfur atom, or an $SO_2$ group].

**23.** The nonaqueous electrolyte solution according to claim 22, wherein the compound of the formula (10) is a compound represented by the following formula.

[Chem. 28]

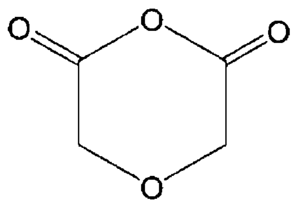

**24.** A nonaqueous electrolyte solution battery, comprising at least a positive electrode, a negative electrode, a separator, and the nonaqueous electrolyte solution according to any one of claims 1 to 23.

**25.** A compound represented by any of the following general formulae (1) to (8):

[Chem. 29]

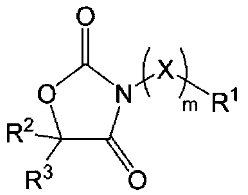 X = 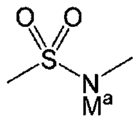 or 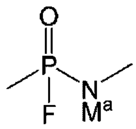 (1)

[wherein in the general formula (1),

m represents an integer of 0 or 1,

$M^a$ represents a hydrogen atom or $(M_1^{A+})_a$, wherein $M_1^{A+}$ represents a metal cation or an onium cation, A represents the valence of the cation, and a represents a number satisfying A × a = 1,

$R^1$ represents $-S(=O)_2R^a$ or $-P(=O)(R^b)(R^c)$,

$R^a$, $R^b$, and $R^c$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_2^{B+})_b$, wherein $M_2^{B+}$ represents a proton, a metal cation, or an onium cation, B represents the valence of the cation, and b represents a number satisfying B × b = 1, wherein $R^b$ and $R^c$ may together form a 5-membered ring or a 6-membered ring together with the phosphorus atom, and

$R^2$ and $R^3$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^2$ and $R^3$ may be integrated to form a carbonyl group]

[Chem. 30]

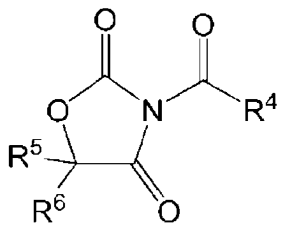 (2)

[wherein in the general formula (2),

$R^4$ represents a halogen atom, $-(O)_{x1}-S(=O)_2R^d$, $-(O)_{y1}-P(=O)(R^e)(R^f)$, or $-O^-(M_3^{C+})_c$, wherein $M_3^{C+}$ represents a proton, a metal cation, or an onium cation, C represents the valence of the cation, c represents a number satisfying C × c = 1, and x1 and y1 each represent an integer of 0 or 1,

$R^d$, $R^e$, and $R^f$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10

carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^{-}(M_4^{D+})_d$, wherein $M_4^{D+}$ represents a proton, a metal cation, or an onium cation, D represents the valence of the cation, and d represents a number satisfying $D \times d = 1$, and

$R^5$ and $R^6$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^5$ and $R^6$ may be integrated to form a carbonyl group]

[Chem. 31]

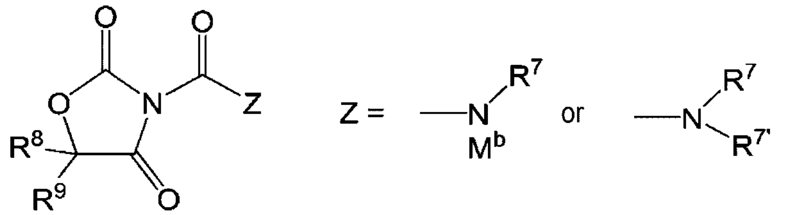

(3)

[wherein in the general formula (3),

$M^b$ represents a hydrogen atom or $(M_5^{E+})_e$, wherein $M_5^{E+}$ represents a metal cation or an onium cation, E represents the valence of the cation, and e represents a number satisfying $E \times e = 1$,

$R^7$ and $R^{7'}$ each independently represent a hydrogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), $-S(=O)_2R^g$, or $-P(=O)(R^h)(R^i)$,

$R^g$, $R^h$, and $R^i$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^{-}(M_6^{F+})_f$, wherein $M_6^{F+}$ represents a proton, a metal cation, or an onium cation, F represents the valence of the cation, and f represents a number satisfying $F \times f = 1$, and

$R^8$ and $R^9$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^8$ and $R^9$ may be integrated to form a carbonyl group]

[Chem. 32]

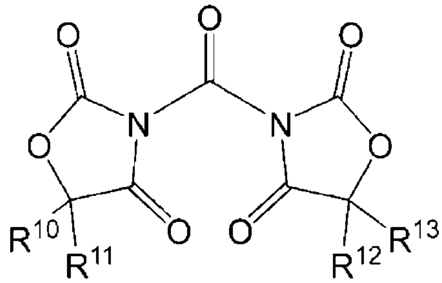

(4)

[wherein in the general formula (4),

$R^{10}$, $R^{11}$, $R^{12}$, and $R^{13}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{10}$ and $R^{11}$ or $R^{12}$ and $R^{13}$ may be each independently integrated to form a carbonyl group]

[Chem. 33]

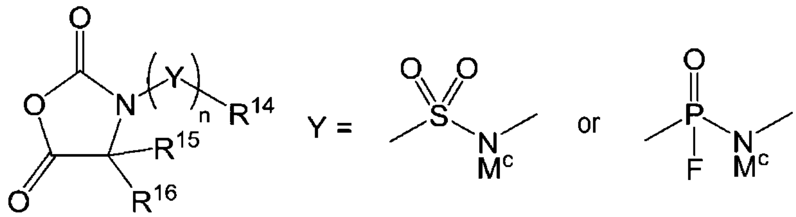

(5)

[wherein in the general formula (5),

n represents an integer of 0 or 1,

$M^c$ represents a hydrogen atom or $(M_7^{G+})_g$, wherein $M_7^{G+}$ represents a metal cation or an onium cation, G represents the valence of the cation, and g represents a number satisfying G × g = 1,

$R^{14}$ represents $-S(=O)_2R^j$ or $-P(=O)(R^k)(R^l)$, wherein $R^j$, $R^k$, and $R^l$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_8^{H+})_h$, wherein $M_8^{H+}$ represents a proton, a metal cation, or an onium cation, H represents the valence of the cation, and h represents a number satisfying H × h = 1, and

$R^{15}$ and $R^{16}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{15}$ and $R^{16}$ may be integrated to form a carbonyl

group]

[Chem. 34]

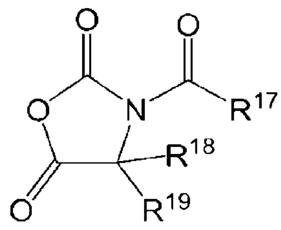

(6)

[wherein in the general formula (6),

$R^{17}$ represents a halogen atom, $-(O)_{x2}-S(=O)_2R^m$, $-(O)_{y2}-P(=O)(R^n)(R^o)$, or $-O^-(M_9{}^{l+})_i$, wherein $M_9{}^{l+}$ represents a proton, a metal cation, or an onium cation, l represents the valence of the cation, and i represents a number satisfying $l \times i = 1$,

x2 and y2 each represent an integer of 0 or 1,

$R^m$, $R^n$, and $R^o$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_{10}{}^{J+})_j$, wherein $M_{10}{}^{J+}$ represents a proton, a metal cation, or an onium cation, J represents the valence of the cation, and j represents a number satisfying $J \times j = 1$, and

$R^{18}$ and $R^{19}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{18}$ and $R^{19}$ may be integrated to form a carbonyl group]

[Chem. 35]

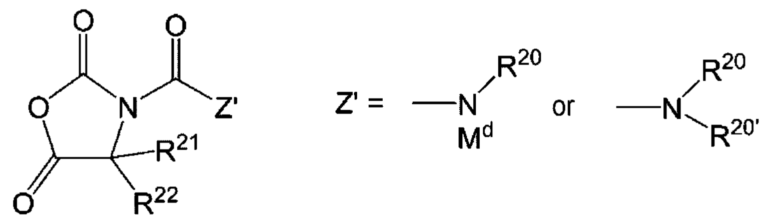

(7)

[wherein in the general formula (7),

$M^d$ represents a hydrogen atom or $(M_{11}{}^{K+})_k$, wherein $M_{11}{}^{K+}$ represents a metal cation or an onium cation, K represents the valence of the cation, and k represents a number satisfying $K \times k = 1$,

$R^{20}$ and $R^{20'}$ each independently represent a hydrogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to

10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), $-S(=O)_2R^p$, or $-P(=O)(R^q)(R^r)$,

$R^p$, $R^q$, and $R^r$ each independently represent a halogen atom, a hydrocarbon group selected from the group consisting of an alkyl group having 1 to 10 carbon atoms, an alkoxy group having 1 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 8 carbon atoms, a cycloalkoxy group having 3 to 8 carbon atoms, a cycloalkenyl group having 3 to 8 carbon atoms, a cycloalkenyloxy group having 3 to 8 carbon atoms, an aryl group having 6 to 10 carbon atoms, an aryloxy group having 6 to 10 carbon atoms, a heteroaryl group having 6 to 10 carbon atoms, and a heteroaryloxy group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), or $-O^-(M_{12}^{L+})_1$, wherein $M_{12}^{L+}$ represents a proton, a metal cation, or an onium cation, L represents the valence of the cation, and 1 represents a number satisfying $L \times 1 = 1$, and

$R^{21}$ and $R^{22}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{21}$ and $R^{22}$ may be integrated to form a carbonyl group]

[Chem. 36]

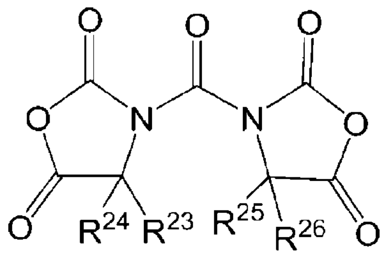

(8)

[wherein in the general formula (8),

$R^{23}$, $R^{24}$, $R^{25}$, and $R^{26}$ each independently represent a hydrogen atom, a halogen atom, or a hydrocarbon group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 8 carbon atoms, a cycloalkenyl group having 3 to 8 carbon atoms, an aryl group having 6 to 10 carbon atoms, and a heteroaryl group having 6 to 10 carbon atoms (a part or all of the hydrogen atoms of the hydrocarbon group may be each independently substituted with a halogen atom, a cyano group, an isocyano group, a cycloalkyl group, or an aryl group, and a carbonyl group, an oxygen atom, a sulfur atom, or a nitrogen atom may be interposed between any carbon-carbon atoms of the hydrocarbon group), wherein $R^{23}$ and $R^{24}$ or $R^{25}$ and $R^{26}$ may be each independently integrated to form a carbonyl group.]

**26.** The compound according to claim 25, wherein the compound represented by the general formula (1) is selected from the following compounds.

[Chem. 37]

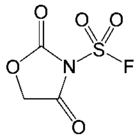
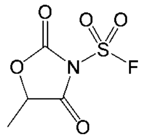
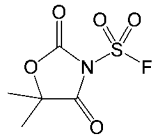
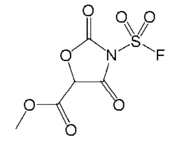
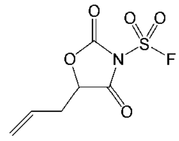
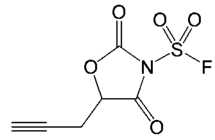

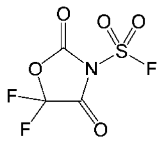
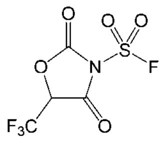
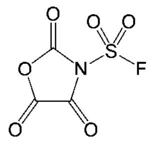
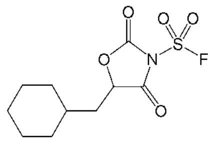
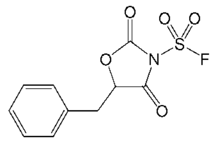

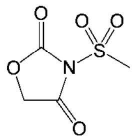
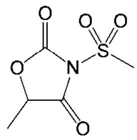
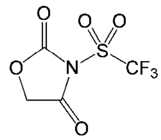
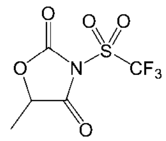
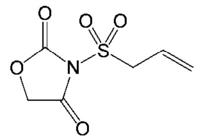

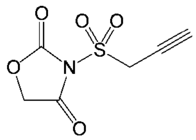
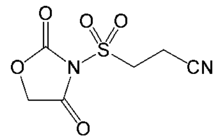
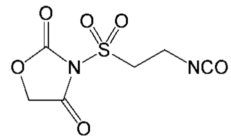
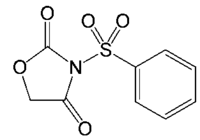

[Chem. 38]

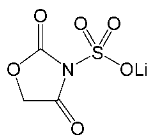
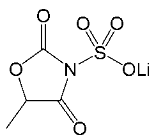
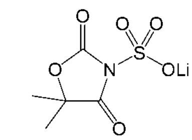
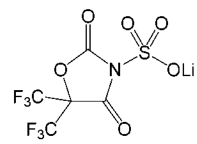
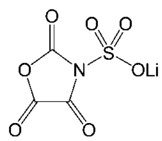

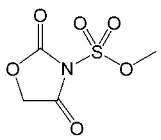
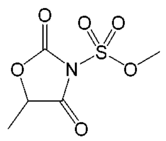
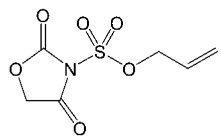
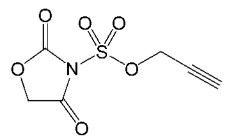
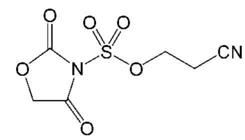

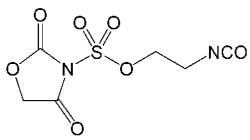
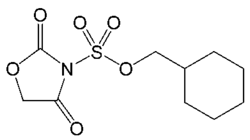
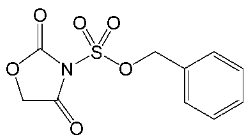
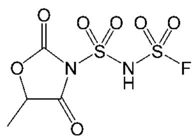
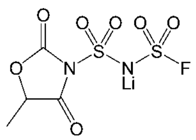

[Chem. 39]

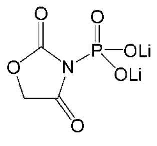 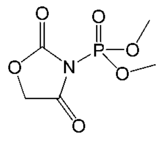 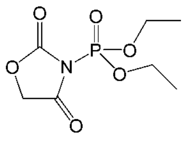 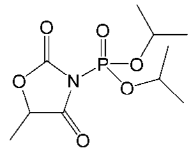 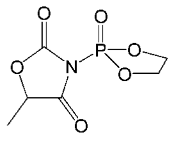

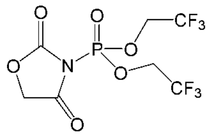 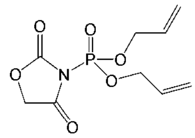 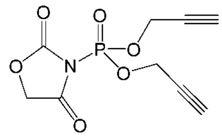 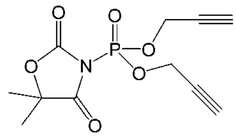 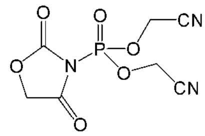

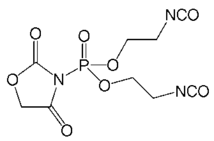 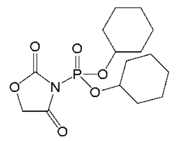 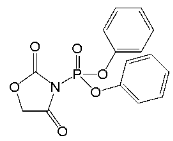 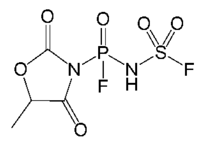 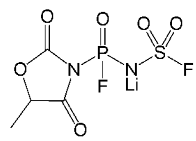

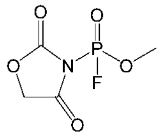 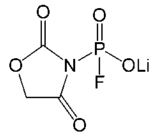 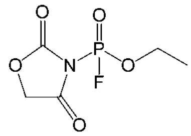 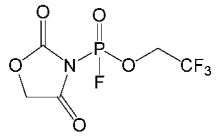 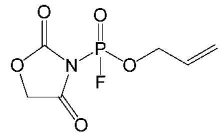

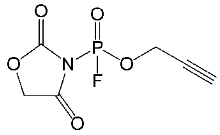 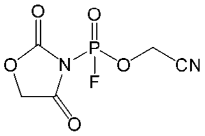 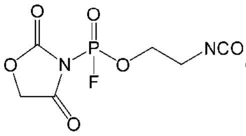 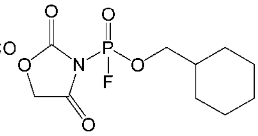 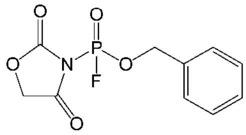

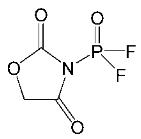 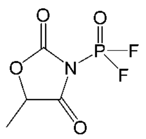 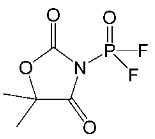 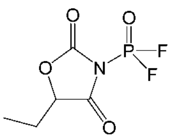 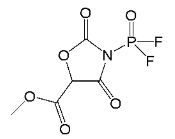 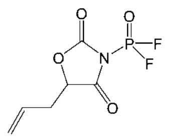

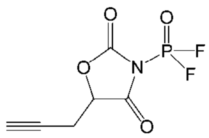 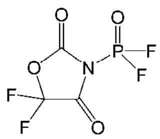 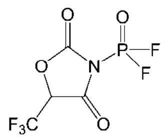 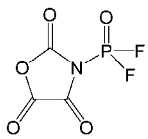 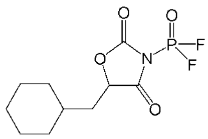 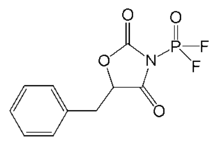

**27.** The compound according to claim 25, wherein the compound represented by the general formula (2) is selected from the following compounds.

[Chem. 40]

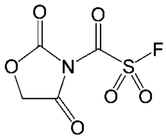 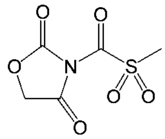 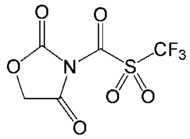 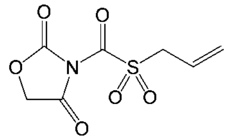 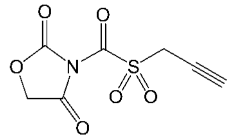

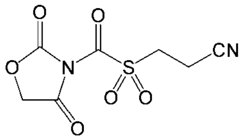 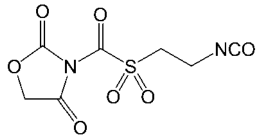 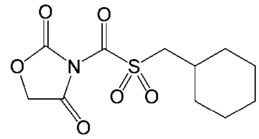 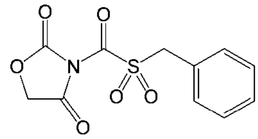

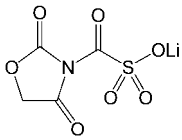 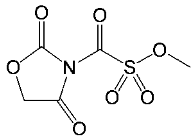 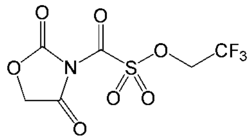 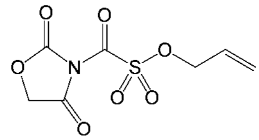

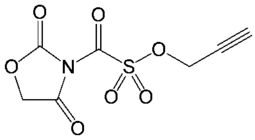 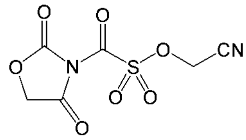 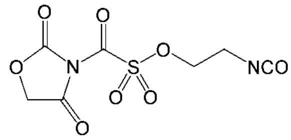 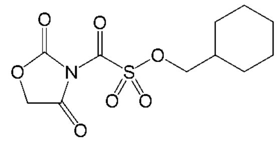

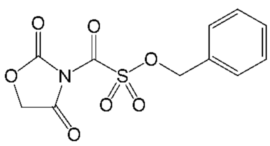 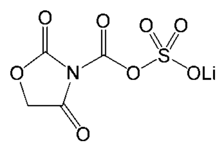 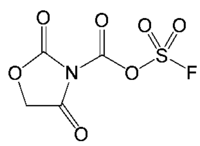 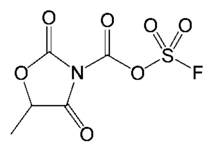

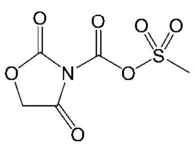 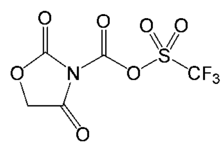 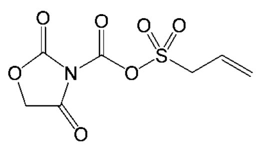 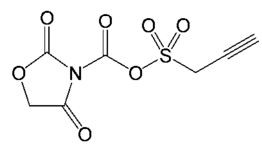

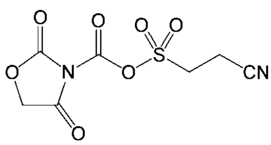 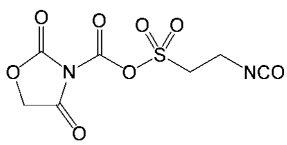 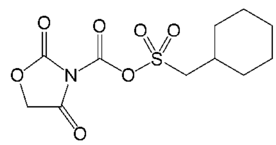 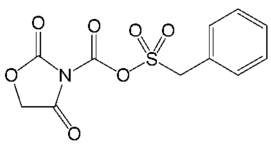

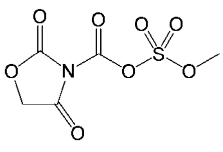 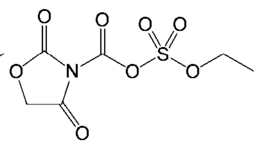 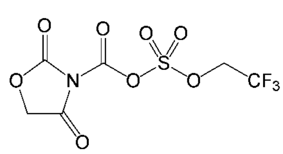 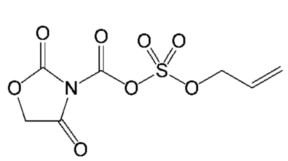

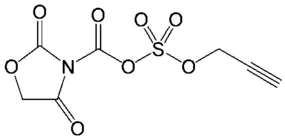 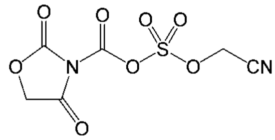 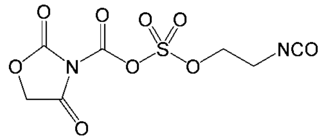

[Chem. 41]

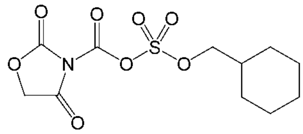 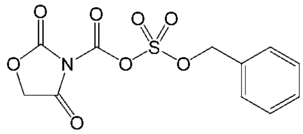

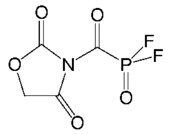 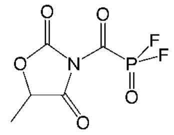 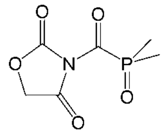 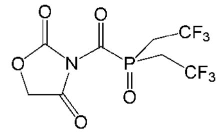 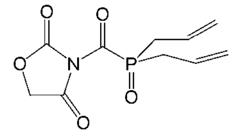

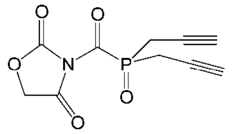 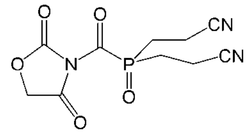 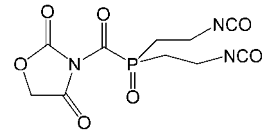

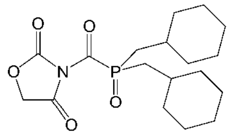 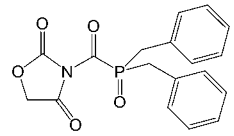

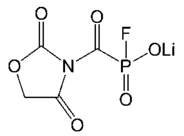 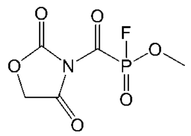 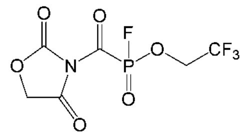 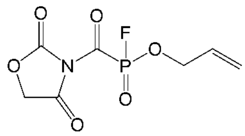 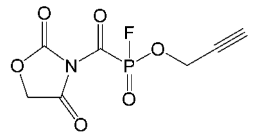

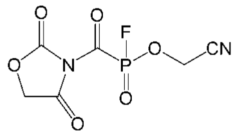 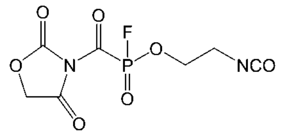 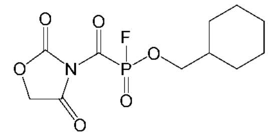 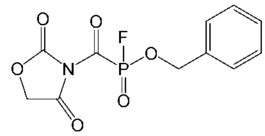

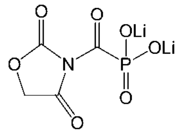 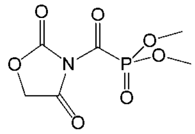 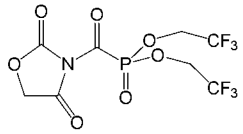 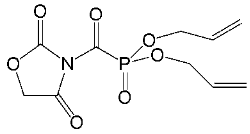

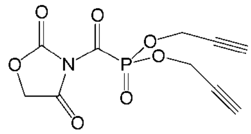 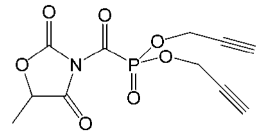 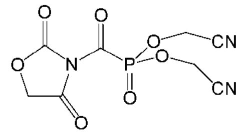

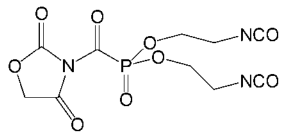 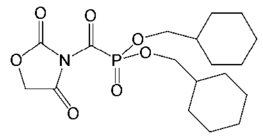 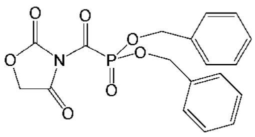

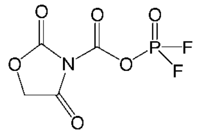 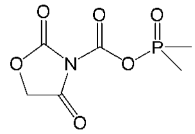 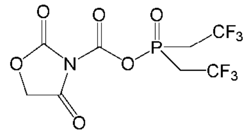 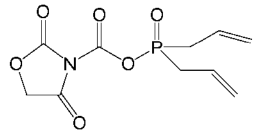

[Chem. 42]

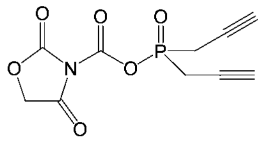 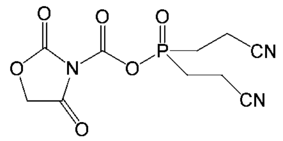 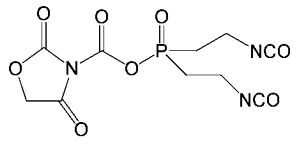 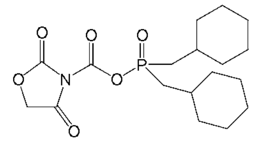

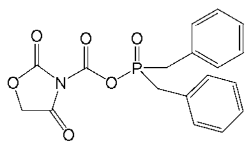 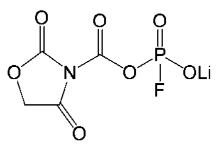 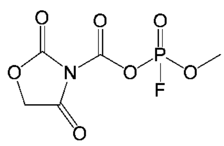 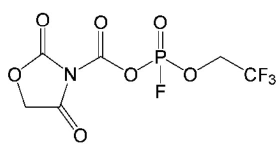

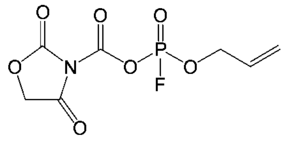 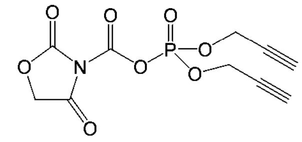 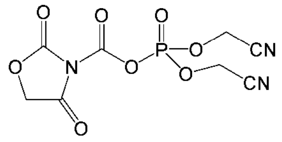 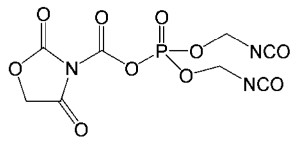

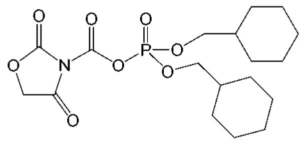 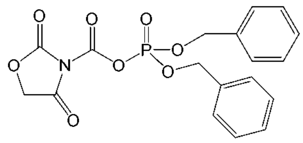

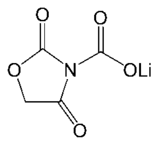 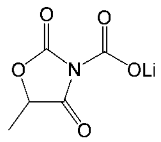 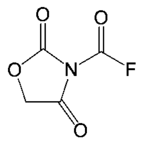 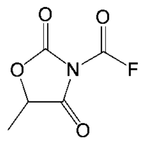 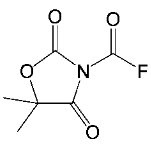

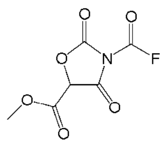 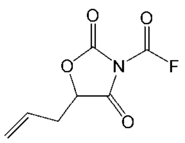 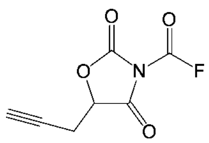 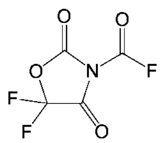

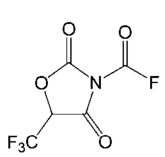 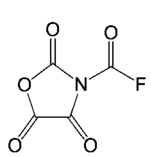 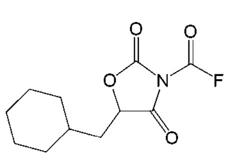 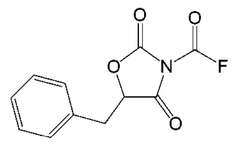

**28.** The compound according to claim 25, wherein the compound represented by the general formula (3) is selected from the following compounds.

[Chem. 43]

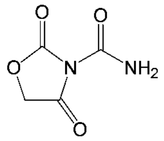 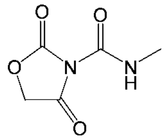 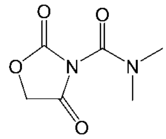 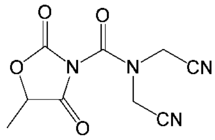

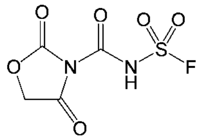 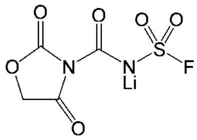 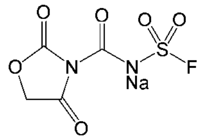 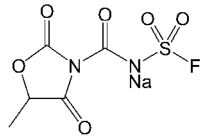 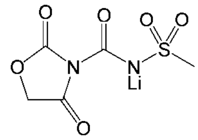

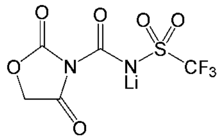 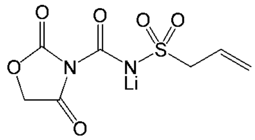 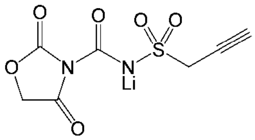

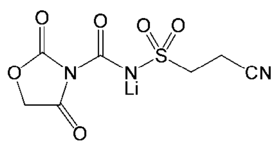 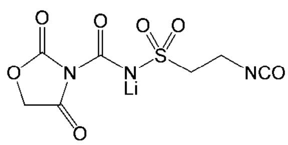 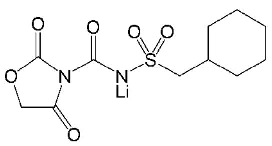 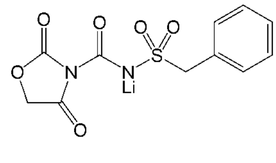

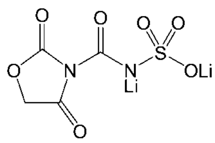 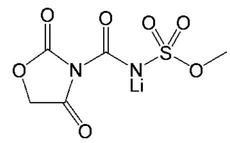 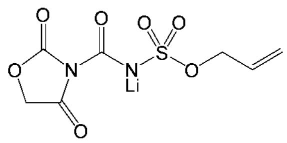 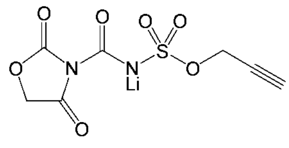

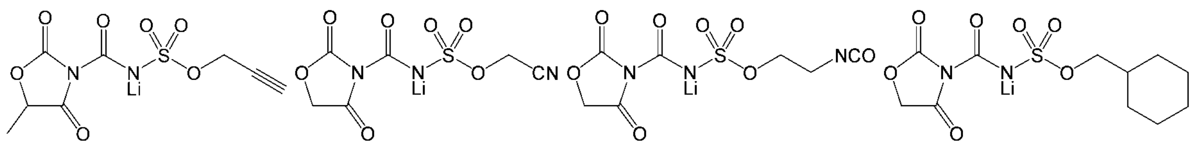

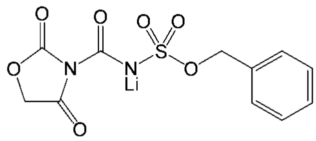 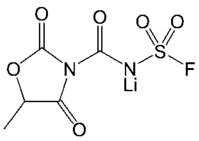 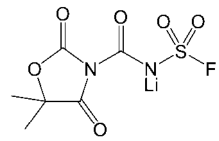 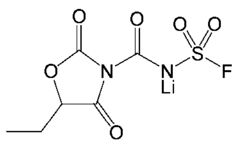

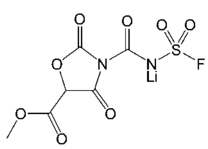 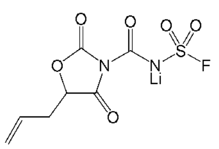 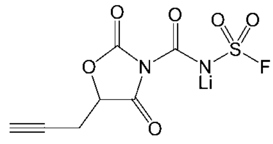 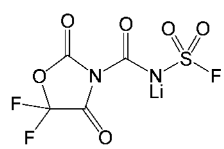

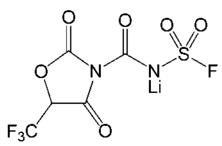 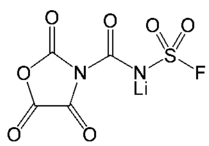 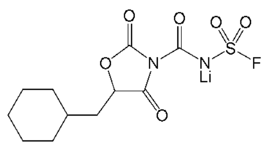 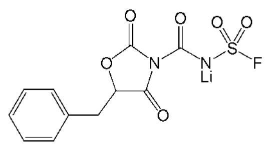

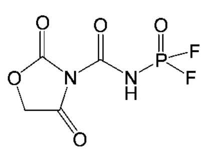 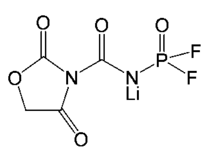 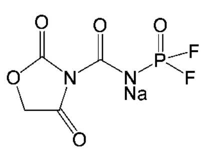 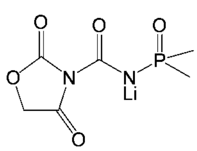 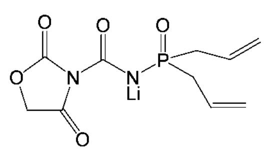

[Chem. 44]

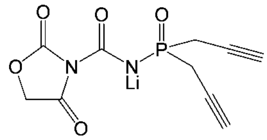 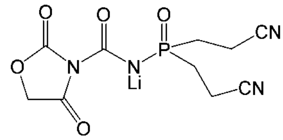 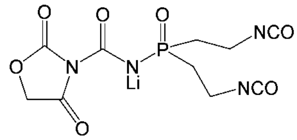

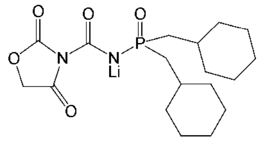 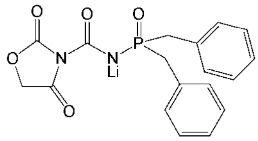

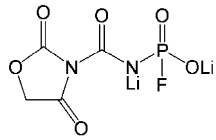 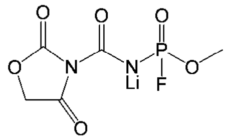 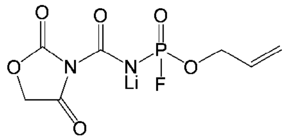 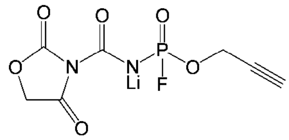

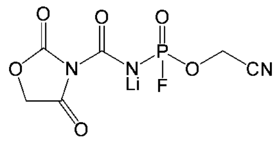 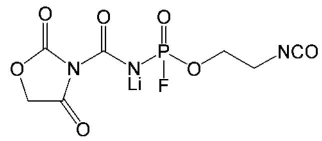 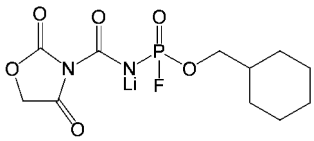

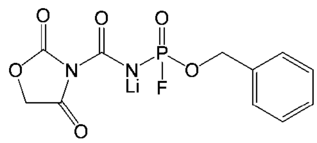 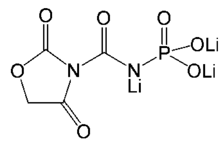 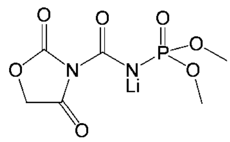 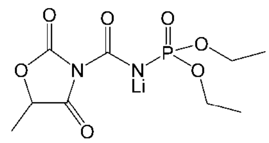

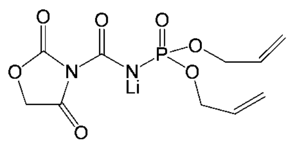 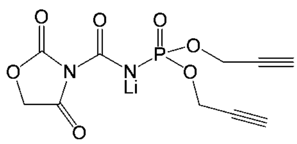 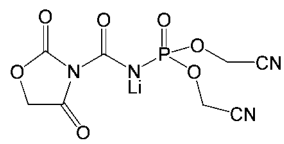

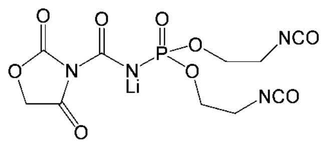 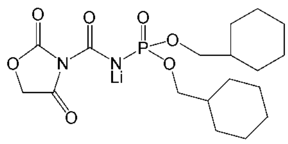 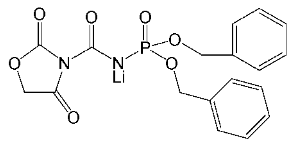

**29.** The compound according to claim 25, wherein the compound represented by the general formula (4) is selected from the following compounds.

[Chem. 45]

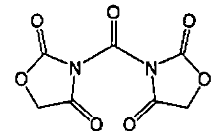 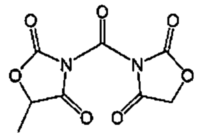 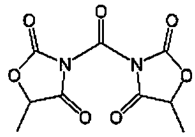 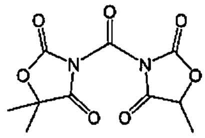 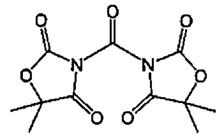

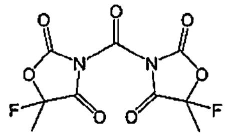 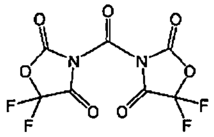 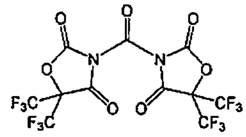 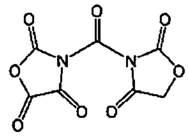 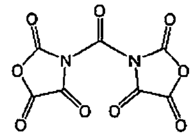

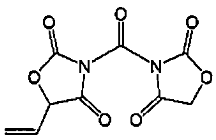 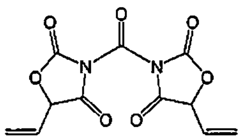 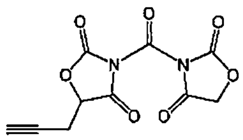 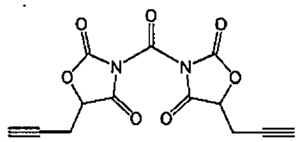

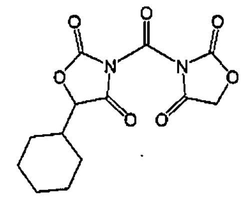 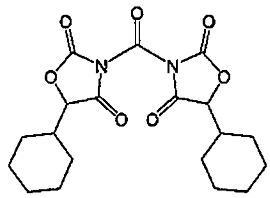 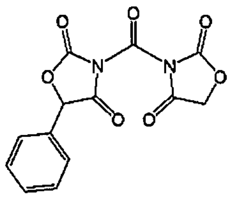 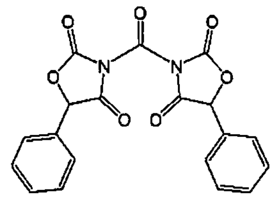

**30.** The compound according to claim 25, wherein the compound represented by the general formula (5) is selected from the following compounds.

[Chem. 46]

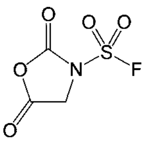 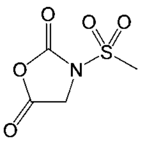 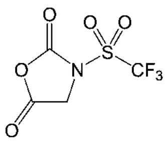 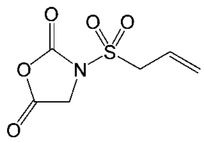

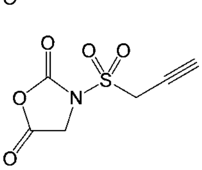 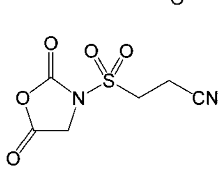 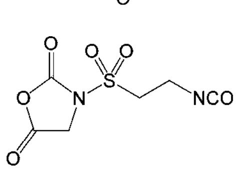 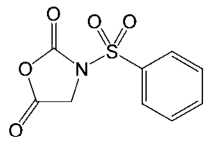

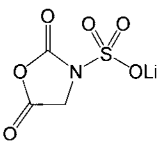 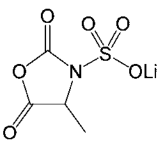 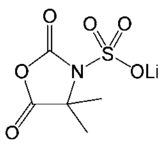 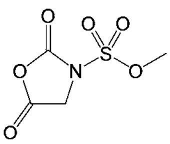 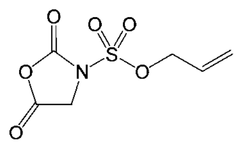

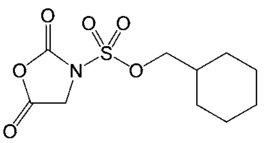 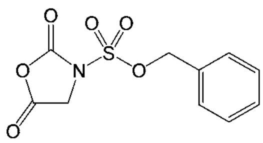 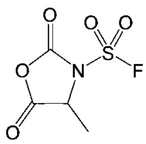 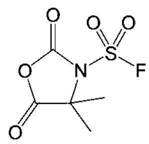 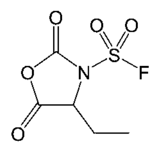

[Chem. 47]

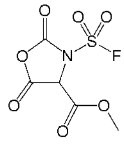
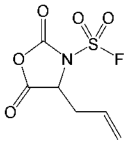
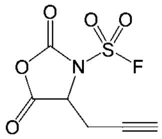
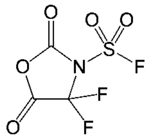

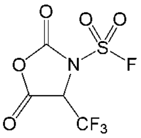
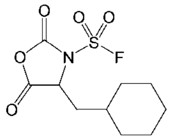
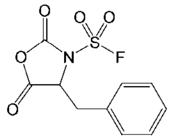

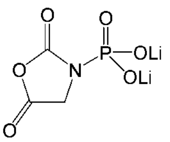
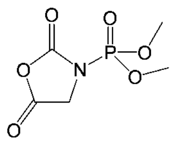
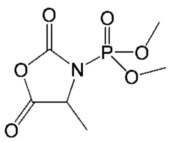
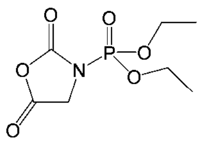
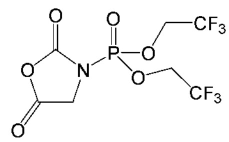

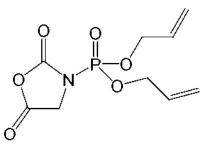
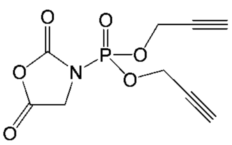
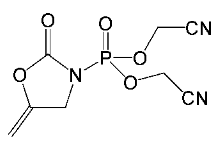
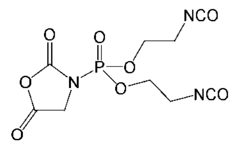

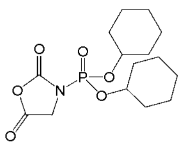
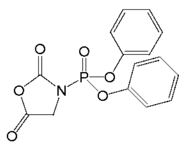
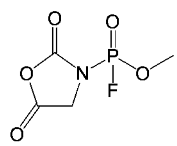
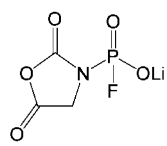
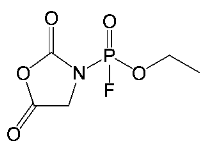

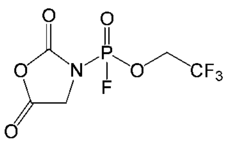
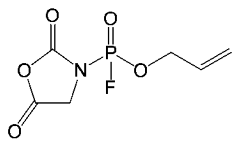
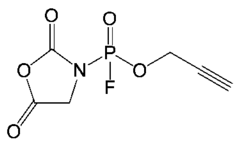

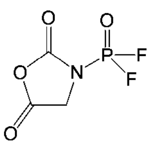
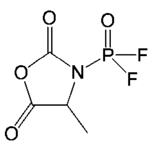
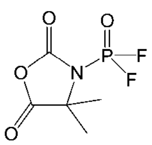

**31.** The compound according to claim 25, wherein the compound represented by the general formula (6) is selected from the following compounds.

[Chem. 48]

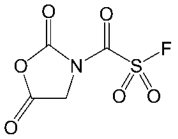 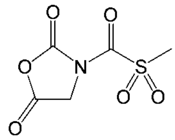 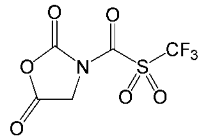 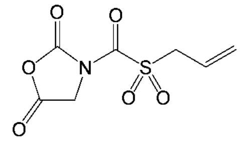 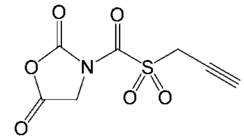

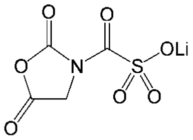 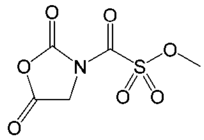 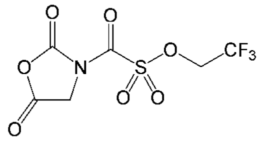 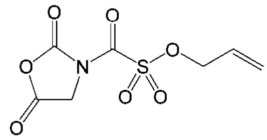

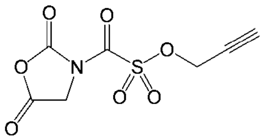 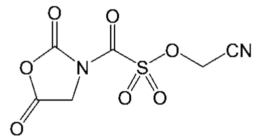 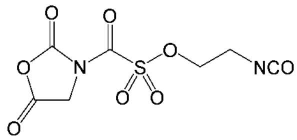

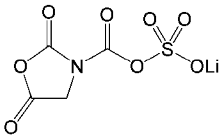 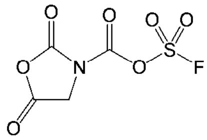 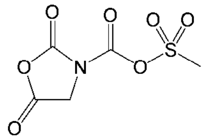 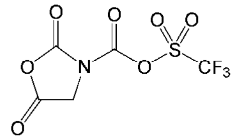

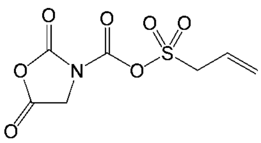 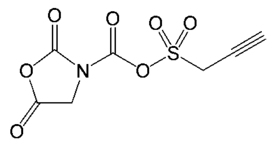 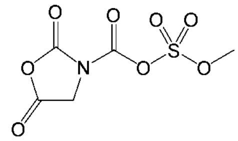 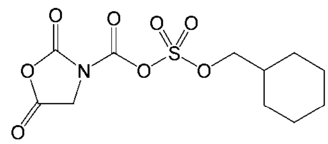

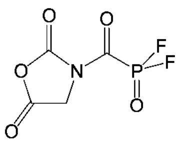 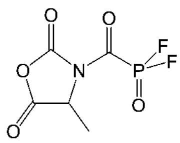 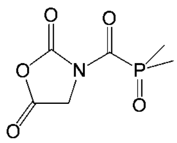 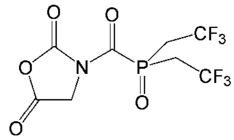

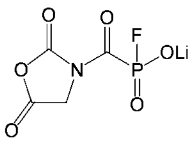 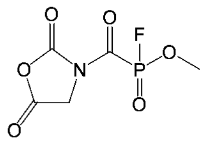 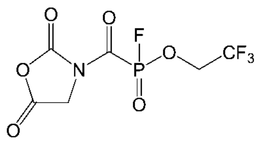 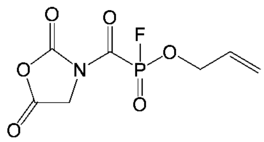

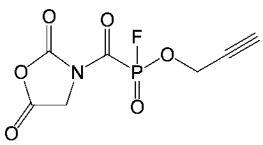 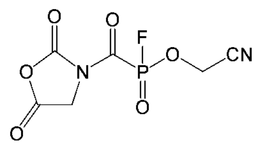 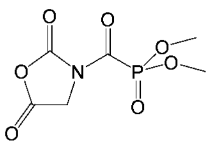 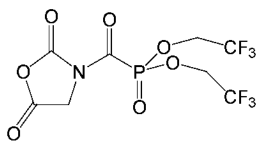

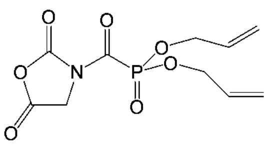 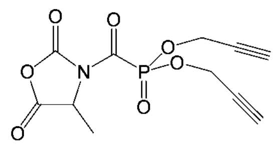 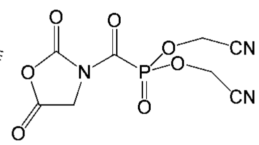 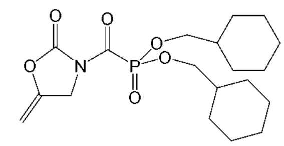

[Chem. 49]

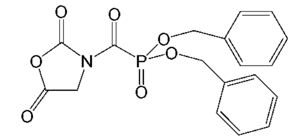 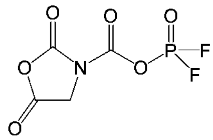 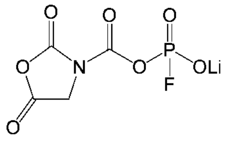

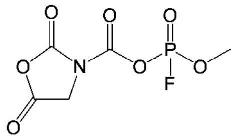 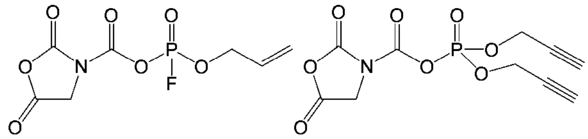

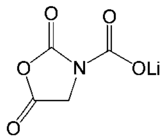 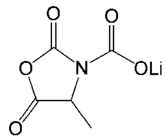 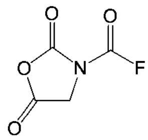 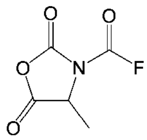

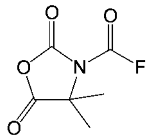 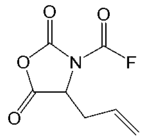 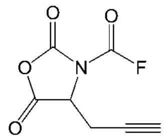 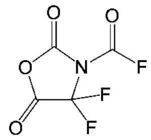

**32.** The compound according to claim 25, wherein the compound represented by the general formula (7) is selected from the following compounds.

[Chem. 50]

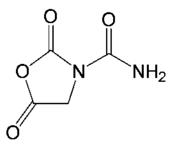 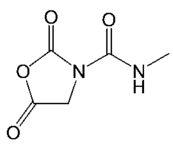 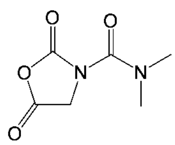 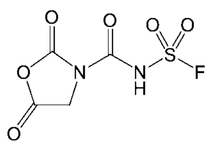 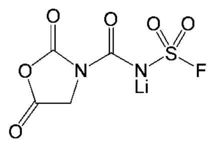

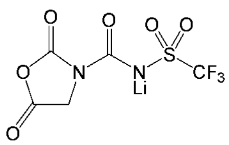 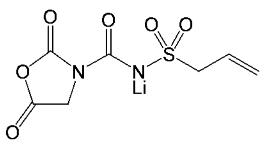 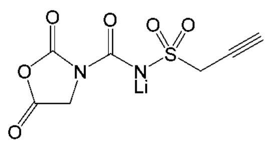 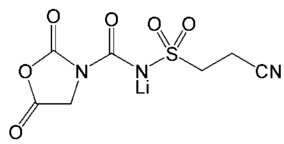

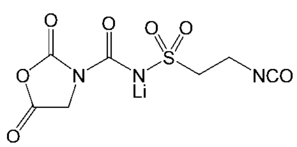 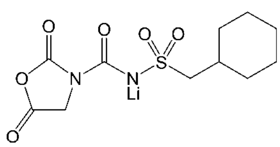 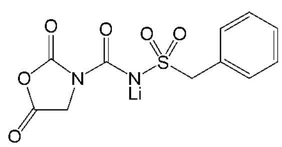 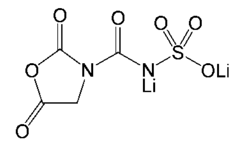

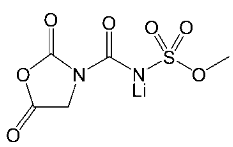 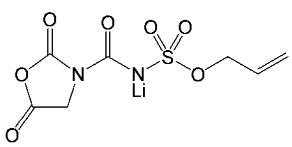 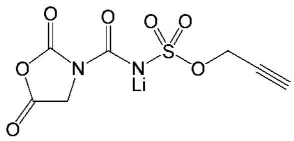 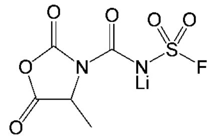

[Chem. 51]

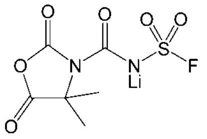
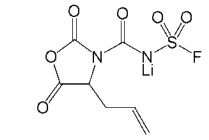
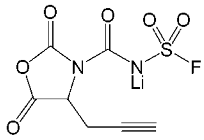
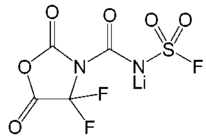

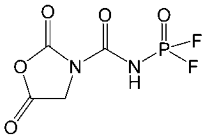
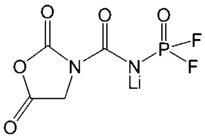
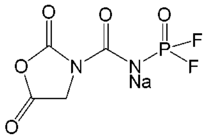
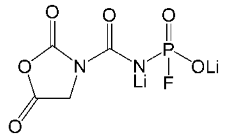

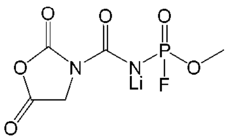
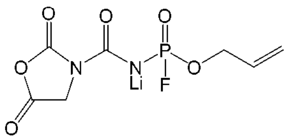
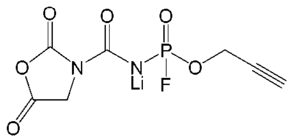
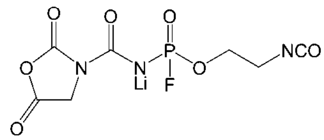

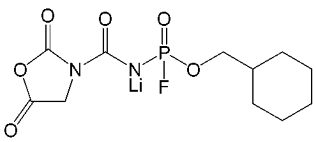
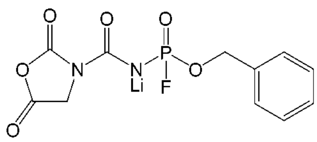
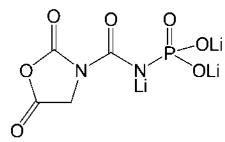
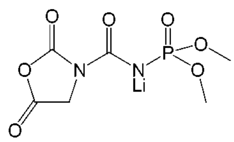

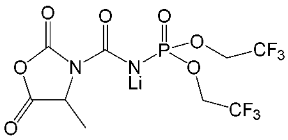
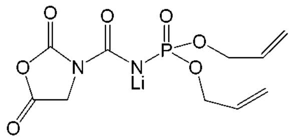
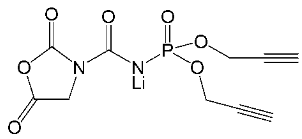
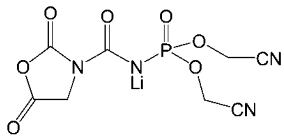

**33.** The compound according to claim 25, wherein the compound represented by the general formula (8) is selected from the following compounds.

[Chem. 52]

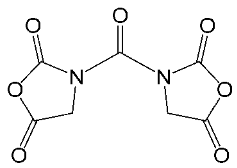
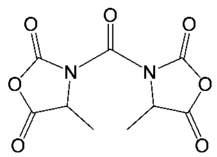
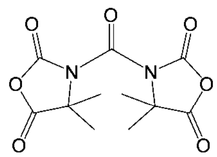
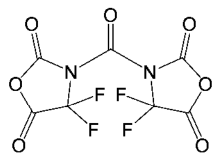

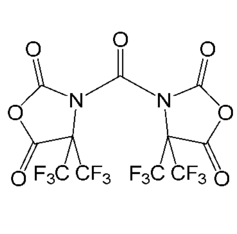
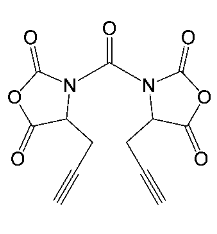
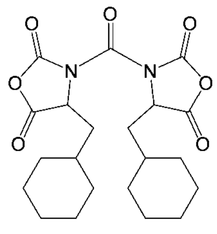
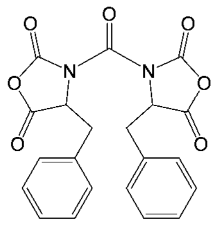

# INTERNATIONAL SEARCH REPORT

International application No.
**PCT/JP2024/034775**

## A. CLASSIFICATION OF SUBJECT MATTER

***H01M 10/0567***(2010.01)i; ***C07D 263/44***(2006.01)i; ***C07F 9/24***(2006.01)i; ***C07F 9/26***(2006.01)i; ***C07F 9/40***(2006.01)i; ***C07F 9/42***(2006.01)i; ***H01G 11/60***(2013.01)i; ***H01G 11/62***(2013.01)i; ***H01G 11/64***(2013.01)i; ***H01M 10/052***(2010.01)i; ***H01M 10/054***(2010.01)i; ***H01M 10/0568***(2010.01)i; ***H01M 10/0569***(2010.01)i

FI: H01M10/0567; C07D263/44 CSP; C07F9/24 G; C07F9/26; C07F9/40 Z; C07F9/42; H01G11/60; H01G11/62; H01G11/64; H01M10/052; H01M10/054; H01M10/0568; H01M10/0569

According to International Patent Classification (IPC) or to both national classification and IPC

## B. FIELDS SEARCHED

Minimum documentation searched (classification system followed by classification symbols)

H01M10/0567; C07D263/44; C07F9/24; C07F9/26; C07F9/40; C07F9/42; H01G11/60; H01G11/62; H01G11/64; H01M10/052; H01M10/054; H01M10/0568; H01M10/0569

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

## C. DOCUMENTS CONSIDERED TO BE RELEVANT

| Category* | Citation of document, with indication, where appropriate, of the relevant passages | Relevant to claim No. |
|---|---|---|
| A | JP 2012-113826 A (SHARP KABUSHIKI KAISHA) 14 June 2012 (2012-06-14) | 1-26 |
| A | WO 2015/045989 A1 (MITSUI CHEMICALS, INC.) 02 April 2015 (2015-04-02) | 1-26 |
| A | JP 2002-270229 A (SONY CORPORATION) 20 September 2002 (2002-09-20) | 1-26 |
| A | ANTONIETTA, D. et al. Electrochemical Studies on Haloamides. Part XII, Electrosynthesis of Oxazolidine-2,4-diones. Tetrahedron. 1995, vol. 51, no. 20, pp. 5891-5900<br>pp. 5891-5900 | 1-26 |
| A | SANTANA, A. B. et al. N-Acyl and N-sulfonyloxazolidine-2,4-diones are pseudo-irreversivle inhibitors of serine proteases. Bioorganic & Medicinal Chemistry Letters. 2012, vol. 22, pp. 3993-3997<br>pp. 3993-3997 | 1-26 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 December 2024** | **24 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/034775**

**C. DOCUMENTS CONSIDERED TO BE RELEVANT**

| Category* | Citation of document, with indication, where appropriate, of the relevant passages | Relevant to claim No. |
|---|---|---|
| A | QUACH, L. et al. Molecular Design of Film-Forming Additives for Lithium-Ion Batteries: Impact of Molecular Substrate Parameters on Cell Performance. ACS Applied Energy Materials. 2023, vol. 6, pp. 9837-9850 | 1-26 |

Form PCT/ISA/210 (second sheet) (July 2022)

**INTERNATIONAL SEARCH REPORT**

International application No.
**PCT/JP2024/034775**

**Box No. III Observations where unity of invention is lacking (Continuation of item 3 of first sheet)**

This International Searching Authority found multiple inventions in this international application, as follows:

The invention in claims 1-26 (an embodiment containing a compound represented by general formula (1) (where $X = -SO_2NMa^-$)) "oxazoline-2,4-dione," is described in document 1 (Japanese Unexamined Patent Publication No. 2012-113826) and is therefore not a special technical feature. However, the invention has the special technical feature of "$X = -SO_2NMa^-$."

The invention in claims 1-33 (an embodiment that does not contain a compound represented by general formula (1), (where $X = -SO_2NMa^a-$)) shares with the invention in claims 1-26 (an embodiment that contains a compound represented by general formula (1), (where $X = -SO_2NMa^a-$)) the common technical feature of "oxazoline-2,4-dione." However, the technical feature is described in document 1 (Japanese Unexamined Patent Publication No. 2012-113826) and does not make a contribution over the prior art. Therefore, it cannot be said to be a special technical feature. Also, there are no other same as or corresponding special technical features between these inventions.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-26**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
**Information on patent family members**

International application No.
**PCT/JP2024/034775**

| Patent document cited in search report | Publication date (day/month/year) | Patent family member(s) | Publication date (day/month/year) |
|---|---|---|---|
| JP 2012-113826 A | 14 June 2012 | US 2012/0129046 A1 | |
| | | CN 102569884 A | |
| WO 2015/045989 A1 | 02 April 2015 | US 2016/0211551 A1 | |
| | | EP 3051619 A1 | |
| | | CN 105474452 A | |
| | | KR 10-2016-0032215 A | |
| JP 2002-270229 A | 20 September 2002 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5109213 B
- JP 4302366 B
- JP 5439345 B
- JP 2009137834 A
- US 7135252 B
- JP 2008270201 A
- WO 2013118661 A
- JP 2013030284 A


### Non-patent literature cited in the description


- *Can. J. Chem*, 2001, vol. 79, 1040-1048